# EUROPEAN PATENT SPECIFICATION

(11) **EP 1 444 211 B1**
(45) Date of publication and mention of the grant of the patent: **21.01.2009**
(21) Application number: 02774961.3
(22) Date of filing: 31.10.2002
(51) Int. Cl.: C07D 239/94, C07D 401/14, C07D 401/12, C07D 407/12, C07D 409/12, C07D 403/12, A61K 31/505, A61P 35/00

(54) **QUINAZOLINE DERIVATIVES AS ANTITUMOR AGENTS**
QUINAZOLIN DERIVATE ALS ANTITUMOR-MITTEL
DERIVES DE LA QUINAZOLINE, AGENTS ANTI-TUMORAUX

(30) Priority: 03.11.2001 GB 0126433
(43) Date of publication of application: 11.08.2004
(73) Proprietor: AstraZeneca AB, 151 85 Södertälje (SE)
(72) Inventor: HENNEQUIN, Laurent, Francois, Andre, Macclesfield, Cheshire SK10 4TG (GB); KETTLE, Jason, Grant, Macclesfield, Cheshire SK10 4TG (GB); PASS, Martin, Macclesfield, Cheshire SK10 4TG (GB); BRADBURY, Robert, Hugh, Macclesfield, Cheshire SK10 4TG (GB)
(86) International application number: PCT/GB2002/004932
(87) International publication number: WO 2003/040109

(56) References cited:
- EP-A- 0 566 226
- WO-A-96/09294
- WO-A-96/15118
- WO-A-98/38984
- WO-A-98/50370
- WO-A-01/094341
- US-A- 6 004 967

## Description

The invention concerns certain novel quinazoline derivatives, or pharmaceutically-acceptable salts thereof, which possess anti-tumour activity and are accordingly useful in methods of treatment of the human or animal body. The invention also concerns processes for the manufacture of said quinazoline derivatives, to pharmaceutical compositions containing them and to their use in therapeutic methods, for example in the manufacture of medicaments for use in the prevention or treatment of solid tumour disease in a warm-blooded animal such as man.

Many of the current treatment regimes for diseases resulting from the abnormal regulation of cellular proliferation such as psoriasis and cancer, utilise compounds that inhibit DNA synthesis and cellular proliferation. To date, compounds used in such treatments are generally toxic to cells however their enhanced effects on rapidly dividing cells such as tumour cells can be beneficial. Alternative approaches to these cytotoxic anti-tumour agents are currently being developed, for example selective inhibitors of cell signalling pathways. These types of inhibitors are likely to have the potential to display an enhanced selectivity of action against tumour cells and so are likely to reduce the probability of the therapy possessing unwanted side effects.

Eukaryotic cells are continually responding to many diverse extracellular signals that enable communication between cells within an organism. These signals regulate a wide variety of physical responses in the cell including proliferation, differentiation, apoptosis and motility. The extracellular signals take the form of a diverse variety of soluble factors including growth factors as well as paracrine and endocrine factors. By binding to specific transmembrane receptors, these ligands integrate the extracellular signal to the intracellular signalling pathways, therefore transducing the signal across the plasma membrane and allowing the individual cell to respond to its extracellular signals. Many of these signal transduction processes utilise the reversible process of the phosphorylation of proteins that are involved in the promotion of these diverse cellular responses. The phosphorylation status of target proteins is regulated by specific kinases and phosphatases that are responsible for the regulation of about one third of all proteins encoded by the mammalian genome. As phosphorylation is such an important regulatory mechanism in the signal transduction process, it is therefore not surprising that aberrations in these intracellular pathways result in abnormal cell growth and differentiation and so promote cellular transformation (reviewed in Cohen et al, Curr Opin Chem Biol, 1999, 3, 459-465).

It has been widely shown that a number of these tyrosine kinases are mutated to constitutively active forms and/or when over-expressed result in the transformation of a variety of human cells. These mutated and over-expressed forms of the kinase are present in a large proportion of human tumours (reviewed in Kolibaba et al, Biochimica et Biophysica Acta, 1997, 133, F217-F248). As tyrosine kinases play fundamental roles in the proliferation and differentiation of a variety of tissues, much focus has centred on these enzymes in the development of novel anti-cancer therapies. This family of enzymes is divided into two groups - receptor and non-receptor tyrosine kinases e.g. EGF Receptors and the SRC family respectively. From the results of a large number of studies including the Human Genome Project, about 90 tyrosine kinase have been identified in the human genome, of this 58 are of the receptor type and 32 are of the non-receptor type. These can be compartmentalised in to 20 receptor tyrosine kinase and 10 non-receptor tyrosine kinases sub-families (Robinson et al, Oncogene, 2000, 19, 5548-5557).

The receptor tyrosine kinases are of particular importance in the transmission of mitogenic signals that initiate cellular replication. These large glycoproteins, which span the plasma membrane of the cell possess an extracellular binding domain for their specific ligands (such as Epidermal Growth Factor (EGF) for the EGF Receptor). Binding of ligand results in the activation of the receptor's kinase enzymatic activity that is encoded by the intracellular portion of the receptor. This activity phosphorylates key tyrosine amino acids in target proteins, resulting in the transduction of proliferative signals across the plasma membrane of the cell.

It is known that the erbB family of receptor tyrosine kinases, which include EGFR, erbB2, erbB3 and erbB4, are frequently involved in driving the proliferation and survival of tumour cells (reviewed in Olayioye et al., EMBO J., 2000,19, 3159). One mechanism in which this can be accomplished is by overexpression of the receptor at the protein level, generally as a result of gene amplification. This has been observed in many common human cancers (reviewed in Klapper et al., Adv. Cancer Res., 2000, 77, 25) such as breast cancer (Sainsbury et al., Brit. J. Cancer, 1988, 58, 458; Guerin et al., Oncogene Res., 1988, 3, 21; Slamon et al., Science, 1989, 244, 707; Klijn et al., Breast Cancer Res. Treat., 1994, 29, 73 and reviewed in Salomon et al., Crit. Rev. Oncol. Hematol., 1995, 19, 183), non-small cell lung cancers (NSCLCs) including adenocarcinomas (Cerny et al., Brit. J. Cancer, 1986, 54, 265; Reubi et al., Int. J. Cancer, 1990, 45, 269; Rusch et al., Cancer Research, 1993, 53, 2379; Brabender et al, Clin. Cancer Res., 2001, 7, 1850) as well as other cancers of the lung (Hendler et al., Cancer Cells, 1989, 7 347; Ohsaki et al. Oncol.Rep., 2000, 7, 603), bladder cancer (Neal et al., Lancet, 1985, 366; Chow et al., Clin. Cancer Res., 2001, 7, 1957, Zhau et al., Mol Carcinog., 3, 254), oesophageal cancer (Mukaida et al., Cancer, 1991, 68, 142), gastrointestinal cancer such as colon, rectal or stomach cancer (Bolen et al., Oncogene Res., 1987, 1, 149; Kapitanovic et al., Gastroenterology, 2000, 112, 1103; Ross et al., Cancer Invest., 2001, 19, 554), cancer of the prostate (Visakorpi et al., Histochem. J., 1992, 24, 481; Kumar et al., 2000, 32, 73; Scher et al., J. Natl. Cancer Inst., 2000, 92, 1866), leukaemia (Konaka et al., Cell, 1984, 37, 1035, Martin-Subero et al., Cancer Genet Cytogenet., 2001, 127,174), ovarian (Hellstrom et al., Cancer Res., 2001, 61, 2420), head and neck (Shiga et al., Head Neck, 2000, 22, 599) or pancreatic cancer (Ovotny et al., Neoplasma, 2001, 48, 188). As more human tumour tissues are tested for expression of the erbB family of receptor tyrosine kinases it is expected that their widespread prevalence and importance will be further enhanced in the future.

As a consequence of the mis-regulation of one or more of these receptors (in particular erbB2), it is widely believed that many tumours become clinically more aggressive and so correlate with a poorer prognosis for the patient (Brabender et al, Clin. Cancer Res., 2001, 7, 1850; Ross et al, Cancer Investigation, 2001, 19, 554, Yu et al., Bioessays, 2000, 22.7, 673). In addition to these clinical findings, a wealth of pre-clinical information suggests that the erbB family of receptor tyrosine kinase are involved in cellular transformation. This includes the observations that many tumour cell lines overexpress one or more of the erbB receptors and that EGFR or erbB2 when transfected into non-tumour cells have the ability to transform these cells. This tumourigenic potential has been further verified as transgenic mice that overexpress erbB2 spontaneously develop tumours in the mammary gland. In addition to this, a number of pre-clinical studies have demonstrated that anti-proliferative effects can be induced by knocking out one or more erbB activities by small molecule inhibitors, dominant negatives or inhibitory antibodies (reviewed in Mendelsohn et al., Oncogene, 2000, 19, 6550). Thus it has been recognised that inhibitors of these receptor tyrosine kinases should be of value as a selective inhibitor of the proliferation of mammalian cancer cells (Yaish et al. Science, 1988, 242, 933, Kolibaba et al, Biochimica et Biophysica Acta, 1997, 133, F217-F248; Al-Obeidi et al, 2000, Oncogene, 19, 5690-5701; Mendelsohn et al, 2000, Oncogene, 19, 6550-6565). In addition to this pre-clinical data, findings using inhibitory antibodies against EGFR and erbB2 (c-225 and trastuzumab respectively) have proven to be beneficial in the clinic for the treatment of selected solid tumours (reviewed in Mendelsohn et al, 2000, Oncogene, 19, 6550-6565).

Amplification and/or activity of members of the ErbB type receptor tyrosine kinases have been detected and so have been implicated to play a role in a number of non-malignant proliferative disorders such as psoriasis (Ben-Bassat, Curr. Pharm. Des., 2000, 6, 933; Elder et al., Science, 1989, 243, 811), benign prostatic hyperplasia (BPH) (Kumar et al., Int. Urol. Nephrol., 2000, 32,73), atherosclerosis and restenosis (Bokemeyer et al., Kidney Int., 2000, 58, 549). It is therefore expected that inhibitors of erbB type receptor tyrosine kinases will be useful in the treatment of these and other non-malignant disorders of excessive cellular proliferation.

International Patent Applications WO 96/33977, WO 96/33978, WO 96/33979, WO 96/33980 and WO 96/33981 disclose that certain quinazoline derivatives which bear an anilino substituent at the 4-position possess receptor tyrosine kinase inhibitory activity.

EP-A-0566226 discloses quinazoline derivatives and their receptor tyrosine inhibitory properties in the treatment of cancer.

US-A-6,004,967 discloses methods and compositions for treating hyperproliferative skin disorders utilizing a quinazoline derivative as an active ingredient. WO 96/15118 discloses aniline derivatives and their use in the treatment of proliferative disease such as cancer.

A review of the structure activity relationship of various quinazoline derivatives is disclosed by G. W. Rewcastle et al (J. Med. Chem. 1995, 38, 3428-3487), including a number of 5-substituted compounds. However, such 5-substituted compounds are stated to have low in-vitro activity as EGFR tyrosine kinase inhibitors compared to quinazolines substituted at the 6- and 7- positions.

WO 96/09294 discloses 4-anilinoquinazoline derivatives, including 5-chloro and 5-methoxy substituted quinazoline derivatives as protein tyrosine kinase inhibitors.

WO 01/94-341 (Co-pending International Patent Application PCT/GB01/02424) discloses that certain quinazoline derivatives which carry a 5-substituent are inhibitors of the Src family of non-receptor tyrosine kinases, such as c-Src, c-Yes and c-Fyn.

We have now found that surprisingly certain 5-substituted quinazoline derivatives possess potent anti-tumour activity. Without wishing to imply that the compounds disclosed in the present invention possess pharmacological activity only by virtue of an effect on a single biological process, it is believed that the compounds provide an anti-tumour effect by way of inhibition of one or more of the erbB family of receptor tyrosine kinases that are involved in the signal transduction steps which lead to the proliferation of tumour cells. In particular, it is believed that the compounds of the present invention provide an anti-tumour effect by way of inhibition of EGFR and/or erbB2 receptor tyrosine kinases.

Generally the compounds of the present invention possess potent inhibitory activity against the erbB receptor tyrosine kinase family, for example by inhibition of EGFR and/or erbB2 and/or erbB4 receptor tyrosine kinases, whilst possessing less potent inhibitory activity against other kinases. Furthermore, certain compounds of the present invention possess substantially better potency against the erbB2 over that of the EGFR tyrosine kinase, thus potentially providing effective treatment for erbB2 driven tumours. Additionally, certain of the compounds according to the present invention possess substantially better potency against the EGFR over that of the erbB2 tyrosine kinase. The invention also includes compounds that are active against all or a combination of EGFR, erbB2 and erbB4 receptor tyrosine kinases, thus potentially providing treatments for conditions mediated by one or more of these receptor tyrosine kinases.

According to a first aspect of the invention there is provided a quinazoline derivative of the Formula I 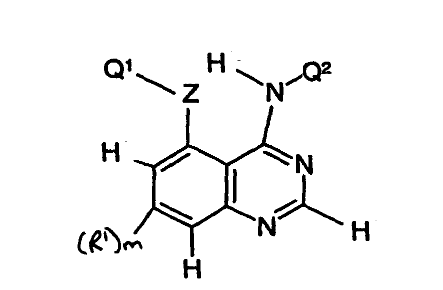 wherein
**m** is 0 or 1 and the **R¹** group, when present, is located at the 7-position and is selected from hydroxy, amino, methyl, ethyl, propyl, methoxy, ethoxy, propoxy, butoxy, pentoxy, pyrrolidin-1-yl, 2-pyrrolidin-1-ylethoxy, 3-pyirolidin-1-ylpropoxy, 2-piperidinoethoxy, 3-piperidinopropoxy, 2-piperidin-3-ylethoxy, 3-piperidin-3-ylpropoxy, 2-piperidin-4-ylethoxy, 3-piperidin-4-ylpropoxy, 2-piperazin-1-ylethoxy, 3-piperazin-1-ylpropoxy, 2-morpholinoethoxy, 3-morpholinopropoxy, 2-homopiperidinoethoxy, 3-homopiperidinopropoxy, 2-homopiperazin-1-ylethoxy and 3-homopiperazin-1-ylpropoxy
and wherein adjacent carbon atoms in any (2-6C)alkoxy chain within a R¹ substituent are optionally separated by the insertion into the chain of a group selected from O, NH and N(CH₃),
and wherein any terminal CH₃ group within a (1-6C)alkoxy chain in a R¹ substituent optionally bears on the terminal CH₃ group a substituent selected from hydroxy, amino and N-(1-methylpyrrolidin-3-yl)-N-methylamino,
and wherein any pyrrolidinyl or piperidinyl group within a R¹ substituent optionally bears a substituent selected from hydroxy, methyl, amino, methylamino and dimethylamino,
and wherein any piperazin-1-yl or homopiperazin-1-yl group within a R¹ substituent optionally bears a substituent at the 4-position selected from methyl, ethyl, isopropyl, 2-methoxyethyl, tetrahydrofurfuryl, 2-morpholinoethyl and 1-methylpiperidin-4-yl;
the **Q¹-Z**- group is selected from cyclopentyloxy, tetrahydrofuran-3-yloxy, tetrahydropyran-4-yloxy, tetrahydrothiopyran-4-yloxy, 1,1-dioxotetrahydrothiopyran-4-yloxy, 1-oxotetrahydrothiopyran-4-yloxy, tetrahydrothien-3-yloxy, 1,1-dioxotetrahydrothien-3-yloxy, 1-oxotetrahydrothien-3-yloxy, pyrrolidin-3-yloxy, pyrolidin-2-yloxy, piperidin-3-yloxy, piperidin-4-yloxy, homopiperidin-3-yloxy, homopiperidin-4-yloxy and azetidin-3-yloxy,
and wherein the azetidinyl, pyrrolidinyl, piperidinyl or homopiperidinyl group within the Q¹-Z- group is optionally N- substituted by a substituent selected from methyl, ethyl, n-propyl, isopropyl, n-butyl, isobutyl, tert-butyl, allyl, 2-propynyl, acetyl, propionyl, methoxycarbonyl, ethoxycarbonyl, propoxycarbonyl, tert-butoxycarbonyl, methylsulphonyl, ethylsulphonyl, 2-methoxyethyl, carbamoylmethyl, N-methylcarbamoylmethyl, N,N-dimethylcarbamoylmethyl, 2-carbamoylethyl, 2-(N-methylcarbamoyl)ethyl, 2-(N,N-dimethylcarbamoyl)ethyl, acetylmethyl, 2-acetylethyl, methoxycarbonylmethyl and 2-methoxycarbonylethyl,
and wherein any heterocyclyl group within the Q¹-Z- group optionally bears 1 or 2 oxo substituents;
**Q²** is an aryl group of formula Ib 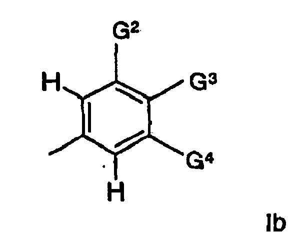 wherein G³ and G⁴ together form a group of formula:- -CH=CH-NH-, -NH-CH=CH-, -NH-N=CH- or -CH=N-NH-,
and the 9-membered bicyclic heteroaryl ring formed when G³ and G⁴ are linked together optionally bears on a NH group of the heteroaryl portion of the bicyclic ring a group of the formula:

-X¹² -Q¹¹

wherein X¹² is a direct bond or is selected from SO₂ and CO, wherein Q¹¹ is phenyl, benzyl, 2-phenylethyl, 2-furyl, furfuryl, 3-furyl, 3-furylmethyl, 2-oxazolyl, 4-oxazolyl, 2-oxazolylmethyl, 4-oxazolylmethyl, 2-imidazolyl, 4-imidazolyl, 2-imidazolylmethyl, 4-imidazolylnaethyl, 2-, 3-or 4-pyridyl, 2-, 3-or 4-pyridylmethyl, 2-(2-, 3-or 4-pyridyl)ethyl, 2-, 4- or 5-pyrimidinyl, 2-, 4- or 5-pyrimidinylmethyl, 2-(2-, 4- or 5-pyrimidinyl)ethyl, 1,2,4-triazol-3-yl, 1,2,4-triazol-5-ylmethyl, triazol-3-ylmethyl, 1,2,4-triazol-5-yl, 2-thienyl, 3-thienyl, 2-thienylmethyl, 3-thienylmethyl, 2-(2-thienyl)ethyl, 2-(3-thienyl)ethyl, 2-thiazolyl, 4-thiazolyl, 2-thiazolylmethyl, 4-thiazolylmethyl,1,2,5-thiadiazol-3-yl, 1,2,5-thiadiazol-3-ylinethyl, 2-(1,2,5-thiadiazol-3-yl)ethyl which optionally bears 1 or 2 substituents, which may be the same or different, selected from fluoro, chloro, bromo, cyano, hydroxy, methyl and ethyl,
and the 9- membered bicyclic heteroaryl ring formed when G³ and G⁴ together are linked optionally bears on an available carbon atom in the heteroaryl portion of the bicyclic ring 1 substituent selected from fluoro, chloro, bromo, cyano, hydroxy, amino, methyl, ethyl, vinyl, ethynyl, methylamino and di-methylamino,
and G² is selected from hydrogen, fluoro, chloro, bromo, trifluoromethyl, cyano, hydroxy, amino, methyl, ethyl, vinyl, ethynyl, methylamino and dimethylamino;
or a pharmaceutically acceptable salt thereof.

In this specification the generic term "alkyl" includes both straight-chain and branched-chain alkyl groups such as propyl, isopropyl and tert-butyl, and (3-7C)cycloalkyl groups such as cyclopropyl, cyclobutyl, cyclopentyl, cyclohexyl and cycloheptyl. However references to individual alkyl groups such as "propyl" are specific for the straight-chain version only, references to individual branched-chain alkyl groups such as "isopropyl" are specific for the branched-chain version only and references to individual cycloalkyl groups such as "cyclopentyl" are specific for that 5-membered ring only. An analogous convention applies to other generic terms, for example (1-6C)alkoxy includes methoxy, ethoxy, cyclopropyloxy and cyclopentyloxy, (1-6C)alkylamino includes methylamino, ethylamino, cyclobutylamino and cyclohexylamino, and di-[(1-6Calkyl]amino includes dimethylamino, diethylamino, N-cyclobutyl-N-methylamino and N-cyclohexyl-N-ethylamino.

It is to be understood that, insofar as certain of the compounds of Formula I defined above may exist in optically active or racemic forms by virtue of one or more asymmetric carbon atoms, the invention includes in its definition any such optically active or racemic form which possesses the above-mentioned activity. The synthesis of optically active forms may be carried out by standard techniques of organic chemistry well known in the art, for example by synthesis from optically active starting materials or by resolution of a racemic form. Similarly, the above-mentioned activity may be evaluated using the standard laboratory techniques referred to hereinafter.

It is to be understood that the present invention includes in its definition any and all tautomeric forms of the compounds of the formula I which possess the above mentioned activity.

It is also to be understood that in so far as certain compounds of the formula 1 may exist in solvated forms as well as unsolvated forms, for example, hydrated forms, the present invention includes any and all such solvated forms, which possess the above mentioned activity.

Suitable values for the generic radicals referred to above include those set out below.

A suitable value for any one of the 'Q' groups (Q¹, Q¹¹) when it is aryl or for the aryl group within a 'Q' group is, for example, phenyl or naphthyl, preferably phenyl.

A suitable value for any one of the 'Q' groups (Q¹, Q³) when it is (3-7C)cycloalkyl or for the (3-7C)cycloalkyl group within a 'Q' group is, for example, cyclopropyl, cyclobutyl, cyclopentyl, cyclohexyl, cycloheptyl or bicyclo[2.2.1]heptyl and a suitable value for any one of the 'Q' groups (Q¹, Q³) when it is (3-7C)cycloalkenyl or for the (3-7C)cycloalkenyl group within a 'Q' group is, for example, cyclobutenyl, cyclopentenyl, cyclohexenyl or cycloheptenyl.

A suitable value for any one of the 'Q' groups (Q¹, Q³, Q¹¹) when it is heteroaryl or for the heteroaryl group within a 'Q' group is, for example, an aromatic 5- or 6-membered monocyclic ring or a 9- or 10-membered bicyclic ring with up to five ring heteroatoms selected from oxygen, nitrogen and sulphur, which, unless specified otherwise, may be carbon or nitrogen linked. Examples of suitable values of "heteroaryl" include furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl, 1,3,5-triazenyl, 1,3-benzodioxolyl, benzofuranyl, indolyl, benzothienyl, benzoxazolyl, benzimidazolyl, benzothiazolyl, indazolyl, benzofurazanyl, quinolyl, isoquinolyl, quinazolinyl, quinoxalinyl, cinnolinyl or naphthyridinyl.

A suitable value for any one of the 'Q' groups (Q¹, Q³ Q¹¹) when it is heterocyclyl or for the heterocyclyl group within a 'Q' group is, for example, a non-aromatic saturated or partially saturated 3 to 10 membered monocyclic or bicyclic ring with up to five heteroatoms selected from oxygen, nitrogen and sulphur, which, unless specified otherwise, may be carbon or nitrogen linked. Examples of suitable values of "heterocyclyl" include oxiranyl, oxetanyl, azetidinyl, tetrahydrofuranyl, tetrahydropyranyl, oxepanyl, pyrrolinyl, pyrrolidinyl, morpholinyl, tetrahydro-1,4-thiazinyl, 1,1-dioxotetrahydro-1,4-thiazinyl, piperidinyl, homopiperidinyl, piperazinyl, homopiperazinyl, dihydropyridinyl, tetrahydropyridinyl, dihydropyrimidinyl, tetrahydropyrimidinyl, tetrahydrothienyl, tetrahydrothiopyranyl, decahydroisoquinolinyl or decahydroquinolinyl, preferably tetrahydrofuranyl, tetrahydropyranyl, pyrrolidinyl, morpholinyl, 1,4-oxazepanyl, thiamorpholinyl 1,1-dioxotetrahydro-4H-1,4-thiazinyl, piperidinyl or piperazinyl, more preferably tetrahydrofuran-3-yl, tetrahydropyran-4-yl, tetrahydrothien -3-yl, tetrahydrothiopyran-4-yl, pyrrolidin-3-yl, morpholino, 1,1-dioxotetrahydro-4H-1,4-thiazin-4-yl, piperidino, piperidin-4-yl, piperidin-3-yl or piperazin-1-yl. A nitrogen or sulphur atom within a heterocyclyl group may be oxidized to give the corresponding N or S oxide, for example 1,1-dioxotetrahydrothienyl, 1-oxotetrahydrothienyl, 1,1-dioxotetrahydrothiopyranyl or 1-oxotetrahydrothiopyranyl. A suitable value for such a group which bears 1 or 2 oxo or thioxo substituents is, for example, 2-oxopyrrolidinyl, 2-thioxopyrrolidinyl, 2-oxoimidazolidinyl, 2-thioxoimidazolidinyl, 2-oxopiperidinyl, 2,5-dioxopyrrolidinyl, 2,5-dioxoimidazolidinyl or 2,6-dioxopiperidinyl.

A suitable value for a 'Q' group when it is heteroaryl-(1-6C)alkyl is, for example, heteroarylmethyl, 2-heteroarylethyl and 3-heteroarylpropyl. The invention comprises corresponding suitable values for 'Q' groups when, for example, rather than a heteroaryl-(1-6C)alkyl group, an aryl-(1-6C)alkyl, (3-7C)cycloalkyl-(1-6C)alkyl, (3-7C)cycloalkenyl-(1-6C)alkyl or heterocyclyl-(1-6C)alkyl group is present.

Suitable values for any of the 'R' groups (R¹), or for various groups within an R¹ substituent, or for G³ or for various groups within G³, or for any of the other 'G' groups (G² or G⁴) within Q², or for various groups within Q², or for Q¹ or for various groups within Q¹, or for various groups within the Q¹-Z- group include:-

| | |
|---|---|
| for halogeno | fluoro, chloro, bromo and iodo; |
| for (1-6C)alkyl: | methyl, ethyl, propyl, isopropyl and tert-butyl; |
| for (2-8C)alkenyl: | vinyl, isopropenyl, allyl and but-2-enyl; |
| for (2-8C)alkynyl: | ethynyl, 2-propynyl and but-2-ynyl; |
| for (1-6C)alkoxy: | methoxy, ethoxy, propoxy, isopropoxy and butoxy; |
| for (2-6C)alkenyloxy: | vinyloxy and allyloxy; |
| for (2-6C)alkynyloxy: | ethynyloxy and 2-propynyloxy; |
| for (1-6C)alkylthio: | methylthio, ethylthio and propylthio; |
| for (1-6C)alkylsulphinyl: | methylsulphinyl and ethylsulphinyl; |
| for (1-6C)alkylsulphonyl: | methylsulphonyl and ethylsulphonyl; |
| for (1-6C)alkylamino: | methylamino, ethylamino, propylamino, isopropylamino and butylamino; |
| for di-[(1-6C)alkyl]amino: | dimethylamino, diethylamino, N-ethyl-N-methylamino and diisopropylamino; |
| for (1-6C)alkoxycarbonyl: | methoxycarbonyl, ethoxycarbonyl, propoxycarbonyl and tert-butoxycarbonyl; |
| for N-(1-6C)alkylcarbamoyl: | N-methylcarbamoyl, N-ethylcarbamoyl and N-propylcarbamoyl; |
| for N,N-di-[(1-6C)alkyl]carbamoyl: | N,N-dimethylcarbamoyl, N-ethyl-N-methylcarbamoyl and N,N-diethylcarbamoyl; |
| for (2-6C)alkanoyl: | acetyl and propionyl; |
| for (2-6C)alkanoyloxy: | acetoxy and propionyloxy; |
| for (2-6C)alkanoylamino: | acetamido and propionamido; |
| for N-(1-6C)alkyl-(2-6C)alknoylamino: | N-methylacetamido and N-methylpropionamido; |
| for amino(2-6C)alkanoyl: | aminoacetyl and 2-aminopropionyl; |
| for N-(1-6C)alkylamino(2-6C)alkanoyl: | N-methylaminoacetyl and 2-(N-methylaminopropionyl; |
| for N,N-di-[(1-6C)alkyl]amino(2-6C)alkanoyl | N,N-di-methylaminoacetyl; |
| for N-(1-6C)alkylsulphamoyl: | N-methylsulphamoyl and N-ethylsulphamoyl; |
| for N,N-di-[(1-6C)alkyl]sulphamoyl: | N,N-dimethylsulphamoyl; |
| for (1-6C)alkanesulphonylamino: | methanesulphonylamino and ethanesulphonylamino; |
| for N-(1-6C)alkyl-(1-6C)alkanesulphony | N-methylmethanesulphonylamino and N-methylethanesulphonylamino; |
| for (3-6C)alkenoylamino: | acrylamido, methacrylamido and crotonamido; |
| for N-(1-6C)alkyl-(3-6C)alkenoylamino: | N-methylacrylamido and N-methylcrotonamido; |
| for (3-6C)alkynoylamino: | propiolamido; |
| for N-(1-6C)alkyl-(3-6C)alkynoylamino: | N-methylpropiolamido; |
| for amino-(1-6C)alkyl: | aminomethyl, 2-aminoethyl, 1-aminoethyl and 3-aminopropyl; |
| for (1-6C)alkylamino-(1-6C)alkyl: | methylaminomethyl, ethylaminomethyl, 1-methylaminoethyl, 2-methylaminoethyl, 2-ethylaminoethyl and 3-methylaminopropyl; |
| for di-[(1-6C)alkyl]amino-(1-6C)alkyl: | dimethylaminomethyl, diethylaminomethyl, 1-dimethylaminoethyl, 2-dimethylaminoethyl and 3-dimethylaminopropyl; |
| for halogeno-(1-6C)alkyl: | chloromethyl, 2-chloroethyl, 1-chloroethyl and 3-chloropropyl; |
| for hydroxy-(1-6C)alkyl: | hydroxymethyl, 2-hydroxyethyl, 1-hydroxyethyl and 3-hydroxypropyl; |
| for (1-6C)alkoxy-(1-6C)alkyl: | methoxymethyl, ethoxymethyl, 1-methoxyethyl, 2-methoxyethyl, 2-ethoxyethyl and 3-methoxypropyl; |
| for cyano-(1-6C)alkyl: | cyanomethyl, 2-cyanoethyl, 1-cyanoethyl and |
| | 3-cyanopropyl; |
| for carboxy-(1-6C)alkyl: | carboxymethyl, 2-carboxyethyl, 1-carboxyethyl and 3-carboxypropyl; |
| for (1-6C)alkylthio-(1-6C)alkyl: | methylthiomethyl, ethylthiomethyl, 2-methylthioethyl, 1-methylthioethyl and 3-methylthiopropyl; |
| for (1-6C)alkylsulphinyl-(1-6C)alkyl: | methylsulphinylmethyl, ethylsulphinylmethyl, 2-methylsulphinylethyl, 1-methylsulphinylethyl and 3-methylsulphinylpropyl; |
| for (1-6C)alkylsulphonyl-(1-6C)alkyl: | methylsulphonylmethyl, ethylsulphonylmethyl, 2-methylsulphonylethyl, 1-methylsulphonylethyl and 3-methylsulphonylpropyl; |
| for (2-6C)alkanoylamino-(1-6C)alkyl: | acetamidomethyl, propionamidomethyl and 2-acetamidoethyl; |
| for (1-6C)alkoxycarbonyl-(1-6C)alkyl: | methoxycarbonylmethyl, 2-methoxycarbonylethyl and 2-ethoxycarbonylethyl; |
| for (1-6C)alkoxycarbonylamino-(1-6C)al alkyl: | methoxycarbonylaminomethyl, ethoxycarbonylaminomethyl, tert-butoxycarbonylaminomethyl and 2-methoxycarbonylaminoethyl; |
| for carbamoyl-(1-6C)alkyl: | carbamoylmethyl, 1-carbamoylethyl, 2-carbamoylethyl and 3-carbamoylpropyl; |
| for (2-6C)alkanoyl-(1-6C)alkyl: | acetylmethyl and 2-acetylethyl; |
| for N-(1-6C)alkylcarbamoyl-(1-6C)alkyl: | N-methylcarbamoylmethyl, N-ethylcarbamoylmethyl, N-propylcarbamoylmethyl, 1-(N-methylcarbamoyl)ethyl, 1-(N-ethylcarbamoyl)ethyl, 2-(N-methylcarbamoyl)ethyl, 2-(N-ethylcarbamoyl)ethyl and 3-(N-methylcarbamoyl)propyl; and |
| for N,N-di[(1-6C)alkyl]carbamoyl-(1-6C) alkyl: | N,N-dimethylcarbamoylmethyl, N,N-diethylcarbamoylmethyl, |
| | 2-(N,N-dimethylcarbamoyl)ethyl, and |
| | 3-(N,N-dimethylcarbamoyl)propyl. |

When in this specification reference is made to a (1-4C)alkyl group it is to be understood that such groups refer to alkyl groups containing up to 4 carbon atoms. A skilled person will realise that representative examples of such groups are those listed above under (1-6C)alkyl that contain up to 4 carbon atoms, such as methyl, ethyl, propyl and butyl. Similarly, reference to a (1-3C)alkyl group refers to alkyl groups containing up to 3 carbon atoms such as methyl, ethyl and propyl. A similar convention is adopted for the other groups listed above such as (1-4C)alkoxy, (2-4C)alkenyl, (2-4C)alkynyl and (2-4C)alkanoyl.

When, an R¹ group forms a group of the formula Q³-X¹- and, for example, X¹ is a OC(R⁴)₂ linking group (wherein each R⁴ is, independently, hydrogen or (1-6C) alkyl) it is the carbon atom, not the oxygen atom, of the OC(R⁴)₂ linking group which is attached to the quinazoline ring and the oxygen atom is attached to the Q³ group.

Adjacent carbon atoms in any (2-6C)alkylene chain within a R¹ substituent may be optionally separated by the insertion into the chain of a group such as O, CON(R⁵), N(R⁵) or C≡C (wherein R⁵ is hydrogen or (1-6C) alkyl). For example, insertion of a C≡C group into the ethylene chain within a 2-morpholinoethoxy group gives rise to a 4-morpholinobut-2-ynyloxy group and, for example, insertion of a CONH group into the ethylene chain within a 3-methoxypropoxy group gives rise to, for example, a 2-(2-methoxyacetamido)ethoxy group. It is to be understood that the term (2-6C)alkylene chain refers to any CH₂CH₂ group within R¹ and includes, for example alkylene chains within a (1-6C)alkyl, (1-6C)alkoxy, (2-8C)alkenyl, (2-8C)alkenyloxy, (2-8C)alkynyl and (2-8C)alkynyloxy group. For example the insertion of a N(CH₃) group between the third and fourth carbon atoms in a hex-5-enyloxy group in R¹ gives rise to a 3-(N-methyl-N-allylamino)propoxy group.

When any CH₂=CH- or HC≡C- group within a R¹ substituent optionally bears at the terminal CH₂= or HC≡ position a substituent such as a group of the formula Q⁴-X²-wherein X² is, for example, NHCO and Q⁴ is a heterocyclyl-(1-6C)alkyl group, suitable R¹ substituents so formed include, for example, N-[heterocyclyl-(1-6C)alkyl]carbamoylvinyl groups such as N-(2-pyrrolidin-1-ylethyl)carbamoylvinyl or N-[heterocyclyl-(1-6C)alkyl]carbamoylethynyl groups such as N-(2-pyrrolidin-1-ylethyl)carbamoylethynyl.

When any CH₂ or CH₃ group within a R¹ substituent optionally bears on each said CH₂ or CH₃ group one or more halogeno or (1-6C)alkyl substituents, there are suitably 1 or 2 halogeno or (1-6C)alkyl substituents present on each said CH₂ group and there are suitably 1, 2 or 3 such substituents present on each said CH₃ group.

When any CH₂ or CH₃ group within a R¹ substituent optionally bears on each said CH₂ or CH₃ group a substituent, suitable R¹ substituents so formed include, for example, hydroxy-substituted heterocyclyl-(1-6C)alkoxy groups such as 2-hydroxy-3-piperidinopropoxy and 2-hydroxy-3-morpholinopropoxy, hydroxy-substituted amino-(2-6C)alkoxy groups such as 3-amino-2-hydroxypropoxy, hydroxy-substituted (1-6C)alkylamino-(2-6C)alkoxy groups such as 2-hydroxy-3-methylaminopropoxy, hydroxy-substituted di-[(1-6C)alkyl]amino-(2-6C)alkoxy groups such as 3-dimethylamino-2-hydroxypropoxy, hydroxy-substituted heterocyclyl-(1-6C)alkylamino groups such as 2-hydroxy-3-piperidinopropylamino and 2-hydroxy-3-morpholinopropylamino, hydroxy-substituted amino-(2-6C)alkylamino groups such as 3-amino-2-hydroxypropylamino, hydroxy-substituted (1-6C)alkylamino-(2-6C)alkylamino groups such as 2-hydroxy-3-methylaminopropylamino, hydroxy-substituted di-[(1-6C)alkyl]amino-(2-6C)alkylamino groups such as 3-dimethylamino-2-hydroxypropylamino, hydroxy-substituted (1-6C)alkoxy groups such as 2-hydroxyethoxy, (1-6C)alkoxy-substituted (1-6C)alkoxy groups such as 2-methoxyethoxy and 3-ethoxypropoxy, (1-6C)alkylsulphonyl-substituted (1-6C)alkoxy groups such as 2-methylsulphonylethoxy and heterocyclyl-substituted (1-6C)alkylamino-(1-6C)alkyl groups such as 2-morpholinoethylaminomethyl, 2-piperazin-1-ylethylaminomethyl and 3-morpholinopropylaminomethyl.

Similar considerations apply to the attachments and substitutions within the -Z-Q¹ group.

It is to be understood that when any CH₂ or CH₃ group within a R¹ substituent or a Q¹-Z- group optionally bears on each said CH₂ or CH₃ group a substituent as defined hereinbefore, the optional substituent may be present on any CH₂ or CH₃ group within a R¹ substituent or a Q¹-Z- group, including those on the hereinbefore defined substituents that may be present on an aryl, heteroaryl or heterocyclyl groups within R¹ or Q¹-Z-. For example, if Q¹ is a 1-(1-6C)alkyl-pipezidin-4-yl group, the (1-6C)alkyl group may be optionally substituted by, for example a (2-6C)alkanoyl group to give a 1-((2-6C)alkanoyl-(1-6C)alkyl)-piperidin-4-yl group such as 1-(acetylmethyl)piperidin-4-yl or 1-(2-acetylethyl)piperidin-4-yl. Other suitable groups that may be so formed by Q¹ include, (1-6C)alkoxycarbonyl-(1-6C)alkyl substituted heterocyclyl groups, such as 1-(methoxycarbonylmethyl)piperidin-4-yl or 1-(2-methoxycarbonylethyl)piperidin-4-yl, carbamoyl-(1-6C)alkyl substituted heterocyclyl groups such as 1-(carbamoylmethyl)piperidin-4-yl, or (1-6C)alkoxy-(1-6C)alkyl substituted heterocyclyl groups, such as 1-(2-methoxyethyl)piperidin-4-yl. Similarly when R¹ is a (1-6C)alkyl substituted aryl, or heteroaryl group, the (1-6C)alkyl group may be optionally substituted by one of the hereinbefore defined substituents that may be present on a CH₂ or CH₃ group. For example if R¹ is a heteroaryl group substituted by (1-6C)alkylamino-(1-6C)alkyl, the terminal CH₃ group of the alkyl substituent may be further substituted by, for example, a(1-6C)alkylsulphonyl group. By way of example if R¹ is a 2-(ethylaminomethyl)-5-furyl group, the ethyl group may be optionally substituted by a methylsulphonyl group to give a 2-(2-methylsulphonylethylaminomethyl)-5-furyl group.

Similar considerations apply to substituents that are optionally present on the terminal group of a CH₂=CH- or HC≡C- group within a R¹ substituent or a Q¹-Z- group.

When G³ and G⁴ together form, for example, a group of formula -O-CH=CH-, it is the oxygen atom, not the carbon atom, which is attached to the G³ para-position of the phenyl ring and the carbon atom is attached to the adjacent G⁴ meta-position of the phenyl ring.

A suitable pharmaceutically-acceptable salt of a compound of the Formula I is, for example, an acid-addition salt of a compound of the Formula I, for example an acid-addition salt with an inorganic or organic acid such as hydrochloric, hydrobromic, sulphuric, trifluoroacetic, citric or maleic acid; or, for example, a salt of a compound of the Formula I which is sufficiently acidic, for example an alkali or alkaline earth metal salt such as a calcium or magnesium salt, or an ammonium salt, or a salt with an organic base such as methylamine, dimethylamine, trimethylamine, piperidine, morpholine or tris-(2-hydroxyethyl)amine.

Particular novel compounds of the invention include, for example, quinazoline derivatives of the Formula I, or pharmaceutically-acceptable salts thereof, wherein, unless otherwise stated, each of m, R¹, Z, Q¹ and Q² has any of the meanings defined hereinbefore or in paragraphs (a) to (P) hereinafter :-
(a) m is 1 and the R¹ group is located at the 7-position and is selected from hydroxy, amino, methyl, ethyl, propyl, methoxy, ethoxy, propoxy, butoxy, pentoxy, pyrrolidin-1-yl, 2-pyrrolidin-1-ylethoxy, 3-pyrrolidin-1-ylpropoxy, 2-piperidinoethoxy, 3-piperidinopropoxy, 2-piperidin-3-ylethoxy, 3-piperidin-3-ylpropoxy, 2-piperidin-4-ylethoxy, 3-piperidin-4-ylpropoxy, 2-piperazin-1-ylethoxy, 3-piperazin-1-ylpropoxy, 2-morpholinoethoxy, 3-morpholinopropoxy, 2-homopiperidinoethoxy, 3-homopiperidinopropoxy, 2-homopiperazin-1-ylethoxy and 3-homopiperazin-1-ylpropoxy
   and wherein adjacent carbon atoms in any (2-6C)alkoxy chain within a R¹ substituent are optionally separated by the insertion into the chain of a group selected from O, NH and N(CH₃),
   and wherein any terminal CH₃ group within a (1-6C)alkoxy chain in a R¹ substituent optionally bears on the terminal CH₃ group a substituent selected from hydroxy, amino and N-(1-methylpyrrolidin-3-yl)-N-methylamino,
   and wherein any pyrrolidinyl, or piperidinyl group within a R¹ substituent optionally bears a substituent selected from hydroxy, methyl, amino, methylamino and dimethylamino,
   and wherein any piperazin-1-yl or homopiperazin-1-yl group within a R¹ substituent optionally bears a substituent at the 4-position selected from methyl, ethyl, isopropyl, 2-methoxyethyl, tetrahydrofurfuryl, 2-morpholinoethyl and 1-methylpiperidin-4-yl;
(b) m is 0;
(c) m is 1 and R¹ is located at the 7-position;
(d) Z is O;
(e) the Q¹-Z- group is selected from cyclopentyloxy, tetrahydrofuran-3-yloxy, tetrahydropyran-4-yloxy, tetrahydrothiopyran-4-yloxy, 1,1-dioxotetrahydrothiopyran-4-yloxy, 1-oxotetrahydrothiopyran-4-yloxy, tetrahydrothien-3-yloxy, 1,1-dioxodotetrahydrothien-3-yloxy, 1-oxotetrahydrothien-3-yloxy, pyrrolidin-3-yloxy, pyrrolidin-2-yloxy, piperidin-3-yloxy, piperidin-4-yloxy, homopiperidin-3-yloxy, homopiperidin-4-yloxy and azetidin-3-yloxy,
   and wherein the azetidinyl, pyrrolidinyl, piperidinyl or homopiperidinyl group within the Q¹-Z- group is optionally N- substituted by a substituent selected from methyl, ethyl, n-propyl, isopropyl, n-butyl, isobutyl, tert-butyl, allyl, 2-propynyl, acetyl, propionyl, methoxycarbonyl, ethoxycarbonyl, propoxycarbonyl, tert-butoxycarbonyl, methylsulphonyl, ethylsulphonyl, 2-methoxyethyl, carbamoylmethyl, N-methylcarbamoylmethyl, N,N-di-methylcarbamoylmethyl, 2-carbamoylethyl, 2-(N-methylcarbamoyl)ethyl, 2-(N,N-di-methylcarbamoyl)ethyl, acetylmethyl, 2-acetylethyl, methoxycarbonylmethyl and 2-methoxycarbonylethyl, and wherein any heterocyclyl group within the Q¹-Z- group optionally bears 1 or 2 oxo substituents;
(f) Q² is an aryl group of formula Ib wherein G³ and G⁴ together form a group of formula:- -CH=CH-NH-, -NH-CH=CH-, -NH-N=CH- or -CH=N-NH-,
   and the 9-membered bicyclic heteroaryl ring formed when G³ and G⁴ are linked together optionally bears on a NH group of the heteroaryl portion of the bicyclic ring a group of the formula:

   -X¹²-Q¹¹

   wherein X¹² is a direct bond or is selected from SO₂ and CO, wherein Q¹¹ is phenyl, benzyl, 2-phenylethyl, 2-furyl, furfuryl, 3-furyl, 3-furylmethyl, 2-oxazolyl, 4-oxazolyl, 2-oxazolylmethyl, 4-oxazolylmethyl, 2-imidazolyl, 4-imidazolyl, 2-imidazolylmethyl, 4-imidazolylmethyl, 2-, 3-or 4-pyridyl, 2-, 3-or 4-pyridylmethyl, 2-(2-, 3-or 4-pyridyl)ethyl, 2-, 4- or 5- pyrimidinyl, 2-, 4- or 5- pyrimidinylmethyl, 2-(2-, 4- or 5- pyrimidinyl)ethyl, 1,2,4-triazol-3-yl, 1,2,4-triazol-5-ylmethyl, 1,2,4-triazol-3-ylmethyl, 1,2,4-triazol-5-yl 2-thienyl, 3-thienyl, 2-thienylmethyl, 3-thienylmethyl, 2-(2-thienyl)ethyl, 2-(3-thienyl)ethyl, 2-thiazolyl, 4-thiazolyl, 2-thiazolylmethyl, 4-thiazolylmethyl, 1,2,5-thiadiazol-3-yl, 1,2,5-thiadiazol-3-ylmethyl, 2-(1,2,5-thiadiazol-3-yl)ethyl which optionally bears 1 or 2 substituents, which may be the same or different, selected from fluoro, chloro, bromo, cyano, hydroxy, methyl and ethyl,
   and the 9- membered bicyclic heteroaryl ring formed when G³ and G⁴ together are linked optionally bears on an available carbon atom in the heteroaryl portion of the bicyclic ring 1 substituent selected from fluoro, chloro, bromo, cyano, hydroxy, amino, methyl, ethyl, vinyl, ethynyl, methylamino and di-methylamino,
   and G² is selected from hydrogen, fluoro, chloro, bromo, trifluoromethyl, cyano, hydroxy, amino, methyl, ethyl, vinyl, ethynyl, methylamino and di-methylamino;
(g) Q² is an aryl group of formula Ib wherein G³ and G⁴ together form a group of formula:- -NH-CH=CH- or -NH-N=CH-,
   and the 9-membered bicyclic heteroaryl ring formed when G³ and G⁴ are linked together optionally bears on a NH group of the heteroaryl portion of the bicyclic ring a group of the formula:

   -X¹² -Q¹¹

   wherein X¹² is a direct bond or is SO₂ and Q¹¹ is benzyl or 2-pyridylmethyl, which optionally bears 1 or 2 substituents, which may be the same or different, selected from fluoro, chloro, bromo, cyano, hydroxy and methyl,
   and the 9- membered bicyclic heteroaryl ring formed when G³ and G⁴ together are linked optionally bears at the 3-position in the heteroaryl portion of the bicyclic ring 1 substituent selected from fluoro, chloro, bromo, cyano, hydroxy, amino, methyl, ethyl and ethynyl,
   and G² is selected from hydrogen, fluoro, chloro, bromo, cyano, hydroxy, amino, methyl, ethyl and ethynyl;
(h) m is 1 and the R¹ group is located at the 7-position and is selected from 3-pyrrolidin-1-ylpropoxy and 3-morpholinopropoxy,
   and wherein any heterocyclyl group within R¹ optionally bears an oxo substituent;
(i) m is 1 and the R¹ group is located at the 7-position and is methoxy or ethoxy;
(j) m is 1 and the R¹ group is located at the 7-position and is methoxy;
(k) the Q¹ -z- group is selected from tetrahydrofuran-3-yloxy and piperidin-4-yloxy and wherein any piperidin-4-yl group optionally bears a substituent at the 1-position selected from methyl, carbamoylmethyl, and N,N-dimethylcarbamoylmethyl;
(l) Q¹Z is 1-methylpiperidin-4-yloxy;
(m) Q¹Z is tetrahydrofuran-3-yloxy;
(n) Q¹Z is tetrahydropyran-4-yloxy;
(o) Q² N(H) is a group of the formula Ic: 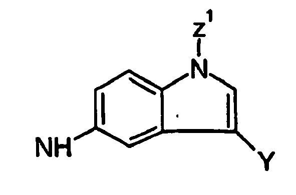 wherein Z¹ is hydrogen
   and Y¹ is selected from chloro and bromo;
(p) Q² is a group of formula Ib wherein:
   G² is hydrogen, and
   G³ and G⁴ together form a group of the formula: -NH-CH=CH-, and the indolyl ring so formed by G³ and G⁴ together with the carbon atoms to which they are attached is substituted at the 1-position by a group of the formula:

      -X¹²-Q¹¹
wherein X¹² is a direct bond and Q¹¹ is benzyl which is optionally substituted by 1 or 2 substituents, which may be the same or different, selected from fluoro, chloro, bromo, cyano, methyl and ethyl, (for example Q¹¹ is 2-fluorobenzyl or 3-fluorobenzyl), and
and wherein the indolyl ring so formed by G³ and G⁴ together with the carbon atoms to which they are attached is optionally substituted at the 3-position by a substituent selected from chloro and bromo;
and wherein m is 1, R¹ is located at the 7-position and wherein R¹ has any of the meanings defined herein;

A further embodiment of the invention is a quinazoline derivative of the Formula I wherein m is 0 or 1 and the **R¹** group, when present, is located at the 7-position and is methoxy,
**Q¹-Z**- is 1-methylpiperidin-4-yloxy,
and
**Q²** is an aryl group of formula Ib as hereinbefore defined wherein,
G² is hydrogen,
and G³ and G⁴ together form a group of formula:- -NH-CH=CH- or -NH-N=CH-,
and the 9-membered bicyclic heteroaryl ring formed when G³ and G⁴ are linked together optionally bears on a NH group of the heteroaryl portion of the bicyclic ring a group of the formula:

-X¹²-Q¹¹

wherein X¹² is a direct bond or is SO₂, and Q¹¹ is phenyl, benzyl, or 2-pyridylmethyl which optionally bears a fluoro substituent,
and the 9- membered bicyclic heteroaryl ring formed when G³ and G⁴ together are linked optionally bears at the 3-position a chloro substituent;
or a pharmaceutically acceptable salt thereof.

Suitable values for Q² is this embodiment include, for example 1-benzenesulphonylindol-5-yl, 1-benzylindol-5-yl, 1-(2-pyridylmethyl)indol-5-yl, 1-(2-pyridylmethyl)indazol-5-yl and 1-(3-fluorobenzyl)indazol-5-yl.

A further embodiment of the invention is a quinazoline derivative of the Formula **I** wherein:
**m** is 0 or 1 and the **R¹** group, when present is located at the 7 position and is methoxy;
the Q¹-Z- group is 1-methylpiperidin-4-yloxy; and
**Q²N(H)** is a group of the formula Ic: 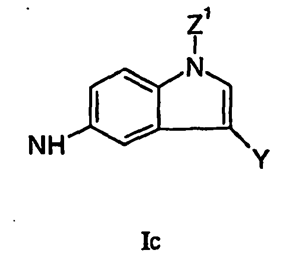
wherein Z¹ is hydrogen, and
Y is selected from hydrogen, chloro and bromo; or a pharmaceutically acceptable salt thereof.

A further embodiment of the invention is a quinazoline derivative of the Formula **I** wherein:
**m** is 1 and the **R¹** group is located at the 7 position and is methoxy;
**the Q¹-Z-** group is 1-methylpiperidin-4-yloxy;
   and
Q² is a group of formula Ib as hereinbefore defined wherein:
   G² is hydrogen, and
   G³ and G⁴ together form a group of the formula: -NH-CH=CH-, and the indolyl ring so formed by G³ and G⁴ together with the carbon atoms to which they are attached is substituted at the 1-position by a group of the formula:

      -X¹²-Q¹¹
wherein X¹² is a direct bond and Q¹¹ is benzyl which is optionally substituted by 1 fluoro substituent, (for example Q¹¹ is 2-fluorobenzyl or 3-fluorobenzyl); or a pharmaceutically acceptable salt thereof.

A further particular preferred compound of the invention is, for example, a quinazoline derivative of the Formula **I** selected from:
4-(3-Chloroindol-5-ylamino)-5-(1-methylpiperidin-4-yloxy)quinazoline;
4-(3-Chloroindol-5-ylamino)-7-methoxy-5-(1-methylpiperidin-4-yloxy)quinazoline;
4-(Indol-5-ylamino)-7-methoxy-5-(1-methylpiperidin-4-yloxy)quinazoline;
or a pharmaceutically acceptable acid addition salt thereof;

A further particular preferred compound of the invention is, for example, a quinazoline derivative of the Formula I selected from:
4-(3-Bromoindol-5-ylamino)-7-methoxy-5-(1-methylpiperidin-4-yloxy)quinazoline;
4-(1-(3-Fluorobenzyl)indol-5-ylamino)-7-methoxy-5-(1-methylpiperidin-4-yloxy)quinazoline; and
4-(1-(2-Fluorobenzyl)indol-5-ylamino)-7-methoxy-5-(1-methylpiperidin-4-yloxy)quinazoline;
or a pharmaceutically acceptable acid addition salt thereof.

A quinazoline derivative of the Formula I, or a pharmaceutically-acceptable salt thereof, may be prepared by any process known to be applicable to the preparation of chemically-related compounds. Such processes, when used to prepare a quinazoline derivative of the Formula I are provided as a further feature of the invention and are illustrated by the following representative process variants in which, unless otherwise stated, Q¹, Z, m, R¹, and Q² have any of the meanings defined hereinbefore. Necessary starting materials may be obtained by standard procedures of organic chemistry. The preparation of such starting materials is described in conjunction with the following representative process variants and within the accompanying Examples. Alternatively necessary starting materials are obtainable by analogous procedures to those illustrated which are within the ordinary skill of an organic chemist.

### Process (a) The reaction, conveniently in the presence of a suitable base, of a quinazoline of the Formula II

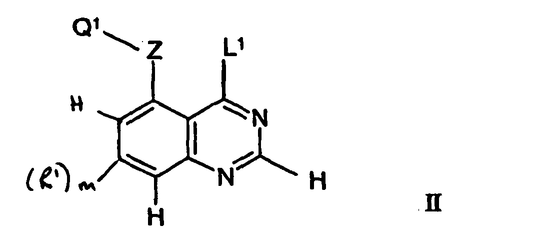

wherein L¹ is a displaceable group and Q¹, Z, m and R¹ have any of the meanings defined hereinbefore except that any functional group is protected if necessary, with an compound of the Formula

Q²NH₂

wherein Q² has any of the meanings defined hereinbefore except that any functional group is protected if necessary, whereafter any protecting group that is present is removed by conventional means.

A suitable base is, for example, an organic amine base such as, for example, pyridine, 2,6-lutidine, collidine, 4-dimethylaminopyridine, triethylamine, di-isopropylethylamine, N-methylmorpholine or diazabicyclo[5.4.0]undec-7-ene, or, for example, an alkali or alkaline earth metal carbonate, for example sodium carbonate, potassium carbonate, calcium carbonate, or, for example, an alkali metal hydride, for example sodium hydride.

A suitable displaceable group L¹ is, for example, a halogeno, alkoxy, aryloxy, mercapto, alkylthio, arylthio, alkylsulphinyl, arylsulphinyl, alkylsuphonyl, arylsulphonyl or sulphonyloxy group, for example a chloro, bromo, methoxy, phenoxy, pentafluorophenoxy, methylthio, methanesulphonyl, methanesulphonyloxy or toluene-4-sulphonyloxy group. The reaction is conveniently carried out in the presence of a suitable inert solvent or diluent, for example an alcohol or ester such as methanol, ethanol, isopropanol or ethyl acetate, a halogenated solvent such as methylene chloride, chloroform or carbon tetrachloride, an ether such as tetrahydrofuran or 1,4-dioxan, an aromatic solvent such as toluene, or a dipolar aprotic solvent such as N,N-dimethylformamide, N,N-dimethylacetamide, N-methylpyrrolidin-2-one or dimethylsulphoxide. The reaction is conveniently carried out at a temperature in the range, for example, 10 to 250°C, preferably in the range 40 to 80°C.

The quinazoline of the Formula **II** may also be reacted with a compound of the formula Q²NH₂ in the presence of a protic solvent such as isopropanol, conveniently in the presence of an acid, for example hydrogen chloride gas in diethyl ether or dioxane, or hydrochloric acid. Alternatively, this reaction may be conveniently carried out in an aprotic solvent, such as dioxane or a dipolar aprotic solvent such as N,N-dimethylacetamide in the presence of an acid, for example hydrogen chloride gas in diethyl ether or dioxane, or hydrochloric acid. The above reactions are conveniently carried out at a temperature in the range, for example, 0 to 150°C, preferably at or near the reflux temperature of the reaction solvent.

The quinazoline derivative of the Formula II, wherein L¹ is halogeno, may be reacted with a compound of the formula Q²NH₂ in the absence of an acid or a base. In this reaction displacement of the halogeno leaving group L¹ results in the formation of the acid HL¹ in-situ and the auto-catalysis of the reaction. Conveniently the reaction is carried out in a suitable inert organic solvent, for example iso propanol, dioxane or N,N-dimethylacetamide. Suitable conditions for this reaction are as described above

The quinazoline derivative of the Formula I may be obtained from this process in the form of the free base or alternatively it may be obtained in the form of a salt with the acid of the formula H-L¹ wherein L¹ has the meaning defined hereinbefore. When it is desired to obtain the free base from the salt, the salt may be treated with a suitable base, for example, an alkali or alkaline earth metal carbonate or hydroxide, for example sodium carbonate, potassium carbonate, calcium carbonate, sodium hydroxide or potassium hydroxide.

Protecting groups may in general be chosen from any of the groups described in the literature or known to the skilled chemist as appropriate for the protection of the group in question and may be introduced by conventional methods. Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule.

Specific examples of protecting groups are given below for the sake of convenience, in which "lower", as in, for example, lower alkyl, signifies that the group to which it is applied preferably has 1-4 carbon atoms. It will be understood that these examples are not exhaustive. Where specific examples of methods for the removal of protecting groups are given below these are similarly not exhaustive. The use of protecting groups and methods of deprotection not specifically mentioned are, of course, within the scope of the invention.

A carboxy protecting group may be the residue of an ester-forming aliphatic or arylaliphatic alcohol or of an ester-forming silanol (the said alcohol or silanol preferably containing 1-20 carbon atoms). Examples of carboxy protecting groups include straight or branched chain (1-12C)alkyl groups (for example isopropyl, and tert-butyl); lower alkoxy- lower alkyl groups (for example methoxymethyl, ethoxymethyl and isobutoxymethyl); lower acyloxy-lower alkyl groups, (for example acetoxymethyl, propionyloxymethyl, butyryloxymethyl and pivaloyloxymethyl); lower alkoxycarbonyloxy-lower alkyl groups (for example 1-methoxycarbonyloxyethyl and 1-ethoxycarbonyloxyethyl); aryl-lower alkyl groups (for example benzyl, 4-methoxybenzyl, 2-nitrobenzyl, 4-nitrobenzyl, benzhydryl and phthalidyl); tri(lower alkyl)silyl groups (for example trimethylsilyl and tert-butyldimethylsilyl); tri(lower alkyl)silyl-lower alkyl groups (for example trimethylsilylethyl); and (2-6C)alkenyl groups (for example allyl). Methods particularly appropriate for the removal of carboxyl protecting groups include for example acid-, base-, metal- or enzymically-catalysed cleavage.

Examples of hydroxy protecting groups include lower alkyl groups (for example tert-butyl), lower alkenyl groups (for example allyl); lower alkanoyl groups (for example acetyl); lower alkoxycarbonyl groups (for example tert-butoxycarbonyl); lower alkenyloxycarbonyl groups (for example allyloxycarbonyl); aryl-lower alkoxycarbonyl groups (for example benzyloxycarbonyl, 4-methoxybenzyloxycarbonyl, 2-nitrobenzyloxycarbonyl and 4-nitrobenzyloxycarbonyl); tri(lower alkyl)silyl (for example trimethylsilyl and tert-butyldimethylsilyl) and aryl-lower alkyl (for example benzyl) groups.

Examples of amino protecting groups include formyl, aryl-lower alkyl groups (for example benzyl and substituted benzyl, 4-methoxybenzyl, 2-nitrobenzyl and 2,4-dimethoxybenzyl, and triphenylmethyl); di-4-anisylmethyl and furylmethyl groups; lower alkoxycarbonyl (for example tert-butoxycarbonyl); lower alkenyloxycarbonyl (for example allyloxycarbonyl); aryl-lower alkoxycarbonyl groups (for example benzyloxycarbonyl, 4-methoxybenzyloxycarbonyl, 2-nitrobenzyloxycarbonyl and 4-nitrobenzyloxycarbonyl); lower alkanoyloxyalkyl groups (for example pivaloyloxymethyl); trialkylsilyl (for example trimethylsilyl and tert-butyldimethylsilyl); alkylidene (for example methylidene) and benzylidene and substituted benzylidene groups.

Methods appropriate for removal of hydroxy and amino protecting groups include, for example, acid-, base-, metal- or enzymically-catalysed hydrolysis for groups such as 2-nitrobenzyloxycarbonyl, hydrogenation for groups such as benzyl and photolytically for groups such as 2-nitrobenzyloxycarbonyl. For example a tert butoxycarbonyl protecting group may be removed from an amino group by an acid catalysed hydrolysis using trifluoroacetic acid.

The reader is referred to Advanced Organic Chemistry, 4th Edition, by J. March, published by John Wiley & Sons 1992, for general guidance on reaction conditions and reagents and to Protective Groups in Organic Synthesis, 2nd Edition, by T. Green et al., also published by John Wiley & Son, for general guidance on protecting groups.

Quinazoline starting materials of the Formula **II** may be obtained by conventional procedures. For example, a 3,4-dihydroquinazolin-4-one of Formula III 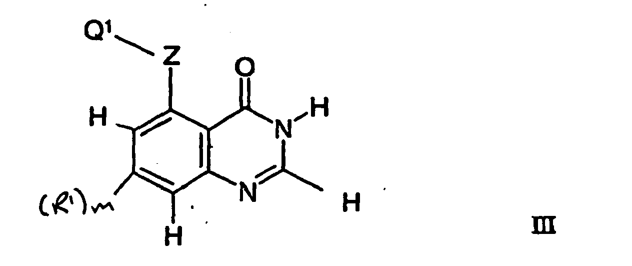 wherein m, R¹, Q¹ and Z have any of the meanings defined hereinbefore except that any functional group is protected if necessary, may be reacted with a halogenating agent such as thionyl chloride, phosphoryl chloride or a mixture of carbon tetrachloride and triphenylphosphine whereafter any protecting group that is present is removed by conventional means. The reaction is conveniently carried out in a suitable inert solvent, for example 1,2-dichloroethane or N,N-dimethylformamide conveniently in the presence of an base such as an organic base, for example di-isopropylethylamine. The reaction is conveniently carried out at a temperature in the range, for example, 0 to 150°C, preferably at or near the reflux temperature of the reaction solvent.

The 4-chloroquinazoline so obtained may be converted, if required, into a 4-pentafluorophenoxyquinazoline by reaction with pentafluorophenol in the presence of a suitable base such as potassium carbonate and in the presence of a suitable solvent such as N,N-dimethylformamide.

The compound of Formula **I** may be also be prepared using a telescoped process stating from the compound of Formula **III**, wherein the compound of the Formula Q²NH₂ is reacted with the compound of Formula **II** following halogenation of the compound of Formula III. The use of such a telescoped process avoids the need to isolate the compound of Formula II prior to reaction with the compound of formula Q²NH₂.

The 3,4-dihydroquinazolin-4-one of Formula III may be obtained using conventional procedures. For example when Z is an oxygen atom the compound of Formula III may be prepared as illustrated by Reaction Scheme 1 starting with the compound of Formula IV. 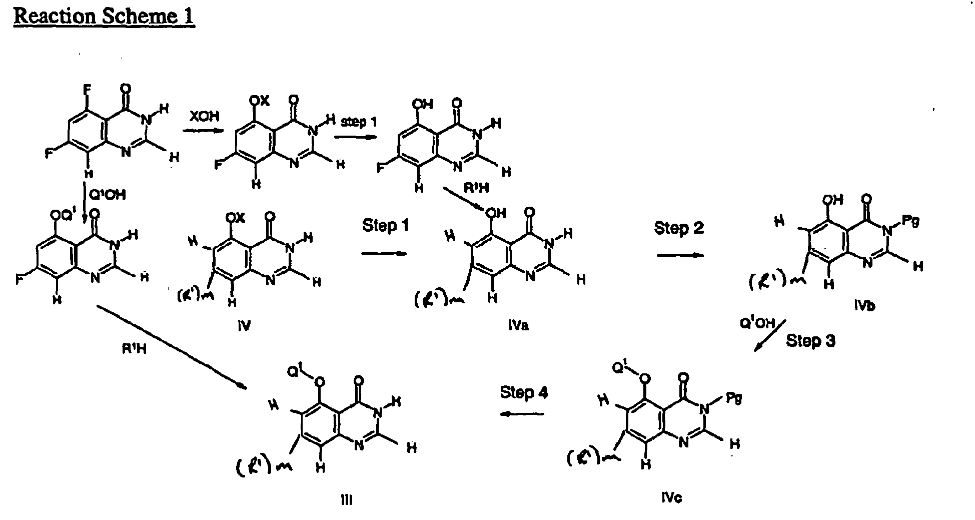 wherein R¹ and Q¹ are as hereinbefore defined, X is a suitable hydroxy protecting group such as (1-6C)alkyl (for example methyl) or benzyl, and Pg is a suitable amine protecting group.
Notes:
Step (1)
   When X is (1-6C)alkyl, it may be may be cleaved from the compound of formula IV by conventional methods, such as by treatment of the compound of Formula IV with, for example:
   (i) an alkali metal (1-6C)alkylsulphide such as sodium ethanethiolate;
   (ii) an alkali metal diarylphosphide such as lithium diphenylphosphide;
   (iii) a boron or aluminium trihalide such as boron tribromide;
   (iv) magnesium bromide, preferably in the presence of a suitable base, such as an organic base, for example pyridine; or
   (v) pyridine hydrochloride in pyridine.
      Generally the cleavage reaction is carried out at a temperature in the range of from, for example, 40 to 150°C.

   When X is benzyl, it may be may be cleaved from the compound of formula IV by, for example, acid catalysed hydrolysis, for example by treatment of the compound of Formula IV with trifluoroacetic acid. Conveniently the reaction is carried out at a temperature in the range of 30 to 120°C, for example 70°C.
Step (2)
   The protecting group Pg is added to the 3,4-dihydro-5-hydroxyquinazolin-4-one of Formula IVa using conventional techniques. For example a suitable Pg is a pivaloyloxymethyl group that may be added to the compound of Formula IVa by reacting the compound of Formula IVa with chloromethylpivalate in the presence of a suitable base such as sodium hydride.
Step (3)
   The Q¹O group may be introduced by coupling the compound of Formula IVb with an alcohol of the Formula Q¹OH in the presence of a suitable dehydrating agent. Suitable conditions for the coupling reaction are described below with reference to process (b).
Step (4)
   The protecting group Pg may be removed using conventional methods, for example when Pg is a pivaloyloxymethyl group it may be removed by treating the compound of Formula IVc with a methanolic ammonia solution.
   The compound of formula IV may be prepared starting from an aniline of the Formula V as illustrated in Reaction Scheme 2. 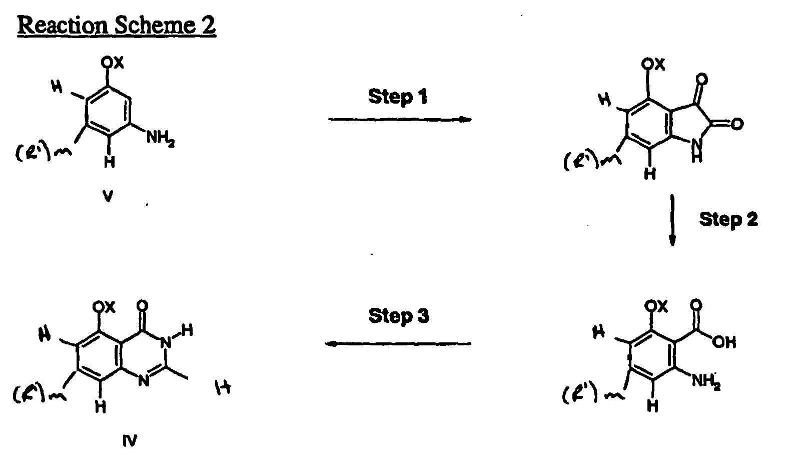
wherein R¹, m and X are as hereinbefore defined.

### Notes:

Steps 1 and 2 may be carried out using analogous conditions to the processes described in Organic Syntheses, Coll Vol 1, p 327-330; J Org Chem 1969, 34, 3484-3489.

Step 3 may be carried out using analogous conditions to the process described in J. Org. Chem. 1952, 17, 141-148; J Med Chem 1994, 37, 2106-2111.

Anilines of Formula V are commercially available compounds, or they are known in the literature, or can be prepared using well known processes in the art.

The quinazoline starting materials of the formula II may also be prepared using alternative synthetic routes to those described above using conventional techniques in organic chemistry. Representative examples of suitable synthetic methods for the preparation of the starting quinazoline material of the formula II and the intermediates described above in Reaction Schemes 1 and 2 are provided by the examples herein.

Compounds of the Formula Q²NH₂ are commercially available compounds, or they are known in the literature, or can be prepared using conventional synthetic methods.

Process (b) For the production of those compounds of the Formula I wherein Z is an oxygen atom, the coupling, conveniently in the presence of a suitable dehydrating agent, of an alcohol of the Formula

Q¹-OH

wherein Q¹ has any of the meanings defined hereinbefore except that any functional group is protected if necessary with a quinazoline of the Formula VI 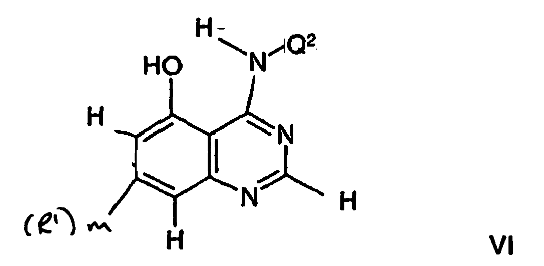 wherein m, R¹, and Q² have any of the meanings defined hereinbefore except that any functional group is protected if necessary, whereafter any protecting group that is present is removed by conventional means.

A suitable dehydrating agent is, for example, a carbodiimide reagent such as dicyclohexylcarbodiimide or 1-(3-dimethylaminopropyl)-3-ethylcarbodiimide or a mixture of an azo compound such as diethyl or di-tert-butyl azodicarboxylate and a phosphine such as triphenylphosphine. The reaction is conveniently carried out in the presence of a suitable inert solvent or diluent, for example a halogenated solvent such as methylene chloride, chloroform or carbon tetrachloride and at a temperature in the range, for example, 0 to 150°C, preferably at or near ambient temperature.

The quinazoline of the Formula VI may be obtained by conventional procedures. For example, by cleavage of the group represented by X from the compound of the Formula VII 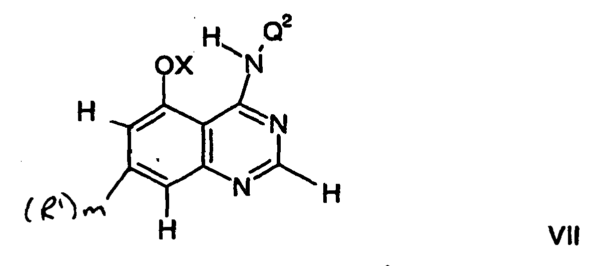 wherein X is as defined hereinbefore and m, R¹, Q² and m have any of the meanings defined hereinbefore except that any functional group is protected if necessary, whereafter any protecting group that is present is removed by conventional means.

The cleavage reaction is conveniently carried out as hereinbefore described in relation to step (1) in Reaction Scheme 1.

The compound of Formula VII may be prepared by for example reacting the compound of the Formula (IV) as hereinbefore defined, with a halogenating agent such as thionyl chloride, phosphoryl chloride or a mixture of carbon tetrachloride and triphenylphosphine. The resulting compound is then reacted with a compound of the Formula

Q²NH₂

wherein Q² has any of the meanings defined hereinbefore except that any functional group is protected if necessary, whereafter any protecting group that is present is removed by conventional means. The halogenation reaction may be performed under analogous conditions to those described above in relation to the reaction with the compound of the Formula III. The subsequent reaction with the compound of the Formula Q²NH₂ may be performed under analogous conditions to those described above in relation to the reaction with the compound of the Formula II.

Process (c) : For the production of those compounds of the Formula I wherein Z is O, the reaction, conveniently in the presence of a suitable base, of an alcohol of the Formula

Q¹-OH

wherein Q¹ has any of the meanings defined hereinbefore except that any functional group is protected if necessary with a quinazoline of the Formula VIII 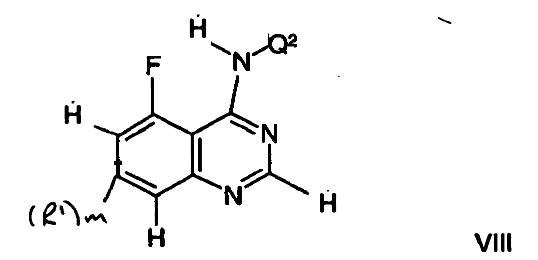 wherein m, R¹, and Q² have any of the meanings defined hereinbefore except that any functional group is protected if necessary, whereafter any protecting group that is present is removed by conventional means.

A suitable base includes, for example a strong non-nucleophilic base such as an alkali metal hydride, for example sodium hydride, or an alkali metal amide, for example lithium di-isopropylamide (LDA).

The reaction is conveniently carried out in the presence of a suitable inert solvent or diluent, for example a halogenated solvent such as methylene chloride, chloroform or carbon tetrachloride, an ether such as tetrahydrofuran or 1,4-dioxan, an aromatic solvent such as toluene, or a dipolar aprotic solvent such as N,N-dimethylformamide,
N,N-dimethylacetamide, N-methylpyrrolidin-2-one or dimethylsulphoxide. The reaction is conveniently carried out at a temperature in the range, for example, 10 to 250°C, preferably in the range 40 to 150°C. This process is particularly suitable for the preparation of those compounds of formula I in which m=0.

The quinazoline of the Formula VIII may be obtained by conventional procedures. For example, a quinazoline of the Formula IX 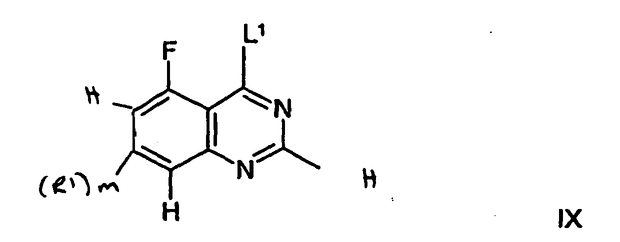 wherein L¹ is a displaceable group as defined hereinbefore (such as halogeno, for example chloro) and m and R¹ have any of the meanings defined hereinbefore except that any functional group is protected if necessary, may be reacted with a compound of the Formula

Q²NH₂

wherein Q² has any of the meanings defined hereinbefore except that any functional group is protected if necessary, whereafter any protecting group that is present is removed by conventional means. The reaction may be performed under analogous conditions to those described above under Process (a).

The quinazoline of Formula IX may be obtained using conventional methods, for example a 3,4-dihydroquinazolin-4-one of Formula X 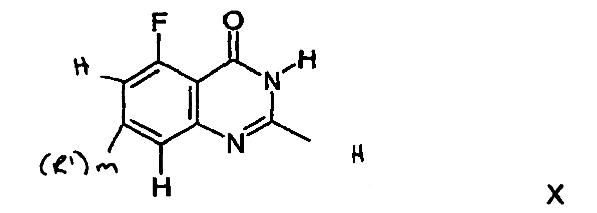 wherein m and R¹ have any of the meanings defined hereinbefore except that any functional group is protected if necessary, may be reacted with a halogenating agent such as thionyl chloride, phosphoryl chloride or a mixture of carbon tetrachloride and triphenylphosphine whereafter any protecting group that is present is removed by conventional means.

Conveniently compounds of formula VIII may be prepared directly starting from the compound of formula X using a telescoped process. In this process the 3,4-dihydroquinazolin-4-one of Formula X is halogenated as described above using a suitable halogenating agent. The resulting product is then reacted directly with the compound of the formula Q²NH₂ as described above, to give a compound of the formula VIII. This process enables compounds of formula VIII to be prepared without isolating the intermediate compound of the formula IX.

The quinazoline starting materials of Formula X are known or may be prepared using conventional methods. For example the compound of the formula X wherein m=0 is described in described in J. Org. Chem. 1952, 17, 164-176.

In an alternative process the 3,4-dihydroquinazolin-4-one of Formula X is reacted with the alcohol of the Formula Q¹-OH as described above to give a compound of Formula III. The compound of Formula III may then be converted to a compound of Formula I by halogenation and reaction with the compound of the formula Q²NH₂ as described above under Process (a).

Process (d) For the production of those compounds of the Formula I wherein m is 1 and R¹ is a group of the formula

Q³-X¹-

wherein Q³ is a - heterocyclyl-(1-6C)alkyl group as defined above and X¹ is O, the coupling, conveniently in the presence of a suitable dehydrating agent as defined hereinbefore, of a quinazoline of the Formula XI 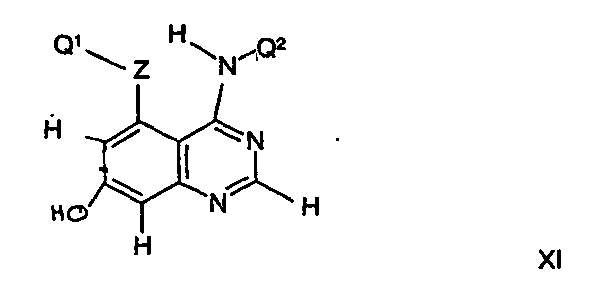 wherein Q¹, Z, and Q² have any of the meanings defined hereinbefore except that any functional group is protected if necessary, with an alcohol of the formula Q³OH wherein any functional group in Q³ is protected if necessary, whereafter any protecting group that is present is removed by conventional means.

The reaction is conveniently carried out in the presence of a suitable inert solvent or diluent, for example a halogenated solvent such as methylene chloride, chloroform or carbon tetrachloride and at a temperature in the range, for example, 10 to 150°C, preferably at or near ambient temperature.

The compound of Formula XI may, for example, be prepared according to process (a) described above.

Process (e) For the production of those compounds of the Formula I wherein R¹ is a hydroxy group, the cleavage of a quinazoline derivative of the Formula I wherein R¹ is a (1-6C)alkoxy or arylmethoxy group.

The cleavage reaction may conveniently be carried out by any of the many procedures known for such a transformation. The cleavage reaction of a compound of the Formula I wherein R¹ is a (1-6C)alkoxy group may be carried out, for example, by treatment of the quinazoline derivative with an alkali metal (1-6C)alkylsulphide such as sodium ethanethiolate or, for example, by treatment with an alkali metal diarylphosphide such as lithium diphenylphosphide. Alternatively the cleavage reaction may conveniently be carried out, for example, by treatment of the quinazoline derivative with a boron or aluminium trihalide such as boron tribromide. The cleavage reaction of a compound of the Formula I wherein R¹ is a arylmethoxy group may be carried out, for example, by hydrogenation of the quinazoline derivative in the presence of a suitable metallic catalyst such as palladium or by reaction with an organic or inorganic acid, for example trifluoroacetic acid. Such reactions are preferably carried out in the presence of a suitable inert solvent or diluent as defined hereinbefore and at a temperature in the range, for example, 10 to 150°C, preferably at or near ambient temperature.

Process (f) For the production of those compounds of the Formula I wherein Q¹, R¹ or Q² contains a primary or secondary amino group, the cleavage of the corresponding compound of Formula I wherein Q¹, R¹ or Q² contains a protected primary or secondary amino group.

Suitable protecting groups for an amino group are, for example, any of the protecting groups disclosed hereinbefore for an amino group. Suitable methods for the cleavage of such amino protecting groups are also disclosed hereinbefore. In particular, a suitable protecting group is a lower alkoxycarbonyl group such as a tert-butoxycarbonyl group which may be cleaved under conventional reaction conditions such as under acid-catalysed hydrolysis, for example in the presence of trifluoroacetic acid.

Process (g) For the production of those compounds of the Formula I wherein Q¹, R¹ or Q² contains a (1-6C)alkoxy or substituted (1-6C)alkoxy group or a (1-6C)alkylamino or substituted (1-6C)alkylamino group, the alkylation, conveniently in the presence of a suitable base as defined hereinbefore, of a quinazoline derivative of the formula I wherein Q¹, R¹ or Q² contains a hydroxy group or a primary or secondary amino group as appropriate.

A suitable alkylating agent is, for example, any agent known in the art for the alkylation of hydroxy to alkoxy or substituted alkoxy, or for the alkylation of amino to alkylamino or substituted alkylamino, for example an alkyl or substituted alkyl halide, for example a (1-6C)alkyl chloride, bromide or iodide, a substituted (1-6C)alkyl chloride, bromide or iodide, or a substituted (1-6C)alkyl-tosylate, conveniently in the presence of a suitable base as defined hereinbefore, in a suitable inert solvent or diluent as defined hereinbefore and at a temperature in the range, for example, 10 to 140°C, conveniently at or near ambient temperature. An analogous procedure may be used to introduce optionally substituted (2-6C)alkenyloxy, (2-6C)alkenylamino, (2-6C)alkynloxy or (2-6C)alkynylamino groups into Q¹, R¹ or Q².

Process (h) For the production of those compounds of the Formula I wherein Q¹, R¹ or Q² contains an amino-hydroxy-disubstituted (1-6C)alkoxy group (such as 2-hydroxy-3-piperidinopropoxy, 2-hydroxy-3-methylaminopropoxy, 3-dimethylamino-2-hydroxypropoxy or 3-[N-(3-dimethylaminopropyl)-N-methylamino]-2-hydroxypropoxy), the reaction of a compound of the Formula I wherein Q¹, R¹ or Q² contains an epoxy-substituted (1-6C)alkoxy group with a heterocyclyl compound or an appropriate amine.

The reaction is conveniently carried out in the presence of a suitable inert diluent or carrier as defined hereinbefore and at a temperature in the range 10 to 150°C, preferably at or near ambient temperature.

Process (i) The reaction, conveniently in the presence of a suitable base as defined hereinbefore, of a quinazoline of the Formula XII 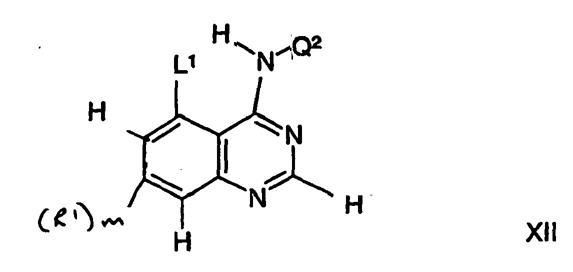 wherein L¹ is a displaceable group as defined hereinbefore and m, R¹ and Q² have any of the meanings defined hereinbefore except that any functional group is protected if necessary, with a compound of the Formula

Q¹ZH

wherein Q¹ and Z have any of the meanings defined hereinbefore except that any functional group is protected if necessary, whereafter any protecting group that is present is removed by conventional means.

The reaction is conveniently carried out in the presence of a suitable base, such as an alkali metal hydride, for example sodium hydride.

The reaction is conveniently carried out in the presence of a suitable inert diluent or carrier as defined hereinbefore and at a temperature in the range 10 to 150°C, preferably at or near 50°C.

The compound of Formula XII may be prepared using an analogous procedure to that described for the preparation of Formula VIII, except that the 5-fluoro atom is replaced by L¹.

Process (j) For the production of those compounds of the Formula I wherein Q¹, R¹ or Q² contains an amino-substituted (1-6C)alkoxy group (such as 3-piperidinopropoxy, 3-methylaminopropoxy or 3-dimethylaminopropoxy), the reaction of a compound of the Formula I wherein Q¹, R¹ or Q² contains a halogeno-substituted (1-6C)alkoxy group with a heterocyclyl compound or an appropriate amine.

The reaction is conveniently carried out in the presence of a suitable inert diluent or carrier as defined hereinbefore and at a temperature in the range 10 to 150°C, preferably at or near ambient temperature.

Process (k) For the production of those compounds of the Formula I wherein a heterocyclyl group in R¹, Q¹ or Q³ contains an S- or N-oxide the oxidation of a ring N or S atom in a compound of the Formula (I). Suitable oxidizing agents include, for example, a peracid (such as m-chloroperbenzoic acid) or perphthalic acid. The oxidation is conveniently carried out in a suitable inert solvent or diluent (such as dichloromethane) at a suitable temperature (such as -5 to 50°C).

Process (l) For the production of those compounds of the Formula I wherein R¹, Q¹ or Q² contains an (1-6C)alkylamino or substituted (1-6C)alkylamino group or a nitrogen linked heterocyclyl group, the reductive amination of an aldehyde or ketone group in a compound of formula 1, with a (1-6C)alkylamine, substituted (1-6C)alkylamine group or a heterocycle containing an NH group in the presence of a suitable reducing agent. A suitable reducing agent is, for example, a hydride reducing agent, for example an alkali metal aluminium hydride such as lithium aluminium hydride, formic acid or, preferably, an alkali metal borohydride such as sodium borohydride, sodium cyanoborohydride, sodium triethylborohydride, sodium trimethoxyborohydride and sodium triacetoxyborohydride. The reaction is conveniently performed in a suitable inert solvent or diluent, for example tetrahydrofuran or diethyl ether for the more powerful reducing agents such as lithium aluminium hydride, and, for example, methylene chloride or a protic solvent such as methanol and ethanol for the less powerful reducing agents such as sodium triacetoxyborohydride and sodium cyanoborohydride. The reaction is conveniently performed at a temperature in the range, for example, 10 to 100°C, conveniently at or near ambient temperature.

An analogous reductive amination to that described above may be used to introduce an alkyl or substituted alkyl group onto a primary or secondary amine group in a compound of the formula I by reductive amination with a corresponding ketone in the presence of a suitable reducing agent. For example, for the production of those compounds of the Formula I wherein Q¹ or Q² contains a N-methyl group, the corresponding compound containing an NH group may be reacted with formaldehyde in the presence of a suitable reducing agent as described above.

Process (m) The conversion of one compound of the Formula I into another compound of the Formula I.

It will be appreciated that certain of the various ring substituents in the compounds of the present invention may be introduced by standard aromatic substitution reactions or generated by conventional functional group modifications either prior to or immediately following the processes mentioned above (for example as in process (r)), and as such are included in the process aspect of the invention. Such reactions and modifications include, for example, introduction of a substituent by means of an aromatic substitution reaction, reduction of substituents, alkylation of substituents and oxidation of substituents. The reagents and reaction conditions for such procedures are well known in the chemical art. Particular examples of aromatic substitution reactions include the introduction of a nitro group using concentrated nitric acid, the introduction of an acyl group using, for example, an acyl halide and Lewis acid (such as aluminium trichloride) under Friedel Crafts conditions; the introduction of an alkyl group using an alkyl halide and Lewis acid (such as aluminium trichloride) under Friedel Crafts conditions; and the introduction of a halogeno group. Particular examples of modifications include the oxidation of alkylthio to alkylsulphinyl or alkylsulphonyl; the substitution of an NH group in Q¹ or Q² by the reaction with an optionally substituted alkyl halide, an optionally substituted alkenyl halide, an optionally substituted alkynyl halide or optionally substituted alkanoyl halide; the introduction of a halogeno group into an aromatic or heteroaromatic ring (for example within an indole) by reaction with an N-halogeno-succinimide; and the introduction of a cyano group into an aromatic ring by reaction with an isocyanate, for example chlorosuphonyl isocyanate.

When a pharmaceutically-acceptable salt of a quinazoline derivative of the Formula I is required, for example an acid-addition salt, it may be obtained by, for example, reaction of said quinazoline derivative with a suitable acid using a conventional procedure.

### Biological Assays

The inhibitory activities of compounds were assessed in non-cell based protein tyrosine kinase assays as well as in cell based proliferation assays before their *in vivo* activity was assessed in Xenograft studies.

### a) Protein Tyrosine Kinase phosphorylation Assays

This test measures the ability of a test compound to inhibit the phosphorylation of a tyrosine containing polypeptide substrate by an enzyme selected from the EGFR kinase, erbB2 kinase and erb4 kinase.

Recombinant intracellular fragments of EGFR, erbB2 and erbB4 (accession numbers X00588, X03363 and L07868 respectively) were cloned and expressed in the baculovirus/Sf21 system. Lysates were prepared from these cells by treatment with ice-cold lysis buffer (20mM N-2-hydroxyethylpiperizine-N'-2-ethanesulphonic acid (HEPES) pH7.5, 150mM NaCl, 10% glycerol, 1% Triton X-100, 1.5mM MgCl₂, 1mM ethylene glycol-bis (β-aminoethyl ether) N',N',N',N'-tetraacetic acid (EGTA), plus protease inhibitors and then cleared by centrifugation.

Constitutive kinase activity of these recombinant proteins was determined by their ability to phosphorylate a synthetic peptide (made up of a random co-polymer of Glutamic Acid, Alanine and Tyrosine in the ratio of 6:3:1). Specifically, Maxisorb^{™} 96-well immunoplates were coated with synthetic peptide (0.2µg of peptide in a 200µl phosphate buffered saline (PBS) solution and incubated at 4°C overnight). Plates were washed in 50mM HEPES pH 7.4 at room temperature to remove any excess unbound synthetic peptide. EGFR, erbB2 or erbB4 activities were assessed by incubation in peptide coated plates for 20 minutes at room temperature in 100mM HEPES pH 7.4 at room temperature, adenosine trisphosphate (ATP) at Km concentration for the respective enzyme, 10mM MnCl₂, 0.1mM Na₃VO₄, 0.2mM DL-dithiothreitol (DTT), 0.1% Triton X-100 with test compound in DMSO (final concentration of 2.5%). Reactions were terminated by the removal of the liquid components of the assay followed by washing of the plates with PBS-T (phosphate buffered saline with 0.5% Tween 20).

The immobilised phospho-peptide product of the reaction was detected by immunological methods. Firstly, plates were incubated for 90 minutes at room temperature with anti-phosphotyrosine primary antibodies that were raised in the mouse (4G10 from Upstate Biotechnology). Following extensive washing, plates were treated with Horseradish Peroxidase (HRP) conjugated sheep anti-mouse secondary antibody (NXA931 from Amersham) for 60 minutes at room temperature. After further washing, HRP activity in each well of the plate was measured colorimetrically using 22'-Azino-di-[3-ethylbenzthiazoline sulphonate (6)] diammonium salt crystals (ABTS^{™} from Roche) as a substrate.

Quantification of colour development and thus enzyme activity was achieved by the measurement of absorbance at 405nm on a Molecular Devices ThermoMax microplate reader. Kinase inhibition for a given compound was expressed as an IC₅₀ value. This was determined by calculation of the concentration of compound that was required to give 50% inhibition of phosphorylation in this assay. The range of phosphorylation was calculated from the positive (vehicle plus ATP) and negative (vehicle minus ATP) control values.

### b) KB cell proliferation assay

This assay measures the ability of a test compound to inhibit the proliferation of KB cells (human naso-pharangeal carcinoma obtained from the American Type Culture Collection (ATCC).

KB cells (human naso-pharangeal carcinoma obtained from the ATCC were cultured in Dulbecco's modified Eagle's medium (DMEM) containing 10% foetal calf serum, 2 mM glutamine and non-essential amino acids at 37°C in a 7.5% CO₂ air incubator. Cells were harvested from the stock flasks using Trypsin/ethylaminediaminetetraacetic acid (EDTA). Cell density was measured using a haemocytometer and viability was calculated using trypan blue solution before being seeded at a density of 1.25x10³ cells per well of a 96 well plate in DMEM containing 2.5% charcoal stripped serum, 1mM glutamine and non-essential amino acids at 37°C in 7.5% CO₂ and allowed to settle for 4 hours.

Following adhesion to the plate, the cells are treated with or without EGF (final concentration of 1ng/ml) and with or without compound at a range of concentrations in dimethylsulphoxide (DMSO) (1% final) before incubation for 4 days. Following the incubation period, cell numbers were determined by removal of the media by aspiration and incubating with 50ul of 3-(4,5-Dimethylthiazol-2-yl)-2,5-diphenyltetrazolium bromide (MTT) (stock 5mg/ml) for 2 hours. MTT solution was then removed by aspiration, allowed to air dry and the cells dissolved upon the addition of 100µl of DMSO.

Absorbance of this solubilised cells was read at 540nm to quantify cell biomass. Inhibition of proliferation was expressed as an IC₅₀ value. This was determined by calculation of the concentration of compound that was required to give 50% inhibition of proliferation. The range of proliferation was calculated from the positive (vehicle plus EGF) and negative (vehicle minus EGF) control values.

### c) H16N-2 cell proliferation assay

This assay measures the ability of a test compound to inhibit heregulin β or EGF driven proliferation of H16N-2 cells. These non-neoplastic eptihelial cells respond in a proliferative manner to stimulation with either EGF or heregulin β (Ram, G.R.and Ethier, S.P.(1996) Cell Growth and Differentiation, 7,551-561) were isolated human mammary tissue (Band, V. and Sager, R. Tumour progression in breast cancer. In: J. S. Rhim and A. Dritschilo (eds.), Neoplastic Transformation in human Cell Culture, pp 169-178. Clifton, NJ: Humana Press, 1991) and were obtained from the Dana-Farber Cancer Institute, 44 Binney Street, Boston, Massachusetts 02115.

H16N-2 cells were routinely cultured in culture medium (a 1:1 mix of Gibco F12 and Ham's αMEM media containing 1 % foetal calf serum, 10mM HEPES, 1µg/ml Insulin, 12.5ng/ml EGF, 2.8µM Hydrocortisone, 2nM Estradiol 5µM Ascorbic Acid, 10µg/ml Transferrin, 0.1mM Phosphoethanolamine, 15nM Sodium Selenite, 2mM Glutamine, 10nM Tri-iodo-thrynoine, 35µg/ml Bovine pituitary Extract and 0.1mM Ethanolamine) at 37°C in a 7.5% CO₂ air incubator. Cells were harvested from the stock flasks using Trypsin/ethylaminediaminetetraacetic acid (EDTA). Cell density was measured using a haemocytometer and viability was calculated using trypan blue solution before being seeded at a density of 1.0x10³ cells per well of a 96 well plate in the above media at 37°C in 7.5% CO₂ and allowed to settle for 72 hours.

Following this, the cells were starved of serum for 24 hours upon the addition of starvation medium (a 1:1 mix of Gibco F12 and Ham's αMEM media containing, 10mM HEPES, 2nM Estradiol, 5µM Ascorbic Acid, 10µg/ml Transferrin, 0.1mM Phosphoethanolamine, 15nM Sodium Selenite, 2mM Glutamine, and 0.1mM Ethanolamine) and incubated at 37°C in 7.5% CO₂. The cells were then treated with or without compound at a range of concentrations in dimethylsulphoxide (DMSO) (1% final) for two hours before the addition of exogenous ligand (at a final concentration of 100ng/ml of heregulin β or 5ng/ml of EGF) and incubation with both ligand and compound for 4 days at 37°C in 7.5% CO₂. Following the incubation period, cell numbers were determined by removal of the media by aspiration and incubating with 50µl of 3-(4,5-Dimethylthiazol-2-yl)-2,5-diphenyltetrazolium bromide (MTT) (stock 5mg/ml) for 2 hours. MTT solution was then removed by aspiration, allowed to air dry and the cells dissolved upon the addition of 100µl of DMSO.

Absorbance of this solubilised cells was read at 540nm to quantify cell biomass. Inhibition of proliferation was expressed as an IC₅₀ value. This was determined by calculation of the concentration of compound that was required to give 50% inhibition of proliferation. The range of proliferation was calculated from the positive (vehicle plus ligand) and negative (vehicle minus ligand) control values.

### d) In vivo LoVo Xenograft assay

This assay measures the ability of a test compound to inhibit the growth of a LoVo tumour cell xenograft (colorectal adenocarcinoma obtained from the ATCC) in Female Swiss athymic mice (Alderley Park, *nu*/*nu* genotype).

Female Swiss athymic (*nu*/*nu* genotype) mice were bred and maintained in Alderley Park in negative pressure Isolators (PFI Systems Ltd.). Mice were housed in a barrier facility with 12hr light/dark cycles and provided with sterilised food and water *ad libitum.* All procedures were performed on mice of at least 8 weeks of age. LoVo tumour cell xenografts were established in the hind flank of donor mice by sub-cutaneous injections of 1x10⁷ freshly cultured cells in 100µl of serum free media per animal. On day 5 post-implant, mice were randomised into groups of 7 prior to the treatment with compound or vehicle control that was administered once daily at 0.1ml/kg body weight. Tumour volume was assessed twice weekly by bilateral Vernier calliper measurement, using the formula (length x width) x √(length x width) x (π/6), where length was the longest diameter across the tumour, and width was the corresponding perpendicular. Growth inhibition from start of treatment was calculated by comparison of the mean changes in tumour volume for the control and treated groups, and statistical significance between the two groups was evaluated using a Students *t* test.

### e) In vivo BT-474 Xenograft assay

This assay measures the ability of a test compound to inhibit the growth of a BT-474 tumour cell xenograft (human mammary carcinoma obtained from Dr Baselga, Laboratorio Recerca Oncologica, Paseo Vall D'Hebron 119-129, Barcelona 08035, Spain) in Female Swiss athymic mice (Alderley Park, *nu*/*nu* genotype) (Baselga, J. et al. (1998) Cancer Research, 58, 2825-2831).

Female Swiss athymic (*nu*/*nu* genotype) mice were bred and maintained in Alderley Park in negative pressure Isolators (PFI Systems Ltd.). Mice were housed in a barrier facility with 12hr light/dark cycles and provided with sterilised food and water *ad libitum.* All procedures were performed on mice of at least 8 weeks of age. BT-474 tumour cell xenografts were established in the hind flank of donor mice by sub-cutaneous injections of 1x10⁷ freshly cultured cells in 100µl of serum free media with 50% Matrigel per animal. On day 14 post-implant, mice were randomised into groups of 10 prior to the treatment with compound or vehicle control that was administered once daily at 0.1ml/kg body weight. Tumour volume was assessed twice weekly by bilateral Vernier calliper measurement, using the formula (length x width) x √(length x width) x (π/6), where length was the longest diameter across the tumour, and width was the corresponding perpendicular. Growth inhibition from start of treatment was calculated by comparison of the mean changes in tumour volume for the control and treated groups, and statistical significance between the two groups was evaluated using a Students t test.

Although the pharmacological properties of the compounds of the Formula I vary with structural change as expected, in general activity possessed by compounds of the Formula I, may be demonstrated at the following concentrations or doses in one or more of the above tests (a), (b), (c), (d) and (e):-

| | |
|---|---|
| Test (a):- | IC₅₀ in the range, for example, 0.001- 10 µM; |
| Test (b):- | IC₅₀ in the range, for example, 0.001 - 20 µM; |
| Test (c):- | IC₅₀ in the range, for example, 0.001 - 20 µM; |
| Test (d):- | activity in the range, for example, 1-200 mg/kg/day; |
| Test (e):- | activity in the range, for example, 1-200 mg/kg/day; |

No physiologically unacceptable toxicity was observed in Test (d) or (e) at the effective dose for compounds tested of the present invention. Accordingly no untoward toxicological effects are expected when a compound of Formula I, or a pharmaceutically-acceptable salt thereof, as defined hereinbefore is administered at the dosage ranges defined hereinafter.

According to a further aspect of the invention there is provided a pharmaceutical composition which comprises a quinazoline derivative of the Formula I, or a pharmaceutically-acceptable thereof, as defined hereinbefore in association with a pharmaceutically-acceptable diluent or carrier.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents.

The amount of active ingredient that is combined with one or more excipients to produce a single dosage form will necessarily vary depending upon the host treated and the particular route of administration. For example, a formulation intended for oral administration to humans will generally contain, for example, from 0.5 mg to 0.5 g of active agent (more suitably from 0.5 to 100 mg, for example from 1 to 30 mg) compounded with an appropriate and convenient amount of excipients which may vary from about 5 to about 98 percent by weight of the total composition.

The size of the dose for therapeutic or prophylactic purposes of a compound of the Formula I will naturally vary according to the nature and severity of the conditions, the age and sex of the animal or patient and the route of administration, according to well known principles of medicine.

In using a compound of the Formula I for therapeutic or prophylactic purposes it will generally be administered so that a daily dose in the range, for example, 0.1 mg/kg to 75 mg/kg body weight is received, given if required in divided doses. In general lower doses will be administered when a parenteral route is employed. Thus, for example, for intravenous administration, a dose in the range, for example, 0.1 mg/kg to 30 mg/kg body weight will generally be used. Similarly, for administration by inhalation, a dose in the range, for example, 0.05 mg/kg to 25 mg/kg body weight will be used. Oral administration is however preferred, particularly in tablet form. Typically, unit dosage forms will contain about 0.5 mg to 0.5 g of a compound of this invention.

We have found that the compounds of the present invention possess anti-proliferative properties such as anti-cancer properties that are believed to arise from their erbB family receptor tyrosine kinase inhibitory activity, particularly inhibition of the EGFR and/or erbB2 and/or erbB4 receptor tyrosine kinases. Accordingly the compounds of the present invention are expected to be useful in the treatment of diseases or medical conditions mediated alone or in part by erbB receptor tyrosine kinases, i.e. the compounds may be used to produce a erbB receptor tyrosine kinase inhibitory effect in a warm-blooded animal in need of such treatment. Thus the compounds of the present invention may be useful in the treatment of malignant cells characterised by inhibition of one or more of the erbB family of receptor tyrosine kinases. Particularly the compounds of the invention may be used to produce an anti-proliferative and/or pro-apoptotic and/or anti-invasive effect mediated alone or in part by the inhibition of erbB receptor tyrosine kinases. Particularly, the compounds of the present invention are expected to be useful in the prevention or treatment of those tumours that are sensitive to inhibition of one or more of the erbB receptor tyrosine kinases, such as EGFR and/or erbB2 and/or erbB4 kinase that are involved in the signal transduction steps which drive proliferation and survival of these tumour cells. Accordingly the compounds of the present invention are expected to be useful in the treatment and/or prevention of a number of hyperproliferative disorders by providing an anti-proliferative effect. These disorders include, for example psoriasis, benign prostatic hyperplasia (BPH), atherosclerosis and restenosis and, in particular, EGF and/or erbB2 receptor tyrosine kinase driven tumours. Such benign or malignant tumours may affect any tissue and include non-solid tumours such as leukaemia, multiple myeloma or lymphoma, and also solid tumours, for example bile duct, bone, bladder, brain/CNS, breast, colorectal, endometrial, gastric, head and neck, hepatic, lung, neuronal, oesophageal, ovarian, pancreatic, prostate, renal, skin, testicular, thyroid, uterine and vulval cancers.

Certain compounds according to the present invention possess potent inhibitory activity against EGFR tyrosine kinase whilst possessing less potent activity against other erb receptor tyrosine kinases such as erbB2. Such compounds are expected to useful as selective receptor tyrosine inhibitors. Furthermore, certain compounds according to the present invention are potent inhibitors of both EGFR and erbB2 tyrosine kinases and are expected to be useful in the treatment of conditions mediated by both EGFR and erbB2 tyrosine kinases.

According to this aspect of the invention there is provided a compound of the formula I, or a pharmaceutically acceptable salt thereof, for use as a medicament.

Thus according to this aspect of the invention there is provided the use of a quinazoline derivative of the formula I, or a pharmaceutically-acceptable salt thereof, as defined hereinbefore in the manufacture of a medicament for use in the production of an anti-proliferative effect in a warm-blooded animal such as man.

According to a further feature of this aspect of the invention there is provided a method for producing an anti-proliferative effect in a warm-blooded animal, such as man, in need of such treatment which comprises administering to said animal an effective amount of a quinazoline derivative of the formula I, or a pharmaceutically acceptable salt thereof, as hereinbefore defined.

According to a further aspect of the invention there is provided a compound of the formula I, or a pharmaceutically acceptable salt thereof, for use in the production of an anti-proliferative effect in a warm-blooded animal such as man.

According to a further aspect of the invention there is provided the use of a quinazoline derivative of the Formula I, or a pharmaceutically-acceptable salt thereof, as defined hereinbefore in the manufacture of a medicament for use in the prevention or treatment of those tumours which are sensitive to inhibition of erbB receptor tyrosine kinases, such as EGFR and/or erbB2 and/or erbB4, that are involved in the signal transduction steps which lead to the proliferation of tumour cells.

A method for the prevention or treatment of those tumours which are sensitive to inhibition of one or more of the erbB family of receptor tyrosine kinases, such as EGFR and/or erbB2 and/or erbB4, that are involved in the signal transduction steps which lead to the proliferation and/or survival of tumour cells in a warm-blooded animal, such as man, in need of such treatment may comprise administering to said animal an effective amount of a quinazoline derivative of the Formula I, or a pharmaceutically-acceptable salt thereof, as defined hereinbefore.

According to a further aspect of the invention there is provided a compound of the formula I, or a pharmaceutically acceptable salt thereof, for use in the prevention or treatment of those tumours which are sensitive to inhibition of one or more of the erbB family of receptor tyrosine kinases, such as EGFR and/or erbB2 and/or erbB4, that are involved in the signal transduction steps which lead to the proliferation and/or survival of tumour cells.

According to a further aspect of the invention there is provided the use of a quinazoline derivative of the Formula I, or a pharmaceutically-acceptable salt thereof, as defined hereinbefore in the manufacture of a medicament for use in providing a EGFR and/or erbB2 and/or erbB4 kinase inhibitory effect.

A method for providing a EGFR and/or an erbB2 and/or an erbB4 kinase inhibitory effect in a warm-blooded animal, such as man, in need of such treatment may comprise administering to said animal an effective amount of a quinazoline derivative of the Formula I, or a pharmaceutically-acceptable salt thereof, as defined hereinbefore.

According to a further aspect of the invention there is provided a compound of the formula I, or a pharmaceutically acceptable salt thereof, for use in providing a EGFR and/or an erbB2 and/or an erbB4 kinase inhibitory effect.

According to a further aspect of the invention there is provided the use of a quinazoline derivative of the Formula I, or a pharmaceutically-acceptable salt thereof, as defined hereinbefore in the manufacture of a medicament for use in providing a selective EGFR kinase inhibitory effect.

A method for providing a selective EGFR kinase inhibitory effect in a warm-blooded animal, such as man, in need of such treatment, may comprise administering to said animal an effective amount of a quinazoline derivative of the Formula I, or a pharmaceutically-acceptable salt thereof, as defined hereinbefore.

According to a further aspect of the invention there is provided a compound of the formula I, or a pharmaceutically acceptable salt thereof, for use in providing a selective EGFR kinase inhibitory effect.

By "a selective EGFR kinase inhibitory effect" is meant that the quinazoline derivative of formula I is more potent against EGFR tyrosine kinase than it is against other kinases. In particular the quinazoline derivative of formula I is more potent against EGFR tyrosine kinase than it is against other erbB receptor tyrosine kinases such as erbB2. For example in a cellular assay (such as in the H16N-2 assay described herein) the quinazoline derivative of formula I is at least 5 times, preferably at least 10 times more potent against EGFR tyrosine kinase driven proliferation than it is against erbB2 receptor tyrosine kinase driven proliferation, as determined from the relative IC₅₀ values

According to a further aspect of the present invention there is provided the use of a quinazoline derivative of the Formula I, or a pharmaceutically-acceptable salt thereof, as defined hereinbefore in the manufacture of a medicament for use in the treatment of a cancer selected from leukaemia, multiple myeloma, lymphoma, bile duct, bone, bladder, brain/CNS, breast, colorectal, endometrial, gastric, head and neck, hepatic, lung, neuronal, oesophageal, ovarian, pancreatic, prostate, renal, skin, testicular, thyroid, uterine and vulval cancer.

A method for treating a cancer selected from selected from leukaemia, multiple myeloma, lymphoma, bile duct, bone, bladder, brain/CNS, breast, colorectal, endometrial, gastric, head and neck, hepatic, lung, neuronal, oesophageal, ovarian, pancreatic, prostate, renal, skin, testicular, thyroid, uterine and vulval cancer in a warm-blooded animal, such as man, in need of such treatment, may comprise administering to said animal an effective amount of a quinazoline derivative of the Formula I, or a pharmaceutically-acceptable salt thereof, as defined hereinbefore.

According to a further aspect of the invention there is provided a compound of the formula I, or a pharmaceutically acceptable salt thereof, for use in the treatment of a cancer selected from leukaemia, multiple myeloma, lymphoma, bile duct, bone, bladder, brain/CNS, breast, colorectal, endometrial, gastric, head and neck, hepatic, lung, neuronal, oesophageal, ovarian, pancreatic, prostate, renal, skin, testicular, thyroid, uterine and vulval cancer.

The anti-proliferative treatment defined hereinbefore may be applied as a sole therapy or may involve, in addition to the quinazoline derivative of the invention, conventional surgery or radiotherapy or chemotherapy. Such chemotherapy may include one or more of the following categories of anti-tumour agents :-
(i) antiproliferative/antineoplastic drugs and combinations thereof, as used in medical oncology, such as alkylating agents (for example cis-platin, carboplatin, cyclophosphamide, nitrogen mustard, melphalan, chlorambucil, busulphan and nitrosoureas); antimetabolites (for example antifolates such as fluoropyrimidines like 5-fluorouracil and tegafur, raltitrexed, methotrexate, cytosine arabinoside and hydroxyurea; antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, amsacrine, topotecan and camptothecin);
(ii) cytostatic agents such as antioestrogens (for example tamoxifen, toremifene, raloxifene, droloxifene and iodoxyfene), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5 α-reductase such as finasteride;
(iii) agents which inhibit cancer cell invasion (for example metalloproteinase inhibitors like marimastat and inhibitors of urokinase plasminogen activator receptor function);
(iv) other inhibitors of growth factor function, for example such inhibitors include growth factor antibodies, growth factor receptor antibodies (for example the anti-erbb2 antibody trastuzumab [Herceptin™] and the anti-erbb1 antibody cetuximab [C225]), farnesyl transferase inhibitors, tyrosine kinase inhibitors and serine/threonine kinase inhibitors, for example inhibitors of the epidermal growth factor family (for example EGFR family tyrosine kinase inhibitors such as N-(3-chloro-4-fluorophenyl)-7-methoxy-6-(3-morpholinopropoxy)quinazolin-4-amine (gefitinib, AZD1839), N-(3-ethynylphenyl)-6,7-bis(2-methoxyethoxy)quinazolin-4-amine (erlotinib, OSI-774) and 6-acrylamido-N-(3-chloro-4-fluorophenyl)-7-(3-morpholinopropoxy)quinazolin-4-amine (CI 1033)), for example inhibitors of the platelet-derived growth factor family and for example inhibitors of the hepatocyte growth factor family;
(v) antiangiogenic agents such as those which inhibit the effects of vascular endothelial growth factor, (for example the anti-vascular endothelial cell growth factor antibody bevacizumab [Avastin™], compounds such as those disclosed in International Patent Applications WO 97/22596, WO 97/30035, WO 97/32856 and WO 98/13354) and compounds that work by other mechanisms (for example linomide, inhibitors of integrin αvβ3 function and angiostatin);
(vi) vascular damaging agents such as Combretastatin A4 and compounds disclosed in International Patent Applications WO 99/02166, WO00/40529, WO 00/41669, WO01/92224, WO02/04434 and WO02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(ix) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

Such conjoint treatment may be achieved by way of the simultaneous, sequential or separate dosing of the individual components of the treatment. Such combination products employ the compounds of this invention within the dosage range described hereinbefore and the other pharmaceutically-active agent within its approved dosage range.

According to this aspect of the invention there is provided a pharmaceutical product composing a quinazoline derivative of the formula I as defined hereinbefore and an additional anti-tumour agent as defined hereinbefore for the conjoint treatment of cancer.

Although the compounds of the Formula I are primarily of value as therapeutic agents for use in warm-blooded animals (including man), they are also useful whenever it is required to inhibit the effects of the erbB receptor tyrosine protein kinases. Thus, they are useful as pharmacological standards for use in the development of new biological tests for the evaluation of the effects of inhibitors of cell cycle activity in laboratory animals such as cats, dogs, rabbits, monkeys, rats and mice, and in the search for new pharmacological agents.

The invention will now be illustrated by the following examples in which, unless stated otherwise:
(i) temperatures are given in degrees Celsius (°C); operations were carried out at room or ambient temperature, that is, at a temperature in the range of 18-25°C;
(ii) organic solutions were dried over anhydrous magnesium sulphate; evaporation of solvent was carried out using a rotary evaporator under reduced pressure (600-4000 Pascals; 4.5-30mmHg) with a bath temperature of up to 60°C;
(iii) chromatography means flash chromatography on silica gel; thin layer chromatography (TLC) was carried out on silica gel plates;
(iv) in general, the course of reactions was followed by TLC and / or analytical LC-MS, and reaction times are given for illustration only;
(v) final products had satisfactory proton nuclear magnetic resonance (NMR) spectra and/or mass spectral data;
(vi) yields are given for illustration only and are not necessarily those which can be obtained by diligent process development; preparations were repeated if more material was required;
(vii) when given, NMR data is in the form of delta values for major diagnostic protons, given in parts per million (ppm) relative to tetramethylsilane (TMS) as an internal standard, determined at 300 MHz using perdeuterio dimethyl sulphoxide (DMSO-d₆) as solvent unless otherwise indicated; the following abbreviations have been used: s, singlet; d, doublet; t, triplet; q, quartet; m, multiplet; b, broad;
(viii) chemical symbols have their usual meanings; SI units and symbols are used;
(ix) solvent ratios are given in volume:volume (v/v) terms; and
(x) mass spectra were run with an electron energy of 70 electron volts in the chemical ionization (CI) mode using a direct exposure probe; where indicated ionization was effected by electron impact (EI), fast atom bombardment (FAB) or electrospray (ESP); values for m/z are given; generally, only ions which indicate the parent mass are reported; and unless otherwise stated, the mass ion quoted is (MH)⁺ which refers to the protonated mass ion; reference to M⁺ is to the mass ion generated by loss of an electron; and reference to M-H⁺ is to the mass ion generated by loss of a proton;
(xi) unless stated otherwise compounds containing an asymmetrically substituted carbon and/or sulphur atom have not been resolved;
(xii) where a synthesis is described as being analogous to that described in a previous example the amounts used are the millimolar ratio equivalents to those used in the previous example;
(xvi) the following abbreviations have been used:

| | |
|---|---|
| THF | tetrahydrofuran; |
| DMF | *N,N*-dimethylformamide; |
| DMA | *N,N*-dimethylacetamide; |
| NMP | 1-methyl-2-pyrrolidinone; |
| DCM | dichloromethane; |
| DMSO | dimethylsulphoxide; |
| HATU | O-(7-Azabenzotriazol-1-yl)-N,N,N',N-Tetramethyluronium Hexafluoro-Phosphate; |
| *m*-CPBA | *Meta*-Chloroperbenzoic acid; |
| IPA | isopropanol; and |
| ether | diethyl ether. |

(xvii) where a synthesis is described as leading to an acid addition salt (e.g. HCl salt), the stoichiometry of the salt was not determined. Unless otherwise stated, all NMR data is reported on free-base material, with isolated salts converted to the free-base form prior to characterisation by treating a solution of the salt in aqueous methanol with a base such as ammonium hydroxide or sodium bicarbonate thereby precipitating the free base, or by chromatography on silica using an eluant containing a base such as ammonia. Alternatively the free base may be obtained by an extraction method wherein the compound is partioned between an organic solvent and a basic aqueous medium. The free base is then isolated from the organic medium by, for example evaporation of the organic solvent.

The illustrative examples provided below are included for illustrative purposes only and do not form part of the present invention.

### Illustrative Example 1

### 4-(3-Bromoanilino)-7-(3-(R)-dimethylaminopyrrolidin-1-yl)-5-(1-methylpiperidin-4-yloxy)quinazoline hydrochloride

A mixture of 4-chloro-7-(3-(*R*)-dimethylaminopyrrolidin-1-yl)-5-(1-methylpiperidin-4-yloxy)quinazoline (reference example 16) (0.2 g), 3-bromoaniline (0.066 ml) and HCl in dioxane (4M, 0.5 ml) in IPA (3 ml) was heated at reflux for 6 hours. The reaction was cooled, and the resulting precipitate filtered, washed with IPA and ether, and dried *in vacuo* to yield the title compound as a yellow solid (0.115 g, 43%); Mass spectrum M⁺ 527, 525.

The procedure described above was repeated using the appropriate 4-chloroquinazoline and aniline. Thus were obtained the compounds described below:

### Illustrative Example 1.1

### 4-(3-Chloroanilino)-7-(3-(R)-dimethylaminopyrrolidin-1-yl-5-(1-methylpiperidin-4-yloxy)quinazoline hydrochloride

Obtained by reacting 4-chloro-7-(3-(*R*)-dimethylaminopyrrolidin-1-yl)-5-(1-methylpiperidin-4-yloxy)quinazoline (reference example 16) with 3-chloroaniline in 53% yield; Mass spectrum M⁺ 481.

### Illustrative Example 1.2

### 4-(3-Methylanilino)-5-(1-methylpiperidine-4-yloxy)quinazoline hydrochloride

Obtained by reacting 4-chloro-5-(1-methylpiperidin-4-yloxy)quinazoline (**reference example 16.1**) with *meta*-toluidine in 25% yield; Mass spectrum MH⁺ 349.

### Illustrative Example 1.3

### 4-(3-Chloroanilino)-5-(1-methylpiperidine-4-yloxy)quinazoline hydrochloride

Obtained by reacting 4-chloro-5-(1-methylpiperidin-4-yloxy)quinazoline (**reference example 16.1**) with 3-chloroaniline in 29% yield; Mass spectrum M⁺ 369.

### Illustrative Example 1.4

### 4-(3-Bromoanilino)-5-(1-methylpiperidin-4-yloxy)quinazoline hydrochloride

Obtained by reacting 4-chloro-5-(1-methylpiperidin-4-yloxy)quinazoline (**reference example 16.1**) with 3-bromoaniline in 36% yield; Mass spectrum M⁺ 415, 413.

### Illustrative Example 1.5

### 4-(3-Ethynylanilino)-5-(1-methylpiperidin-4-yloxy)quinazoline hydrochloride

Obtained by reacting 4-chloro-5-(1-methylpiperidin-4-yloxy)quinazoline (**reference example 16.1**) with 3-ethynylaniline in 27% yield; Mass spectrum M-H⁺ 357.

### Illustrative Example 1.6

### 4-(3-Fluoroanilino)-5-(1-methylpiperidin-4-yloxy)quinazoline hydrochloride

Obtained by reacting 4-chloro-5-(1-methylpiperidin-4-yloxy)quinazoline (**reference example 16.1**) with 3-fluoroaniline in 26% yield; Mass spectrum MH⁺ 353.

### Example 1.7

### 4-(3-Indol-5-ylamino)-5-(1-methylpiperidin-4-yloxy)quinazoline hydrochloride

Obtained by reacting 4-chloro-5-(1-methylpiperidin-4-yloxy)quinazoline (**reference example 16.1**) with 5-aminoindole in 30% yield; Mass spectrum MH⁺ 374.

### Example 1.8

### 4-(3-Indazol-5-ylamino)-5-(1-methylpiperidin-4-yloxy)quinazoline hydrochloride

Obtained by reacting 4-chloro-5-(1-methylpiperidin-4-yloxy)quinazoline (**reference example 16.1**) with 5-aminoindazole in 30% yield; Mass spectrum MH⁺ 375.

### Illustrative Example 1.9

### 4-(3-Chloro-4-(azepan-1-ylcarbonyl)anilino)-7-methoxy-5-(1-methylpiperidin-4-yloxy)quinazoline hydrochloride

Obtained by reacting 4-chloro-7-methoxy-5-(1-methylpiperidin-4-yloxy)quinazoline (**reference example 16.2**) with 4-(azepan-1-ylcarbonyl)-3-chloroaniline (**reference example** 29) in 45% yield; NMR Spectrum (CDCl₃) 1.50 (bs, 6H), 1.84 (bs, 2H), 1.99 (m, 2H), 2.30 (m, 4H), 2.34 (s, 3H), 2.81 (m, 2H), 3.31 (m. 2H), 3.72 (m, 2H), 3.92 (s, 3H), 4.57 (m, 1H), 6.52 (d, 1H), 6.85 (d, 1H), 7.24 (d, 1H), 7.56 (dd, 1H), 8.09 (d, 1H), 8.61 (s, 1H), 9.99 (s, 1H); Mass Spectrum MH⁺ 524.

### Example 1.10

### 4-(3-Bromoindol-5-ylamino)-7-methoxy-5-(1-methylpiperidin-4-yloxy)quinazoline hydrochloride

Obtained by reacting 4-chloro-7-methoxy-5-(1-methylpiperidin-4-yloxy)quinazoline (**reference example 16.2**) and 5-amino-3-bromoindole (reference example 25.2) in 19% yield; NMR Spectrum (CDCl₃) 2.06 (m, 2H), 2.22 (m, 2H), 2.33 (s, 3H), 2.40 (m, 2H), 2.75 (m, 2H), 3.92 (s, 3H), 4.65 (m, 1H), 6.51 (d, 1H), 6.83 (d, 1H), 7.22 (d, 1H), 7.35 (d, 1H), 7.47 (dd, 1H), 7.84 (s, 1H), 8.47 (bs, 1H), 8.53 (s, 1H), 9.84 (s, 1H); Mass Spectrum MH⁺ 482, 484.

### Example 1.11

### 4-(3-Chloroindol-5-ylamino)-5-(1-methylpiperidin-4-yloxy)quinazoline hydrochloride hydrochloride

Obtained by reacting 4-chloro-5-(1-methylpiperidin-4-yloxy)quinazoline (**reference example 16.1**) and 5-amino-3-chloroindole (**reference example 26.2**) in 11% yield; Mass Spectrum M⁺ 408.

### Example 1.12

### 4-(3-Cyanoindol-5-ylamino)-5-(1-methylpiperidin-4-yloxy)quinazoline hydrochloride

Obtained by reacting 4-chloro-5-(1-methylpiperidin-4-yloxy)quinazoline (**reference example 16.1**) with 5-aminoindole-3-carbonitrile (**reference example 27.2**) in 28% yield; NMR spectrum (DMSO-d6) 2.3 (m, 4H), 2.7 (m, 3H), 3.2 - 3.6 (m, 4H) 5.1. (m, 1H), 7.5 (m; 3H), 7.7 (m, 1H), 8.0 (m, 1H), 8.2 (s, 1H), 8.3 (s, 1H), 8.9 (s, 1H); Mass spectrum MH⁺ 399.

### Illustrative Example 2

### 4-(3-Bromoanilino)-7-methoxy-5-(1-methylpiperidin-4-yloxy)quinazoline

A solution of hydrochloric acid in dioxane (4M, 0.5 ml) was added to a mixture of 4-chloro-7-methoxy-5-(1-methylpiperidin-4-yloxy)quinazoline (**reference example 16.2**) (308 mg) and 3-bromoaniline (172 mg) in dioxane (20 ml). The resulting suspension was heated at reflux for 4 hours, then allowed to cool to room temperature. The reaction mixture was partitioned between saturated aqueous sodium hydrogen carbonate solution and DCM. Combined organic extracts were dried (sodium sulphate) and concentrated to give an orange oil, which was purified by chromatography using 0-5% methanol in DCM as eluent to give the title compound as a white solid (190 mg, 43%); NMR Spectrum (CDCl₃) 2.10 (m,2H), 2.38 (m, 4H), 2.42 (s, 3H), 2.90 (m, 2H), 4.00 (s, 3H), 4.66 (m, 1H), 6.60 (d, 1H), 6.93 (d, 1H), 7.31 (m, 2H), 7.65 (m, 1H), 8.22 (m, 1H), 8.68 (s, 1H), 9.98 (s, 1H); Mass Spectrum MH⁺ 443, 445.

The procedure described above was repeated using the appropriate 4-chloroquinazoline and aniline. Thus were obtained the compounds described below:

### Example 2.1

### 4-(3-Chloroindol-5-ylamino)-7-methoxy-5-(1-methylpiperidin-4-yloxy)quinazoline

Obtained by reacting 4-chloro-7-methoxy-5-(1-methylpiperidin-4-yloxy)quinazoline (**reference example 16.2**) and 5-amino-3-chloroindole (**reference example 26.2**) in 51% yield; NMR Spectrum (DMSO-d6) 1.94 (m, 2H), 2.14 (m, 2H), 2.17 (s, 3H), 2.34 (m, 2 H), 2.64 (m, 2H), 3.92 (s, 3H), 4.86 (m, 1H), 6.81 (s, 2H), 7.34 (dd, 1H), 7.47 (d, 1H), 7.55 (d, 1H), 9.97, (s, 1H). 11.37 (s, 1H); Mass Spectrum MH⁺ 438, 440.

### Illustrative Example 2.2

### 4-(3-Ethynylanilino)-7-methoxy-5-(1-methylpiperidin-4-yloxy)quinazoline

Obtained by reacting 4-chloro-7-methoxy-5-(1-methylpiperidin-4-yloxy)quinazoline (**reference example 16.2**) and 3-ethynylaniline in 41% yield; NMR Spectrum (CDCl₃) 2.10 (m, 2H), 2.28 (m, 4H), 2.35 (s, 3H). 2.83 (m, 2H), 3.10 (s, 1H), 3.93 (s, 3H), 4.59 (m, 1H), 6.53 (d, 1H), 6.85 (d, 1H), 7.24 - 7.36 (m, 2H), 7.76 (d, 1H), 7.94 (d, 1H), 8.59 (s, 1H), 9.90 (s, 1H); Mass Spectrum MH⁺ 389.

### Example 2.3

### 4-(Indazol-5-ylamino)-7-methoxy-5-(1-methylpiperidin-4-yloxy)quinazoline

Obtained by reacting 4-chloro-7-methoxy-5-(1-methylpiperidin-4-yloxy)quinazoline (**reference example 16.2**) and 5-aminoindazole in 28% yield; NMR Spectrum (DMSO-d6). 1.93 (m, 2H), 2.19 (bs, 4H), 2.32 (t, 2H), 2.63 (m, 2H), 3.91 (s, 3H), 4.84 (m, 1H), 6.82 (s, 2H), 7.52 (d, 1H), 7.59 (d, 1H). 8.10 (s, 1H), 8.34 (s, 1H), 8.44 (s, 1H), 13.05 (s, 1H); Mass Spectrum MH⁺ 405.

### Illustrative Example 2.4

### 4-(3-Chloro-4-fluoroanilino)-7-methoxy-5-(1-methylpiperidin-4-yloxy)quinazoline

Obtained by reacting 4-chloro-7-methoxy-5-(1-methylpiperidin-4-yloxy)quinazoline (**reference example 16.2**) and 3-chloro-4-fluoroaniline in 31 % yield; NMR Spectrum (CDCl₃) 2.00 (m, 2H), 2.31 (m, 7H), 2.80 (m, 2H), 3.92 (s, 3H), 4.58 (m, 1H), 6.51 (d,1H), 6.84 (d, 1H), 7.12 (t, 1H), 7.46 (m, 1H), 8.00 (dd, 1H). 8.56 (s, 1H), 9.83 (s, 1H); Mass Spectrum MH⁺ 417, 419.

### Illustrative Example 2.5

### 4-(3-Chloroanilino)-7-methoxy-5-(1-methylpiperidin-4-yloxy)quinazoline

Obtained by reacting 4-chloro-7-methoxy-5-(1-methylpiperidin-4-yloxy)quinazoline (**reference example 16.2**) and 3-chloroaniline in 36% yield; NMR Spectrum (CDCl₃) 2.01 (m, 2H), 2.30 (m, 7H), 2.81 (m, 2H), 3.92 (s, 3H), 4.57 (m, 1H), 6.52 (d, 1H), 6.85 (d, 1H), 7.08 (m, 1H), 7.28 (t, 1H), 7.51 (dm, 1H), 7.99 (t, 1H), 8.59 (s, 1H), 9.92 (s, 1H); Mass Spectrum MH⁺ 399, 401.

### Illustrative Example 2.6

### 7-Methoxy-4-(3-methylanilino)-5-(1-methylpiperidin-4-yloxy)quinazoline

Obtained by reacting 4-chloro-7-methoxy-5-(I-methylpiperidin-4-yloxy)quinazoline (**reference example 16.2**) and *meta*-toluidine in 76% yield; NMR Spectrum (CDCl₃) 2.03 (m, 2H), 2.34 (m, 7H), 2.40 (s, 3H), 2.82 (m, 2H), 3.92 (s, 3H), 4.58 (m, 1H), 6.51 (d, 1H), 6.84 (d, 1H), 7.27 (m, 1H), 7.53 (d, 1H). 7.56 (s, 1H). 8.56 (s, 1H), 9.83 (s, 1H); Mass Spectrum MH⁺ 379.

### Illustrative Example 2.7

### 4-(3-Fluoroanilino)-7-methoxy-5-(1-methylpiperidin-4-yloxy)quinazoline

Obtained by reacting 4-chloro-7-methoxy-5-(1-methylpiperidin-4-yloxy)quinazoline (**reference example 16.2**) and 3-fluoroaniline in 41% yield; NMR Spectrum (CDCl₃) 2.01 (m, 2H), 2.28 (m, 4H), 2.33 (s, 3H), 2.83 (m, 2H), 3.92 (s, 3H), 4.57 (m, 1H), 6.52 (d, 1H), 6.80 (m, 1H), 6.85 (d, 1H), 7.29 (m, 2H), 7.88 (m, 1H), 8.59 (s, 1H), 9.98 (s, 1H); Mass Spectrum MH⁺ 383.

### Example 2.8

### 4-(Indol-5-ylamino)-7-methoxy-5-(1-methylpiperidin-4-yloxy)quinazoline

Obtained by reacting 4-chloro-7-methoxy-5-(1-methylpiperidin-4-yloxy)quinazoline (**reference example 16.2**) and 5-aminoindole in 17% yield; NMR Spectrum (CDCl₃) 2.06 (m, 2H), 2.22 - 2.38 (m, 7H), 2.77 (m, 2H), 3.91 (s, 3H), 4.61 (m, 1H), 6.50 (d, 1H), 6.36 (m, 1H), 7.22 (t, 1H), 7.38 (s, 2H), 7.96 (s, 1H), 8.25 (bs, 1H), 8.51 (s, 1H), 9.82 (s, 1H); Mass Spectrum MH⁺ 404.

### Illustrative Example 2.9

### 4-(3-Chloro-4-hydroxyanilino)-7-methoxy-5-(1-methylpiperidin-4-yloxy)quinazoline

Obtained by reacting 4-chloro-7-methoxy-5-(1-methylpiperidin4-yloxy)quinazoline (**reference example 16.2**) and 4-amino-2-chlorophenol in 72% yield; NMR spectrum (DMSO-d6) 1.9 (m, 2H), 2.1 (m, 2H), 2.2 (s. 3H), 23 (m, 2H), 2.6 (m, 2H), 3.9 (s, 3H), 4.8 (m, 1H) 6.8 (s, 1H), 7.0 (d, 3H), 7.3 (dd, 1H), 8.0 (d, 1H), 8.4 (s, 1H). 9.8 (s, 1H), 10.0 (bs, 1H); Mass spectrum MH⁺ 415.

### Illustrative Example 2.10

### 4-(3-Methyl-4-hydroxyanilino)-7-methoxy-5-(1-methylpiperidin-4-yloxy)quinazoline

Obtained by reacting 4-chloro-7-methoxy-5-(1-methylpiperidin-4-yloxy)quinazoline (**reference example 16.2**) and 4-aminocresol in 84% yield; NMR spectrum (DMSO-d6) 1.9 (m, 2H), 2.1 (m, 2H), 2.2 (s, 3H), 2.2 (s, 3H), 2.3 (m, 2H), 2.6 (m, 2H), 3.9 (s, 3H), 4.8 (m, 1H) 6.8 (s, 2H), 6.8 (d, 1H), 7.4 (dd, 1H), 7.4 (s, 1H), 8.4 (s, 1H), 9.2 (s, 1H), 9.7 (s, 1H), 10.0 (bs, 1H); Mass spectrum MH⁺ 395.

### Illustrative Example 3

### 4-(3-Methylbenzisothiazol-5-ylamino)-5-(1-methylpiperidin-4-yloxy)quinazoline hydrochloride

A mixture of 4-chloro-5-(1-methylpiperidin-4-yloxy)quinazoline (**reference example 16.1**) (0.42 g) and 5-amino-3-methylbenzisothiazole (**reference example 27**) (0.25 g) in IPA (10 ml) were heated at reflux for 16 hours, then allowed to cool to room temperature. A solid precipitated from the mixture and this was filtered, washed with IPA and diethyl ether, and dried *in vacuo* to give the title compound as a yellow solid (0.4 g, 60%); Mass Spectrum MH⁺ 406.

The procedure described above was repeated using the appropriate aniline and chloroquinazoline. Thus were obtained the compounds described below:

### Illustrative Example 3.1

### 4-(3-Ethynyl 4-(2-fluorobenzyloxy)anilino)-7-methoxy-5-(1-methylpiperidin-4-yloxy)quinazoline hydrochloride

Obtained by reacting 4-chloro-7-methoxy-5-(1-methylpiperidin-4-yloxy)quinazoline (**reference example 16.2**) with 3-ethynyl-4-(2-fluorobenzyloxy)aniline (**reference example 42**) in 67% yield; Mass Spectrum MH⁺ 514.

### Illustrative Example 3.2

### 4-(3-Ethynyl-4-(3-fluorobenzyloxy)anilino)-7-methoxy-5-(1-methylpiperidin-4-yloxy)quinazoline hydrochloride

Obtained by reacting 4-chloro-7-methoxy-5-(1-methylpiperidin-4-yloxy)quinazoline (**reference example 16.2**) with 3-ethynyl-4-(3-fluorobenzyloxy)aniline (reference example 42.1) in 60% yield; Mass Spectrum MH⁺ 514.

### Illustrative Example 3.3

### 4-(3-Fluoro-4-(1-methyl-1H-imidazol-2-ylthio)anilino)-7-methoxy-5-(1-methylpiperidin-4-yloxy)quinazoline hydrochloride

Obtained by reacting 4-chloro-7-methoxy-5-(1-methylpiperidin-4-yloxy)quinazoline (**reference example 16.2**) with 3-fluoro-4-(1-methyl-1*H*-imidazol-2-ylthio)aniline (**reference example 26.3**) in 54% yield; NMR spectrum (DMSO-d6, 373K) 1.9 - 2.0 (m, 2H), 2.1- 2.2 (m, 2H), 2.25 (s, 3H), 2.25 - 2.35 (m, 2H), 2.6 - 2.7 (m, 2H), 3.7 (s, 3H), 3.9 (s. 3H), 4.7 - 4.8 (m, 1H), 6.8 (d, 1H), 6.85 (d, 1H), 7.0 (s, 1H), 7.1- 7.2 (t, 1H), 7.3 (s, 1H), 7.3 - 7.4 (dd, 1H), 8.0 - 8.1 (d, 1H), 8.5 (s, 1H), 10.0 (bs, 1H); Mass Spectrum MH⁺ 495.

### Example 4

### 4-(3-Bromoindazol-5-ylamino)-5-(1-methylpiperidin-4-yloxy)quinazoline

Di-iso-propylethylamine (94 µl) was added to a mixture of 4-chloro-5-(1-methylpiperidin-4-yloxy)quinazoline (**reference example 16.1**) (75 mg) and 5-amino-3-bromoindazole (**reference example 25**) (115 mg) in IPA (12 ml). The resulting suspension was heated at reflux for 2 hours, then allowed to cool to room temperature. A solid precipitated from the mixture and this was filtered, washed with IPA and diethyl ether, and dried *in vacuo* to afford the title compound as a white solid (114 mg, 93%); NMR Spectrum (DMSO-d6) 1.97 (m, 2H), 2.20 (m, 5H), 2.38 (m, 2H), 2.70 (m, 2H), 4.82 (m, 1H), 7.20 (d, 1H), 7.37 (d, 1H), 7.57-7.74 (m, 3H), 8.15 (s, 1H), 8.51 (s, 1H), 10.10 (s, 1H), 13.10 (bs, 1H); Mass Spectrum MH⁺ 453, 455.

The procedure described above was repeated using the appropriate 4-chloroquinazoline and aniline. Thus were obtained the compounds described below:

### Example 4.1

### 4-(3-Chloroindazol-5-ylamino)-5-(1-methylpiperidin-4-yloxy)quinazoline

Obtained by reacting 4-chloro-5-(1-methylpiperidin-4-yloxy)quinazoline (**reference example 16.1**) and 5-amino-3-chloroindazole (reference example 25.1) in 85% yield; NMR Spectrum (DMSO-d6) 1.96 (m, 2H), 2.18 (m, 2H), 2.25 (s, 3H), 2.41 (m, 2H), 2.74 (m, 2H), 4.82 (m, 1H), 7.23 (d, 1H), 7.33 (d, 1H), 7.51- 7.74 (m, 3H), 8.39 (s, 1H), 8.53(s, 1H), 10.21 (s, 1H), 13.29 (bs, 1H); Mass spectrum MH⁺ 409.

### Example 4.2

### 4-(3-Chloro-1-(2-pyridylmethyl)indol-5-ylamino)-7-methoxy-5-(1-methylpiperidin-4-yloxy)quinazoline

Obtained by reacting 4-chloro-7-methoxy-5-(1-methylpiperidin-4-yloxy)quinazoline (**reference example 16.2**) and 5-amino-3-chloro-1-(2-pyridylmethyl)indole (**reference example 26**) in 10% yield; NMR Spectrum (CDCl₃) 2.03 (m, 2H), 2.22 (m, 2H), 2.32 (s, 3H), 2.37 (m, 2H), 2.75 (m, 2H), 3.91 (s, 3H), 4.61 (m, 1H), 5.39 (s, 2H), 6.50 (d, 1H), 6.82 (m, 2H), 7.18 (m, 2H), 7.27 (d, 1H), 7.44 (dd, 1H), 7.56 (dt, 1H), 7.95 (d, 1H), 8.52 (s, 1H), 8.59 (d, 1H), 9.83 (s, 1H); Mass Spectrum MH⁺ 529.

### Example 4.3

### 4-(3-Chloro-1-(2-pyridylmethyl)indazol-5-ylamino)-7-methoxy-5-(1-methylpiperidin-4-yloxy)quinazoline

Obtained by reacting 4-chloro-7-methoxy-5-(1-methylpiperidin-4-yloxy)quinazoline (**reference example 16.2**) and 5-amino-3-chloro-1-(2-pyridylmethyl)indazole (**reference example 26.1**) in 9% yield; NMR Spectrum (CDCl₃) 2.03 (m, 2H), 2.25 (m, 2H), 2.32 (s, 3H), 2.37 (m, 2H), 2.77 (m, 2H), 3.92 (s, 3H), 4.60 (m, 1H), 5.66 (s, 2H), 6.51 (d, 1H), 6.84 (d, 1H), 6.98 (d, 1H), 7.19 (dd, 2H), 7.39 (d, 1H), 7.58 (m, 2H), 8.13 (d, 1H), 8.55 (s, 1H), 8.58 (d, 1H),9.88 (s, 1H); Mass Spectrum MH⁺ 530.

### Illustrative Example 4.4

### 7-Methoxy-4-(3-methyl-4-(2-pyridylmethoxy)anilino)-5-(1-methylpiperidin-4-yloxy)quinazoline

Obtained by reacting 4-chloro-7-methoxy-5-(1-methylpiperidin-4-yloxy)quinazoline (**reference example 16.2**) and 3-methyl-4-(2-pyridylmethoxy)aniline (obtained using the method of Example 13 of WO 96/15118) in 18% yield; NMR Spectrum (CDCl₃) 2.02(m, 2H), 2.25 (m, 2H), 2.32 (s, 3H), 2.38 (s, 3H), 2.76 (m, 2H), 3.90 (s, 3H), 4.58 (m, 1H), 5.23 (s, 2H), 6.48 (d, 1H), 6.81 (d, 1H), 7.22 (dd, 1H), 7.44 (m, 2H), 7.56 (d, 1H), 7.23 (dt, 1H), 8.50 (s, 1H), 8.59 (d, 1H), 9.65 (s, 1H): Mass Spectrum MH⁺ 486.

### Example 4.5

### 4-(3-Methylindol-5-ylamino)-5-(1-methylpiperidin-4-yloxy)quinazoline

Obtained by reacting 4-chloro-5-(1-methylpiperidin-4-yloxy)quinazoline (**reference example 16.1**) and 3-methylindol-5-ylamine (**reference example 27.1**) in 10% yield; NMR spectrum (DMSO-d6); 2.2 - 2.4 (m, 5H), 2.8 (s, 3H), 3.2 - 3.7 (m, 6H), 5.1 (m, 1H), 7.2 (s, 1H), 7.3-7.6 (m, 4H), 7.8 - 8.0 (m, 2H), 8.8 (d, 1H): Mass spectrum MH⁺388.

### Illustrative Example 4.6

### 4-(3-Chloro-4-hydroxyanilino)-5-(1-methylpiperidin-4-yloxy)quinazoline

Obtained by reacting 4-chloro-5-(1-methylpiperidin-4-yloxy)quinazoline (**reference example 16.1**) and 3-chloro-4-hydroxyaniline in 60% yield; NMR spectrum (DMSO-d6) 1.9 (m, 2H), 2.1 (m, 5H), 2.3 (m, 2H), 2.6 (m, 2H), 4.8 (m, 1H), 7.0 (d, 1H), 7.2 (d, 1H), 7.3 (m, 2H), 7.7 (m, 1H), 8.0 (d, 1H), 8.4 (s, 1H), 10.0 (s, 1H); Mass spectrum NM⁺ 385.

### Illustrative Example 5

### 5-(1-Methylpiperidin-4-yloxy)-4-((1R)-1-Phenylethylamino)quinazoline

4-Chloro-5-(1-methylpiperidin-4-yloxy)quinazoline (**reference example 16.1**) (0.3 g), (*R*)-α-methylbenzylamine (0.28 ml) and di-*iso*-propylethylamine (0.94 ml) were heated at reflux in dioxane (20 ml) for 3 hours. The solution was concentrated *in vacuo* and the residue triturated with ether to give the title compound as a white solid (0.29 g, 74%); Mass spectrum MH⁺ 363.

The procedure described above was repeated using the appropriate 4-chloroquinazoline and amine. Thus was obtained the compound described below:

### Illustrative Example 5.1

### 7-Methoxy-5-(1-methylpiperidin-4-yloxy)-4-((1R)-1-Phenylethylamino)quinazoline

Obtained by reacting 4-chloro-7-methoxy-5-(1-methylpiperidin-4-yloxy)quinazoline (reference example 16.2) and (*R*)-α-methylbenzylamine in 47% yield; Mass Spectrum MH⁺ 393.

### Illustrative Example 6

### 4-(3-Chloro-4-fluroanilino)-7-(3-(R)-dimethylaminopyrrolidin-1-yl)-5-(1-methylpiperidin-4-yloxy)quinazoline

Di-iso-propylethylamine (0.18 ml) was added to 7-(3-(*R*)-dimethylaminopyrrolidin-1-yl)-5-(1-methylpiperidin-4-yloxy)-3,4-dihydroquinazolin-4-one (**reference example 14**) (50 mg) dissolved in anhydrous 1, 2-dichloroethane (5 ml) and the resulting solution cooled to 0°C. POCl₃ (40 µl) was added dropwise and the reaction heated at 80°C for 3 hours. The reaction mixture was concentrated *in vacuo* to give an orange oil which was used without further purification. 3-Chloro-4-fluoroaniline (20 mg) was added to this oil dissolved in IPA (300 µl), followed by di-iso-propylethylamine (11 µl). The resulting mixture was heated at 80°C for 12 hours to give a yellow precipitate. The reaction mixture was cooled to room temperature, the solid filtered, washed with IPA, then diethyl ether and dried *in vacuo* to afford the title compound as a yellow solid (25 mg, 37%); NMR spectrum (DMSO-d6, 373K) 2.3 - 2.6 (m, 3H), 2.8 (s, 3H), 2.9 (s, 6H), 3.0 - 3.6 (m, 7H), 3.8 (m, 1H), 3.9 (m, 2H), 4.1 (m, 1H), 5.3 (m, 1H), 6.5 (s, 1H), 6.7 (s, 1H), 7.5 (m, 1H), 7.7 (m, 1H), 8.1 (m, 1H), 8.6 (s, 1H), 10.1 (m, 1H); Mass spectrum MH⁺ 499.

The procedure described above was repeated using the appropriate 3,4-dihydroquinazolin-4-one and aniline. Thus were obtained the compounds described below:

### Illustrative Example 6.1

### 4-(3-Chloro-4-fluoroanilino)-7-methoxy-5-(tetrahydrofuran-3-yloxy)quinazoline

Obtained by reacting 7-methoxy-5-(tetrahydrofuran-3-yloxy)-3,4-dihydroquinazolin-4-one (**reference example 14.1**) and 3-chloro-4-fluoroaniline in 44% yield; NMR Spectrum (DMSO-d6) 2.20 (m, 1H), 2.35 (m, 1H), 3.84 (m, 3H), 3.95 (s, 3H), 4.19 (d, 1H), 5.56 (m, 1H), 7.01 (s, 2H), 7.53 (t, 1H), 7.63 (m, 1H), 8.11 (dd, 1H), 8.81 (s, 1H), 10.41 (s, 1H); Mass spectrum MH⁺ 390.

### Illustrative Example 6.2

### 4-(3-Chloro-4-fluoroanilino)-7-methoxy-5-(tetrahydropyran-4-yloxy)quinazoline

Obtained by reacting 7-methoxy-5-(tetrahydropyran-4-yloxy)-3,4-dihydroquinazolin-4-one (**reference example 14.2**) and 3-chloro-4-fluoroaniline in 56% yield; NMR Spectrum (DMSO-d6) 1.95 (m, 2H). 2.15 (m, 2H), 3.53 (m, 2H), 3.89 (m, 2H), 3.95 (s, 3H), 5.08 (m, 1H), 7.00 (d, 1H), 7.09 (d, 1H), 7.59 (m, 2H), 8.06 (dd, 1H), 8.81 (s, 1H), 10.46 (s, 1H); Mass spectrum MH⁺ 404.

### Illustrative Example 6.3

### 5-(1-tert-Butoxycarbonylpiperidin-4-yloxy)-4-(3-chloro-4-fluoroanilino-7-methoxyquinazoline

Obtained by reacting 5-(1-*tert*-butoxycarbonylpiperidin-4-yloxy)-7-methoxy-3,4-dihydroquinazolin-4-one (**reference example 15**) and 3-chloro-4-fluoroaniline in 54% yield; NMR Spectrum (CDCl₃) 1.48 (s, 9H), 1.86 (m, 2 H), 2.24 (m, 2H), 3.20 (m, 2H), 3.92 (s, 3H), 4.00 (m, 2H), 4.69 (m, 1H), 6.53 (d, 1H), 6.87 (d, 1H), 7.14 (t, 1H), 7.39 (m, 1H), 8.02 (dd, 1H), 8.57 (s, 1H), 9.70 (s, 1H); Mass Spectrum MH⁺ 503.

### Illustrative Examples 6.4

### 4-(3-Chloro-4-(3-fluorobenzyloxy)anilino)-7-(3-(R)-dimethylaminopyrrolidin-1-yl)-5-(1-methylpiperidin-4-yloxy)quinazoline

Obtained by reacting 7-(3-(*R*)-dimethylaminopyrrolidin-1-yl)-5-(1-methylpiperidin-4-yloxy)-3,4-dihydroquinazolin-4-one (**reference example 14**) and 3-chloro-4-(3-fluorobenzyloxy)aniline (**reference example 28**) in 61% yield; Mass Spectrum M⁺ 605.

### Illustrative Example 6.5

### 4-(3-Chloroanilino)-7-(3-(S)-dimethylaminopyrrolidin-1-yl)-5-(tetrahydropyran-4-yloxy)quinazoline

Obtained by reacting 7-(3-(S)-dimethylaminopyrrolidin-1-yl)-5-(tetrahydropyran-4-yloxy)-3,4-dihydroquinazolin-4-one (**reference example 11.1**) and 3-chloroaniline in 73% yield; Mass Spectrum M⁺ 468.

### Illustrative Example 7

### 4-(3-Chloro-4-fluoroanilino)-7-methoxy-5-(tetrahydrothiophen-3-yloxy)quinazoline

4-(3-Chloro-4-fluoroanilino)-5-hydroxy-7-methoxyquinazoline (**reference example 10**) (200 mg), triphenylphosphine (247 mg) and 3-hydroxytetrahydrothiophene (98 mg) were dissolved in anhydrous DCM (30 ml) under a nitrogen atmosphere and cooled to 0°C. Di-*tert-*butyl azodicarboxylate (217 mg) in DCM (1 ml) was added dropwise to the reaction, maintaining the internal temperature < 5 °C. The reaction was allowed to warm up to room temperature over 1 hour and then stirred at room temperature for 1 hour. The reaction was concentrated *in vacuo* and the residue purified by chromatography using 1-5% methanol in DCM as eluent to afford the title compound as a white solid (24 mg, 10%); Mass Spectrum MH⁺ 404.

The procedure described above was repeated using the appropriate 5-hydroxyquinazoline and alcohol. Thus was obtained the compound described below:

### Illustrative Example 7.1

### 4-(3-Chloro-4-fluoroanilino)-7-methoxy-5-(1-iso-propylazetidin-3-yloxy)quinazoline

Obtained by reacting 4-(3-chloro-4-fluoroanilino)-5-hydroxy-7-methoxyquinazoline (**reference example 10**) and 3-hydroxy-1-iso-propylazetidine (obtained as described in J. Org. Chem., 1967, 32, 2972-75) in 13% yield; Mass spectrum MH⁺417.

### Illustrative Example 8

### 4-(3-Chloro-4-fluoroanilino)-5-(tetrahydrothiopyran-4-yloxy)quinazoline

Sodium hydride (180 mg, 60% dispersion in oil) was added portionwise to 4-hydroxy-tetrahydrothiopyran **(reference example 36)** (320 mg) and 4-(3-chloro-4-fluoroanilino)-5-fluoroquinazoline hydrochloride **(reference example 18)** (300 mg) in DMF (5 ml) at room temperature. When the foaming had subsided the reaction was heated at 120°C for 2 hours to give a black solution. The reaction mixture was concentrated *in vacuo* and the residue purified by chromatography using 1-5% methanol in DCM as eluent to give a colourless oil which crystallised on standing. Diethyl ether was added and the product filtered to afford the title compound as a white solid (235 mg, 65%); NMR Spectrum (DMSO-d6) 1.82 (m, 1H), 1.98 (m, 3H), 2.53 - 2.71 (m, 2H), 2.98 - 3.01 (m, 1H), 3.15 (m, 1H). 5.03 (m, 1H), 7.22 (d, 1H). 7.35 (d, 1H), 7.43 (t, 1H), 7.71 (m, 2H), 8.22 (dd, 1H), 8.53 (s, 1H), 10.32 (s, 1H); Mass spectrum MH⁺ 390.

The procedure described above was repeated using the appropriate alcohol and 5-fluoroquinazoline. Thus were obtained the compounds described below:

### Illustrative Example 8.1

### 4-(3-Chloro-4-fluoroanilino)-5-(tetrahydrofuran-3-yloxy)quinazoline

Obtained by reacting 4-(3-chloro-4-fluoroanilino)-5-fluoroquinazoline hydrochloride **(reference example 18)** and 3-hydroxytetrahydrofuran in 72% yield; Mass spectrum MH⁺ 360.

### Illustrative Example 8.2

### 4-(3-Chloro-4-fluoroanilino)-5-(tetrahydropyran-4-yloxy)quinazoline

Obtained by reacting 4-(3-chloro-4-fluoroanilino)-5-fluoroquinazoline hydrochloride **(reference example 18)** and 4-hydroxy-tetrahydropyran in 45% yield; Mass spectrum MH⁺ 374.

### Illustrative Examples 8.3

### 4-(3-Chloro-4-fluoroanilino)-5-cyclopentyloxyquinazoline

Obtained by reacting 4-(3-chloro-4-fluoroanilino)-5-fluoroquinazoline hydrochloride **(reference example 18)** and cyclopentanol in 40% yield; Mass spectrum MH⁺ 358.

### Illustrative Example 8.4

### 4-(3-Chloro-4-fluoroanilino)-5-(1-methylpyrrolidin-3-yloxy)quinazoline

Obtained by reacting 4-(3-chloro-4-fluoroanilino)-5-fluoroquinazoline hydrochloride **(reference example 18)** and 3-hydroxy-1-methylpyrrolidine in 34% yield; Mass spectrum MH⁺ 371.

### Illustrative Example 8.5

### 4-(3-Chloro-4-fluoroanilino)-5-(1-iso-propylazetidin-3-yloxy)quinazoline

Obtained by reacting 4-(3-chloro-4-fluoroanilino)-5-fluoroquinazoline hydrochloride **(reference example 18)** and 3-hydroxy-1-iso-propylazetidine in 54% yield; Mass spectrum MH⁺ 387.

### Illustrative Example 8.6

### 4-(3-Chloro-4-fluoroanilino)-5-(tetrahydrothiophen-3-yloxy)quinazoline

Obtained by reacting 4-(3-chloro-4-fluoroanilino)-5-fluoroquinazoline hydrochloride **(reference example 18)** and 3-hydroxytetrahydrothiophene in 66% yield; Mass spectrum MH⁺ 376.

### Illustrative Example 8.7

### 4-(3-Chloro-4-fluoroanilino)-5-(1-methylpiperidin-3-yloxy)quinazoline

Obtained by reacting 4-(3-chloro-4-fluoroanilino)-5-fluoroquinazoline hydrochloride **(reference example 18)** and 3-hydroxy-1-methylpiperidine in 51% yield; NMR Spectrum (DMSO-d6) 1.25 (t, 1H), 1.47-1.72 (m, 3H), 2.02 (m, 2H), 2.30 (s, 3H), 2.81 (m, 1H), 3.14 (m, 1H), 5.08 (m, 1H), 7.19 (d, 1H), 7.33 (d, 1H), 7.44 (t, 1H), 7.73 (t, 1H), 8.00 (m, 1H), 8.19 (dd, 1H), 8.56 (s, 1H), 10.78 (bs, 1H); Mass spectrum MH⁺ 387.

### Illustrative Example 8.8

### 4-(3-Chloro-4-fluoroanilino)-5-(1-methylpiperidin-4-yloxy)quinazoline

Obtained by reacting 4-(3-chloro-4-fluoroanilino)-5-fluoroquinazoline hydrochloride **(reference example 18)** and 4-hydroxy-1-methylpiperidine in 21% yield; Mass spectrum M⁺ 387.

### Illustrative Example 8.9

### 5-(1-tert-Butoxycarbonylazetidin-3-yloxy)-4-(3-chloro-4-fluoroanilino)quinazoline

Obtained by reacting 4-(3-chloro-4-fluoroanilino)-5-fluoroquinazoline hydrochloride **(reference example 18)** and 1-*tert*-butoxycarbonylazetidin-3-ol (obtained as described in J. Med Chem., (2001), 44(1), 94-104) in 16% yield; NMR spectrum (DMSO-d6); 1.4 (s, 9H), 4.1 (m, 2H), 4.4 (m, 2H), 5.2 (m, 1H), 6.8 (d, 1H), 7.4 (m, 2H), 7.7 (m, 2H), 8.2 (d, 1H), 8.6 (s, 1H), 9.8 (s, 1H); Mass spectrum MH⁺ 445.

### Illustrative Example 9

### 4-(3-Chloro-4-fluoroanilino)-5-(1,1-dioxo-tetrahydrothiophen-3-yloxy)quinazoline and 4-(3-Chloro-4-fluoroanilino)-5-(1-oxo-tetrahydrothiophen-3-yloxy)quinazoline

*m*-CPBA (240 mg) was added to 4-(3-chloro-4-fluoroanilino)-5-(tetrahydrothiophen-3-yloxy)quinazoline **(example 8.6)** (192 mg) in DCM (10 ml) at 0°C. The reaction was stirred at this temperature for 30 minutes then concentrated and the residue purified by chromatography using 1-8% methanol in DCM as eluent to afford firstly 4-(3-chloro-4-fluoroanilino)-5-(1,1-dioxo-tetrahydrothiophen-3-yloxy)quinazoline as a beige solid (64.8 mg, 62%); Mass spectrum M-H⁺ 408; followed by 4-(3-chloro-4-fluoroanilino)5-(1-oxotetrahydrothiophen-3-yloxy)quinazoline as a beige solid (33.4 mg, 33%); Mass spectrum M-H⁺392.

The procedure described above was repeated using the appropriate sulphide. Thus were obtained the compounds described below:

### Illustrative Example 9.1

### 4-(3-Chloro-4-fluoroanilino)-5-((tetrahydrothiopyran-1,1-dioxide)-4-yloxy)quinazoline and 4-3-(Chloro-4-fluoanilino)-5-((tetrahydrothiopyran-1-oxide)-4-yloxy)quinazoline

Obtained from 4-(3-chloro-4-fluoroanilino)-5-(tetrahydrothiopyran-4-yloxy)quinazoline (example 8) in 94% yield; Mass spectrum M-H⁺ 422; and 6% yield; Mass spectrum M-H⁺ 406, respectively.

### Illustrative Example 10

### 4-(3-Chloro-4-(3-fluorobenzyloxy)anilino)-7-methoxy-5-(1-methylpiperidin-4-yloxy)quinazoline

3-Fluorobenzyl chloride (80 mg) was added to a mixture of 4-(3-chloro-4-hydroxyanilino)-7-methoxy-5-(1-methylpiperidin-yloxy)quinazoline (example 2.9) (207 mg) and potassium carbonate (700 mg) in DMF (15 ml). The mixture was stirred vigorously at room temperature for 72 hours, then concentrated *in vacuo.* The resultant oil was partitioned between water and ethyl acetate. The combined organic extracts were then dried (sodium sulphate) and concentrated to give the crude product, which was purified by chromatography using 0-10% methanol in DCM as eluent to give the title compound as a white solid (110 mg, 42%); NMR spectrum (CDCl₃) 2.0 (m, 2H), 2.3 (m, 4H), 2.3 (s, 3H), 2.8 (m, 2H), 3.9 (s, 3H), 4.6 (m, 1H) 5.1 (s, 2H), 6.8 (d, 1H), 6.9 (d, 1H), 7.0 (t, 1H), 7.2 (m, 2H), 7.3 (m, 1H), 7.5 (dd, 1H), 7.9 (d, 1H), 8.5 (s, 1H), 9.7 (s, 1H); Mass spectrum MH⁺ 523.

### Illustrative Example 10.1

### 4-(3-Chloro-4-(5-methylisoxazol-3-ylmethoxy)anilino)-7-methoxy-5-(1-methylpiperidin-4-yloxyl)quinazoline

Obtained by reacting 4-(3-chloro-4-hydroxyanilino)-7-methoxy-5-(1-methylpiperidin-4-yloxy)quinazoline (example 29) with 3-chloromethyl-5-methylisoxazole in 60% yield; NMR spectrum (CDCl₃) 2.0 (m, 2H), 2.3 (m, 4H), 2.3 (s. 3H), 2.4 (s, 3H), 2.8 (m, 2H), 3.9 (s, 3H), 4.6 (m, 1H) 5.2 (s, 2H), 6.2 (s, 1H), 6.5 (d, 1H), 6.8 (d, 11-1), 7.0 (d, 1H), 7.4 (dd, 1H). 7.9 (d, 1H), 8.5 (s, 1H), 9.7 (s, 1H); Mass spectrum MH⁺ 510.

### Illustrative Example 10.2

### 4-(3-Chloro-4-(thiazol-4-ylmethoxy)anilino)-7-methoxy-5-(1-methylpiperidin-4-yloxy)quinazoline

Obtained by reacting 4-(3-chloro-4-hydroxyanilino)-7-methoxy-5-(1-methylpiperidin-4-yloxy)quinazoline **(example 2.9)** with 4-chloromethylthiazole in 60% yield; NMR spectrum (CDCl₃) 2.0 (m, 2H), 2.3 (m, 7H), 2.8 (m, 2H), 3.9 (s, 3H), 4.6 (m,1H) 5.4 (s, 2H), 6.5 (d, 1H), 6.8 (d, 1H), 7.0 (d, 1H), 7.5 (m, 2H), 7.9 (d, 1H), 8.5 (s, 1H), 8.8 (d, 1H), 9.7 (s, 1H); Mass spectrum MH⁺ 512.

### Illustrative Example 10.3

### 4-(3-Chloro-4-(4-pyridylmethoxy)anilino)-7-methoxy-5-(1-methylpiperidin-4-yloxy)quinazoline

Obtained by reacting 4-(3-chlom-4-hydroxyanilino)-7-methoxy-5-(1-methylpiperidin-4-yloxy)quinazoline **(example 2.9)** with 4-picolyl chloride in 45% yield; NMR spectrum (CDCl₃) 2.0 (m, 2H), 2.3 (m, 7H), 2.8 (m, 2H), 3.9 (s, 3H), 4.6 (m, 1H) 5.2 (s, 2H), 6.5 (s, 1H), 6.8 (d, 1H), 6.9 (d, 1H), 7.4 (d, 2H), 7.5 (d, 1H), 7.9 (d, 1H), 8.5 (s, 1H), 8.6 (d, 2H), 9.8 (s, 1H); Mass spectrum MH⁺ 506.

### Illustrative Example 10.4

### 4-(3-Chloro-4-benzyloxyanilino)-7-methoxv-5-(1-methylpiperidin-4-yloxy)quinazoline

Obtained by reacting 4-(3-chloro-4-hydroxyanilino)-7-methoxy-5-(1-methylpiperidin-4-yloxy)quinazoline **(example 2.9)** with benzyl chloride in 47% yield; NMR spectrum (CDCl₃) 2.0 (m, 2H), 2.3 (m, 7H), 2.8 (m, 2H), 3.9 (s, 3H), 4.6 (m, 1H), 5.2 (s, 2H), 6.5 (d, 1H), 6.8 (d, 1H), 7.0 (d, 1H), 7.4 (m, 6H), 7.9 (d, 1H), 8.5 (s, 1H), 9.7 (s, 1H); Mass spectrum MH⁺ 505.

### Illustrative Example 10.5

### 4-(3-Chloro-4-(2-cyanobenzyloxy)anilino)-7-methoxy-5-(1-methylpiperidin-4-yloxy)quinazoline

Obtained by reacting 4-(3-chloro-4-hydroxyanilino)-7-methoxy-5-(1-methylpiperidin-4-yloxy)quinazoline **(example 2.9)** with 2-chloromethylbenzonitrile in 63% yield; NMR spectrum (CDCl₃) 2.0 (m, 2H), 2.3 (m, 7H), 2.8 (m, 2H), 3.9 (s, 3H), 4.6 (m, 1H) 5.3 (s, 2H), 6.5 (s, 1H), 6.8 (s, 1H), 7.0 (d, 1H), 7.4 (m, 2H), 7.7 (m, 2H), 7.8 (d, 1H), 8.0 (s, 1H), 8.5 (s, 1H), 9.8 (s, 1H); Mass spectrum MH⁺ 530.

### Illustrative Example 10.6

### 4-(4-Benzyloxy-3-methylanilino)-7-methoxy-5-(1-methylpideridin-4-yloxy)quinazoline

Obtained by reacting 4-(3-methyl-4-hydroxyanilino)-7-methoxy-5-(1-methylpiperidin-4-yloxy)quinazoline **(example 2.10)** and benzyl chloride in 63% yield; NMR spectrum (CDCl₃) 2.0 (m, 2H), 2.2 (m, 2H). 2.3 (s, 3H), 2.3 (s, 3H), 2.4 (m, 2H), 2.8 (m, 2H), 3.9 (s, 3H), 4.6 (m, 1H) 5.1 (s, 2H), 6.5 (d, 1H), 6.8 (d, 1H), 6.9 (d, 1H), 7.3 - 7.5 (m, 7H), 8.5 (s, 1H), 9.6 (s, 1H); Mass spectrum MH⁺ 485.

### Illustrative Example 10.7

### 4-(4-(2-Fluorobenzyloxy)-3-methylanilino)-7-methoxy-5-(1-methylpiperidin-4-yloxy)quinazoline

Obtained by reacting 4-(3-methyl-4-hydroxyanilino)-7-methoxy-5-(1-methylpiperidin-4-yloxy)quinazoline **(example 2.9)** with 2-fluorobenzyl chloride in 72% yield; NMR spectrum (CDCl₃) 2.0 (m, 2H), 2.2 - 2.4 (m, 10H), 2.8 (m, 2H), 3.9 (s, 3H), 4.6 (m, 1H) 5.2 (s, 2H), 6.5 (s, 1H), 6.8 (s, 1H), 6.9 (d, 1H), 7.1 (m, 2H), 7.3 (m, 1H), 7.5 (m, 3H), 8.5 (s, 1H), 9.7 (s, 1H); Mass spectrum MH⁺ 503.

### Illustrative Example 10.8

### 4-(4-(2,6-Difluorobenzyloxy)-3-methylanilino)-7-methoxy-5-(1-methylpiperidin-4-yloxy)quinazoline

Obtained by reacting 4-(3-methyl-4-hydroxyanilino)-7-methoxy-5-(1-methylpiperidin-4-yloxy)quinazoline **(example 2.10)** with 2,6-difluorobenzyl chloride in 81% yield; NMR spectrum (CDCl₃) 2.0 (m, 2H), 2.2 (m, 5H), 2.3 (m, 5H). 2.8 (m, 2H), 3.9 (s, 3H), 4.6 (m, 1H), 5.1 (s, 2H), 6.5 (d, 1H), 6.8 (d, 1H), 6.9 (t, 2H), 7.0 (d, 1H), 7.3 (m, 1H), 7.4 (m, 1H). 7.5 (dd, 1H), 8.5 (s, 1H), 9.7 (s, 1H); Mass spectrum MH⁺ 521.

### Illustrative Example 10.9

### 4-(4-(2-Cyanobenzyloxy)-3-methylanilino)-7-methoxy-5-(1-methylpiperidin-4-yloxy)quinazoline

Obtained by reacting 4-(3-methyl-4-hydroxyanilino)-7-methoxy-5-(1-methylpiperidin-4-yloxy)quinazoline **(example 2.10)** with 2-chloromethyl benzonitrile in 83% yield; NMR spectrum (CDCl₃) 2.0 (m, 2H), 2.2 (m, 2H), 2.3 (s, 3H), 2.3 (s, 3H), 2.4 (m, 2H), 2.8 (m, 2H), 3.9 (s, 3H), 4.6 (m, 1H) 5.3 (s, 2H), 6.5 (s, 1H), 6.8 (s, 1H), 6.9 (m, 1H), 7.4 (m, 3H), 7.6 (t, 1H), 7.7 (m, 2H), 8.5 (s, 1H), 9.7 (s, 1H); Mass spectrum MH⁺ 510.

### Illustrative Example 10.10

### 4-(3-Methyl-4-(5-methylisoxazol-3-ylmethoxy)anilino)-7-methoxy 5-(1-methylpiperidin-4-yloxy)quinazoline

Obtained by reacting 4-(3-methyl-4-hydroxyanilino)-7-methoxy-5-(1-methylpiperidin-4-yloxy)quinazoline **(example 2.10)** with 3-chloromethyl-5-methylisoxazole in 81% yield; NMR spectrum (CDCl₃) 2.0 (m, 2H), 2.2 - 2.4 (m, 4H), 2.3 (s, 3H), 2.3 (s, 3H), 2.4 (s, 3H), 2.8 (m, 2H), 3.9 (s, 3H), 4.6 (m, 1H) 5.1 (s, 2H), 6.1 (s, 1H), 6.5 (s, 1H), 6.8 (s, 1H), 6.9 (m, 1H), 7.5 (m, 2H), 8.5 (s, 1H), 9.7 (s, 1H); Mass spectrum MH⁺ 490.

### Illustrative Example 10.11

### 4-(3-Methyl-4-(thiazol-4-ylmethoxy)anilino)-7-methoxy-5-(1-methylpiperidin-4-yloxy)quinazoline

Obtained by reacting 4-(3-methyl-4-hydroxyanilino)-7-methoxy-5-(1-methylpiperidin-4-yloxy)quinazoline **(example 2.10)** with 4-chloromethylthiazole in 31% yield; NMR spectrum (CDCl₃) 2.0 (m, 2H), 2.2 - 2.4 (m, 10H), 2.8 (m, 2H), 3.9 (s, 3H), 4.6 (m, 1H) 5.3 (s, 2H), 6.5 (d, 1H), 6.8 (d, 1H), 6.9 (d, 1H). 7.4 - 7.5 (m, 3H), 8.5 (s, 1H), 8.8 (d, 1H), 9.7 (s, 1H); Mass spectrum MH⁺ 492.

### Illustrative Example 10.12

### 4-(4-(3-Fluorobenzyloxy)-3-methylanilino)-7-methoxy-5-(1-methylpiperidin-4-yloxy)quinazoline

Obtained by reacting 4-(3-methyl-4-hydroxyanilino)-7-methoxy-5-(1-methylpiperidin-4-yloxy)quinazoline **(example 2.10)** with 3-fluorobenzyl chloride in 57% yield; NMR spectrum (CDCl₃) 2.0 (m, 2H), 2.2 - 2.4 (m, 10H), 2.8 (m, 2H), 3.9 (s, 3H), 4.6 (m, 1H) 5.1 (s, 2H), 6.5 (d, 1H), 6.8 (d, 1H), 6.9 (d, 1H), 7.0 (m, 1H), 7.2 (m, 2H), 7.3 (m, 1H), 7.4 - 7.5 (m, 2H), 8.5 (s, 1H), 9.7 (s, 1H); Mass spectrum MH⁺ 503.

### Illustrative Example 11

### 4-(3-Chloro-4-fluoroanilino)-7-(2-methoxyethoxy)-5-(tetrahydropyran-4-yloxy)quinazoline

Potassium carbonate (0.14 g) and 2-bromoethyl methyl ether (73 µl) were added to a suspension of 4-(3-chloro-4-fluoroanilino)-7-hydroxy-5-(tetrahydropyran-4-yloxy)quinazoline trifluoroacetate **(reference example 20)** in DMF (3 ml). The mixture was stirred at room temperature for 20 hours, then more 2-bromoethyl methyl ether (97 µl) was added and the mixture was stirred at room temperature for a further 20 hours. The mixture was then concentrated in *vacuo,* the residue was cooled and cold water was added. The resulting solid was filtered, washed with cold water and dried *in vacuo* to give the title compound as a beige solid (0.09g, 78%); NMR Spectrum (DMSO-d6) 1.85 (m, 2H), 2.17 (m, 2H), 3.31 (s, 3H), 3.54 (t, 2H), 3.70 (m, 2H), 3.89 (m, 2H), 4.23 (m, 2H), 4.98 (m, 1H), 6.80 (d, 2H), 6.87 (d, 1H), 7.41 (t, 1H), 7.51-7.58 (m, 1H), 8.28 (m, 1H), 8.47 (s, 1H), 9.89 (s, 1H); Mass spectrum MH⁺ 446.

The procedure described above was repeated using the appropriate 7-hydroxyquinazoline and alkyl halide or tosylate. Thus were obtained the compounds described below:

### Illustrative Example 11.1

### 4-(3-Bromoanilino)-7-(2-methoxyethoxy)-5-(1-methylpiperidin-4-yloxy)quinazoline

Obtained by reacting 4-(3-bromoanilino)-7-hydroxy-5-(1-methylpiperidin-4-yloxy)quinazoline trifluoroacetate **(reference example 20.1)** and 2-bromoethyl methyl ether in 43% yield; NMR spectrum (DMSO-d6) 2.2 (m, 2H), 2.4 - 2.6 (m, 7H), 2.7 (m, 2H), 3.3 (s, 3H), 3.7 (m, 2H), 4.2 (m, 2H), 5.0 (m, 1H), 6.9 (dd, 2H), 7.3 (m, 2H) 7.6 (m, 1H), 8.3 (s, 1H), 8.5 (s, 1H); Mass spectrum MH⁺ 489.

### Illustrative Example 11.2

### 4-(3-Chloro-4-fluoroanilino)-7-(2-methoxyethoxy)-5-(tetrahydrofuran-3-yloxy)quinazoline

Obtained by reacting 4-(3-chloro-4-fluoroanilino)-7-hydroxy-5-(tetrahydrofuran-3-yloxy)quinazoline trifluoroacetate **(reference example 20.2)** and 2-bromoethyl methyl ether in 79% yield; NMR Spectrum (DMSO-d6) 2.16 (m, 1H), 2.32 (m, 1H), 3.32 (s, 3H), 3.71 (m, 2H), 3.78-3.97 (m, 3H), 4.21 (m, 3H), 5.47 (m, 1H), 6.82 (s, 2H), 7.42 (t, 1H), 7.60 (m, 1H), 8.28 (m, 1H), 8.49 (s, 1H), 9.91 (s, 1H); Mass spectrum MH⁺ 434.

### Illustrative Example 11.3

### 4-(3-Chloro-4-fluoroanilino)-5-cyclopentyloxy-7-(2-methoxyethoxy)quinazoline

Obtained by reacting 4-(3-chloro-4-fluoroanilino)-5-cyclopentyloxy-7-hydroxyquinazoline trifluoroacetate **(reference example 20.3)** and 2-bromoethyl methyl ether in 96 % yield; NMR Spectrum (DMSO-d6) 1.72 (m, 4H), 2.00 (m, 4H), 3.31 (s, 3H), 3.70 (m, 2H), 4.23 (m, 2H), 5.19 (m, 1H), 6.70 (d, 1H). 6.79 (d, 1H), 7.45 (m, 2H), 8.25 (m, 111), 8.46 (s, 1H), 9.88 (s, 1H); Mass spectrum MH⁺ 432.

### Illustrative Example 11.4

### 7-(2-Methoxyethoxy)-4-(3-methylanilino)-5-(1-methylpiperidin-4-yloxy)quinazoline

Obtained by reacting 7-hydroxy-4-(3-methylanilino)-5-(1-methylpiperidin-4-yloxy)quinazoline trifluoroacetate **(reference example 20.5)** and 2-bromoethyl methyl ether in 36% yield; NMR spectrum (DMSO-d6) 1.8 (m, 2H), 2.1 (m, 5H), 2.3 (m, 5H), 2.6 (m, 2H), 3.3 (s, 3H), 3.7 (m, 2H), 4.2 (m, 2H), 4.8 (m, 1H), 6.8 (m, 2H), 7.0 (m, 1H), 7.2 (m, 1H), 7.5 (m, 1H), 7.6 (s, 1H), 8.4 (s, 1H), 9.9 (s, 1H); Mass spectrum MH⁺ 423.

### Illustrative Examples 11.5

### 4-(3-Chloro-4-fluoroanilino)-7-(2-methoxyethoxy)-5-(1-methylpiperidin-4-yloxy)quinazoline

Obtained by reacting 4-(3-chloro-4-fluoroanilino)-7-hydroxy-5-(1-methylpiperidin-4-yloxy)quinazoline trifluoroacetate **(reference example 20.4)** and 2-bromoethyl methyl ether in 53% yield; NMR spectrum (DMSO-d6) 1.8 (m, 2H), 2.1 (m, 5H), 2.3 (m, 2H), 2.6 (m, 2H), 3.3 (s, 3H), 3.7 (m, 2H), 4.2 (m, 2H), 4.8 (m, 1H), 6.8 (m, 2H), 7.4 (t, 1H), 7.6 (m, 1H), 8.2 (m, 1H), 8.5 (s, 1H), 9.9 (s, 1H); Mass spectrum M-H⁺ 459.

### Illustrative Example 11.6

### 4-(3-Chloro-4-(3-fluorobenzyloxy)anilino)-5-(1-methylpiperidin-4-yloxy)-7-((1-tert-butoxycarbonypiperidin-4-yl)methoxy)quinazoline

Obtained by reacting N-tert-butoxycarbonyl-4-tosyloxymethylpiperidine (reference example 41) and 4-(3-chloro-4-(3-fluorobenzyloxy)anilino)-5-(1-methylpiperidin-4-yloxy)-7-hydroxyquinazoline **(reference example 20.8)** in 65% yield; NMR spectrum (CDCl₃) 1.3 (m, 2H), 1.5 (s, 9H), 1.8 (m, 25, 2.0 (m, 3H), 2.2 - 2.4 (m, 7H), 2.8 (m, 4H), 3.9 (d, 2H), 4.2 (m, 2H), 4.6 (m, 1H), 5.2 (s, 2H), 6.5 (d, 1H), 6.8 (d, 1H), 6.9 (d, 1H), 7.0 (m, 1H), 7.2 (m, 2H), 7.3 (m, 1H), 7.5 (dd, 1H), 7.9 (d, 1H), 8.5 (s, 1), 9.7 (s, 1H): Mass spectrum MH⁺ 706.

### Illustrative Example 11.7

### 4-(3-Chloro-4-(3-fluorobenzyloxy)anilino)-5-(1-methylpiperidin-4-yloxy)-7-(2-methoxyethoxy)quinazoline

Obtained by reacting 2-methoxyethyl bromide and 4-(3-chloro-4-(3-fluorobenzyloxy)anilino)-5-(1-methylpiperidin-4-yloxy)-7-hydroxyquinazoline **(reference example 20.8)** in 64% yield as a white solid; NMR spectrum (CDCl₃) 2.0 (m, 2H), 2.2 - 2.3 (m, 7H), 2.8 (m, 2H), 3.5 (s, 3H), 3.8 (m, 2H), 4.2 (m, 2H), 4.6 (m, 1H). 5.1 (s, 2H), 6.6 (d, 1H), 6.8 (d, 1H), 6.9 (d, 1H), 7.0 (m, 1H), 7.2 (m, 2H), 7.3 (m, 1H), 7.5 (dd, IED, 7.9 (d, 1H), 8.5 (s, 1H), 9.7 (s, 1H); Mass spectrum MH⁺ 567.

### Illustrative example 11.8

### 4-(3-Chloro-4-(3-fluorobenzyloxy)anilino)-5-(tetrahydropyran-4-yloxy)-7-((1-tert-butoxycarbonylpiperidin-4-yl)methoxy)quinazoline

Obtained by reacting *N-tert*-butoxycarbonyl-4-tosyloxymethylpiperidine **(reference example 41)** and 4-(3-chloro-4-(3-fluorobenzyloxy)anilino)-5-(tetrahydropyran-4-yloxy)-7-hydroxyquinazoline **(reference example 20.9)** in 69% yield; NMR spectrum (CDCl₃) 1.3 (m, 2H), 1.5 (s, 9H), 1.8 (m, 2H). 2.0 (m, 3H). 2.2 - 2.3 (m, 2H), 2.8 (m, 2H), 3.6 (m, 2H), 3.9 (d, 2H), 4.0 (dt, 2H), 4.2 (m, 2H), 4.8 (m, 1H), 5.2 (s, 2H), 6.5 (d, 1H), 6.8 (d, 1H), 6.9 (d, 1H), 7.0 (m, 1H, 7.2 (m, 2H), 7.4 (m, 1m, 7.5 (dd, 1H), 7.9 (d, 1H), 8.5 (s, 1H), 9.7 (s, 1H); Mass spectrum MH⁺ 693.

### Illustrative Example 11.9

### 4-(3-Chloro-4-(3-fluorobenzyloxy)anilino)-7-(2-methoxyethoxy)-5-(3-tetrahydrofuranyloxy)quinazoline

Obtained by reacting 2-bromoethyl methyl ether with 4-(3-chloro-4-(3-fluorobenzyloxy)anilino)-7-hydroxy-5-(3-tetrahydrofuranyloxy)quinazoline (125 mg) **(reference example 20.10)** in 35% yield; Mass spectrum MH⁺ 540.

### Illustrative Example 12

### 4-(3-Chloroanilino)7-(1-methylpiperidin-4-ylmethoxy)-5-(1-methylpiperidin-4-yloxy)quinazoline

7-(1-*tert*-Butoxycarbonylpiperidin-4-ylmethoxy)-4-(3-chloroanilino)-5-(1-methylpiperidin-4-yloxy)quinazoline **(reference example 22.1)** (50 mg) was heated at 80°C in formic acid (2 ml) and aqueous formaldehyde (1 ml) for 15 hours. The solvent was removed in *vacuo* to give a pink solid. 7N Ammonia in methanol was added and the solvent removed in *vacuo.* Water was added and the solid thus obtained was filtered and dried to afford the title compound as a beige solid; (30 mg, 71%); NMR spectrum (DMSO-d6) 1.3 (m, 2H), 1.6 - 2.0 (m, 7H), 2.1 (m, 8H), 2.3 (m, 2H), 2.6 (m, 2H), 2.8 (m, 2H), 4.0 (d, 2H), 4.8 (m, 1H), 6.8 (d, 2H), 7.1 (d, 1H), 7.4 (t, 1H), 7.5 (d, 1H), 8.2 (s, 1H), 8.5 (s, 1H), 10.0 (s, 1H);
Mass spectrum MH⁺ 496.

The procedure described above was repeated using the appropriate 1-tert-butoxycarbonyl amine. Thus were obtained the compounds described below:

### Illustrative Example 12.1

### 4-(3-Chloro-4-fluoroanilino)-7-(1-methylpiperidin-4-ylmethoxy)-5-(1-methylpiperidin-4-yloxy)quinazoline

Obtained from 7-(1-*tert*-butoxycarbonylpiperidin-4-ylmethoxy)-4-(3-chloro-4-fluoroanilino)-5-(1-methylpiperidin-4-yloxy)quinazoline **(reference example 22)** in 35% yield; NMR spectrum (DMSO-d6) 1.3 (m, 2H), 1.7 (m,4H), 1.9 (m, 4H), 2.1 (m, 7H), 2.3 (m, 2H), 2.6 (m, 2H), 2.8 (m, 2H), 4.0 (d, 2H), 4.8 (m, 1H), 6.8 (d, 2H), 7.4 (t, 1H), 7.6 (m, 1H), 8.2 (dd, 1H), 8.5 (s, 1H), 9.9 (s, 1H); Mass spectrum MH⁺ 514.

### Illustrative Example 12.2

### 4-(3-Chloro-4-fluoroanilino)-5-(1-methylazetidin-3-yloxy)quinazoline

Obtained from 5-(1-*tert*-butyloxycarbonylazetidin-3-yloxy)-4-(3-chloro-4-fluoroanilino)quinazoline **(example 8.9)** in 18% yield; NMR spectrum (DMSO-d6) 2.3 (s, 3H), 3.3 (m, 2H), 3.8 (m, 2H), 5.1 (m, 1H), 6.9 (d, 1H), 7.4 (m, 2H), 7.7 (m, 2H), 8.3 (d, 1H), 8.6 (s,1H); Mass spectrum MH⁺ 359.

### Illustrative Example 12.3

### 4-(3-Chloro-4-fluoroanilino)-7-(1-methylpiperidin-4-ylmethoxy)-5-(tetrahydrofuran-3-yloxy)quinazoline

Obtained from 4-(3-chloro-4-fluoroanilino)-7-(1-*tert*-butoxycarbonylpiperidin-4-ylmethoxy)-5-(tetrahydrofuran-3-yloxy)quinazoline **(reference example 22.2)** in 94% yield; NMR Spectrum (DMSO-d6) 1.36 (m, 2H), 1.75 (m, 2H), 1.90 (m, 2H), 2.18 (s, 3H), 2.22 - 2.40 (m, 1H), 2.50 (m, 2H), 2.79 (m, 2H), 3.78 - 3.98 (m, 5H), 4.18 (d, 1H), 5.45 (m, 1H), 6.80 (m, 2H), 7.42 (t, 1H), 7.61 (m, 1H), 8.28 (m, 1H), 8.40 (m, 1H). 8.52 (s, 1H), 9.87 (s, 1H); Mass spectrum MH⁺ 488.

### Illustrative Example 13

### 4-(3-Chloro-4-fluoroanilino)-7-methoxy-5-(piperidin-4-yloxy)quinazoline

Trifluoroacetic acid (20 ml) was added to a solid sample of 5-(1-*tert-*butoxycarbonylpiperidin-4-yloxy)-4-(3-chloro-4-fluoroanilino)-7-methoxyquinazoline **(example 6.3)** (200 mg), then stirred at room temperature for 10 minutes. The excess trifluoroacetic acid was removed in *vacuo,* then saturated aqueous sodium hydrogen carbonate was carefully added (effervescence). The product was then extracted into DCM, dried over sodium sulphate and concentrated *in vacuo* to give the crude material, which was purified by chromatography using 0-10% methanol in DCM as eluent, to give the title compound as a white solid (130 mg, 81%); NMR Spectrum (DMSO-d6) 1.84 (m, 2H), 2.24 (m, 2H), 2.83 (m, 2H), 2.40 (m, 2H), 3.12 (m, 2H), 3.35 (bs, 1H), 3.92 (s, 3H), 4.91 (m, 1H), 6.85 (d, 1H), 6.87 (d, 1H), 7.47 (t, 1H), 7.59 (m, 1H), 8.28 (dd, 1H), 8.52 (s, 1H), 9.94 (s, 1H); Mass Spectrum MH⁺ 403.

The procedure described above was repeated using the appropriate *tert-*butoxycarbonyl protected amine. Thus was obtained the compound described below:

### Illustrative Example 13.1

### 4-(3-Chloro-4-fluoroanilino-7-(piperidin-4-ylmethoxy)-5-(tetrahydrofuran-3-yloxy)quinazoline

Obtained from 4-(3-chloro-4-fluoroanilino)-7-(1-*tert-*butoxycarbonylpiperidin-4-ylmethoxy)-5-(tetrahydrofuran-3-yloxy)quinazoline (**reference example 22.2**) in 73% yield; NMR Spectrum (DMSO-d6) 1.48 (m, 2H), 1.95 (m, 2H), 2.02 - 2.20 (m, 2H), 2.30 (m, 1H), 2.92 (m, 2H), 3.30. (m, 2H), 3.78 - 3.98 (m, 3H), 4.03 (d, 2H), 4.19 (d, 1H), 5.45 (m, 1H), 6.80 (m, 2H), 7.42 (t, 1H), 7.61 (m, 1H), 8.28 (m, 1H), 8.43 (m, 1H), 8.52 (s, 1H), 9.87 (s, 1H); Mass spectrum MH⁺ 474.

### Illustrative Example 13.2

### 4-(3-Chloro-4-(3-fluorobenzyloxy)anilino)-5-(1-methylpiperidin-4-yloxy)-7-(piperidin-4-ylmethoxy)quinazoline

Obtained from 4-(3-chloro-4-(3-fluorobenzyloxy)anilino)-5-(1-methylpiperidin-4-yloxy)-7-((1-*tert*-butoxycarbonylpiperidin-4-yl)methoxy)quinazoline (**example 11.6**) in 78% yield; NMR spectrum (CDCl₃) 1.3 (m, 2H), 1.8 (m, 2H), 2.0 (m, 3H), 2.2 - 2.4 (m, 7H), 2.6 - 2.8 (m, 4H), 3.2 (m, 2H), 3.9 (d, 2H), 4.6 (m, 1H), 5.1 (s, 2H), 6.5 (d, 1H), 6.8 (d, 1H), 6.9 (d, 1H), 7.0 (m, 1H), 7.2 (m, 2H), 7.3 (m, 1H), 7.5 (dd, 1H), 7.9 (d, 1H), 8.5 (s, 1H), 9.7 (s, 1H); Mass spectrum MH⁺ 606.

### Illustrative Example 13.3

### 4-(3-Chloro-4-(3-fluorobenzyloxy)anilino)-5-(tetrahydropyran-4-yloxy)-7-(piperidin-4-ylmethoxy)quinazoline

Obtained from 4-(3-chloro-4-(3-fluorobenzyloxy)anilino)-5-(tetrahydropyran-4-yloxy)-7-((1-*tert-*butoxycarbonylpiperidin-4-yl)methoxy)quinazoline (**example 11.8**) in 72% yield; NMR spectrum (CDCl₃) 1.3 (m, 2H), 1.8 (m, 2H), 2.0 (m, 3H), 2.3 (m, 2H), 2.7 (m, 2H), 3.1 (m, 2H), 3.6 (m, 2H), 3.9 (d, 2H), 4.1 (m, 2H), 4.7 (m, 1H), 5.2 (s, 2H, 6.5 (d, 1H), 6.8 (d, 1H), 6.9 (d, 1H), 7.0 (m, 1H), 7.2 (m, 2H), 7.4 (m, 1H), 7.5 (dd, 1H), 7.9 (d, 1H), 8.5 (s, 1H), 9.7 (s, 1H); Mass spectrum NH⁺ 593.

### Illustrative Example 14

### 5-(N-Acetylpiperidin-4-yloxy)-4-(3-chloro-4-fluoroanilino)-7-methoxyquinazoline

Acetyl chloride (0.18 ml) was added to a solution of 4-(3-chloro-4-fluoroanilino)-7-methoxy-5-(piperidin-4-yloxy)quinazoline (90 mg) (example 13) and 4-(dimethylamino)pyridine (approximately 1 mg) in pyridine (20 ml). The mixture was then stirred at room temperature for 1 hour, and concentrated *in vacuo.* The residue was dissolved in DCM, and washed with saturated aqueous sodium hydrogen carbonate, aqueous copper (II) sulphate, then water. Drying over sodium sulphate, followed by concentration *in vacuo* gave a yellow viscous oil. Purification by chromatography, using 0-2% methanol in DCM as eluent, gave the title compound as a yellow foam, which was triturated under cold acetonitrile to give the product as a white solid (30 mg, 29%); NMR Spectrum (CDCl₃) 1.88 (m, 2H), 2.15 (s, 3H), 2.29 (m, 2H), 3.26 (m, 1H), 3.39 (m, 1H), 3.84 (m, 1H), 3.93 (s, 3H), 4.32 (m, 1H), 4.76 (m, 1H), 6.53 (d, 1H), 6.87 (d, 1H), 7.14 (t, 1H), 7.36 (m, 1H), 8.01 (dd, 1H), 8.57 (s, 1H), 9.63 (s, 1H); Mass Spectrum MH⁺ 445.

### Illustrative Example 15

### 4-(3-Chloro-4-fluoroanilino-7-methoxy-5-(1-propylpiperidin-4-yloxy)quinazoline

Sodium triacetoxyborohydride (63 mg) was added to a stirred solution of 4-(3-chloro-4-fluoroanilino)-7-methoxy-5-(piperidin-4-yloxy)quinazoline (example 13) (100 mg), propionaldehyde (0.18 ml) and acetic acid (0.5 ml) in 1, 2-dichloroethane (10 ml) at room temperature. After 15 minutes, the reaction mixture was diluted with water, and solid potassium carbonate (excess) was added. The resultant mixture was extracted into DCM, dried over sodium sulphate, and concentrated *in vacuo* to give the crude material as a colourless oil, which solidified on addition of diethyl ether. Trituration of this solid with cold methanol gave the title compound as a white powder (110 mg, 100%); NMR Spectrum (CDCl₃) 0.92 (t, 3H), 1.53 (m, 2H), 1.98 (m, 2H), 2.25 (m, 6H), 2.87 (m, 2H), 3.91 (s, 3H), 4.57 (m, 1H), 6.51 (d, 1H), 6.84 (d, 1H), 7.12 (t, 1H), 7.46 (m, 1H), 8.00 (dd, 1H), 8.56 (s, 1H), 9.83 (s, 1H); Mass Spectrum MH⁺ 445.

The procedure described above was repeated using the appropriate amine and aldehyde. Thus were obtained the compounds described below:

### Illustrative Example 15.1

### 5-(1-Ethylpiperidin-4-yloxy)-4-(3-chloro-4-fluoroanilino)-7-methoxyquinazoline

Obtained from 4-(3-chloro-4-fluoroanilino)-7-methoxy-5-(piperidin-4-yloxy)quinazoline (**example 13**) and acetaldehyde in 80% yield; NMR Spectrum (CDCl₃) 1.11 (t, 3H), 2.00 (m, 2H), 2.31 (m, 4H), 2.46 (q, 2H), 2.88 (m, 2H), 3.91 (s, 3H), 4.58 (m, 1H), 6.51 (d, 1H), 6.84 (d, 1H), 7.12 (t, 1H), 7.46 (m, 1H), 8.00 (dd, 1H), 8.56 (s, 1H), 9.83 (s, 1H); Mass Spectrum MH⁺ 431.

### Illustrative Example 15.2

### 4-(3-Chloro-4-fluoroanilino)-7-methoxy-5-(1-(2-methoxyethyl)piperidin-4-yloxy)quinazoline

Obtained from 4-(3-chloro-fluoroanilino)-7-methoxy-5-(piperidin-4-yloxy)quinazoline (**example 13**) and 2-methoxyacetaldehyde in 68% yield; NMR Spectrum (CDCl₃) 2.03 (m, 2H), 2.25 (m, 2H), 2.39 (m, 2H), 2.62 (t, 2H), 2.93 (m, 2H), 3.36 (s, 3H), 3.52 (t, 2H), 3.91 (s, 3H), 4.57 (m, 1H), 6.50 (d, 1H), 6.84 (d, 1H), 7.12 (t, 1H), 7.45 (m, 1H), 8.01 (dd, 1H), 8.56 (s, 1H), 9.83 (s, 1H); Mass Spectrum MH⁺ 461.

### Illustrative Example 16

### 4-(3-Chloro-4-fluoroanilino)-5-(1-(2-propynyl)piperidin-4-yloxy)-7-methoxy-quinazoline

Propargyl bromide (80% w/w in toluene, 60 mg) was added to a mixture of 4-(3-chloro-4-fluoroanilino)-7-methoxy-5-(piperidin-4-yloxy)quinazoline (**example 13**) (100 mg) and potassium carbonate (343 mg) in DMF (15 ml). The reaction mixture was stirred at room temperature for 4 hours, then poured into water. The resultant fine white precipitate was recovered by filtration, then purified by preparative LC-MS, to give the title compound as a white solid (56 mg, 51%); NMR Spectrum (CDCl₃) 2.03 (m, 2H), 2.19 (t, 1H), 2.30 (m, 2H), 2.56 (m, 2H), 2.91 (m, 2H), 3.39 (d, 2H), 3.92 (s, 3H), 4.61 (m, 1H), 6.52 (d, 1H), 6.85 (d, 1H), 7.13 (t, 1H), 7.47 (m, 1H), 8.01 (dd, 1H), 8.56 (s, 1H), 9.80 (s, 1H); Mass Spectrum,MH⁺ 441.

The procedure described above was repeated using the appropriate alkyl or alkenyl halide and amine. Thus was obtained the compound described below:

### Illustrative Example 16.1

### 5-(1-Allylpilperidin-4-yloxy)-4-(3-chloro-4-fluoroanilino)-7-methoxyquinazoline

Obtained by reacting 4-(3-chloro-4-fluoroanilino)-7-methoxy-5-(piperidin-4-yloxy)quinazoline (**example 13**) with allyl bromide in 45% yield; NMR Spectrum (CDCl₃) 1.99 (m, 2H), 2.31 (m, 4H), 2.89 (m, 2H), 3.05 (d, 2H), 3.91 (s, 3H), 4.57 (m, 1H), 5.19 (m, 2H), 5.87 (m, 1H), 6.51 (d, 1H), 6.84 (d, 1H), 7.12 (t, 1H), 7.47 (m, 1H), 7.99 (dd, 1H, 8.56 (s, 1H), 9.82 (s, 1H); Mass Spectrum MH⁺ 443.

### Illustrative Example 17

### Methyl 2(4-(4-(3-chloro-4-fluornanilino)-7-methoxyquinazolin-5-yloxy)piperidin-1-yl) acetate

Potassium carbonate (343 mg), methyl chloroacetate (0.036 ml), and 4-(3-chloro-4-fluoroanilino)-7-methoxy-5-(piperidin-4-yloxy)quinazoline (**example 13**) (100 mg) in DMF (2 ml) were stirred and heated in a sealed tube to 120°C using a focussed microwave source The mixture was then cooled, poured into water, and extracted into DCM (containing 2% methanol), dried over sodium sulphate and concentrated *in vacuo.* The resultant crude oil was purified by chromatography, using 0-5% methanol in DCM. This gave the title compound as a colourless oil (72 mg, 61 %); NMR Spectrum (CDCl₃) 2.05 (m, 2H), 2.30 (m, 2H), 2.57 (m, 2H), 2.98 (m, 2H), 3.31 (s, 2H), 3.73 (s, 3H), 3.92 (s, 3H), 4.60 (m, 1H), 6.51 (d, 1H), 6.85 (d, 1H), 7.13 (t, 1H), 7.46 (m, 1H), 8.02 (dd, 1H), 8.56 (s, 1H), 9.79 (s, 1H); Mass Spectrum MH⁺ 475.

The procedure described above was repeated using the appropriate alkyl halide and amine. Thus were obtained the compounds described below:

### Illustrative Example 17.1

### 4-(4-(3-Chloro-4-fluoroanilino)-7-methoxyquinazolin-5-yloxy)piperidin-1-ylmethyl methyl ketone

Obtained by reacting 4-(3-chloro-4-fluoroanilino)-7-methoxy-5-(piperidin-4-yloxy)quinazoline (**example 13**) with chloromethyl methyl ketone in 44% yield; NMR Spectrum (CDCl₃) 2.05 (m, 2H), 2.16 (s, 3H), 2.29 (m, 2H), 2.46 (m, 2H), 2.88 (m, 2H, 3.27 (s, 2H), 3.92 (s, 3H), 4.59 (m, 1H), 6.51 (d, 1H), 6.85 (d, 1H), 7.14 (t, 1H), 7.45 (m, 1H), 8.01 (dd, 1H), 8.57 (s, 1H), 9.79 (s, 1H); Mass Spectrum MH⁺ 459.

### Illustrative Example 17.2

### 2-(4-(4-(3-Chloro-4-fluoroanilino)-7-methoxyquinazolin-5-yloxy)piperidin-1-yl)acetamide

Obtained by reacting 4-(3-chloro4-fluoroanilino)-7-methoxy-5-(piperidin-4-yloxy)quinazoline (**example 13**) with 2-bromoacetamide in 43% yield; NMR Spectrum (DMSO-d6) 1.99 (m, 2H), 2.18 (m, 2H), 2.32 (m, 2H), 2.90 (s, 2H), 3.92 (s, 3H), 4.83 (m, 1H), 6.84 (s, 2H), 7.10 (bs, 1H), 7.25 (bs, 1H), 7.47 (t, 1H), 7.57 (m, 1H), 8.31 (dd, 1H), 8.51 (s, 1H), 9.95 (s, 1H); Mass Spectrum MH⁺ 460.

### Illustrative Example 18

### 4-(3-Chloro-4-fluoroanilino)-5-(1-(methanesulphonyl)piperidin-4-yloxy)-7-methoxy-quinazoline

Methanesulphonyl chloride (42 mg) was added to a stirred solution of 4-(3-chloro-4-fluoroanilino)-7-methoxy-5-(piperidin-4-yloxy)quinazoline (**example 13**) (100 mg) and triethylamine (55 mg) in DCM (20 ml) at room temperature. After 1 hour, the reaction mixture was diluted with DCM, washed with saturated aqueous sodium hydrogen carbonate, dried over sodium sulphate and concentrated *in vacuo* to give the crude material, which was triturated under methanol to give the tide compound as a white solid (85 mg, 71%); NMR Spectrum (CDCl₃) 2.08 (m, 2H), 2.37 (m, 2H), 2.79 (s, 3H), 3.19 (m, 2H), 3.67 (m, 2H), 3.92 (s, 3H), 4.71 (m, 1H), 6.51 (d, 1H), 6.87 (d, 1H), 7.14 (t, 1H), 7.37 (m, 1H), 8.00 (dd, 1H), 8.56 (s, 1H), 9.58 (s, 1H); Mass Spectrum MH⁺ 481.

### Illustrative Example 19

### 4-(3-Chloro-4-(3-fluorobenzyloxy)anilino)-7-(3-(1-methylpinerazin-4-yl)propoxy)-5-cyclopentyloxyquinazoline hydrochloride

A solution of 4-(3-chloro-4-(3-fluorobenzyloxy)anilino)-7-(3-chloropropoxy)-5-cyclopentyloxyquinazoline (**reference example 21.8**) (0.15 g) and 1-methylpiperazine (0.18 ml) in NMP (2 ml) was heated at 80°C for 16 hours. The solution was concentrated *in vacuo* and the residue triturated with ether. The resulting solid was filtered to give the title compound as a white solid (30 mg, 18%); Mass Spectrum MH⁺ 620.

The procedure described above was repeated using the appropriate alkyl halide and amine. Thus were obtained the compounds described below:

### Illustrative Example 19.1

### 4-(3-Chloro-4-(3-fluorobenzyloxy)anilino)-7-(3-(N-(2-methoxyethyl)-N-methylamino)propoxy)-5-cyclopentyloxyquinazoline hydrochloride

Obtained by reacting 4-(3-chloro-4-(3-fluorobenzyloxy)anilino)-7-(3-chloropropoxy)-5-cyclopentyloxyquinazoline (**reference example 21.8**) and *N*-(2-methoxyethyl)-N-methylamine in 60% yield; Mass Spectrum MH⁺ 609.

### Illustrative Example 19.2

### 4-(3-Chloro-4-(3-fluorobenzyloxy)anilino)-7-(2-(1-methylpiperazin-4-yl)ethoxy)-5-cyclopentyloxyquinazoline hydrochloride

Obtained by reacting 4-(3-chloro-4-(3-fluorobenzyloxy)anilino)-7-(2-chloroethoxy-5-cyclopentyloxyquinazoline (**reference example 21.9**) and 1-methylpiperazine in 62% yield; Mass Spectrum MH⁺ 606.

### Illustrative Example 19.3

### 4-(3-Chloro-4-fluoroanilino)-7-(3-(pyrrolidin-1-yl)propoxy)-5-(tetrahydrofuran-3-yloxy)quinazoline

Obtained by reacting pyrrolidine and 4-(3-chloro-4-fluoroanilino)-7-(3-chloropropoxy)-5-(tetrahydrofuran-3-yloxy)quinazoline (**reference example 21**) in 64% yield; NMR Spectrum (DMSO-d6) 1.67 (m, 4H), 1.92 (m, 2H), 2.15 (m, 1H), 2.30 (m, 1H), 2.45 (m, 4H), 2.55 (t, 2H), 3.78 - 3.98 (m, 3H), 4.15 - 4.20 (m, 3H), 5.45 (m, 1H), 6.80 (m, 2H), 7.42 (t, 1H), 7.61 (m, 1H), 8.28 (m, 1H), 8.47 (s, 1H), 9.87 (s, IM; Mass spectrum MH⁺ 488.

### Illustrative Example 19.4

### 4-(3-Chloro-4-fluoroanilino)-7-(3-piperidinopropoxy)-5-(tetrahydrofuran-3-yloxy)quinazoline

Obtained by reacting 4-(3-chloro-4-fluoroanilino)-7-(3-chloropropoxy)-5-(tetrahydrofuran-3-yloxy)quinazoline (**reference example 21**) and piperidine in 64% yield; NMR spectrum (DMSO-d6) 1.38 (m, 2H), 1.48 (m, 4H), 1.90 (m, 2H), 2.18 (m, 1H), 2.22 - 2.40 (m, 7H), 3.78 - 3.98 (m, 3H), 4.10 - 4.20 (m, 3H), 5.45 (m, 1H), 6.79 (m, 2H), 7.42 (t, 1H), 7.61 (m, 1H), 8.28 (m, 1H), 8.50 (s, 1H), 9.85 (s, 1H); Mass spectrum MH⁺ 502.

### Illustrative Example 19.5

### 4-(3-Chloro-4-fluoroanilino)-7-(3-morpholinopropoxy)-5-(tetrahydrofuran-3-yloxy)quinazoline

Obtained by reacting 4-(3-chloro-4-fluoroanilino)-7-(3-chloropropoxy)-5-(tetrahydrofuran-3-yloxy)quinaxoline (**reference example 21**) and morpholine in 64% yield; NMR spectrum (DMSO-d6) 1.92 (m, 2H), 2.18 (m, 1H), 2.22 - 2.45 (m, 7H), 3.58 (m, 4H), 3.78 - 3.98 (m, 3H), 4.10 - 4.20 (m, 3H), 5.48 (m, 1H), 6.79 (m, 2H), 7.41 (t, 1H), 7.61 (m, 1H), 8.28 (m, 1M, 8.50 (s, 1H), 9.83 (s, 1H); Mass spectrum MH⁺ 504.

### Illustrative Example 19.6

### 4-(3-Chloro-4-fluoroanilino)-7-(3-(N-methyl-N-(2-propynyl)amino)propoxy)-5-(tetrahydrofuran-3-yloxy)quinazoline

Obtained by reacting 4-(3-chloro-4-fluoroanilino)-7-(3-chloropropoxy)-5-(tetrahydrofuran-3-yloxy)quinazoline (**reference example 21**) and *N*-methyl-N-propargylamine in 53% yield; NMR spectrum (DMSO-d6) 1.88 (m, 2H), 2.18 (m, 1H), 2.20 (s, 3H), 2.22 - 2.40 (m, 1H), 2.50 (m, 2H), 3.08 (t, 1H), 3.28 (m, 2H), 3.78 - 3.98 (m, 3H), 4.10 - 4.20 (m, 3H), 5.48 (m, 1H), 6.79 (m, 2H), 7.41 (t, 1H), 7.61 (m, 1H), 8.28 (m, 1H), 8.50 (s, 1H), 9.83 (s, 1H); Mass spectrum MH⁺ 486.

### Illustrative Example 19.7

### 4-(3-Chloro-4-fluoroanilino)-7-(3-(N-methyl-N-allylamino)propoxy)-5-(tetrahydrofuran-3-yloxy)quinazoline

Obtained by reacting 4-(3-chloro-4-fluoroanilino)-7-(3-chloropropoxy)-5-(tetrahydrofuran-3-yloxy)quinazoline (**reference example 21**) and *N*-methyl-N-allylamine in 37% yield; NMR spectrum (DMSO-d6) 1.90 (m, 2H), 2.10 - 2.20 (m, 4H), 2.22 - 2.40 (m, 1H), 2.45 (m, 2H), 2.98 (d, 2H), 3.78-3.98 (m, 3H), 4.10 - 4.21 (m, 3H), 5.05 - 5.20 (m, 2H), 5.48 (m, 1H), 5.80 (m, 1H), 6.79 (m, 2H), 7.41 (t, 1H), 7.61 (m, 1H), 8.28 (m, 1H), 8.50 (s, 1H), 9.83 (s, 1H); Mass spectrum MH⁺ 488.

### Illustrative Example 19.8

### 4-(3-Chloro-4-fluroannilino)-7-(3-(4-hydroxypiperidin-1-yl)propoxy)-5-(tetrahydrofuran-3-yloxy)quinazoline

Obtained by reacting 4-(3-chloro-4-fluoroanilino)-7-(3-chloropropoxy)-5-(tetrahydrofuran-3-yloxy)quinazoline (**reference example 21**) and 4-hydroxypiperidine in 78% yield; NMR spectrum (DMSO-d6) 1.38 (m, 2H), 1.70 (m, 2H), 1.90 (m, 2H), 2.01 (m, 2H), 2.18 (m, 2H), 2.32 (m, 1H), 2.40 (m, 2H), 2.70 (m, 2H), 3.42 (m, 1H), 3.78 - 3.98 (m, 3H), 4.10 - 4.21 (m, 3H), 4.50 (m, 1H), 5.48 (m, 1H), 6.80 (m, 2H), 7.41 (t, 1H), 7.61 (m, 1H), 8.28 (m, 1H), 8.50 (s, 1H), 9.93 (s, 1H); Mass spectrum MH⁺ 518.

### Illustrative Example 19.9

### 4-(3-Chloro-4-fluoroanilino)-7-(3-(3-oxo-piperazin-1-yl)propoxy)-5-(tetrahydrofuran-3-yloxy)quinazoline

Obtained by reacting 4-(3-chloro-4-fluoroanilino)-7-(3-chloropropoxy)-5-(tetrahydrofuran-3-yloxy)quinazoline (**reference example 21**) and piperazin-2-one in 76% yield; NMR spectrum (DMSO-d6) 1.90 (m, 2H), 2.18 (m, 1H), 2.32 (m, 1H), 2.45 - 2.60 (m, 4H), 2.95 (s, 2H), 3.15 (m, 2H), 3.78 - 3.98 (m, 3H), 4.13 - 4.21 (m, 3H), 5.48 (m, 1H), 6.80 (m, 2H), 7.41 (t, 1H), 7.61 (m, 1H), 7.70 (s, 1H), 8.28 (m, 1H), 8.50 (s, 1H), 9.93 (s, 1H); Mass spectrum MH⁺ 517.

### Illustrative Example 19.10

### 4-(3-Chloro-4-fluoroanilino)-7-(3-(4-methylpiperazin-1-yl)propoxy)-5-(tetrahydrofuran-3-yloxy)quinazoline

Obtained by reacting 4-(3-chloro-4-fluoroanilino-7-(3-chloropropoxy)-5-(tetrahydrofuran-3-yloxy)quinazoline (**reference example 21**) and 1-methylpiperazine in 41% yield; NMR spectrum (DMSO-d6) 1.90 (m, 2H), 2.10 - 2.20 (m, 4H), 2.21- 2.42 (m, 11H), 3.78 - 3.98 (m, 3H), 4.13 - 4.21 (m, 3H), 5.48 (m, 1H), 6.80 (m, 2H), 7.41 (t, 1H), 7.61 (m, 1H), 8.28 (m, 1H), 8.50 (s, 1H), 9.93 (s, 1H); Mass spectrum MH⁺ 517.

### Illustrative example 19.11

### 4-(3-Chloro-4-fluoroanilino)-7-3-(4-2-methoxyethyl)piperazin-1-yl)propoxy)-5-(tetrahydrofuran-3-yloxy)quinazoline

Obtained by reacting 4-(3-chloro-4-fluoroanilino)-7-(3-chloropropoxy)-5-(tetrahydrofiuan-3-yloxy)quinazoline (**reference example 21**) and 4-(2-methoxyethyl)piperazine in 49% yield; NMR spectrum (DMSO-d6) 1.90 (m, 2H), 2.18 (m, 1H), 2.22 - 2.45 (m, 13H), 3.20 (s. 3H), 3.40 (t, 2H), 3.78 - 3.98 (m, 3H), 4.10 - 4.21 (m, 3H), 5.48 (m, 1H), 6.79 (m, 2H), 7.41 (t, 1H), 7.61 (m, 1H), 8.28 (m, 1H), 8.50 (s, 1H), 9.93 (s, 1H); Mass spectrum MH⁺ 561.

### Illustrative Example 19.12

### 4-(3-Chloro-4-fluoroanilino)-7-(3-(4-(N,N-dimethylcarbamoylmethyl)piperazin-1-yl)propoxy)-5-(tetrahydrofuran-3-yloxy)quinazoline

Obtained by reacting 4-(3-chloro-4-fluoroanilino)-7-(3-chloropropoxy)-5-(tetrahydrofuran-3-yloxy)quinazoline (**reference example 21**) and 1-(N,N-dimethylcarbamoylmethyl)piperazine in 62% yield; NMR spectrum (DMSO-d6) 1.90 (m, 2H), 2.18 (m, 1H), 2.22 - 2.50 (m, 11H), 2.78 (s, 3H), 2.99 (s, 3H), 3.27 (s, 2H), 3.78 - 3.98 (m, 3H), 4.10 - 4.21 (m, 3H), 5.48 (m, 1H), 6.79 (m, 2H), 7.41 (t, 1H), 7.61 (m, 1H), 8.28 (m, 1H), 8.50 (s, 1H), 9.93 (s, 1H); Mass spectrum MH⁺ 588.

### Illustrative Example 19.13

### 4-(3-Chloro-4-fluoroanilino)-7-(3-(4-allylpiperazin-1-yl)propoxy)-5-(tetrahydrofuran-3-yloxy)quinazoline

Obtained by reacting 4-(3-chloro-4-fluoroanilino)-7-(3-chloropropoxy)-5-(tetrahydrofuran-3-yloxy)quinazoline (**reference example 21**) and 1-allylpiperazine in 50% yield; NMR spectrum (DMSO-d6) 1.90 (m, 2H), 2.18 (m, 1H), 2.22 - 2.50 (m, 11H), 2.90 (d, 2H), 3.78 - 3.98 (m, 3H), 4.10 - 4.21 (m, 3H), 5.10 (m, 2H), 5.45 (m, 1H), 5.78 (m, 1H), 6.79 (m, 2H), 7.41 (t, 1H), 7.61 (m, 1H), 8.28 (m, 1H), 8.50 (s, 1H), 9.93 (s, 1H); Mass spectrum MH⁺ 543.

### Illustrative Example 19.14

### 4-(3-Chloro-4-fluoroanilino)-7-(3-(4-(2-propynyl)piperazin-1-yl)propoxy)-5-(tetrahydrofuran-3-yloxy)quinazoline

Obtained by reacting 4-(3-chloro-4-fluoroanilino)-7-(3-chloropropoxy)-5-(tetrahydrofuran-3-yloxy)quinazoline (**reference example 21**) and 1-(2-propynyl)piperazine in 53% yield; NMR spectrum (DMSO-d6) 1.90 (m, 2H), 2.18 (m, 1H), 2.22 - 2.50 (m, 11H), 3.08 (t, 1H), 3.22 (d, 2H), 3.78 - 3.98 (m, 3H), 4.10 - 4.21 (m, 3H), 5.47 (m, 1H), 6.79 (m, 2H), 7.41 (t, 1H), 7.61 (m, 1H), 8.28 (m, 1H), 8.50 (s, 1H), 9.93 (s, 1H); Mass spectrum MH⁺ 541.

### Illustrative Example 19.15

### 4-(3-Chloro-4-fluoroanilino-7-(3-(4-cyanomethylpiperazin-1-yl)propoxy)-5-(tetrahydrofuran-3-yloxy)quinazoline

Obtained by reacting 4-(3-chloro-4-fluoroanilino)-7-(3-chloropropoxy)-5-(tetrahydrofuran-3-yloxy)quinazoline (**reference example 21**) and 1-cyanomethylpiperazine in 40% yield; NMR spectrum (DMSO-d6) 1.90 (m, 2H), 2.18 (m, 1H), 2.22 - 2.50 (m, 11H), 3.68 (s, 2H), 3.78 - 3.98 (m, 3H), 4.10 - 4.21 (m, 3H), 5.47 (m, 1H), 6.79 (m, 2H), 7.41 (t, 1H), 7.61 (m, 1H), 8.28 (m, 1H), 8.50 (s, 1H), 9.93 (s, 1H); Mass spectrum MH⁺ 542.

### Illustrative Example 19.16

### 4-(3-Chloro-4-fluoroanilino)-7-(3-(piperazin-1-yl)propoxy)-5-(tetrahydrofuran-3 yloxy)quinazoline

Obtained by reacting 4-(3-chloro-4-fluoroanilino)-7-(3-chloropropoxy)-5-(tetrahydrofuran-3-yloxy)quinazoline (**reference example 21**) and piperazine in 71% yield; NMR spectrum (DMSO-d6) 1.90 (m, 2H), 2.18 (m, 1H), 2.22 - 2.50 (m, 7H), 2.70 (m, 4H), 3.78 - 3.98 (m, 3H), 4.10 - 4.21 (m, 3H), 5.47 (m, 1H), 6.79 (m, 2H), 7.41 (t, 1H), 7.61 (m, 1H), 8.28 (m, 1H), 8.50 (s, 1H), 9.93 (s, 1H); Mass spectrum MH⁺ 503.

### Illustrative Example 19.17

### 4-(3-Chloro-4-(3-fluorobenzyloxy)anilino)-5-(1-methylpiperidin-4-yloxy)-7-(3-(4-methylpiperazin-1-yl)propoxy)quinazoline

Obtained by reacting 1-methylpiperazine and 4-(3-chloro-4-(3-fluorobenzyloxy)anilino)-5-(1-methylpiperidin-4-yloxy)-7-(3-chloropropyl-1-yloxy)quinazoline (**reference example 21.10**) in 41% yield; NMR spectrum (CDCl₃) 2.0 (m, 4H), 2.2 - 2.4 (m, 10H), 2.4 - 2.6 (m, 10H), 2.8 (m, 2H), 4.1 (t, 2H), 4.6 (m, 1H), 5.1 (s, 2H), 6.5 (d, 1H), 6.8 (d, 1H), 6.9 (d, 1H), 7.0 (m, 1H), 7.2 (m, 2M, 7.3 (m, 1H), 7.5 (dd, 1H), 7.9 (d, 1H), 8.5 (s, 1H), 9.7 (s, 1H); Mass spectrum MH⁺ 649.

### Illustrative Example 19.18

### 4-(3-Chloro-4-(3-fluorobenzyloxy)anilino)-5-(1-methylpiperidin-4-yloxy)-7-(3-piperidinopropoxy)quinazoline

Obtained by reacting 4-(3-chloro-4-(3-fluorobenzyloxy)anilino)-5-(1-methylpiperidin-4-yloxy)-7-(3-chloropropyl-1-yloxy)quinazoline (**reference example 21.10**) with piperidine in 44% yield; NMR spectrum (CDCl₃) 1.5 (m, 2H), 1.6 (m, 4H), 2.0 (m, 4H), 2.2 - 2.4 (m, 11H), 2.5 (m, 2H), 2.8 (m, 2H), 4.1 (t, 2H), 4.6 (m, 1H), 5.1 (s, 2H), 6.5 (d, 1H), 6.8 (d, 1H), 6.9 (d, 1H), 7.0 (m, 1H), 7.2 (m, 2H), 7.4 (m, 1H), 7.5 (dd, 1H), 7.9 (d, 1H), 8.5 (s, 1H), 9.7 (s, 1H); Mass spectrum MH⁺ 634.

### Illustrative Example 19.19

### 4-(3-Chloro-4-(3-fluorobenzyloxy)anilino)-5-(1-methylpiperidin-4-yloxy)-7-(3-morpholinopropoxy)quinazoline

Obtained by reacting 4-(3-chloro-4-(3-fluorobenzyloxy)anilino)-5-(1-methylpiperidin-4-yloxy)-7-(3-chloropropyl-1-yloxy)quinazoline (**reference example 21.10**) with morpholine in 39% yield; NMR spectrum (CDCl₃) 2.0 - 2.2 (m, 4H), 2.3 (m, 2H), 2.4 (s, 3H), 2.5 - 2.6 (m, 7H), 2.8 (m, 2m, 3.6 (d, 1H), 3.8 (m, 4H), 4.2 (t, 2H), 4.6 (m, 1H) 5.1 (s, 2H), 6.5 (d, 1H), 6.8 (d, 1H), 6.9 (d, 1H), 7.0 (m, 1H), 7.2 (m, 2H), 7.4 (m, 1H), 7.4 (dd, 1H), 7.9 (d, 1H), 8.5 (s, 1H), 9.7 (s, 1H); Mass spectrum MH⁺ 636.

### Illustrative Example 19.20

### 4-(3-Chloro-4-(3-fluorobenzyloxy)anilino)-5-(1-methylpiperidin-4-yloxy)-7-(3-(N-(2-methoxethyl)-N-methylamino)propoxy)quinazoline

Obtained by reacting 4-(3-chloro-4-(3-fluorobenzyloxy)anilino)-5-(1-methylpiperidin-4-yloxy)-7-(3-chloropropyl-1-yloxy)quinazoline (**reference example 21.10**) with N-(2-methoxyethyl)-N-methylamine in 35% yield; NMR spectrum (CDCl₃) 2.0 (m, 4H), 2.2 - 2.4 (m, 10H), 2.6 (m, 4H), 2.8 (m, 2H), 3.3 (s, 3H), 3.5 (t, 2H), 4.1 (t, 2H), 4.6 (m, 1H), 5.1 (s, 2H), 6.5 (d, 1H), 6.8 (d, 1H), 6.9 (d, 1H), 7.0 (m, 1H), 7.2 (m, 2H), 7.4 (m, 1H), 7.5 (dd, 1H). 7.9 (d, 1H). 8.5 (s, 1H), 9.7 (s, 1H); Mass spectrum MH⁺ 638.

### Illustrative Example 19.21

### 4-(3-Chloro-4-(3-fluorobenzyloxy)anilino)-5-(tetrahydropyran-4-yloxy)-7-(2-(4,4-difluoropiperidin-1-yl)ethoxy)quinazoline

Obtained by reacting 4,4-difluoropipendine and 4-(3-chloro-4-(3-fluorobenzyloxy)anilino)-5-(tetrahydropyran-4-yloxy)-7-(2-chloroethoxy)quinazoline (**reference example 21.11**) in 23% yield; NMR spectrum (CDCl₃) 1.9 - 2.1 (m, 6H), 2.3 (m, 2H), 2.8 (t, 4H), 3.0 (t, 2H), 3.6 (m, 2H), 4.1 (dt, 2H), 4.3 (t, 2H), 4.8 (m, 1H) 5.2 (s, 2H), 6.6 (d, 1H). 7.0 (d, 1H), 7.0 (m, 2H), 7.2 (m, 2H), 7.4 (m, 1H), 7.5 (dd, 1H), 7.8 (d, 1H), 8.6 (s, 1H), 9.9 (s, 1H); Mass spectrum MH⁺ 643.

### Illustrative Example 19.22

### 4-(3-Chloro-4-(3-fluorobenzyloxy)anilino-5-(tetrahydropyran-4-yloxy)-7-(3-(N-(2-methoxyethyl)-N-methylamino)propoxy)quinazoline

Obtained by reacting 4-(3-chloro-4-(3-fluorobenzyloxy)anilino)-5-(tetrahydropyran-4-yloxy)-7-(3-chloropropyl-1-yloxy)quinazoline (**reference example 21.12**) with N-(2-methoxyethyl)-N-methylaminee in 52% yield; NMR spectrum (DMSO-d6) 1.8 - 2.0 (m, 2H), 2.0 - 2.2 (m, 4H). 2.7 (m, 3H), 3.1 (m, 4H), 3.3 (s, 3H), 3.6 (m. 2H), 3.7 (m, 2H), 3.9 (m, 2H), 4.2 (m, 2H). 5.0 (m, 1H), 5.3 (s, 2H), 6.9 (m, 2H), 7.2 - 7.4 (m, 4H), 7.5 (m, 2H), 8.2 (d, 1H), 8.5 (s, 1H), 9.9 (s, 1H); Mass spectrum MH⁺ 625.

### Illustrative Example 19.23

### 4-(3-Chloro-4-(3-fluorobenzyloxy)anilino)-5-(tetrahydropyran-4-yloxy)-7-(3-piperidinopropoxy)quinazoline

Obtained by reacting 4-(3-chloro4-(3-fluorobenzyloxy)anillino)-5-(tetrahydropyran-4-yloxy)-7-(3-chloropropyl-1-yloxy)quinazoline (**reference example 21.12**) with piperidine in 39% yield; NMR spectrum (DMSO-d6) 1.6 (m, 2H), 1.8 - 2.0 (m, 6H), 2.2 (m, 4H), 3.2 (m, 6H), 3.6 (t, 2H), 4.0 (m. 2H). 4.3 (m, 2H), 5.0 (m, 1H), 5.3 (s, 2H), 6.9 (s, 2H), 7.2 - 7.4 (m, 4H), 7.5 (m, 2H), 8.2 (d, 1H), 8.5 (s, 1H), 9.9 (s, 1H); Mass spectrum MH⁺ 621.

### Illustrative Example 19.24

### 4-(3-Chloro4-(3-fluorobenzyloxy)anilino)-5-(tetrahydropyran-4-yloxy)-7-(2-(4-methylpiperazin-1-yl)ethoxy)quinazoline

Obtained by reacting 4-(3-chloro-4-(3-fluorobenzyloxy)anilino)-5-(tetrahydropyran-4-yloxy)-7-(2-chloroethoxyquinazoline (**reference example 21.11**) with 1-methylpiperazine in 43% yield; NMR spectrum (CDCl₃) 1.9 (m, 2H), 2.2 - 2.3 (m, 5H), 2.5 (m, 4H), 2.6 (m, 4H), 2.9 (t, 2H), 3.6 (m, 2H), 4.1 (dt, 2H), 4.2 (t, 2H), 4.7 (m, 1H) 5.1 (s, 2H), 6.6 (d, 1H), 6.8 (d, 1H), 6.9 (d, 1H), 7.0 (m, 1H), 7.2 (m, 2H), 7.4 (m, 1H), 7.5 (dd, 1H), 7.9 (d, 1H), 8.5 (s, 1H), 9.7 (s, 1H); Mass spectrum MH⁺ 622.

### Illustrative Example 19.25

### 4-(3-Chloro-4-(3-fluorobenzyloxy)anilino)-5-(tetrahydropyran-4-yloxy)-7-(3-(4-methyl-piperazin-1-yl)propoxy)quinazoline

Obtained by reacting 4-(3-chloro-4-(3-fluorobenzyloxy)anilino)-5-(tetrahydropyran-4-yloxy)-7-(3-chloropropyl-1-yloxy)quinazoline (**reference example 21.12**) with 1-methylpiperazine in 59% yield; NMR spectrum (CDCl₃) 2.0 (m, 4H), 2.3 (m, 5H), 2.4 - 2.6 (m, 10H), 3.6 (m, 2H), 4.1 (dt, 2H), 4.1 (t, 2H), 4.8 (m, 1H), 5.2 (s, 2H), 6.5 (d, 1H), 6.8 (d, 1H), 6.9 (d, 1H), 7.0 (m, 1H), 7.2 (m, 2H), 7.4 (m, 1H), 7.5 (dd, 1H), 7.9 (d, 1H), 8.5 (s, 1H), 9.7 (s, 1H); Mass spectrum MH⁺ 636.

### Illustrative Example 19.26

### 4-(3-Chloro-4(3-fiuorobenzyloxy)anilino)-7-(3-(4-methyl-piperazin-1-yl)propoxy)-5-(tetrahydrofuran-3-yloxy)quinazoline

Obtained by reacting 1-methylpiperazine with 4-(3-chloro-4-(3-fluorobenzyloxy)anilino)-7-(3-chloropropyloxy)-5-(tetrahydrofuran-3-yloxy)quinazoline (**reference example 21.13**) in 43% yield; NMR spectrum (DMSO-d6) 2.0 (m, 2H), 2.2 (m, 5H), 2.3 - 2.5 (m, 10H), 3.8 - 4.0 (m, 3H), 4.2 (m, 3H), 5.3 (s, 2H) 5.5 (m, 1H), 6.8 (m, 2H), 7.2 - 7.4 (m, 4H), 7.5 - 7.6 (m, 2H), 8.2 (d, 1H), 8.5 (s, 1H), 9.9 (s, 1H); Mass spectrum MH⁺ 622.

### Illustrative Example 19.27

### 4-(3-Chloro-4-(3-fluorobenzyloxy)anilino)-7-(3-piperidinopropxy)-5-(tetrahydrofuran-3-yloxy)quinazoline

Obtained by reacting 4-(3-chloro-4-(3-fluorobenzyloxy)anilino)-7-(3-chloropropyloxy)-5-(tetrahydrofuran-3-yloxy)quinazoline (**reference example 21.13**) with piperidine in 23% yield; NMR spectrum (DMSO-d6) 1.4 (m, 2H), 1.5 - 1.6 (m, 4H), 1.9 - 2.0 (m, 2H), 2.2 - 2.3 (m, 1H), 2.3 - 2.5 (m, 7H), 3.8 - 4.0 (m, 3H), 4.2 (m, 3H), 5.3 (s, 2H), 5.5 (m, 1H), 6.8 (m, 2H), 7.1-7.4 (m, 4H), 7.5 (m, 1H), 7.6 (dd, 1H). 8.2 (d, 1H), 8.5 (s, 1H), 9.9 (s, 1H); Mass spectrum MH⁺ 607.

### Illustrative Example 19.28

### 4-(3-Chloro-4-(3-fluorobenzyloxy)anilino)-5-(tetrahydrofuran-3-yloxy)-7-(2-(4-methyl piperazin-1-yl)ethoxy)quinazoline

Obtained by reacting 1-methylpiperazine with 4-(3-chloro-4-(3-fluorobenzyloxy)anilino)-7-(2-chloroethoxy)-5-(tetrahydrofuran-3-yloxy)quinazoline (reference example 21.14) in 53% yield; NMR spectrum (DMSO-d6) 2.2 - 2.3 (m, 4H), 2.3 - 2.4 (m, 5H), 2.5 (m, 2H - hidden under DMSO signal), 2.8 (m, 2H), 3.4 (m, 2H - partially obscured by water signal), 3.8 - 4.0 (m, 3H), 4.2 - 4.3 (m, 3H), 5.3 (s, 2H), 5.5 (m, 1H), 6.9 . (m, 2H), 7.2 - 7.4 (m, 4H), 7.5-7.6 (m, 2H), 8.2 (m, 1H), 8.5 (s, 1H), 9.9 (s, 1H); Mass spectrum MH⁺ 622.

### Illustrative Example 19.29

### 4-(3-Chloro-4-(3-fluorobenzyloxy)anilino)-7-(3-morpholinopropoxy)-5-(tetrahydrofuran-3-yloxy)quinazoline

Obtained by reacting 4-(3-chloro-4-(3-fluorobenzyloxy)anilino)-7-(3-chloropropyloxy)-5-(tetrahydrofuran-3-yloxy)quinazoline (**reference example 21.13**) with morpholine in 14% yield; NMR spectrum (DMSO-d6) 2.0 (m, 2H), 2.2 (m, 1H), 2.3 - 2.5 (m, 7H), 3.6 (m. 4H), 3.8 - 4.0 (m, 3H), 4.2 (m, 3H), 5.3 (s, 2H), 5.5 (m, 1H), 6.8 (m, 2H), 7.2 - 7.4 (m, 4H), 7.5 - 7.6 (m, 2H), 8.2 (d, 1H), 8.5 (s, 1H), 9.8 (s, 1H); Mass spectrum MH⁺ 609.

### Illustrative Example 19.30

### 4-(3-Chloro-4-(3-fluorobenzyloxy)anilino)-7-(2-morpholinoethoxy)-5-(tetrahydrofuran-3-yloxo)-quinazoline

Obtained by reacting 7-(2-chloroethoxy)-4-(3-chloro-4-(3-fluorobenzyloxy)anilino)-5-(tetrahydrofuran-3-yloxy)quinazoline (**reference example 21.14**) with morpholine in 33% yield; NMR spectrum (DMSO-d6) 2.2 (m, 1H), 2.4 (m, 1H), 2.5 (m, 2H - hidden under DMSO signal), 2.8 (t, 2H), 3.3 (m, 2H- partially obscured by water signal), 3.6 (m, 4H), 3.8 - 4.0 (m, 3H), 4.2 (d, 1H), 4.3 (t, 2H), 5.3 (s, 2H), 5.5 (m, 1H), 6.8 (d, 1H), 6.9 (d, 1H), 7.2-7.4 (m, 4H), 7.5 (m, 1H), 7.6 (dd, 1H), 8.2 (d,1H), 8.5 (s. 1H), 9.9 (s, 1H); Mass spectrum MH⁺ 595.

### Illustrative Example 19.31

### 4-(3-Chloro-4-(3-fluorobenzyloxy)anilino)-7-(2-[N-2-methoxyethyl)-N-methylamino]ethoxy)-5-(tetrahydrofuran-3-yloxy)quinazoline

Obtained by reacting 7-(2-chloroethoxy)-4-(3-chloro-4-(3-fluorobenzyloxy)anilino)-5-(tetrahydrofuran-3-yloxy)quinazoline (**reference example 21.14**) with N-(2-methoxyethyl)-N-methylamine in 25% yield; NMR spectrum (DMSO-d6) 2.2 (m, 1H), 2.3 (s, 3H), 2.7 (t, 2H), 2.9 (t, 2H), 3.3 (s, 3H), 3.3 (m, 1H), 3.5 (t, 2H), 3.8 - 4.0 (m, 3H), 4.2 (m, 3H), 5.3 (s, 2H), 5.5 (m, 1H), 6.8 (m, 210, 7.2 - 7.4 (m, 4H), 7.5 - 7.6 (m, 2H), 8.2 (d, 1H), 8.5 (s, 1H), 9.9 (s, 1H); Mass spectrum MH⁺ 597.

### Illustrative Example 19.32

### 4-(3-Chloro-4-(3-fluorobenzyloxy)anilino)-7-(2-piperidinoethoxy)-5-(tetrahydrofuran-3-yloxy)quinazoline

Obtained by reacting 7-(2-chloroethoxy)-4-(3-chloro-4-(3-fluorobenzyloxy)anilino)-5-(tetrahydrofuran-3-yloxy)quinazoline (**reference example 21.14**) with piperidine in 34% yield; NMR spectrum (DMSO-d6) 1.4 (m, 2H), 1.5 - 1.6 (m, 4H), 2.2 - 2.3 (m, 1H), 2.3 - 2.4 (m, 1H), 2.5 (m, 2H), 2.8 (m, 2H), 3.2 (m, 2H), 3.9 - 4.0 (m, 3H), 4.2 - 4.3 (m, 3H), 5.3 (s, 2H), 5.5 (m, 1H), 6.8 (m, 2H), 7.2 - 7.4 (m, 4H), 7.5 (m, 1H), 7.6 (dd, 1H), 8.2 (d, 1H), 8.5 (s, 1H), 9.9 (s, 1H); Mass spectrum MH⁺ 593.

### Illustrative Example 20

### 4-(3-Chloro-4-fluoroanilino)-7-(3-(4-acetylpiperazin-1-yl)propoxy)-5-(tetrahydrofuran-3-yloxy)quinazoline

Triethylamine (38 µl) and acetic anhydride (26 µl) were added, each in one portion, to a stirred solution of 4-(3-chloro-4-fluoroanilino)-7-(3-(piperazin-1-yl)propoxy)-5-(tetrahydrofuran-3-yloxy)quinazoline (**example 19.16**) (115 mg) in DCM (2 ml) at 0°C. The solution was stirred at 0°C under a nitrogen atmosphere for 1 hour and then DCM (10 ml) and saturated aqueous sodium hydrogen carbonate (15 ml) were added. The layers were separated and the aqueous layer was extracted with DCM (2 × 10 ml). The combined organic extracts were dried and concentrated *in vacuo* to leave a white solid which was purified by chromatography using 0 - 8% 7N ammonia in methanol in DCM as eluent. This gave the title compound as a white solid (105 mg, 84%); NMR Spectrum (DMSO-d6) 1.90 - 2.00 (m, 5H), 2.18 (m, 1H), 2.22 - 2.50 (m, 7H), 3.42 (m, 4H), 3.78 - 3.98 (m, 3H). 4.10 - 4.21 (m, 3H), 5.47 (m, 1H), 6.80 (m, 2H), 7.41 (t, 1H), 7.61 (m, 1H), 8.28 (m, 1H), 8.50 (s, 1H), 9.93 (s, 1H); Mass spectrum MH⁺ 545.

### Illustrative Example 21,

### 4-(3-Chloro-4-(3-fluorobenzyloxy)anilino)-5-(1-methylpiperidin-4-yloxy)-7-(1-methylpiperidin-4-ylmethoxy)quinazoline

4-(3-Chloro-4-(3-fluorobenzyloxy)anilino-5-(1-methylpiperidin-4-yloxy)-7-(piperidin-4-ylmethoxy)quinazoline (130 mg) (**example 13.2**) was added to a mixture of formic acid (0.58 ml) and formaldehyde (37 wt. % aqueous solution, 0.88 ml), and the resultant mixture was heated at 85°C for 2 hours. An excess of saturated aqueous sodium hydrogen carbonate solution was added, and the product was extracted into DCM. The combined organic extracts were dried and concentrated *in vacuo* to give the crude product, which was triturated under cold methanol to give the title compound as a white solid (20 mg, 15%); NMR spectrum (CDCl₃) 1.5 (m, 2H), 1.8 (m, 3H), 2.0 (m, 4H), 2.2 - 2.4 (m, 10H), 2.8 (m, 2H), 2.9 (m, 2H), 3.9 (d, 2H), 4.6 (m, 1H) 5.1 (s, 2H), 6.5 (d, 1H). 6.8 (d, 1H). 6.9 (d, 1H), 7.0 (m; 1H), 7.2 (m, 2m, 7.4 (m, 1H). 7.5 (dd, 1H), 7.9 (d, 1H), 8.5 (s, 1H), 9.7 (s, 1H); Mass spectrum MH⁺ 620.

### Example 22

### 4-(1-(2-Cyanobenzyl)indol-5-ylamino-7-methoxy-5-(1-methylpiperidin-4-yloxy)quinazoline

Sodium hydride (13.1 mg) was added to a solution of 4-(indol-5-ylamino)-7-methoxy-5-(1-metllylpiperidin-4-yloxy)quinazoline (**example 2.8**) (120 mg) in DMA (1 ml), and stirred at room temperature for 30 minutes. This mixture was then added dropwise to a solution of 2-chloromethylbenzonitrile (50 mg) in DMA (1 ml), and allowed to stir for 5 hours at room temperature. Excess water was added, which gave the product as a thick gum, which was decanted off. The gum was then purified by chromatography, using 0-10% methanol in DCM as eluent to give the product as a gum, which was triturated under water, to give the title compound as a solid (10 mg, 6%); Mass Spectrum MH⁺ 519.

The procedure described above was repeated using the appropriate alkyl halide. Thus were obtained the compounds described below:

### Example 22.1

### 4-(1-(3-Fluorobenzyl)indol-5-ylamino)-7-methoxy-5-(1-methylpiperidin-4-yloxy)quinazoline

Obtained by reacting 4-(indol-5-ylamino)-7-methoxy-5-(1-methylpiperidin-4-yloxy)quinazoline (**example 2.8**) with 3-fluorobenzyl chloride in 14% yield; Mass Spectrum MH⁺ 512.

### Example 22.2

### 4-(1-(2-Fluorobenzyl)indol-5-ylamino)-7-methoxy-5-1-methylpiperidin-4-yloxy)quinazoline

Obtained by reacting 4-(indol-5-ylamino-7-methoxy-5-(1-methylpiperidin-4-yloxy)quinazoline (**example 2.8**) with 2-fluorobenzyl chloride in 5% yield; Mass Spectrum MH⁺ 512.

### Illustrative Example 22.3

### 4-(1-(5-methylisoxazol-3-ylmethyl)indol-5-ylamino)-7-methoxy-5-(1-methylpiperidin-4*-yloxy)quinazoline

Obtained by reacting 4-(indol-5-ylamino)-7-methoxy-5-(1-methylpiperidin-4-yloxy)quinazoline (**example 2.8**) with 3-(chloromethyl)-5-methylisoxazole in 74% yield; Mass Spectrum MH⁺ 499.

### Example 22.4

### 4-(1-Benzylindol-5-ylamino)-7-methoxy-5-(1-methylpiperidin-4-yloxy)quinazoline

Obtained by reacting 4-(indol-5-ylamino)-7-methoxy-5-(1-methylpiperidin-4-yloxy)quinazoline (example 2.8) with benzyl chloride in 46% yield; Mass Spectrum MH⁺ 494.

### Example 22.5

### 7-Methoxy-5-(1-methylpiperidin-4-yloxy)-4-(1-(2-pyridylmethyl)indol-5-ylamino)quinazoline

Obtained by reacting 4-(indol-5-ylamino-7-methoxy-5-(1-methylpiperidin-4-yloxy)quinazoline (**example 2.8**) with 2-picolyl chloride in 35% yield; Mass Spectrum MH⁺ 495.

### Example 22.6

### 7-Methoxy-5-(1-methylpiperidin-4-yloxy)-4-(1-(thiazol-4-ylmethyl)indol-5-ylamino)quinazoline

Obtained by reacting 4-(indol-5-ylamino)-7-methoxy-5-(1-methylpiperidin-4-yloxy)quinazoline (**example 2.8**) with 4-(chloromethyl)thiazole in 57% yield; Mass Spectrum MH⁺ 501.

### Example 22.7

### 4-(1-(2,6-Difluorobenzyl)indol-5-ylamino)-7-methoxy-5-(1-methylpiperidin-4-yloxy)quinazoline

Obtained by reacting 4-(indol-5-ylamino)-7-methoxy-5-(1-methylpiperidin-4-yloxy)quinazoline (**example 2.8**) with 2,6-difluorobenzyl chloride in 43% yield; Mass Spectrum MH⁺ 530.

### Illustrative Example 23

The compounds shown in bold in Table 1 were prepared as follows:
Amines (1.2 mM) were dissolved in NMP (1 ml) and 50 µl of each solution transferred to a 96 well plate. Stock solutions of the 4 substrates;
**Substrate A** 4-(3-chloro-4-fluoroanilino)-7-(3-chloropropoxy)-5-(1-methylpiperidin-4-yloxy)quinazoline (**reference example 21.2**) (120mg);
**Substrate B** 7-(2-chloroeuioxy)-4-(3-chloro-4-fluoroanilino)-5-(1-methylpiperidin-4-yloxy)quinazoline (**reference example 21.6**) (116mg);
**Substrate C** 4-(3-chloro-4-fluoroanilino)-7-(3-chloropropoxy)-5-(tetrahydropyran-4-yloxy)quinazoline (**reference example 21.3**) (117mg) and
**Substrate D** 7-(2-chloroethoxy)-4-(3-chloro-4-fluoroanilino)-5-(tetrahydropyran-4-yloxy)quinazoline (**reference example 21.7**) (113 mg) in NMP (1.25 ml) were prepared, and 50 µl aliquots added to each well containing the amine solution shown in Table 1. The plate was heated and agitated at 80°C for 60 hours, allowed to cool, then concentrated *in vacuo.* To each well was added DMSO (550 µl). Aliquots of 50 µl were then taken from each well for LCMS purity determination. LCMS purity was determined on a Phenomenex Synergi column (reverse phase silica, 50 x 2 mm, flow rate 1.1 ml/minute), eluting with acetonitrile-water containing formic acid (0.05%) on a gradient from 5-95% over 4.5 minutes, with UV detection at 254 nm. There was thus obtained the compound shown in bold in Table 1.

**Table 1 In table 1, EG refers to Illustrative Example, RT refers to the LCMS retention time (minutes)**

| **EG** | **Compound** | ***M*-H⁺** | **RT** |
|---|---|---|---|
| 23.1 | **4-(3-Chloro-4-fluoroanilino)-7-[3-(N-(2-hydroxyethyl)-N- methylamino)propoxy]-5-(1-methylpiperidin-4-yloxy)quinazoline** | 517 | 0.84 |
| | Obtained by reacting Substrate A with N-(2-hydroxyethyl)-N-methylamine | | |
| 23.2 | **4-(3-Chloro-4-fluoroanilino)-7-(3-(3-hydroxypyrrolidin-1-yl)propoxy)- 5-(1-methylpiperidin-4-yloxy)quinazoline** | 528 | 0.86 |
| | Obtained by reacting Substrate A with 3-hydroxypyrrolidine | | |
| 23.3 | **4-(3-Chloro-4-fluoroanilino)-7-(3-(4-methylpiperazin-1-yl)propoxy)-5- (1-methylpiperidin-4-yloxy)quinazoline** | 542 | 0.93 |
| | Obtained by reacting Substrate A with 1-methylpiperazine | | |
| 23.4 | **4-(3-Chloro-4-fluoroanilino)-5-(1-methylpiperidin-4-yloxy)-7-(3- piperidinopropoxy)quinazoline** | 527 | 0.96 |
| | Obtained by reacting Substrate A with piperidine | | |
| 23.5 | **4-(3-Chloro-4-fluoroanilino)-7-[3-(N-methyl-N-(1-methylpyrrolidin-3- yl)-amino)propoxy]-5-(1-methylpiperidin-4-yloxy)quinazoline** | 556 | 0.88 |
| | Obtained by reacting Substrate A with *N*-(1-methylpyrrolidin-3-yl)-*N*-methylamine | | |
| 23.6 | **4-(3-Chloro-4-fluoroanilino)-7-(3-(4-(2-methoxyethyl)piperazin-1- yl)propoxy)-5-(1-methylpiperidin-4-yloxy)quinazoline** | 586 | 0.98 |
| | Obtained by reacting Substrate A with 1-(2-methoxyethyl)piperazine. | | |
| 23.7 | **4-(3-Chloro-4-fluoroanilino)-5-(1-methylpiperidin-4-yloxy)-7-(3- pyrrolidin-1-ylpropoxy)quinazoline** | 513 | 0.90 |
| | Obtained by reacting Substrate A with pyrrolidine | | |
| 23.8 | **4-(3-Chloro-4-fluoroanilino)-5-(1-methylpiperidin-4-yloxy)-7-(3- morpholinopropoxy)quinazoline** | 529 | 1.06 |
| | Obtained by reacting Substrate A with morpholine | | |
| 23.9 | **4-(3-Chloro-4-fluoroanilino)-7-(3-homopiperidin-1-ylpropoxy)-5-(1-methylpiperidin-4-yloxy)quinazoline** | 542 (*M*H⁺) | 1.00 |
| | Obtained by reacting Substrate A with homopiperidine | | |
| 23.10 | **4-(3-Chloro-4-fluoroanilino)-7-[3-(N-(2-dimethylaminoethyl)-N-methylamino)propoxy]-5-(1-methylpiperridin-4-yloxy)quinazoline** | 544 | 0.97 |
| | Obtained by reacting Substrate A with N,N,N'-trimethylethylene diamine | | |
| 23.11 | **4-(3-Chloro-4-fluoroanilino)-7-(3-(4-methylhomopiperazin-1-yl)propoxy)-5-(1-methylpiperidin-4-yloxy)quinazoline** | 555 (*M*H⁺) | 0.92 |
| | Obtained by reacting Substrate A with 1-methylhomopiperaizine | | |
| 23.12 | **4-(3-Chloro-4-fluoroanilino)-7-[2-(N-(2-hydroxyethyl)-N-methylamino)ethoxy]-5-(1-methylpiperidin4-yloxy)quinazoline** | 503 | 0.77 |
| | Obtained by reacting Substrate B with N-(2-hydroxyethyl)-N-methylamine | | |
| 23.13 | **4-(3-Chloro-4-fluoroanilino)-7-(2-(3-hydroxypyrrolidin-1-yl)ethoxy)-5-(1-methylpiperidin-4-yloxy)quinazoline** | 515 | 0.76 |
| | Obtained by reacting Substrate B with 3-hydroxypyrrolidine | | |
| 23.14 | **4-(3-Chloro-4-fluoroanilino)-7-(2-(4-methylpiperazin-1-yl)ethoxy)-5-(1-methylpiperidin-4-yloxy)quinazoline** | 528 | 0.84 |
| | Obtained by reacting Substrate B with 1-methylpiperazine | | |
| 23.15 | **4-(3-Chloro-4-fluoroanilino)-5-(1-methylpiperidin-4-yloxy)-7-(2-piperidinoethoxy)quinazoline** | 514 (*M*H⁺) | 1.01 |
| | Obtained by reacting Substrate B with piperidine | | |
| 23.16 | **4-(3-Chloro-4-fluoroanilino)-7-[2-(N-methyl-N-(1-methylpyrrolidin-3-yl)amino)ethoxy]-5-(1-methylpiperidin-4-yloxy)quinazoline** | 544 (*M*H⁺) | 0.90 |
| | Obtained by reacting Substrate B with N-(1-methylpyrrolidin-3-yl)-N-methylamine | | |
| 23.17 | **4-(3-Chloro-4-fluoroanilino)-7-(2-(4-(2-methoxyethyl)piperazin-1-yl)etuoxy)-5-(1-methylpiperidin-4-yloxy)quinazoline** | 574 (*M*H⁺) | 1.06 |
| | Obtained by reacting Substrate B with 1-(2-methoxyethyl)piperazine | | |
| 23.18 | **4-(3-Chloro-4-fluoroanilino)-5-(1-methylpiperidin-4-yloxy)-7-(2-pyrrolidin-1-ylethoxy)quinazoline** | 499 | 0.89 |
| | Obtained by reacting Substrate B with pyrrolidine | | |
| 23.19 | **4-(3-Chloro-4-fluoroanilino)-5-(1-methylpiperidin-4-yloxy)-7-(2-morpholinoethoxy)quinazoline** | 515 | 1.22 |
| | Obtained by reacting Substrate B with morpholine | | |
| 23.20 | **4-(3-Chloro-4-fluoroanilino)-7-(2-homopiperidin-1-ylethoxy)-5-(1-methylpiperidin-4-yloxy)quinazoline** | 527 | 1.03 |
| | Obtained by reacting Substrate B with homopiperidine | | |
| 23.21 | **4-(3-Chloro-4-fluoroanilino)-7-[2-(N-(2-dimethylaminoethyl)-N-methylamino)ethoxy]-5-(1-methylpiperidin-4-yloxy)quinazoline** | 530 | 0.93 |
| | Obtained by reacting Substrate B with N,N,N'-trimethylethylene diamine | | |
| 23.22 | **4-(3-Chloro-4-fluoroanilino)-7-(2-(4-methylhomopiperazin-1-yl)ethoxy)-5-(1-methylpiperidin-4-yloxy)quinazoline** | 544 (*M*H⁺) | 0.96 |
| | Obtained by reacting Substrate B with 1-methylhomopiperazine | | |
| 23.23 | **4-(3-Chloro-4-fluoroanilino)-5-(1-methylpiperidin-4-yloxy)-7-(2-(4-isopropylpiperazin-1-yl)ethoxy)quinazoline** | 556 | 0.99 |
| | Obtained by reacting Substrate B with 1-isopropylpiperazine | | |
| 23.24 | **4-(3-Chloro-4-fluoroanilino)-7-[2-(N-(2-methoxyethyl)-N-methylamino)ethoxy]-5-(1-methylpiperidin-4-yloxy)quinazoline** | 517 | 0.95 |
| | Obtained by reacting Substrate B with N-(2-methoxyethyl)-N-methylamine | | |
| 23.25 | **4-(3-Chloro-4-fluoroanilino)-5-(1-methylpiperidin-4-yloxy)-7-(2-(4-(2-morpholinoethyl)piperazin-1-yl)ethoxy)quinazoline** | 627 | 1.04 |
| | Obtained by reacting Substrate B with 1-(2-morpholino-ethyl)piperazine | | |
| 23.26 | **4-(3-Chloro-4-fluoroanilino)-5-(1-methylpiperidin-4-yloxy)-7-[2-(4-(tetrahydrofuran-2-ylmethyl)piperazin-1-ylethoxyl]quinazoline** | 598 | 1.10 |
| | Obtained by reacting Substrate B with 1-(tetrahydrofuran-2-yl-methyl)piperazine | | |
| 23.27 | **4-(3-Chloro-4-fluoroanilino)-7-(2-(3-dimethylaminopyrrolidin-1-yl)ethoxy)-5-(1-methylpiperidin-4-yloxy)quinazoline** | 542 | 0.98 |
| | Obtained by reacting Substrate B with 3-dimethylaminopyrrolidine | | |
| 23.28 | **4-(3-Chloro-4-fluoroanilino)-5-(1-methylpiperidin-4-yloxy)-7-[2-(4-(1-methylpiperidin-4-yl)piperazin-1-yl)ethoxy]quinazoline** | 611 | 1.08 |
| | Obtained by reacting Substrate B with 1-(1-methylpiperidin-4-yl)piperazine | | |
| 23.29 | **4-(3-Chloro-4-fluoroanilino)-7-[3-(N-(2-ydroxyethyl)-N-methylamino)propoxy]-5-(tetrahydropyran-4-yloxy)quinazoline** | 505 (*M*H⁺) | 1.56 |
| | Obtained by reacting Substrate C with N-(2-hydroxyethyl)-N-methylamine | | |
| 23.30 | **4-(3-Chloro-4-fluoroanilino)-7-(3-(3-hydroxypyrrolidin-1-yl)propoxy)- 5-(tetrahydropyran-4-yloxy)quinazoline** | 516 | 1.54 |
| | Obtained by reacting Substrate C with 3-hydroxypyrrolidine | | |
| 23.31 | **4-(3-Chloro-4-fluoroanilino)-7-(3-(4-methylpiperazin-1-yl)propoxy)-5-(tetrahydropyran-4-yloxy)quinazoline** | 531 (*M*H⁺) | 1.64 |
| | Obtained by reacting Substrate C with 1-methylpiperazine | | |
| 23.32 | **4-(3-Chloro-4-fluoroanilino)-7-(3-piperidinopropoxy)-5-(tetrahydropyran-4-yloxy)quinazoline** | 514 | 1.64 |
| | Obtained by reacting Substrate C with piperidine | | |
| 23.33 | **4-(3-Chloro-4-fluoroanilino)-7-[3-(4-(2-methoxyethyl)piperazin-1-yl)propoxy]-5-(tetrahydropyran-4-yloxy)quinazoline** | 574 (*M*H⁺) | 1.68 |
| | Obtained by reacting Substrate C with 1-(2-methoxyethyl)piperazine | | |
| 23.34 | **4-(3-Chloro-4-fluoroanilino)-7-(3-pyrrolidin-1-ylpropoxy)-5-(tetrahydropyran-4-yloxy)quinazoline** | 500 | 1.59 |
| | Obtained by reacting Substrate C with pyrrolidine | | |
| 23.35 | **4-(3-Chloro-4-fluoroanilino)-7-(3-morpholinopropoxy)-5-(tetrahydropyran-4-yloxy)quinazoline** | 516 | 1.85 |
| | Obtained by reacting Substrate C with morpholine | | |
| 23.36 | **4-(3-Chloro-4-fluoroanilino)-7-[3-(N-(2-dimethylaminoethyl)-N-methylamino)propoxy]-5-(tetrahydropyran-4-yloxy)quinazoline** | 531 | 1.67 |
| | Obtained by reacting Substrate C with 1,1, 2-trimethylethylene diamine | | |
| 23.37 | **4-(3-Chloro-4-fluoroanilino)-7-(3-(4-methylhomopiperazin-1-yl)propoxy)-5-(tetrahydropyran-4-yloxy)quinazoline** | 543 | 1.55 |
| | Obtained by reacting Substrate C with 1-methylhomopiperazine | | |
| 23.38 | **4-(3-Chloro-4-fluoroanilino)-7-(3-(4-isopropylpiperazin-1-yl)propoxy)-5-(tetrahydropyran-4-yloxy)quinazoline** | 557 | 1.66 |
| | Obtained by reacting Substrate C with 1-isopropylpiperazine | | |
| 23.39 | **4-(3-Chloro-4-fluoroanilino)-7-[3-(N-(2-methoxyethyl)-N-methylamino)propoxy]-5-(tetrahydropyran-4-yloxy)quinazoline** | 518 | 1.64 |
| | Obtained by reacting Substrate C with N-(2-methoxyethyl)-N-methylamine | | |
| 23.40 | **4-(3-Chloro-4-fluoroanilino)-7-[3-(4-(2-morpholinoethyl)piperazin-1-yl)propoxy]-5-(tetrahydropyran-4-yloxy)quinazoline** | 628 | 1.40 |
| | Obtained by reacting Substrate C with 1-(2-morpholinoethyl)piperazine | | |
| 23.41 | **4-(3-Chloro-4-fluoroanilino)-7-[3-(4-(tetrahydrofuran-2- ylmethyl)piperazin-1-yl)propoxy]-5-(tetrahydropyran-4- yloxy)quinazoline** | 601 (*M*H⁺) | 1.76 |
| | Obtained by reacting Substrate C with 1-(tetrahydrofuran-2-yl-methyl)piperazine | | |
| 23.42 | **4-(3-Chloro-4-fluoroanilino)-7-(3-(3-dimethylaminopyrrolidin-1- yl)propoxy)-5-(tetrahydropyran-4-yloxy)quinazoline** | 545 (*M*H⁺) | 1.61 |
| | Obtained by reacting Substrate C with 3-dimethylaminopyrrolidine | | |
| 23.43 | **4-(3-Chloro-4-fluoroanilino)-7-[3-(4-(1-methylpiperidin-4-yl)piperazin-1-yl)propoxy]-5-(tetrahydropyran-4-yloxy)quinazoline** | 612 | 1.27 |
| | Obtained by reacting Substrate C with 1-(1-methylpiperidin-4-yl)piperazine | | |
| 23.44 | **4-(3-Chloro-4-fluoroanilino)-7-[2-(N-(2-hydroxyethyl)-N- methylamino)ethoxy]-5-(tetrahydropyran-4-yloxy)quinazoline** | 490 | 1.53 |
| | Obtained by reacting Substrate D with N-(2-hydroxyethyl)-N-methylamine | | |
| 23.45 | **4-(3-Chloro-4-fluoroanilino)-7-(2-(4-methylpiperazin-1-yl)ethoxy)-5- (tetrahydropyran-4-yloxy)quinazoline** | 515 | 1.59 |
| | Obtained by reacting Substrate D with 1-methylpiperazine | | |
| | | | |
| 23.46 | **4-(3-Chloro-4-fluoroanilino)-7-(2-piperidinoethoxy)-5-(tetrahydropyran-4-yloxy)quinazoline** | 500 | 1.64 |
| | Obtained by reacting Substrate D with piperidine | | |
| 23.47 | **4-(3-Chloro-4-fluoroanilino)-7-[2-(N-methyl-N-(1-methylpyrrolidin-3-yl)amino)ethoxy]-5-(tetrahydropyran-4-yloxy)quinazoline** | 529 | 1.54 |
| | Obtained by reacting Substrate D with *N*-(1-methylpyrrolidin-3-yl)-*N*-methylamine | | |
| 23.48 | **4-(3-Chloro-4-fluoroanilino)-7-(2-(4-(2-methoxyethyl)piperazin-1-yl)ethoxy)-5-(tetrahydropyran-4-yloxy)quinazoline** | 559 | 1.66 |
| | Obtained by reacting Substrate D with 1-(2-methoxyethyl)piperazine | | |
| 23.49 | **4-(3-Chloro-4-fluoroanilino)-7-(2-(homopiperidin-1-yl)ethoxy)-5-(tetrahydropyran-4-yloxy)quinazoline** | 514 | 1.69 |
| | Obtained by reacting Substrate D with homopiperidine | | |
| 23.50 | **4-(3-Chloro-4-fluoroanilino)-7-[2-(N-(2-diniethylaminoethyl)-N-methylamino)ethoxy]-5-(tetrahydropyran-4-yloxy)quinazoline** | 517 | 1.60 |
| | Obtained by reacting Substrate D with N,N,N'-trimethylethylene diamine | | |
| 23.51 | **4-(3-Chloro-4-fluoroanilino)-7-(2-(4-methylhomopiperazin-1-yl)ethoxy)-5-(tetrahydropyran-4-yloxy)quinazoline** | 529 | 1.60 |
| | Obtained by reacting Substrate D with 1-methylhomopiperazine | | |
| 23.52 | **4-(3-Chloro-4-fluoroanilino)-7-(2-(4-isopropylpiperazin-1-yl)ethoxy)-5-(tetrahydropyran-4-yloxy)quinazoline** | 543 | 1.61 |
| | Obtained by reacting Substrate D with 1-isopropylpiperazine | | |
| 23.53 | **4-(3-Chloro-4-fluoroanilino)-7-[2-(N-methyl-N-(2-methoxyethyl)amino)ethoxy]-5-(tetrahydropyran-4-yloxy)quinazoline** | 504 | 1.65 |
| | Obtained by reacting Substrate D with N-(2-methoxyethyl)-N-methylamine | | |
| 23.54 | **4-(3-Chloro-4-fluoroanilino)-7-[2-(4-(2-morpholinoethyl)piperazin-1-yl)ethoxy]-5-(tetrahydropyran-4-yloxy)quinazoline** | 616 (*M*H⁺) | 1.48 |
| | Obtained by reacting Substrate D with 1-(2-morpholinoethyl)piperazine | | |
| 23.55 | **4-(3-Chloro-4-fluroanilino)-7-[2-(4-(tetrahydrofuran-2- ylmethyl)piperazin-1-yl)ethoxy]-5-(tetrahydropyran-4- yloxy)quinazoline** | 587 (*M*H⁺) | 1.71 |
| | Obtained by reacting Substrate D with 1-(tetrahydrofuran-2-yl-methyl)piperazine | | |
| 23.56 | **4-(3-Chloro-4-fluoroanilino)-7-[2-(3-dimethylaminopyrrolidin-1-yl)ethoxy]-5-(tetrahydropyran-4-yloxy)quinazoline** | 529 | 1.59 |
| | Obtained by reacting Substrate D with 3-dimethylaminopyrrolidine | | |
| 23.57 | **4-(3-Chloro-4-fluoroanilino)-7-[2-(4-(1-methylpiperidin-4- yl)piperazin-1-yl)ethoxy]-5-(tetrahydropyran-4-yloxy)quinazoline** | 598 | 1.35 |
| | Obtained by reacting Substrate D with 1-(1-methylpiperidin-4-yl)piperazine | | |

### Illustrative Example 24

The compounds shown in bold in Table 2 were prepared as follows:
Amines (1.2 mM) were dissolved in NMP (1 ml) and 50 µl of each solution transferred to a 96 well plate. Stock solutions of the 4 substrates;
**Substrate E** 4-(3-chloro-4-fluoroanilino)-7-(3-chloropropoxy)-5-(tetrahydrofuran-3-yloxy)quinazoline (**reference example 21**) (113mg);
**Substrate F** 7-(2-chloroethoxy)-4-(3-chloro-4-fluoroanilino-5-(tetrahydrofuran-3-yloxy)quinazoline (**reference example 21.4**) (110 mg);
**Substrate G** 4-(3-chloro-4-fluoroanilino)-7-(3-chloropropoxy)-5-cyclopentyloxyquinazoline (**reference example 21.1**) (113mg) and
**Substrate H** 7-(2-chloroethoxy-4-(3-chloro-4-fluoroanilino)-5-cyclopentyloxyquinazoline (**reference example 21.5**) (109mg) in NMP (1.25 ml) were prepared, and 50 µl aliquots added to each well containing the amine solution shown in Table 2. The plate was heated and agitated at 80°C for 60 hours, allowed to cool, then concentrated *in vacuo.* To each well was added DMSO (550 µl). Aliquots of 50 µl were then taken from each well for LCMS purity determination. LCMS purity was determined on a Phenomenex Synergi column (reverse phase silica, 50 x 2 mm, flow rate 1.1 ml/minute), eluting with acetonitrile-water containing formic acid (0.05%) on a gradient from 5-95% over 4.5 minutes, with UV detection at 254 nm. There was thus obtained the compounds shown in bold in Table 2.

**Table 2 In Table 2 EG refers to Illustrative Example, RT refers to the LCMS retention time (minutes)**

| ***EG*** | **Compound** | ***M*-H⁺** | **RT** |
|---|---|---|---|
| 24.1 | **4-(3-Chloro-4-fluoroanilino)-7-[3-(N-(2-hydroxyethyl)-N-methylamino)propoxy]-5-(tetrahydrofuran-3-yloxy)quinazoline** | 490 | 1.09 |
| | Obtained by reacting Substrate E with N-(2-hydroxyethyl)-N-methylamine | | |
| 24.2 | **4-(3-Chloro-4-fluoroanilino)-7-(3-(3-hydroxypyrrolidin-1-yl)propoxy)-5-(tetrahydrofuran-3-yloxy)quinazoline** | 502 | 1.14 |
| | Obtained by reacting Substrate E with 3-hydroxypyrrolidine | | |
| 24.3 | **4-(3-Chloro-4-fluoroanilino)-7-(3-(4-methylpiperazin-1-yl)propoxy)-5-(tetrahydrofuran-3-yloxy)quinazoline** | 515 | 1.08 |
| | Obtained by reacting Substrate E with 1-methylpiperazine | | |
| 24.4 | **4-(3-Chloro-4-fluoroanilino)-7-(3-piperidinopropoxy)-5-(tetrahydrofuran-3-yloxy)quinazoline** | 500 | 1.20 |
| | Obtained by reacting Substrate E with piperidine | | |
| 24.5 | **4-(3-Chloro-4-fluoroanilino)-7-[3-(4-(2-methoxyethyl)piperazin-1-yl)propoxy]-5-(tetrahydrofuran-3-yloxy)quinazoline** | 559 | 1.13 |
| | Obtained by reacting Substrate E with 1-(2-methoxyethyl)piperazine | | |
| 24.6 | **4-(3-Chloro-4-fluoroanilino)-7-(3-pyrrolidin-1-ylpropoxy)-5-(tetrahydrofuran-3-yloxy)quinazoline** | 486 | 1.19 |
| | Obtained by reacting Substrate E with pyrrolidine | | |
| 24.7 | **4-(3-Chloro-4-fluoroanilino)-7-(3-morpholinopropoxy)-5-(tetrahydrofuran-3-yloxy)quinazoline** | 502 | 1.14 |
| | Obtained by reacting Substrate E with morpholine | | |
| 24.8 | **4-(3-Chloro-4-fluoroanilino)-7-(3-homopiperidin-1-ylpropoxy)-5-(tetrahydrofuran-3-yloxy)quinazoline** | 514 | 1.26 |
| | Obtained by reacting Substrate E with homopiperidine | | |
| 24.9 | **4-(3-Chloro-4-fluoroanilino)-7-[3-(N-(2-dimethylaminoethyl)-N-methylamino)propoxy]-5-(tetrahydrofuran-3-yloxy)quinazoline** | 517 | 0.94 |
| | Obtained by reacting Substrate E with N,N,N'-trimethylethylene diamine | | |
| 24.10 | **4-(3-Chloro-4-fluoroanilino)-7-(3-(4-methylhomopiperazin-1-yl)propoxy)-5-(tetrahydrofuran-3-yloxy)quinazoline** | 529 | 0.91 |
| | Obtained by reacting Substrate E with 1-methylhomopiperazine | | |
| 24.11 | **4-(3-Chloro-4-fluoroanilino)-7-(3-(4-isopropylpiperazin-1-yl)propoxy)-5-(tetrahydrofuran-3-yloxy)quinazoline** | 543 | 1.12 |
| | Obtained by reacting Substrate E with 1-isopropylpiperazine | | |
| 24.12 | **4-(3-Chloro-4-fluoroanilino)-7-[3-(N-(2-methoxyethyl)-N-methylamino)propoxy]-5-(tetrahydrofuran-3-yloxy)quinazoline** | 504 | 1.19 |
| | Obtained by reacting Substrate E with N-(2-methoxyethyl)-N-methylamine | | |
| 24.13 | **4-(3-Chloro-4-fluoroanilino)-7-[3-(4-(2-morpholinoethyl)piperazin-1-yl)propoxy]-5-(tetrahydrofuran-3-yloxy)quinazoline** | 614 | 0.93 |
| | Obtained by reacting Substrate E with 1-(2-morpholinoethyl)piperazine | | |
| 24.14 | **4-(3-Chloro-4-fluoroanilino)-7-[3-(4-(tetrahydrofuran-2-ylmethyl)piperazin-1-yl)propoxy]-5-(tetrahydrofuran-3-yloxy)quinazoline** | 585 | 1.18 |
| | Obtained by reacting Substrate E with 1-(tetrahydrofuran-2-yl-methyl)piperazine | | |
| 24.15 | **4-(3-Chloro-4-fluoroanilino)-7-[3-(3-dimethylaminopyrrolidin-1-yl)propoxy]-5-(tetrahydrofuran-3-yloxy)quinazoline** | 529 | 0.94 |
| | Obtained by reacting Substrate E with 3-dimethylaminopyrrolidine | | |
| 24.16 | **4-(3-Chloro-4-fluoroanilino)-7-[3-(4-(1-methylpiperidin-4-yl)piperazin-1-yl)propoxy]-5-(tetrahydrofuran-3-yloxy)quinazoline** | 598 | 0.92 |
| | Obtained by reacting Substrate E with 1-(1-methylpiperidin-4-yl)piperazine | | |
| 24.17 | **4-(3-Chloro-4-fluoroanilino)-7-[2-(N-(2-hydroxyethyl)-N-methylamino)ethoxy]-5-(tetrahydrofuran-3-yloxy)quinazoline** | 477 (*M*H⁺) | 1.12 |
| | Obtained by reacting Substrate F with N-(2-hydroxyethyl)-N-methylamine | | |
| 24.18 | **4-(3-Chloro-4-fluoroanilino)-7-[2-(3-hydroxypyrrolidin-1-yl)ethoxy]-5-(tetrahydrofuran-3-yloxy)quinazoline** | 488 | 1.12 |
| | Obtained by reacting Substrate F with 3-hydroxypyrrolidine | | |
| 24.19 | **4-(3-Chloro-4-fluoroanilino)-7-(2-(4-methylpiperazin-1-yl)ethoxy)-5-(tetrahydrofuran-3-yloxy)quinazoline** | 501 | 1.09 |
| | Obtained by reacting Substrate F with 1-methylpiperazine | | |
| 24.20 | **4-(3-Chloro-4-fluoroanilino)-7-(2-piperidinoethoxy)-5-(tetrahydrofuran-3-yloxy)quinazoline** | 486 | 1.19 |
| | Obtained by reacting Substrate F with piperidine | | |
| 24.21 | **4-(3-Chloro-4-fluoroanilino)-7-[2-(4-(2-methoxyethyl)piperazin-1-yl)ethoxy]-5-(tetrahydrofuran-3-yloxy)quinazoline** | 545 | 1.15 |
| | Obtained by reacting Substrate F with 1-(2-methoxyethyl)piperazine | | |
| 24.22 | **4-(3-Chloro-4-fluoroanilino)-7-(2-pyrrolidin-1-ylethoxy)-5-(tetrahydrofuran-3-yloxy)quinazoline** | 472 | 1.15 |
| | Obtained by reacting Substrate F with pyrrolidine | | |
| 24.23 | **4-(3-Chloro-4-fluoroanilino)-7-(2-morpholinoethoxy)-5-(tetrahydrofuran-3-yloxy)quinazoline** | 488 | 1.15 |
| | Obtained by reacting Substrate F with morpholine | | |
| | | | |
| 24.24 | **4-(3-Chloro-4-fluoroanilino)-7-(2-homopiperidin-1-ylethoxy)-5-(tetrahydrofuran-3-yloxy)quinazoline** | 499 | 1.28 |
| | Obtained by reacting Substrate F with homopiperidine | | |
| 24.25 | **4-(3-Chloro-4-fluoroanilino)-7-(2-(4-methylhomopiperazin-1-yl)ethoxy)-5-(tetrahydrofuran-3-yloxy)quinazonne** | 515 | 0.98 |
| | Obtained by reacting Substrate F with 1-methylhomopiperazine | | |
| 24.26 | **4-(3-Chloro-4-fluoroanilino)-7-(2-(4-isopropylpiperazin-1-yl)ethoxy)-5-(tetrahydrofuran-3-yloxy)quinazoline** | 529 | 1.15 |
| | Obtained by reacting Substrate F with 1-isopropylpiperazine | | |
| 24.27 | **4-(3-Chloro-4-fluoroanilino)-5-cyclopentyloxy-7-(3-pyrrolidin-1-ylpropoxy)quinazoline** | 484 | 1.45 |
| | Obtained by reacting Substrate G with pyrrolidine | | |
| 24.28 | **4-(3-Chloro-4-fluoroanilino)-5-cyclopentyloxy-7-(3-morpholinopropoxy)quinazoline** | 500 | 1.42 |
| | Obtained by reacting Substrate G with morpholine | | |
| 24.29 | **4-(3-Chloro-4-fluoroanilino)-5-cyclopentyloxy-7-(3-homopiperidin-1-ylpropoxy)quinazoline** | 512 | 1.54 |
| | Obtained by reacting Substrate G with homopiperidine | | |
| 24.30 | **4-(3-Chloro-4-fluoroanilino)-5-cyclopentyloxy-7-(3-(4-methylhomopiperazin-1-yl)propoxy)quinazoline** | 527 | 1.17 |
| | Obtained by reacting Substrate G with 1-methylhomopiperazine | | |
| 24.31 | **4-(3-Chloro-4-fluoroanilino)-5-cyclopentyloxy-7-[3-(4-isopropylpiperazin-1-yl)propoxy]quinazoline** | 541 | 1.39 |
| | Obtained by reacting Substrate G with 1-isopropylpiperazine | | |
| 24.32 | **4-(3-Chloro-4-fluoroanilino)-5-cyclopentyloxy-7-[3-(N-(2-methoxyethyl)-N-methylamino)propoxy]quinazoline** | 502 | 1.47 |
| | Obtained by reacting Substrate G with N-(2-methoxyethyl)-N-methylamine | | |
| | | | |
| 24.33 | **4-(3-Chloro-4-fluoroanilino)-5-cyclopentyloxy-7-[3-(4-(2-morpholinoethyl)piperazin-1-yl)propoxy]quinazoline** | 612 | 1.16 |
| | Obtained by reacting Substrate G with 1-(2-morpholinoethyl)piperazine | | |
| 24.34 | **4-(3-Chloro-4-fluoroanilino)-5-cyclopentyloxy-7-[3-(4- --(tetrahydrofuran-2-ylmethyl)piperazin-l-yl)propoxy]quinazoline** | 583 | 1.45 |
| | Obtained by reacting Substrate G with 1-(tetrahydrofuran-2-yl-methyl)piperazine | | |
| 24.35 | **4-(3-Chloro-4-fluoroanilino)-5-cyclopentyloxy-7-[3-(3-dimethylaminopyrrolidin-1-yl)propoxy]quinazoline** | 527 | 1.18 |
| | Obtained by reacting Substrate G with 3-dimethylaminopyrrolidine | | |
| 24.36 | **4-(3-Chloro-4-fluoroanilino)-5-cyclopentyloxy-7-[3-(4-(1-methylpiperidin-4-yl)piperazin-1-yl)propoxy]quinazoline** | 596 | 1.16 |
| | Obtained by reacting Substrate G with 1-(1-methylpiperidin-4-yl)piperazine | | |
| 24.37 | **4-(3-Chloro-4-fluoroanilino)-5-cyclopentyloxy-7-[2-(N-(2-hydroxyethyl)-N-methylamino)ethoxy]quinazoline** | 474 | 1.35 |
| | Obtained by reacting Substrate H with N-(2-hydroxyethyl)-N-methylamine | | |
| 24.38 | **4-(3-Chloro-4-fluoroanilino)-5-cyclopentyloxy-7-[2-(3-hydroxypyrrolidin-1-yl)ethoxylquinazoline** | 486 | 1.37 |
| | Obtained by reacting Substrate H with 3-hydroxypyrrolidine | | |
| 24.39 | **4-(3-Chloro-4-fluoroanilino)-5-cyclopentyloxy-7-(2-(4-methylpiperazin-1-yl)ethoxy)quinazoline** | 499 | 1.37 |
| | Obtained by reacting Substrate H with 1-methylpiperazine | | |
| 24.40 | **4-(3-Chloro-4-fluoroanilino)-5-cyclopentyloxy-7-(2-piperidinoethoxy)quinazoline** | 484 | 1.47 |
| | Obtained by reacting Substrate H with piperidine | | |
| 24.41 | **4-(3-Chloro-4-fluoroanilino)-5-cyclopentyloxy-7-[2-(4-(2-methoxyethyl)piperazin-1-yl)ethoxylquinazofine** | 543 | 1.40 |
| | Obtained by reacting Substrate H with 1-(2-methoxyethyl)piperazine | | |
| 24.42 | **4-(3-Chloro4-fluoroanflino)-5-cyclopentyloxy-7-(2-homopiperidin-1-ylethoxy)quinazoline** | 498 | 1.52 |
| | Obtained by reacting Substrate H with homopiperidine | | |
| 24.43 | **4-(3-Chloro-4-fluoroanilino)-5-cyclopentyloxy-7-[2-(4-methylhomopiperazin-1-yl)ethoxy]quinazoline _{.}** | 513 | 1.21 |
| | Obtained by reacting Substrate H with 1-methylhomopiperazine | | |
| 24.44 | **4-(3-Chloro-4-fluoroanilino)-5-cyclopentyloxy-7-[2-(4-(2-morpholinoethyl)piperazin-1-yl)ethoxylquinazoline** | 598 | 1.14 |
| | Obtained by reacting Substrate H with 1-(2-morpholinoethyl)piperazine | | |
| 24.45 | **4-(3-Chloro-4-fluoroanilino)-5-cyclopentyloxy-7-[2-(4-(tetrahydrofuran-2-ylmethyl)piperazin-1-yl)ethoxy]quinazoline** | 569 | 1.45 |
| | Obtained by reacting Substrate H with 1-(tetrahydrofuran-2-yl-methyl)piperazine | | |
| 24.46 | **4-(3-Chloro-4-fluoroanilino)-5-cyclopentyloxy-7-[2-(3-dimethylaminopyrrolidin-1-yl)ethoxy]quinnohne** | 513 | 1.20 |
| | Obtained by reacting Substrate H with 3-dimethylaminopyrrolidine | | |
| 24.47 | **4-(3-Chloro-4-fluoroanilino)-5-cyclopentyloxy-7-[2-(4-(1-methylpiperidin-4-yl)piperazin-1-yl)ethoxiy]quinazoline** | 582 | 1.13 |
| | Obtained by reacting Substrate H with 1-(1-methylpiperidin-4-yl)piperazine | | |

### Example 25

### Pharmaceutical composition

The following illustrates a representative pharmaceutical dosage form of the invention as defined herein (the active ingredient being termed "Compound X"), for therapeutic or prophylactic use in humans:

| (a) | Tablet I | mg/tablet |
|---|---|---|
| | Compound X | 100 |
| | Lactose Ph.Eur | 182.75 |
| | Croscarmellose sodium | 12.0 |
| | Maize starch paste (5% w/v paste) | 2.25 |
| | Magnesium stearate | 3.0 |
| | | |

| (b) | Injection I | (50 mg/ml) |
|---|---|---|
| | Compound X | 5.0% w/v |
| | 1M Sodium hydroxide solution | 15.0% v/v |
| | 0.1M Hydrochloric acid (to adjust pH to 7.6) | |
| | Polyethylene glycol 400 | 4.5% w/v |
| | Water for injection to 100%. | |

The above formulations may be obtained by conventional procedures well known in the pharmaceutical art. For example the tablet may be prepared by blending the components together and compressing the mixture into a tablet.

### Starting Materials

### Reference Example 1

### 4, 6 -Difluoroisatin

A solution of hydroxylamine hydrochloride (41.7 g) in water (100 ml) was added dropwise to a solution of chloral hydrate (31.6 g) and sodium sulphate (228.3 g) in water (50 ml) at 60°C. The resulting solution was then added to a solution of 3, 5-difluoroaniline (25 g) in water (300 ml) and concentrated HCl (16 ml) at 80°C, and the mixture heated at 95°C for 15 minutes. The resulting white solid was filtered and washed with water. This solid was added in portions to concentrated H₂SO₄ (167 ml) at 60 - 80°C, to give a deep red solution which was stirred for an additional 15 minutes. The solution was poured into ice-water and the resulting orange solid filtered, washed with water, and dried *in vacuo* to yield the title compound (24.64 g, 69%); NMR spectrum (DMSO-d6) 6.58 (dd, 1H), 6.85 (dt, 1H), 11.36 (bs, 1H); Mass spectrum M-H⁺ 182.

### Reference Example 2

### 4,6-Dibenzyloxyisatin

3,5-Dibenzyloxyaniline hydrochloride (**reference example 24**) (32.33 g) was added cautiously to oxalyl chloride (100 ml) and the solution heated at reflux for 3 hours. The solution was cooled and concentrated *in vacuo.* Methanol (100 ml) was added to the residue and the mixture heated at reflux for 1 hour. The reaction was allowed to cool, and the resulting precipitate filtered and washed with methanol to give the title compound as a yellow solid (16.22 g, 48%); NMR spectrum (DMSO-d6) 5.22 (s, 2H), 5.24 (s, 2H), 6.10 (s, 1H), 6.38 (s, 1H), 7.30-7.50 (m, 10H), 10.90 (bs, 1H); Mass spectrum M-H⁺ 358.

The procedure described above was repeated using the appropriate aniline hydrochloride. Thus was obtained the compound described below:

### Reference Example 2.1

### 4,6-Dimethoxyisatin

Obtained from 3,5-dimethoxyaniline hydrochloride; NMR spectrum (DMSO-d6) 3.83 (s, 3H), 3.86 (s, 3H), 6.00 (d, 1H), 6.17 (d, 1H), 10.86 (bs, 1H).

### Reference Example 3

### 2-Amino-4,6-difluorobenzoic acid

4,6-Difluoroisatin **(reference example 1)** (10 g) was dissolved in 33 % (w/v) aqueous NaOH (85 ml) at 75°C. To this solution was added H₂O₂ (30%, 16 ml) dropwise over 30 minutes. The reaction was stirred for an hour at 75°C, then cooled to room temperature. Ice was added, and the reaction mixture acidified to pH 1 with concentrated HCl. The resulting precipitate was filtered, washed with water and dried *in vacuo* to give the title compound as a pale yellow solid (6.28 g, 66%) Mass spectrum M⁺ 173.

The procedure described above was repeated using the appropriate isatin. Thus were obtained the compounds described below:

### Reference Example 3.1

### 2-Amino-4,6-dibenzyloxybenzoic acid

Obtained from 4,6-dibenzyloxyisatin **(reference example 2)** in 87% yield; NMR spectrum (DMSO-d6) 4.97 (s, 2H), 5.05 (s, 2H), 5.92 (d, 1H), 5.97 (d, 1H), 7.20 - 7.50 (m, 10H).

### Reference Example 3.2

### 2-Amino-4,6-dimethoxybenzoic acid

Obtained from 4, 6-dimethoxyisatin **(reference example 2.1)** in 63% yield; NMR spectrum (DMSO-d6) 3.69 (s, 3H), 3.75 (s, 3H), 5.77 (d, 1H), 5.92 (d, 1H); Mass spectrum MH⁺ 198.

### Reference Example 4

### Methyl 2-amino-4,6-difluorobenzoate

Dimethyl sulphate (11.76 ml) was added dropwise to a mixture of potassium carbonate (37.8 g) and 2-amino-4,6-difluorobenzoic acid **(reference example 3)** (21.56 g) in DMF (500 ml) at 0°C. The reaction was stirred for 1 hour, then poured into water. The resulting precipitate was filtered, washed with water and dried *in vacuo* to give the title compound as a beige solid (9.39 g, 40%). The filtrate was extracted with ethyl acetate, and combined organic extracts dried and concentrated *in vacuo* to yield more of the title compound as a yellow crystalline solid (6.57 g, 28%); NMR spectrum (DMSO-d6) 3.78 (s, 3M, 6.25 (m, 1H), 6.38 (m, 1H), 6.90 (bs, 2H).

The procedure described above was repeated using the appropriate acid. Thus were obtained the compounds described below:

### Reference Example 4.1

### Methyl 2-amino-4,6-dibenzyloxybenzoate

Obtained from 2-amino-4,6-dibenzyloxybenzoic acid **(reference example 3.1)** in 81% yield; NMR spectrum (DMSO-d6) 3.72 (s, 3H), 5.02 (s, 2H), 5.07 (s, 2H), 5.96 (s, 1H), 6.03 (s, 1H), 6.20 (bs, 2H), 7.22 - 7.48 (m, 10H); Mass spectrum MH⁺ 364.

### Reference Example 4.2

### Methyl 2-amino-4,6-dimethoxybenzoate

Obtained from 2-amino-4,6-dimethoxybenzoic acid **(reference example 3.2)** in 77% yield; NMR spectrum (DMSO-d6) 3.66 (s, 3H), 3.67 (s, 3H), 3.68 (s, 3H), 5.75 (d, 1H), 5.90 (d, 1H), 6.13 (s, 2H).

### Reference Example 5

### 5,7-Difluoro-3,4-dihydroquinazolin-4-one

A solution of methyl 2-amino-4,6-difluorobenzoate **(reference example 4)** (15.96 g) and formamidine acetate (19.58 g) in 2-methoxyethanol (200 ml) was heated at 120°C for 16 hours. The reaction was cooled, concentrated *in vacuo,* and the residue triturated with methanol to give the title compound as a beige solid (8.09 g, 52%); NMR spectrum (DMSO-d6) 7.20 - 7.40 (m, 2H), 8.10 (s, 1H), 12.35 (bs, 1H); Mass spectrum M-H⁺ 181.

The procedure described above was repeated using the appropriate anthranilic ester. Thus were obtained the compounds described below:

### Reference Example 5.1

### 5. 7-Dibenzyloxy-3,4-dihydroquinazolin-4-one

Obtained from methyl 2-amino-4,6-dibenzyloxybenzoate **(reference example 4.1)** in 64% yield; NMR spectrum (DMSO-d6) 5.20 (s, 4H), 6.72 (d, 1H), 6.78 (d, 1H), 7.20 - 7.60 (m, 10H), 7.92 (s, 1H), 11.70 (bs, 1H); Mass spectrum M-H⁺ 357.

### Reference Example 5.2

### 3,4-Dihydro-5,7-dimethoxyquinazolin-4-one

Obtained from methyl 2-amino-4,6-dimethoxybenzoate **(reference example 4.2)** in 88% yield; NMR spectrum (DMSO-d6) 3.80 (s, 3H), 3.84 (s, 3H), 6.51 (d, 1H), 6.63 (d, 1H), 7.88 (s, 1H), 11.62 (bs, 1H); Mass spectrum MH⁺ 207.

### Reference Example 6

### 5.Benzyloxy-3,4-dihydro-7-fluoroquinazolin-4-one

Sodium hydride (0.88 g, 60% dispersion in mineral oil) was added portionwise over 5 minutes to benzyl alcohol (1.71 ml) in DMF (30 ml) at 0°C. The reaction was stirred at 0°C for 10 minutes then 5,7-difluoro-3,4-dihydroquinazolin-4-one **(reference example 5)** (2.00 g) was added in portions over 5 minutes. The resulting solution was allowed to warm to room temperature and stirred for 12 hours. The reaction mixture was concentrated *in vacuo,* water (10 ml) added and then extracted with ethyl acetate (100 ml). A solid precipitated from the organic layer and this was filtered and dried *in vacuo* to afford the title compound as white needles (1.00 g, 34%). The aqueous layer was extracted with ethyl acetate (3 x 100ml), dried, filtered and concentrated *in vacuo* to afford more of the title compound (0.64 g, 22%); NMR spectrum (DMSO-d6) 5.23 (s, 2H), 6.90 (dd, 1H), 7.00 (dd, 1H), 7.30 (t, 1H), 7.36 (t, 2H), 7.58 (d, 2H), 8.00 (s, 1H), 11.96 (bs, 1H); Mass spectrum MH⁺ 271.

The procedure described above was repeated using the appropriate alcohol. Thus was obtained the compound described below:

### Reference Example 6.1

### 7-Fluoro-5-(tetrahydropyran-4-yloxy)-3,4-dihydroquinazolin-4-one

Obtained from 5, 7-difluoro-3,4-dihydroquinazoline **(reference example 5)** and tetrahydropyran-4-ol in 38% yield; NMR spectrum (CDCl₃) 1.92 (m, 2H), 2.08 (m, 2H), 3.64 (m, 2H), 4.10 (m, 2H), 4.70 (m, 1H), 6.67 (dd, 1H), 7.00 (dd, 1H), 8.00 (s, 1H); Mass spectrum MH⁺ 265.

### Reference Example 7

### 5-(1-Methylpiperidin-4-yloxy)- 3,4-dihydroquinazolin-4-one

Sodium hydride (4.1 g, 60%) was added in portions to 4-hydroxy-1-methylpiperidine (10.7 g) in DMA (125 ml). The reaction was stirred at room temperature for 15 minutes, 50°C for 15 minutes then allowed to cool to room temperature. 5-Fluoro-3,4-dihydroquinazolin-4-one (5.1 g) was added in a single portion, and the mixture heated at 80°C for 2 hours. The reaction was cooled, concentrated *in vacuo* and the residue purified by chromatography using DCM - 7N ammonia in methanol (9:1) as eluent to give the title compound as a white solid after trituration with ether (7.3 g, 91%); NMR spectrum (DMSO-d6) 1.72 (m, 2H), 1.88 (m, 2H), 2.15 (s, 3H), 2.19 (m, 2H), 2.63 (m, 2H), 4.46 (m, 1H), 7.00 (d, 1H), 7.14 (d, 1H), 7.61 (t, 1H), 7.91 (s, 1H), 11.75 (bs, 1H).

The procedure described above was repeated using the appropriate alcohol. Thus was obtained the compound described below:

### Reference Example 7_{.}1

### 5-(1-tert-Butoxycarbonylpiperidin-4-yloxy)-3,4-dihydroquinazolin-4-one

Obtained from 1-*tert*-butoxycarbonyl-4-hydroxypiperidine in 87% yield; NMR spectrum (DMSO-d6) 1.39 (s, 9H), 1.6 - 1.87 (m, 4H), 3.32 - 3.43. (m, 2H), 3.47 - 3.60 (m, 2H), 4.75 (m, 1H), 7.08 (d, 1H), 7.17 (d, 1H), 7.64 (t, 1H) 8.84 (s, 1H), 11.80 (bs, 1H); Mass spectrum MH⁺ 346.

### Reference Example 8

### 5-Hydroxy-7-fluoro-3,4-dihydroquinazolin-4-one trifluoroacetate

Trifluoroacetic acid (50 ml) was added to 5-benzyloxy-7-fluoro-3,4-dihydroquinazolin-4-one **(reference example 6)** (1.64 g) and the resulting pale yellow solution was heated at 70°C for 2 hours. The reaction mixture was concentrated *in vacuo* to give an oil. Diethyl ether was added to give a solid which was filtered to afford the title compound as a pink solid (820 mg, 75%); NMR spectrum (DMSO-d6) 6.72 (dd, 1H), 7.86 (dd, 1H), 8.12 (s, 1H), 12.13 (bs, 1H); Mass spectrum MH⁺ 181.

### Reference Example 9

### 7-Benzyloxy-3,4-dihydro-5-hydroxyquinazolin-4-one,

Magnesium bromide (4.3 g) was added cautiously to 5, 7-dibenzyloxy-3,4-dihydroquinazolin-4-one **(reference example 5.1)** (8.37 g) in pyridine (250 ml) and the solution heated at reflux for 1 hour. The reaction mixture was cooled, concentrated *in vacuo* and the residue triturated with water and filtered to yield the title compound as an off-white solid (6.2 g, 99%); Mass spectrum MH⁺ 269.

The procedure described above was repeated using the appropriate 5-alkoxyquinazoline. Thus was obtained the compound described below:

### Reference Example 9.1

### 3,4-Dihydro-5-hydroxy-7-methoxyquinazolin-4-one

Obtained from 3,4-dihydro-5,7-dimethoxyquinazolin-4-one **(reference example 5.2)** in 93% yield; Mass spectrum MH⁺ 193.

### Reference Example 10

### 4-(3-Chloro-4-fluoroanilino)-5-hydroxy-7-methoxyguinazoline

Pyridine hydrochloride (1.08 g) was added to 4-(3-chloro-4-fluoroanilino)-5,7-dimethoxyquinazoline **(reference example 19.1)** (3.29 g) suspended in pyridine (50 ml). The reaction was heated at 115°C for 8 hours then allowed to cool to room temperature. The precipitate formed upon cooling was filtered and washed with water before drying under suction to afford the title compound as a yellow solid (2.21 g, 70%); NMR spectrum (DMSO-d6) 3.8 (s, 3H), 6.4 (d, 2H), 7.4 (t, 1H), 7.6 (m, 1H), 8.0 (d, 1H), 8.5 (s, 1H); Mass spectrum MH⁺ 320.

### Reference Example 11

### 3,4-Dihydro-5-hydroxy-7-(3-(R)-dimethylaminopyrrolidin-1-yl)quinazolin-4-one

3-(R)-(+)-Dimethylaminopyrrolidine (490 µl) was added to 3,4-dihydro-5-hydroxy-7-fluoroquinazolin-4-one trifluoroacetate **(reference example 8)** (400 mg) suspended in NMP (400 µl). The resulting solution was heated at 100°C for 3 hours. The reaction mixture was concentrated *in vacuo* to give a brown oil. Methanol (500 µl) was added and the suspension filtered to afford the title compound as a pink solid (243 mg, 41%); NMR spectrum (DMSO-d6) 1.80 (m, 1H), 2.18 (s, 6H), 2.78 (m, 1H), 3.05 (dd, 1H), 3.24 (m , 2H), 3.47 (m, 2H), 6.00 (d, 1H), 6.10 (d, 1H), 7.88 (s, 1H), 11.85 (bs, 2H); Mass spectrum MH⁺ 273.

The procedure described above was repeated using the appropriate 7-fluoroquinazoline and amine. Thus was obtained the compound described below:

### Reference Example 11.1

### 3,4-Dihydro-7-(3-(S)-dimethylaminopyrrolidin-1-yl)-5-(tetrahydropyran-4-yloxy)quinazolin-4-one

Obtained from 3,4-dihydro-7-fluoro-5-(tetrahydropyranyl-4-oxy)quinazolin-4-one **(reference example 6.1)** and 3-(S)-dimethylaminopyrrolidine in 74% yield; NMR spectrum (DMSO-d6) 1.66 (m, 2H), 1.90 (m, 2H), 2.20 (s, 6H), 2.77 (m, 1H), 3.07 (t, 1H), 3.26 (m, 3H), 3.40 - 3.58 (m, 4H), 3.90 (m, 2H), 4.65 (m, 1H), 6.20 (s, 2H), 7.75 (s, 1H); Mass spectrum MH⁺ 359.

### Reference Example 12

### 7-(3-(R)-Dimethylaminopyrrolidin-1-yl)-5-hydroxy-3-pivaloyloxymethyl-3,4-dihydro quinazolin-4-one

Sodium hydride (40 mg) was added portionwise over 5 minutes to 3,4-dihydro-5-hydroxy-7-(3-(*R*)-dimethylaminopyrrolidin-1-yl)quinazolin-4-one **(reference example 11)** (0.24 g) in DMF (5 ml) at 0°C. Chloromethyl pivalate (130 µl) was added dropwise over 15 minutes to give a clear orange solution. The reaction mixture was allowed to warm to room temperature and stirred for a further 18 hours. Incomplete reaction was seen by tlc, therefore reaction was cooled to 0°C and sodium hydride (10 mg) was added followed by chloromethyl pivalate (26 µl). Reaction was complete after stirring for 1 hour at room temperature. The reaction mixture was concentrated *in vacuo* and purified by chromatography using 2-10% methanol in DCM as eluent to afford the title compound as a cream solid (210 mg, 62%); NMR spectrum (DMSO-d6) 1.10 (s, 9H), 1.83 (m, 1H), 2.22 (s, 6H), 2.81 (m, 1H), 3:13 (m, 1H), 3.33 (m , 2H), 3.45 - 3.60 (m, 2H), 5.80 (s, 2H), 6.08 (d, 1H), 6.18 (d, 1H), 8.21 (s, 1H), 11.39 (s, 1H); Mass spectrum MH⁺ 389.

The procedure described above was repeated using the appropriate 3,4-dihydroquinazolin-4-one. Thus were obtained the compounds described below:

### Reference Example 12.1

### 5-Hydroxy-7-methoxy-3-pivaloyloxymethyl-guinazolin-4-one

Obtained from 3,4-dihydro-5-hydroxy-7-methoxyquinazolin-4-one **(reference example 9.1)** in 67% yield; NMR spectrum (DMSO-d6) 1.11 (s, 9H), 3.85 (s, 3H), 5.86 (s, 2H), 6.51 (d, 1H), 6.66 (d, 1H), 8.37 (s, 1H), 11.42 (s, 1H); Mass spectrum M-H⁺ 305.

### Reference Example 12.2

### 7-Benzyloxy-5-hydroxy-3-pivaloyloxymethyl-3,4-dihydroguinazolin-4-one

Obtained from 7-benzyloxy-3,4-dihydro-5-hydroxyquinazolin-4-one **(reference example 9)** in 93% yield; NMR spectrum (DMSO-d6) 1.11 (s, 9H), 5.23 (s, 2H), 5.86 (s, 2H), 6.59 (d, 1H), 6.74 (d, 1H), 7.29 - 7.47 (m, 5H), 8.37 (s, 1H), 11.42 (s, 1H); Mass spectrum M-H⁺ 383.

### Reference Example 13

### 7-(3-(R)-Dimethylaminopyrrolidin-1-yl)-5-(1-methylpiperidin-4-yloxy)-3-pivaloyloxymethyl-3,4-dihydroquinazolin-4-one

7-(3-(R)-Dimethylaminopyrrolidin-1-yl)-5-hydroxy-3-pivaloyloxymethyl-3,4-dihydro quinazolin-4-one **(reference example 12)** (210 mg), 4-hydroxy-N-methylpiperidine (125 mg) and triphenylphosphine (280 mg) were dissolved in anhydrous DCM (10 ml), under a nitrogen atmosphere at 0°C. A solution of di-tert-butyl azodicarboxylate (250 mg) in DCM (1 ml) was added dropwise over 5 minutes and the resulting yellow solution was allowed to warm to room temperature and stirred for 18 hours. A further 1 equivalent of all reagents was added in the same sequence as above under the same reaction conditions and was left to stir for a further 12 hours at room temperature. The reaction mixture was concentrated *in vacuo* and the residue purified by chromatography using 2-8% methanol in DCM as eluent to afford the title compound as a cream solid (200 mg, 77%); Mass spectrum MH⁺ 486.

The procedure described above was repeated using the appropriate 5-hydroxyquinazoline and alcohol. Thus were obtained the compounds described below:

### Reference Example 13.1

### 7-Methoxy-3-pivaloyloxymethyl-5-(tetrahydrofuran-3-yloxy)-3,4-dihydroquinazolin-4-one

Obtained from 5-hydroxy-7-methoxy-3-pivaloyloxymethyl-3,4-dihydroquinazolin-4-one (reference example 12.1) and tetrahydrofuran-3-ol in 80% yield; Mass spectrum MH⁺ 377.

### Reference Example 13.2

### 7-Methoxy-3-pivaloyloxymethyl-5-(tetrahydropyran-4-yloxy)-3,4-dihydroguinazolin-4-one

Obtained from 5-hydroxy-7-methoxy-3-pivaloyloxymethyl-3,4-dihydroquinazolin-4-one **(reference example 12.1)** and tetrahydropyran-4-ol in 70% yield; NMR spectrum (DMSO-d6) 1.11 (s, 9H), 1.66 (m, 2H), 1.92 (m, 2H), 3.49 (m, 2H), 3.85 (s, 3H), 3.89 (m, 2H), 4.76 (m, 1H), 5.81 (s, 2H), 6.68 (s, 2H), 8.30 (s, 1H).

### Reference Example 13.3

### 7-Benzyloxy-3-pivaloyloxymethyl-5-(tetrahydropyran-4-yloxy)-3,4-dihydroquinazolin-4-one

Obtained from 7-benzyloxy-5-hydroxy-3-pivaloyloxymethyl-3,4-dihydroquinazolin-4-one **(reference example 12.2)** and tetrahydropyran-4-ol in 80% yield; Mass spectrum MH⁺ 467.

### Reference Example 13.4

### Benzyloxy-5-(1-methylpiperidin-4-yloxy)-3-piyaloyloxymethyl-3,4-dihydroquinazolin-4-one

Obtained from 7-benzyloxy-5-hydroxy-3-pivaloyloxymethyl-3,4-dihydroquinazolin-4-one **(reference example 12.2)** and 1-methylpiperidin-4-ol in 100% yield; Mass spectrum MH⁺ 480.

### Reference Example 13.5

### 7-Methoxy-5-(1-methylniperidin-4-yloxy)-3-pivaloyloxymethyl-3,4-dihydroquinazolin-4-one

Obtained from 5-hydroxy-7-methoxy-3-pivaloyloxymethyl-3,4-dihydroquinazolin-4.-one **(reference example 12.1)** and 1-methylpiperidin-4-ol in 56% yield; NMR spectrum (DMSO-d6) 1.11 (s, 9H), 1.71 (m, 2H), 1.87 (m, 2H), 2.13 (s, 3H), 2.18 (m, 2H), 2.57 (m, 2H), 3.84 (s, 3H), 4.52 (m, 1H), 5.79 (s, 2H), 6.61 (d, 1H), 6.67 (d, 1H), 8.16 (s, 1H); Mass spectrum MH⁺ 405.

### Reference Example 13.6

### 7-Benzyloxy-3-pivaloyloxymethyl-5-(tetrahydrofuran-3-yloxy)-3,4-dihydroquinazolin-4-one

Obtained from 7-benzyloxy-5-hydroxy-3-pivaloyloxymethyl-3,4-dihydroquinazolin-4-one **(reference example 12.2)** tetrahydrofuran-3-ol in 83% yield; Mass spectrum MH⁺ 454.

### Reference Example 13.7

### 7-Benzyloxy-5-cyclopentyloxy-3-pivaloyloxymethyl-3,4-dihydroquinazolin-4-one

Obtained from 7-benzyloxy-5-hydroxy-3-pivaloyloxymethyl-3,4-dihydroquinazolin-4-one (reference example 12.2) and cyclopentanol in 88% yield; Mass spectrum MH⁺ 451.

### Reference Example 14

### 3,4-Dihydro-7-(3-(R)-dimethylaminopyrrolidin-1-yl)-5-(1-methylpiperidin-4-yloxy)quinazolin-4-one

7N Ammonia in methanol (20 ml) was added to 7-(3-(R)-dimethylaminopyrrolidin-1-yl)-5-(1-methylpiperidin-4-yloxy)-3-pivaloyloxymethyl-3,4-dihydroquinazolin-4-one **(reference example 13)** (200 mg) and the solution stirred at room temperature for 18 hours. The reaction mixture was concentrated *in vacuo* to give an oil which was triturated with diethyl ether to give an orange solid which was filtered to afford the title compound (100 mg, 66%); NMR spectrum (DMSO-d6) 1.72 (m, 3H), 1.87 (m, 3H), 2.10 (m, 3H), 2.15 (s, 3H), 2.18 (s, 6H), 2.63 (m, 2H), 2.75 (m, 1H), 3.05 (dd, 1H), 3.26 (m, 1H), 3.30 - 3.50 (m, 2H), 4.35 (m, 1H), 6.08 (s, 1H), 6.12 (s, 1H), 7.67 (s, 1H), 11.07 (bs, 1H); Mass spectrum MH⁺" 370.

The procedure described above was repeated using the appropriate 3-pivaloyloxymethylquinazolone. Thus were obtained the compounds described below:

### Reference Example 14.1

### 3,4-Dihydro-7-methoxy-5-(tetrahydrofuran-3-yloxy)quinazolin-4-one

Obtained from 7-methoxy-3-pivaloyloxymethyl-5-(tetrahydrofuran-3-yloxy) -3,4-dihydroquinazolin-4-one **(reference example 13.1)** in 87% yield; Mass spectrum MH⁺ 263.

### Reference Example 14.2

### 3,4-Dihydro-7-methoxy-5-(tetrahydropyran-4-yloxy)quinazolin-4-one

Obtained from 7-methoxy-3-pivaloyloxymethyl-5-(tetrahydropyran-4-yloxy)-3,4-dihydroquinazolin-4-one **(reference example 13.2)** in 91% yield; NMR spectrum (DMSO-d6) 1.65 (m, 2H), 1.91 (m, 2H), 3.48 (m, 2H), 3.83 (s, 3H), 3.89 (m, 2H), 4.70 (m, 1H), 6.60 (d, 2H), 6.65 (d, 2H), 7.88 (s, 1H), 12.12 (bs, 1H); Mass spectrum M-H⁺ 275.

### Reference Example 14.3

### 7-Benzyloxy-3,4-dihydro-5-(tetrahydropyran-4-yloxy)quinazolin-4-one

Obtained from 7-benzyloxy-3-pivaloyloxymethyl-5-(tetrahydropyran-4-yloxy) -3,4-dihydroquinazolin-4-one **(reference example 13.3)** in 76% yield; Mass spectrum MH⁺ 263.

### Reference Example 14.4

### 7-Benzyloxy-3,4-dihydro-5-(1-methylpiperidin-4-yloxy)guinazolin-4-one

Obtained from 7-benzyloxy-5-(1-methylpiperidin-4-yloxy)-3-pivaloyloxymethyl-3,4-dihydroquinazolin-4-one **(reference example 13.4)** in 48% yield; Mass spectrum MH⁺ 366.

### Reference Example 14.5

### 3,4-Dihydro-7-methoxy-5-(1-methylpiperidin-4-yloxy)quinazolin-4-one

Obtained from 7-methoxy-5-(1-methylpiperidin-4-yloxy)-3-pivaloyloxymethyl-3,4-dihydroquinazolin-4-one **(reference example 13.5)** in 75% yield; NMR spectrum (DMSO-d6) 1.68 (m, 2H), 1.84 (m, 2H), 2.11 (s, 3H), 2.18 (m, 2H), 2.61 (m, 2H), 3.82 (s, 3H), 4.45 (m, 1H), 6.53 (d, 2H), 6.64 (d, 2H), 7.86 (s, 1H), 11.60 (bs, 1H); Mass spectrum MH⁺ 290.

### Reference Example 14.6

### 7-Benzyloxy-3,4-dihydro-5-(tetrahydrofuran-3-yloxy)quinazolin-4-one

Obtained from 7-benzyloxy-3-pivaloyloxymethyl-5-(tetrahydrofuran-3-yloxy) -3,4-dihydroquinazolin-4-one **(reference example 13.6)** in 86% yield; NMR spectrum (DMSO-d6) 2.00 (m, 1H), 2.17 (m, 1H), 3.81 (m, 4H), 5.05 (m, 1H), 5.21 (s, 2H), 6.54 (d, 1H), 6.75 (d, 1H), 7.40 (m, 5H), 7.87 (s, 1H), 11.67 (bs, 1H); Mass spectrum MH⁺ 339.

### Reference Example 14.7

### 7-Benzyloxy-5-cyclopentyloxy-3,4-dihydroquinazolin-4-one

Obtained from 7-benzyloxy-5-cyclopentyloxy-3-pivaloyloxymethyl-3,4-dihydroquinazolone **(reference example 13.7)** in 88% yield; Mass spectrum MH⁺ 337.

### Reference Example 15

### 5-(1-tert-Butoxycarbonylpiperidin-4-yloxy)-3,4-dihydro-7-methoxyquinazolin-4-one

Di-tert-butylazodicarboxylate (915 mg) was added to a stirred solution of 5-hydroxy-7-methoxy-3-pivaloyloxymethyl-3,4-dihydroquinazolin-4-one-**(reference example 12.1)** (800 mg), 1-tert-butoxycarbonyl-4-hydroxypiperidine (631 mg), and triphenylphosphine (11.02 g) in dry DCM (13 ml), under an atmosphere of nitrogen. External cooling (ice bath) was applied during the addition. The reaction was then stirred for ten minutes, after which it was allowed to warm to room temperature. After 2 hours, the mixture was concentrated *in vacuo* to give the crude material as an orange oil. A solution of ammonia in methanol (7N) was added to this crude mixture, to give an orange solution, which was stirred at room temperature for 24 hours. The mixture was then concentrated *in vacuo,* and the residue purified by column chromatography, using 0-10% methanol in DCM, to give the title compound as white foam that solidified on drying overnight (892 mg, 91 %); NMR spectrum (CDCl₃) 1.47 (s, 9H), 1.93 (m, 4 H), 3.54 (m, 2H) 3.70 (m, 2H), 3.90 (s, 3H), 4.66 (m, 1H), 6.50 (d, 1H), 6.77 (d, 1H), 7.89 (s, 1H), 10.32 (s, 1H); Mass spectrum M-H⁺ 374.

### Reference Example 16

### 4-Chloro-7-(3-(R)-dimethylaminopyrrolidin-1-yl)-5-(1-methylpiperidin-4-yloxy)quinazoline

Phosphorus oxychloride (1.4 ml) was added to a solution of 3,4-dihydro-7-(3-(R)-dimethylaminopyrrolidin-1-yl)-5-(1-methylpiperidin-4-yloxy)quinazolin-4-one **(reference example 14)** (1.75 g) and di-isopropylethylamine (6.3 ml) in 1, 2-dichloroethane (100 ml), and the resulting solution heated at reflux for 3 hours. The reaction was cooled and concentrated *in vacuo* and the residue purified by chromatography using DCM-methanol-triethylamine (8:1:1) as eluent. The resulting solid was triturated with DCM and filtered. The filtrate was evaporated to yield the title compound as a yellow solid (1.5 g, 81%); Mass spectrum M⁺ 390.

The procedure described above was repeated using the appropriate 3,4-dihydroquinazolin-4-one. Thus was obtained the compound described below:

### Reference Example 16.1

### 4-Chloro-5-(1-methylpiperidin-4-yloxy)quinazoline

Obtained from 3,4-dihydro-5-(1-methylpiperidin-4-yloxy)quinazolin-4-one **(reference example 7)** in 66% yield; NMR spectrum (CDCl₃) 2.10 (m, 2H), 2.23 (m, 2H), 2.42 (s, 3H), 2.60 (m, 2H), 2.84 (m, 2H), 4.73 (m, 1H), 7.04 (d, 1H), 7.62 (d, 1H), 7.81 (t, 1H), 8.93 (s, 1H): Mass spectrum M⁺ 278.

### Reference Example 16.2

### 4-Chloro-7-methoxy-5-(1-methylpiperidin-4-yloxy)quinazoline

Obtained from 3,4-dihydro-7-methoxy-5-(1-methylpiperidin-4-yloxy)quinazolin-4-one **(reference example 14.5)** in 99% yield; NMR spectrum (CDCl₃) 2.10 (m, 4H), 2.35 (s, 3H), 2.44 (m, 2H), 2.74 (m, 2H), 3.95 (s, 3H), 4.58 (s, 1H), 6.60 (d, 1H), 6.94 (d, 1H), 8.80 (s, 1H); Mass spectrum MH⁺ 308.

### Reference Example 16.3

### 5-(1-tert-Butoxycarbonylpiperidin-4-yloxy)-4-chloroquinazoline

Obtained from 5-(1-tert butoxycarbonylpiperidin-4-yloxy)-3,4-dihydroquinazoline **(reference example 7.1)** in 66% yield; NMR spectrum (DMSO-d6) 1.38 (s, 9H), 1.58 - 1.90 (m, 4H), 3.30 - 3.60 (m, 4H), 4.82 (m, 1H), 7.14 - 7.28 (m, 2H), 7.74 (t, 1H), 8.33 (s, 1H).

### Reference Example 17

### 4-Chloro-5-fluoroquinazoline hydrochloride

To a suspension of 3,4-dihydro-5-fluoroquinazolin-4-one (0.5 g) in thionyl chloride (5 ml) was added DMF (0.2 ml). The mixture was heated at reflux under an atmosphere of nitrogen for 3 hours. The mixture was evaporated *in vacuo,* the residue re-suspended in dry toluene, and evaporated again. The residue was dried *in vacuo* to give the title compound as a pale yellow solid (634 mg, 95%), which was used without further manipulation.

### Reference Example 18

### 4-(3-Chloro-4-fluoroanilino)-5-fluoroquinazoline hydrochloride

DMF (1 ml) was added dropwise to 5-fluoro-3,4-dihydroquinazolin-4-one (1.00 g) in thionyl chloride (10 ml). The reaction was heated at 110°C for 18 hours to afford an orange solution. The reaction mixture was concentrated *in vacuo* to give an orange solid. This solid was added portionwise to a flask containing ice (100 g) and saturated aqueous sodium hydrogen carbonate solution (50 ml), maintaining the internal temperature < 5 °C and checking the solution remained basic. The aqueous mixture was then extracted with DCM (3 x 100 ml), organic extracts were combined, dried (MgSO₄), and concentrated *in vacuo* to afford an orange solid. 3-Chloro-4-fluoroaniline (0.88 g) and 1N HCl in diethyl ether (6.09 ml) were added to the orange solid suspended in IPA (50 ml). The resulting mixture was heated at 100°C for 90 minutes then allowed to cool to room temperature. The solid was filtered and washed with IPA (5 ml), then diethyl ether (20 ml) to afford the title compound, as a beige solid (1.37 g, 69%); Mass spectrum MH⁺292.

### Reference Example 19

### 7-Benzyloxy-4-(3-chloro-4-fluoroanilino)-5-(tetrahydropyran-4-yloxy)quinazoline

Di-isopropylethylamine (2.27 ml) was added to 7-benzyloxy-3,4-dihydro-5-(tetrahydropyran-4-yloxy)quinazolin-4-one **(reference example 14.3)** (638 mg) dissolved in anhydrous 1,2-dichloroethane (30 ml) and the resulting solution cooled to 0°C in an ice bath. Phosphorous oxychloride (0.51 ml) was added dropwise and the reaction heated at reflux for 3 hours. The reaction mixture was concentrated *in vacuo* to give an orange oil. 3-Chloro-4-fluoroaniline (99 mg) was added to this oil dissolved in IPA (15 ml), followed by di-isopropylethylamine (0.16 ml). The resulting mixture was heated at reflux for 1.5 hours. The reaction mixture was cooled to room temperature, and the resulting solid filtered, washed with IPA, then diethyl ether and dried *in vacuo* to afford the title compound as a green solid (0.577 g, 67%); Mass spectrum MH⁺ 480.

The procedure described above was repeated using the appropriate 3,4-dihydroquinazolin-4-one and aniline. Thus was obtained the compound described below:

### Reference Example 19.1

### 4-(3-Chloro-4-fluoroanilino)-5,7-dimethoxyquinazoline

Obtained from 3,4-dihydro-5,7-dimethoxyquinazolin-4-one **(reference example 5.2)** and 3-chloro-4-fluoroaniline in 92% yield; NMR spectrum (DMSO-d6) 4.0 (s, 3H), 4.1 (s, 3H), 6.9-7.0 (dd, 2H), 7.5-7.6 (m, 2H), 7.9 (dd, 1H), 8.7 (s, 1H); Mass spectrum M-H⁺334.

### Reference Example 19.2

### 7-Benzyloxy-4-(3-bromoanilino)-5-(1-methylpiperidin-4-yloxy)quinazoline

Obtained from 7-benzyloxy-3,4-dihydro-5-(1-methylpiperidin-4-yloxy)quinazolin-4-one **(reference example 14.4)** and 3-bromoaniline in 38% yield; Mass spectrum MH⁺ 521.

### Reference Example 19.3

### 7-Benzyloxy-4-(3-chloro-4-fluoroanilino)-5-(tetrahydrofuran-3-yloxy)quinazoline

Obtained from 7-benzyloxy-3,4-dihydro-5-(tetrahydrofuran-3-yloxy)quinazolin-4-one (reference example 14.6) and 3-chloro-4-fluoroaniline in 34% yield; Mass spectrum MH⁺ 466.

### Reference Example 19.4

### 7-Benzyloxy-4-(3-chloro-4-fluoroanilino)-5-cyclopentyloxyquinazoline

Obtained from 7-benzyloxy-5-cyclopentyloxy-3,4-dihydroquinazolin-4-one **(reference example 14.7)** and 3-chloro-4-fluoroaniline in 47% yield; NMR spectrum (DMSO-d6) 1.72 (m, 4H), 2.02 (m, 4H), 5.29 (m, 1H), 5.32 (s, 2H), 7.01 (d, 1H), 7.07 (d, 1H), 7.39 (m, 3H) 7.53 (m, 4H), 8.06 (m, 1H), 8.81 (s, 1H), 10.42 (bs, 1H); Mass spectrum MH⁺ 464.

### Reference Example 19.5

### 7-Benzyloxy-4-(3-chloro-4-fluoroanilino)-5-(1-methylniperidin-4-yloxy)quinazoline

Obtained from 7-benzyloxy-3,4-dihydro-5-(1-methylpiperidin-4-yloxy)quinazolin-4-one **(reference example 14.4)** and 3-chloro-4-fluoroaniline in 21% yield; NMR spectrum (DMSO-d6) 2.3 (m, 2H), 2.4 (m, 1H), 2.7 (d, 3H), 3.2 (m, 2H), 3.3 (m, 1H), 3.5 (m, 2H), 5.1 (m, 1H), 5.3 (s, 2H), 7.1 (s, 2H), 7.4 (m, 3H), 7.5 (m, 3H), 7.6 (m, 1H), 8.0 (m, 1H), 8.8 (d, 1H); Mass spectrum MH⁺ 493.

### Reference example 19.6

### 7-Benzyloxy-4-(3-methylanilino)-5-(1-methylpiperidin-4-yloxy)quinazoline

Obtained from 7-benzyloxy-3,4-dihydro-5-(1-methylpiperidin-4-yloxy)quinazolin-4-one **(reference example 14.4)** and 3-methylaniline in 32% yield; NMR spectrum (DMSO-d6) 2.2 (m, 2H), 2.4 - 2.5 (m, 6H), 2.7 (m, 2H), 3.1 (m, 2H), 3.5 (m, 2H), 5.1 (m, 1H), 5.3 (s, 2H), 7.1- 7.2 (m, 2H), 7.3 - 7.6 (m, 8H), 8.0 (m, 1H), 8.8 (m, 1H); Mass spectrum MH⁺455.

### Reference example 19.7

### 7-Benzyloxy-4-(3-chloroanilino)-5-(1-methylpiperidin-4-yloxy)quinazoline

Obtained from 7-benzyloxy-3,4-dihydro-5-(1-methylpiperidin-4-yloxy)quinazolin-4-one **(reference example 14.4)** and 3-chloroaniline in 26% yield; NMR spectrum (DMSO-d6) 2.2 (m, 2H), 2.3 - 2.4 (m, 5H), 2.7 (m, 2H), 3.1 (m, 2H), 3.4 (m, 2H), 5.1 (m, 1H), 5.3 (s, 2H), 7.0 - 7.2 (m, 3H), 7.3 - 7.6 (m, 8H), 8.8 (m, 1H); Mass spectrum MH⁺ 475.

### Reference Example 19.8

### 7-Benzyloxy-4-(3-chloro-4-(3-fluorobenzyloxy)anilino)-5-cyclopentyloxyquinazoline

Obtained from 7-benzyloxy-5-cyclopentyl-3,4-dihydroquinazolin-4-one **(reference example 14.7)** and 3-chloro-4-(3-fluorobenzyloxy)aniline **(reference example 28)** in 62% yield; Mass spectrum MH⁺ 570.

### Reference Example 19.9

### 7-Benzyloxy-4-(3-chloro-4-(3-fluorobenzyloxy)anilino)-5-(1-methylpiperidin-4-yloxy)quinazoline

Obtained by reacting 7-benzyloxy-3,4-dihydro-5-(1-methylpiperidin-4-yloxy)quinazolin-4-one **(reference example 14.4)** and 3-chloro-4-(3-fluorobenzyloxy)aniline (reference example 28) in 96% yield; Mass spectrum MH⁺ 599.

### Reference Example 19.10

### 7-Benzyloxy-4-(3-chloro-4-(3-fluorobenzyloxy)anilino)-5-(tetrahydronyran-4-yloxy)quinazoline

Obtained by reacting 7-benzyloxy-3,4-dihydro-5-(tetrahydropyran-4-yloxy)quinazolin-4-one **(reference example 14.3)** with 3-chloro-4-(3-fluorobenzyloxy)aniline **(reference example 28)** in 70% yield; Mass spectrum MH⁺585.

### Reference Example 19.11

### 7-Benzyloxy-4-(3-chloro-4-(3-fluorobenzyloxy)anilino)-5-(tetrahydrofuran-3-yloxy)quinazoline

Obtained by reacting 7-benzyloxy-3,4-dihydro-5-(tetrahydrofuran-3-yloxy)quinazolin-4-one **(reference example 14.3)** with 3-chloro-4-(3-fluorobenzyloxy)aniline **(reference example 28)** in 70% yield; NMR spectrum (DMSO-d6) 2.2 (m, 1H), 2.3 (m, 1H), 3.8 - 4.0 (m, 3H), 4.2 (d, 1H), 5.2 (s, 2H), 5.2 (s, 2H), 5.5 (m, 1H), 6.9 (d, 1H), 6.9 (d, 1H), 7.1- 7.5 (m, 11H), 8.2 (d, 1H), 8.5 (s, 1H), 9.8 (s, 1H); Mass spectrum MH⁺ 572.

### Reference Example 20

### 4-(3-Chloro-4-fluoroanillno)-7-hydroxy-5-(tetrahydropyran-4-yloxy)quinazoline trifluoroacetate

A mixture of 7-benzyloxy-4-(3-chloro-4-fluoroanilino)-5-(tetrahydropyran-4-yloxy)quinazoline **(reference example 19)** (0.58 g) and trifluoroacetic acid (25 ml) was heated at 70°C for 20 hours. The reaction mixture was cooled, concentrated *in vacuo,* and the residue triturated with diethyl ether to give the title compound as a pale green solid (0.49 g, 82%); NMR spectrum (DMSO-d6) 1.94 (m, 2H), 2.15 (m, 2H), 3.53 (t, 2H), 3.89 (m, 2H), 4.96 (m, 1H), 6.81 (s, 1H), 6.92 (s, 1H), 7.50 (t, 1H), 7.57 (m, 1H). 8.09 (dd, 1H), 8.68 (s, 1H), 10.31 (s, 1H); Mass spectrum MH⁺ 390.

The procedure described above was repeated using the appropriate 7-benzyloxyquinazoline. Thus were obtained the compounds described below:

### Reference Example 20.1

### 4-(3-Bromoanilino)-7-hydroxy-5-(1-methylpiperidin-4-yloxy)quinazoline trifluoroacetate

Obtained from 7-benzyloxy-4-(3-bromoanilino)-5-(1-methylpipendin-4-yloxy)quinazoline **(reference example 19.2)** in 93% yield; Mass spectrum MH⁺ 431.

### Reference Example 20.2

### 4-(3-Chloro-4-fluoroanilino)-7-hydroxy-5-(tetrahydrofuran-3-yloxy)quinazoline trifluoroacetate

Obtained from 7-benzyloxy-4-(3-chloro-4-fluoroanilino)-5-(tetrahydrofuran-3-yloxy)quinazoline **(reference example 19.3)** in 79% yield; Mass spectrum MH⁺ 376.

### Reference Example 20.3

### 4-(3-Chloro-4-fluoroanilino)-5-cyclopentyloxy-7-hydroxyquinazoline trifluoroacetate

Obtained from 7-benzyloxy-4-(3-chloro-4-fluoroanilino)-5-cyclopentyloxyquinazoline **(reference example 19.4)** in 60% yield; NMR spectrum (DMSO-d6) 1.71 (m, 4H), 2.04 (m, 4H), 5.18 (m, 1H), 6.71 (d, 1H), 6.78 (d, 1H), 7.53 (m; 2H), 8.07 (m, 1H), 8.73 (s, 1H), 10.33 (bs, 1H); Mass spectrum MH⁺ 374.

### Reference Example 20.4

### 4-(3-Chloro-4-fluoroanilino)-7-hydroxy-5-(1-methylpiperidin-4-yloxy)quinazoline trifluoroacetate

Obtained from 7-benzyloxy-4-(3-chloro-4-fluoroanilino)-5-(1-methylpiperidin-4-yloxy)quinazoline **(reference example 19.5)** in 93% yield; Mass spectrum MH⁺ 403.

### Reference Example 20.5

### 7-Hydroxy-4-(3-methylanilino)-5-(1-methylpiperidin-4-yloxy)quinazoline trifluoroacetate

Obtained from 7-benzyloxy-4-(3-methylanilino)-5-(1-methylpiperidin-4-yloxy)quinazoline **(reference example 19.6)** in 80% yield; Mass spectrum M-H⁺363.

### Reference Example 20.6

### 4-(3-Chloroanilino)-7-hydroxy-5-(1-methylpiperidin-4-yloxy)quinazoline trifluoroacetate

Obtained from 7-benzyloxy-4-(3-chloroanilino)-5-(1-methylpiperidin-4-yloxy)quinazoline **(reference example 19.7)** in 100% yield; Mass spectrum M-H⁺383.

### Reference Example 20.7

### 4-(3-Chloro-4-(3-fluorobenzyloxy)anilino)-5-cyclopentyloxy-7-hydroxyquinazoline trifluoroacetate

Obtained from 7-benzyloxy-4-(3-chloro-4-(3-fluorobenzyloxy)anilino)-5-cyclopentyloxyquinazoline **(reference example 19.8)** in 43% yield; Mass spectrum MH⁺ 480.

### Reference Example 20.8

### 4-(3-Chloro-4-(3-fluorobenzyloxy)anilino)-7-hydroxy-5-(1-methylpiperidin-4-yloxy)quinazoline trifluoroacetate

Obtained from 4-(3-chloro-4-(3-fluorobenzyloxy)anilino)-5-(1-methylpiperidin-4-yloxy)-7-benzyloxyquinazoline **(reference example 19.9)** in 27% yield; Mass spectrum MH⁺ 509.

### Reference Example 20.9

### 4-(3-Chloro-4-(3-fluorobenzyloxy)anilino)-7-hydroxy-5-(tetrahydropyran-4-yloxy)quinazoline trifluoroacetate

Obtained from 7-benzyloxy-4-(3-chloro-4-(3-fluorobenzyloxy)anilino)-5-(tetrahydropyran-4-yloxy)quinazoline **(reference example 19.10)** in 30% yield; Mass spectrum MH⁺ 496.

### Reference Example 20.10

### 4-(3-Chloro-4-(3-fluorobenzyloxy)anilino)-7-hydroxy-5-(tetrahydrofuran-3-yloxy)quinazoline trifluoroacetate

Obtained from 7-benzyloxy-4-(3-chloro-4-(3-fluorobenzyloxy)anilino)-5-(tetrahydrofuran-3-yloxy)quinazoline **(reference example 19.11)** in > 100% yield; Mass spectrum MH⁺ 482.

### Reference Example 21

### 4-(3-Chloro-4-fluoroanilino)-7-(3-chloropropoxy)-5-(tetrahydrofuran-3-yloxy)quinazoline

Potassium carbonate (0.21 g) and 1-bromo-3-chloropropane (40 µl) were added to a suspension of 4-(3-chloro-4-fluoroanilino)-7-hydroxy-5-(tetrahydrofuran-3-yloxy)quinazoline trifluoroacetate **(reference example 20.2)** (0.19 g) in DMF (4 ml). The mixture was stirred at room temperature for 23 hours, then more 1-bromo-3-chloropropane (19 µl) was added and the mixture was stirred at room temperature for a further 18 hours. The mixture was then concentrated *in vacuo,* the residue was cooled and cold water was added. The resulting solid was filtered, washed with cold water and dried *in vacuo* to give the title compound as a green solid (0.15g, 88%); NMR spectrum (DMSO-d6) 2.22 (m, 4H), 3.85 (m, 5H), 4.22 (m, 3H), 5.46 (m, 1H), 6.80 (d, 1H), 6.83 (d, 1H), 7.42 (t, 1H), 7.59 (m, 1H), 8.27 (m, 1H), 8.49 (s, 1H), 9.91 (s, 1H); Mass spectrum MH⁺ 452.

The procedure described above was repeated using the appropriate 7-hydroxyquinazoline and alkyl halide. Thus were obtained the compounds described below:

### Reference Example 21.1

### 4-(3-Chloro-4-fluoroanilino)-7-(3-chloropropoxy)-5-cyclopentyloxyquinazoline

Obtained from 4-(3-chloro-4-fluoroanilino)-5-cyclopentyloxy-7-hydroxyquinazoline trifluoroacetate **(reference example 20.3)** and 1-bromo-3-chloropropane in 99% yield; NMR spectrum (DMSO-d6) 1.72 (m, 4H), 2.01 (m, 4H), 2.22 (m, 2H), 3.81 (t, 2H), 4.23 (t, 2H), 5.17 (m, 1H), 6.70 (m, 1H), 6.79 (m, 1H), 7.44 (m, 2H), 8.25 (m, 1H), 8.47 (s, 1H), 9.88 (s, 1H); Mass spectrum MH⁺ 450.

### Reference Example 21.2

### 4-(3-Chloro-4-fluoroanilino)-7-(3-chloropropoxy)-5-(1-methylpiperidin-4-yloxy)quinazoline

Obtained from 4-(3-chloro-4-fluoroanilino)-7-hydroxy-5-(1-methylpiperidin-4-yloxy)quinazoline trifluoroacetate **(reference example 20.4)** and 1-bromo-3-chloropropane in 66% yield; Mass spectrum MH⁺ 479.

### Reference Example 21.3

### 4-(3-Chloro-4-fluoroanilino)-7-(3-chloropropoxy)-5-(tetrahydropyran-4-yloxy)quinazoline

Obtained from 4-(3-chloro-4-fluoroanilino)-7-hydroxy-5-(tetrahydropyran-4-yloxy)quinazoline trifluoroacetate **(reference example 20)** and 1-bromo-3-chloropropane in 77% yield; Mass spectrum MH⁺ 467.

### Reference Example 21.4

### 4-(3-Chloro-4-fluoroanilino)-7-(2-chloroethoxy)-5-(tetrahyrofuran-3-yloxy)quinazoline

Obtained from 4-(3-chloro-4-fluoroanilino)-7-hydroxy-5-(tetrahydrofuran-3-yloxy)quinazoline trifluoroacetate **(reference example 20.2)** and 1-bromo-2-chloroethane in 90% yield; NMR spectrum (DMSO-d6) 2.17 (m, 1H), 2.32 (m, 1H), 3.78 - 4.01 (m, 5H), 4.18 (d, 1H), 4.42 (t, 2H), 5.50 (m, 1H), 6.84 (m, 2H), 7.42 (t, 1H), 7.61 (m, 1H), 8.28 (m, 1H), 8.50 (s, 1H), 9.92 (s, 1H); Mass spectrum MH⁺ 438.

### Reference Example 21.5

### 7-(2-Chloroethoxy)-4-(3-chloro-4-fluoroanilino)-5-cyclopentyloxyquinazoline

Obtained from 4-(3-chloro-4-fluoroanilino)-5-cyclopentyloxy-7-hydroxyquinazoline trifluoroacetate **(reference example 20.3)** and 1-bromo-2-chloroethane in 75% yield; NMR spectrum (DMSO-d6) 1.72 (m, 4H), 2.01 (m, 4H), 3.99 (m, 2H), 4.41 (m, 2H), 5.21 (m, 1H), 6.72 (m, 1H), 6.81 (m, 1H), 7.45 (m, 2H), 8.25 (m, 1H), 8.48 (s, 1H), 9.88 (s, 1H); Mass spectrum MH⁺ 436.

### Reference Example 21.6

### 7-(2-Chloroethoxy)-4-(3-chloro-4-fluoroanilino)-5-(1-methylpiperidin-4-yloxy)quinazoline

Obtained from 4-(3-chloro-4-fluoroanilino)-7-hydroxy-5-(1-methylpiperidin-4-yloxy)quinazoline trifluoroacetate (**reference example 20.4**) and 1-bromo-2-chloroethane in 53% yield; Mass spectrum MH⁺ 465.

### Reference Example 21.7

### 7-(2-Chloroethoxy)-4-(3-chloro-4-fluoroanilino)-5-(tetrahydropyran-4-yloxy)quinazoline

Obtained from 4-(3-Chloro-4-fluoroanilino)-7-hydroxy-5-(tetrahydropyran-4-yloxy)quinazoline trifluoroacetate **(reference example 20)** and 1-bromo-2-chloroethane in 85% yield; Mass spectrum MH⁺ 452.

### Reference Example 21.8

### 4-(3-Chloro-4-(3-fluorobenzyloxy)anilino)-7-(3-chloropropoxy)-5-cyclopentyloxyquinazoline

Obtained from 4-(3-chloro-4-(3-fluorobenzyloxy)anilino)-5-cylopentyloxy-7-hydroxyquinazoline trifluoroacetate **(reference example 20.7)** and 1-bromo-3-chloropropane in 100% yield; NMR spectrum (DMSO-d6) 1.60 - 1.82 (m,4H), 1.90 - 2.15 (m, 4H), 2.22 (m, 2H), 3.82 (t, 2H), 4.23 (t, 2H), 5.18 (m, 1H), 5.23 (s, 2H), 6.68 (d, 1H), 6.78 (d, 1H), 7.17 (m, 1H), 7.25 (m, 3H), 7.43 (m, 2H), 8.13 (d, 1H), 8.42 (s, 1H), 9.80 (s, 1H); Mass spectrum MH⁺ 556.

### Reference Example 21.9

### 7-(2-Chloroethoxy)-4-(3-chloro-4-(3-fluorobenzyloxy)anilino)-5-cyclopentyloxyquinazoline

Obtained from 4-(3-chloro-4-(3-fluorobenzyloxy)anilino)-5-cyclopentyloxy-7-hydroxyquinazoline trifluoroacetate **(reference example 20.7)** and 1-bromo-2-chloroethane in 100% yield; Mass spectrum MH⁺ 542.

### Reference Example 21.10

### 4-(3-Chloro-4-(3-fluorobenzyloxy)anilino)-7-(3-chloropropoxy)-5-(1-methylpiperidin-4-yloxy)quinazoline

Obtained from 4-(3-chloro-4-(3-fluorobenzyloxy)anilino)-7-hydroxy-5-(1-methylpiperidin-4-yloxy)quinazoline **(reference example 20.8)** and 1-bromo-3-chloropropane in 78% yield; Mass spectrum MH⁺ 585.

### Reference Example 21.11

### 7-(2-Chloroethoxy)-4-(3-chloro-4-(3-fluorobenzyloxy)anilino)-5-(tetrahydropyran-4-yloxy)quinazoline

Obtained from 4-(3-chloro-4-(3-fluorobenzyloxy)anilino)-7-hydroxy-5-(tetrahydropyran-4-yloxy)quinazoline **(reference example 20.9)** and 1-bromo-2-chloroethane in 83% yield; Mass spectrum MH⁺ 558.

### Reference Example 21.12

### 4-(3-Chloro-4-(3-fluorobenzyloxy)anilino)-7-(3-chloropropoxy)-5-(tetrahydropyran-4-yloxy)quinazoline

Obtained by reacting 4-(3-chloro-4-(3-fluorobenzyloxy)anilino)-5-(tetrahydropyran-4-yloxy)-7-hydroxyquinazoline **(reference example 20.9)** and 1-bromo-3-chloropropane in 100% yield; NMR spectrum (CDCl₃) 2.0 (m, 2H), 2.3 (m, 4H), 2.8 (t, 4H), 3.6 (m, 2H), 3.8 (t, 2H), 4.1 (dt, 2H), 4.2 (t, 2H), 4.8 (m, 1H) 5.2 (s, 2H), 6.5 (d, 1H), 6.8 (d, 1H), 7.0 (d, 1H), 7.0 (m, 1H), 7.2 (m, 2H), 7.4 (m, 1H), 7.5 (dd, 1H), 7.9 (d, 1H), 8.5 (s, 1H), 9.7 (s, 1H); Mass spectrum MH⁺ 572.

### Reference Example 21.13

### 4-(3-Chloro-4-(3-fluorobenzyloxy)anilino)-7-(3-chloropropoxy)-5-(tetrahydrofuran-3-yloxy)quinazoline

Obtained by reacting 4-(3-chloro-4-(3-fluorobenzyloxy)anilino)-5-(tetrahydropyran-4-yloxy)-7-hydroxyquinazoline **(reference example 20.10)** and 1-bromo-3-chloropropane in 91% yield; NMR spectrum (DMSO-d6) 2.1 - 2.4 (m, 4H), 3.8-4.0 (m, 5H), 4.2 - 4.3 (m, 3H), 5.2 (s, 2H), 5.5 (m, 1H), 6.8 (m, 2H), 7.1-7.3 (m, 4H), 7.4 - 7.5 (m, 2H), 8.2 (d, 1H), 8.5 (s, 1H), 9.8 (s, 1H); Mass spectrum MH⁺ 558.

### Reference Example 21.14

### 7-(2-Chloroethoxy)-4-(3-chloro-4-(3-fluorobenzyloxy)anilino)-5-(tetrahydrofuran-3-yloxy)quinazoline

Obtained by reacting 4-(3-chloro-4-(3-fluorobenzyloxy)anilino)-5-(tetrahydropyran-4-yloxy)-7-hydroxyquinazoline **(reference example 20.10)** and 1-bromo-2-chloroethane in 100% yield; NMR spectrum (DMSO-d6) 2.2 (m, 1H), 2.3 (m, 1H), 3.8 - 4.0 (m, 5H), 4.2 (d, 1H), 4.4 (t, 2H), 5.2 (s, 2H), 5.5 (m, 1H), 6.8 (m, 2H), 7.1-7.3 (m, 4H), 7.4 - 7.5 (m, 2H), 8.2 (m, 1H), 8.5 (s, 1H), 9.8 (s, 1H); Mass spectrum MH⁺ 544.

### Reference Example 22

### 7-(1-tert-Butoxycarbonylpiperidin-4-ylmethoxy)-4-(3-chloro-4-fluoroanilino)-5-(1-methylpiperidin-4-yloxy)quinazoline

Potassium carbonate (240 mg) was added to 4-(3-chloro-4-fluoroanilino)-7-hydroxy-5-(1-methylpiperidin-4-yloxy)quinazoline trifluoroacetate **(reference example 20.4)** (175 mg) in DMA (5 ml). 1-(*tert-*Butoxycarbonyl)-4-tosyloxymethylpiperidine **(reference example 41)** (161 mg) was added and the resulting mixture was stirred for 18 hours at room temperature. The reaction was then heated at 60°C for 18 hours and the solvent concentrated *in vacuo* to give a solid. Water was added to this and the solid filtered to afford the title compound as a beige solid (100 mg, 38%); NMR spectrum (DMSO-d6) 1.2 (m, 2H), 1.4 (s, 9H), 1.5 (m, 1H), 1.7 (m, 2H), 1.9 (m, 2H), 2.1 (m, 2H), 2.2 (s, 3H), 2.2 (m, 2H), 3.8 (m, 2H), 4.0 (m, 4H), 4.8 (m, 1H), 6.8 (s, 2H), 7.4 (m, 2H), 7.6 (m, 1H), 7.8 (d, 1H), 8.2 (dd, 1H), 8.5 (s, 1H), 9.9 (s, 1H); Mass spectrum MH⁺ 600.

The procedure described above was repeated using the appropriate 7-hydroxyquinazoline. Thus was obtained the compound described below:

### Reference Example 22.1

### 7-(1-tert-Butoxycarbonylpiperidin4-ylmethoxy)-4-(3-chloroanilino)-5-(1-methylpiperidin-4-yloxy)quinazoline

Obtained from 4-(3-chloroanilino)-7-hydroxy-5-(1-methylpiperidin-4-yloxy)quinazoline trifluoroacetate **(reference example 20.6)** in 35% yield; Mass spectrum MH⁺ 582.

### Reference Example 22.2

### 7-(1-tert-Butoxycarbonylpiperidin-4-ylmethoxy)-4-(3-chloro-4-fluoroanilino)-5-(tetrahydrofuran-3-yloxy)quinazoline

Obtained from 4-(3-chloro-4-fluoroanilino)-7-hydroxy-5-(tetrahydrofuran-3-yloxy)quinazoline **(reference example 20.2)** in 86% yield; Mass spectrum MH⁺ 574.

### Reference Example 23

### N-tert-Butoxycarbonyl-3,5-dibenzyloxyaniline

Di-*iso*propylethylamine (6 ml) and diphenylphosporyl azide (7 ml) were added to a suspension of 3, 5-dibenzyloxybenzoic acid (10 g) in *tert*-butanol (150 ml), and the reaction stirred at 70°C for 5 hours. The reaction was cooled, concentrated *in vacuo* and the residue purified by chromatography (isohexane-5% ethyl acetate) to give the title compound as a white solid (5.8 g, 48%); NMR spectrum (DMSO-d6) 1.43 (s, 9H), 5.00 (s, 4H), 6.30 (s, 1H), 6.80 (s, 1H), 7.10 - 7.42 (m, 10H), 9.24 (s, 1H); Mass spectrum M-H⁺ 404.

### Reference Example 24

### 3,5-Dibenzyloxyaniline trifluoroacetate

Trifluoroacetic acid (20 ml) was added to a solution of 3,5-dibenzyloxy-*N*-*tert-*butoxycarbonylaniline **(reference example 23)** (5.75 g) in DCM (150 ml) and the reaction stirred for 4 hours. The reaction was concentrated *in vacuo* to yield the title compound as a beige solid (7.4 g, >100%); Mass spectrum MH⁺ 306. Alternatively, the product could be isolated as the hydrochloride salt by partitioning between saturated aqueous sodium bicarbonate and ethyl acetate, and acidification of the organic extracts by addition of a 1M HCl solution in ether.

### Reference Example 25

### 5-Amino-3-bromoindazole

Titanium trichloride (10% solution in HCl, 45 ml) was added dropwise to a solution of ammonium acetate (6.36 g) and 3-bromo-5-nitroindazole (obtained as described in Eur. J. Med. Chem., (1986), 21(4), 359-362) (1.0 g) in a mixture of acetone (60 ml) and water (10 ml). The mixture was stirred at room temperature for 30 minutes before pouring into water (150 ml) and neutralising with 10N sodium hydroxide. The aqueous mixture was then extracted with ethyl acetate (3 x 100 ml), organic extracts were washed with saturated brine, then combined, dried and concentrated *in vacuo* to give the title compound as a pale pink solid (0.76 g, 86%); NMR spectrum (DMSO-d6) 5.12 (bs, 2H), 6.54 (s, 1H), 6.83 (d, 1H), 6.86 (d, 1H), 12.88 (bs, 1H); Mass spectrum MH⁺ 212.

The procedure described above was repeated using the appropriate aryl nitro compound. Thus were obtained the compounds described below:

### Reference Example 25.1

### 5-Amino-3-chloroindazole

Obtained from 3-chloro-5-nitroindazole in 87% yield; NMR spectrum (DMSO-d6) 4.99 (bs, 2H), 6.58 (m, 1H), 6.83 (d, 1H), 6.86 (d, 1H), 12.71 (bs, 1H).

### Reference Example 25.2

### 5-Amino-3-bromoindole

Obtained from 3-bromo-5-nitroindole **(reference example 30.1)** in 80% yield; NMR spectrum (DMSO-d6) 5.48 (bs, 2H), 6.61 (m, 2H), 7.15 (d, 1H), 7.32 (d, 2H), 10.99 (s, 1H); Mass spectrum MH⁺ 211.

### Reference Example 26

### 5-Amino-3-chloro-1-(2-pyridylmethyl)indole

A solution of 3-chloro-5-nitro-1-(2-pyridylmethyl)indole **(reference example 33)** (2.5 g) in ethanol (130 ml) was stirred at room temperature. Sodium dithionite (7.6 g) in water (18 ml) was added, and the mixture was heated to 50°C for 5 hours, then cooled to room temperature. The ethanol was removed *in vacuo,* and the residue was partitioned between DCM and water. The DCM layer was separated, dried over sodium sulphate, then concentrated *in vacuo* to give the crude material, which was purified by chromatography using 50% DCM in isohexane then DCM as eluent to give the title compound as an orange solid (488 mg, 23%); NMR spectrum (CDCl₃) 3.53 (s, 2H), 5.27 (s, 2H), 6.61 (dd, 1H), 6.68 (d, 1H), 6.86 (d, 1H), 7.01 (d, 1H), 7.04 (s, 1H), 7.11 (dd, 1H), 7.47 (dt, 1H), 8.55 (m, 1H); Mass spectrum MH⁺ 258.

The procedure described above was repeated using the appropriate aryl nitro compound. Thus were obtained the compounds described below:

### Reference Example 26.1

### 5-Amino-3-chloro-1-(2-pyridylmethyl)indazole

Obtained from 3-chloro-5-nitro-1-(2-pyridylmethyl)indazole **(reference example 33.1)** in 24% yield; NMR spectrum (CDCl₃) 3.3 (bs, 2H), 6.65 (dd, 1H), 6.77 (m, 1H), 6.84 - 7.02 (m, 5H), 7.24 (m, 1H).

### Reference Example 26.2

### 5-Amino-3-chloroindole

Obtained from 3-chloro-5-nitroindole **(reference example 30)** in 17% yield; NMR spectrum (DMSO-d6) 4.7 - 4.9 (bs, 2H), 6.6 (m, 2H), 7.1 (d, 1H), 7.2 (d, 1H).

### Reference Example 26.3

### 3-Fluoro-4-(1-methyl-1H-imidazol-2-ylthio)aniline

Obtained from 3-fluoro-4-(1-methyl-1*H*-imidazol-2-ylthio)nitrobenzene **(reference example 43)** in 86% yield; Mass spectrum MH⁺ 224.

### Reference Example 27

### 5-Amino-3-methylbenzisothiazole

3-Methyl-5-nitrobenzisothiazole (1 g) and 10% Pd/C (0.3 g) in ethanol (40 ml) were stirred for 16 hours under an atmosphere of hydrogen. The solid residues were removed by filtration and the solution concentrated *in vacuo.* The residue was triturated with ether and filtered to give the title compound as a pale yellow solid (0.25 g, 30% yield); NMR spectrum (DMSO-d6) 2.52 (s, 3H), 5.30 (bs, 2H), 6.95 (dd, 1H), 7.03 (dd, 1H), 7.70 (d, 1H).

The procedure described above was repeated using the appropriate nitro compound. Thus was obtained the compound described below:

### Reference Example 27.1

### 5-Amino-3-methylindole

Obtained from 3-methyl-5-nitroindole **(reference example 31)** in 66% yield; NMR spectrum (DMSO-d6) 2.1 (s, 3H), 4.4 (bs, 2H), 6.4 (dd, 1H), 6.6 (d, 1H), 6.8 (d, 1H), 7.0 (d, 1H); Mass spectrum MH⁺ 147.

### Reference Example 27.2

### 5-Aminoindole-3-carbonitrile

Obtained from 5-nitroindole-3-carbonitrile **(reference example 38)** in 71% yield; NMR spectrum (DMSO-d6) 4.8 (bs, 2H), 6.6 (dd, 1H), 6.7 (s, 1H), 7.2 (d, 1H), 7.9 (s, 1H); Mass spectrum MH⁺ 158.

### Reference Example 28

### 3-Chloro-4-(3-fluorobenzyloxy)aniline

To a solution of 2-chloro-1-(3-fluorobenzyloxy)-4-nitrobenzene (reference example 34) (3.74 g) in ethyl acetate (60 ml) was added 10% Pt/C (0.5 g). The resulting solution was subjected to a hydrogen atmosphere for 4 hours at room temperature. The catalyst was then filtered off and the solvent concentrated *in vacuo* to give the title compound as an orange crystalline solid (3.08 g, 92%); NMR spectrum (DMSO-d6) 4.91 (s, 2H), 5.01 (s, 2H), 6.45 (dd, 1H), 6.63 (s, 1H), 6.89 (d, 1H), 7.12 (t, 1H), 7.24 (t, 2H), 7.40 (m, 1H); Mass spectrum MH⁺ 252.

### Reference Example 29

### 4-(Azepan-1-ylcarbonyl)-3-chloroaniline

To 1-(2-chloro-4-nitrobenzoyl)azepane **(reference example 37)** (2.58 g) was added ethyl acetate (100 ml) and tin (II) chloride dihydrate (9 g). This was heated to 70°C for 4 hours, then allowed to cool. The mixture was made basic with 880 ammonia solution, the resulting solid filtered. The filtrate was extracted with water and combined organic extracts were dried and concentrated *in vacuo.* The residue was purified by chromatography using DCM - 30% ethyl acetate as eluent to give the title compound as a white solid (1.85 g, 73%); NMR spectrum (CDCl₃) 1.48 -1.74 (m, 6H), 1.74 - 1.92 (m, 2H), 3.23 - 3.33 (m, 2H), 3.35 - 3.84 (m, 2H), 3.85 (s, 2H), 6.54 (dd, 1H), 6.68 (d, 1H), 7.02 (d, 1H); Mass spectrum MH⁺ 253.

### Reference Example 30

### 3-Chloro-5-nitroindole

*N*-Chlorosuccinimide (1.65 g) was added in portions to a solution of 5-nitroindole (2.00 g) in DMF (20 ml). The resulting solution was stirred at room temperature for 18 hours. The pale brown solution was poured into water (200 ml) to give a yellow precipitate which was filtered, washed with water and dried in vacuo to give the title compound as a yellow solid (2.40 g, 99%). Mass spectrum M-H⁺ 195.

The procedure described above was repeated using the appropriate *N*-halosuccinimide. Thus was obtained the compound described below:

### Reference Example 30.1

### 3-Bromo-5-nitroindole

Obtained from *N*-bromosuccinimide in 89% yield; NMR spectrum (DMSO-d6) 7.60 (d, 1H), 7.82 (d, 1H), 8.04 (dd, 1H), 8.30 (d, 1H), 12.16 (bs, 1H).

### Reference Example 31

### 3-Methyl-5-nitroindole

Tetra-n-butylammonium bromide (1.25 g) and triethylamine (1.37 ml) were added to allyl-(2-bromo-4-nitrophenyl)amine **(reference example 32)** (1.00 g) dissolved in DMF (5 ml). Palladium (II) acetate (50 mg) was added and the reaction was stirred at room temperature for 72 hours. The reaction was filtered through celite after dilution with ethyl acetate. The solution was washed with water (50 ml), 5% aqueous HCl (50 ml), brine (50 ml) and dried. Concentration *in vacuo* gave a brown solid which was purified by chromatography using DCM as eluent to afford the title compound as a yellow solid (680 mg, 99%); NMR spectrum (DMSO-d6) 2.3 (s, 3H), 7.4 (d, 1H), 7.5 (d, 1H), 8.0 (dd, 1H), 8.5 (d, 1H); Mass spectrum M-H⁺ 175.

### Reference Example 32

### Allyl-(2-bromo-4-nitrophenyl)amine

Potassium *tert*-butoxide (2.71 g) was added to 2-bromo-4-nitroaniline (5.00g) in DMF (30 ml) at 0°C. The resulting red solution was stirred at this temperature for 30 minutes. Allyl bromide (2.05 ml) was added dropwise and the reaction mixture was stirred for 18 hours at room temperature. The reaction mixture was poured into 20% NaH₂PO₄ and extracted with ethyl acetate. The combined organic extracts were dried, filtered and concentrated *in vacuo* to afford an orange oil. This was purified by chromatography using ethyl acetate / isohexane (1:9) as eluent to afford the title compound as a yellow crystalline solid (2.25 g, 38%); NMR spectrum (DMSO-d6) 4.0 (m, 2H), 5.1 (d, 1H), 5.2 (dd, 1H), 5.9 (m, 1H), 6.7 (d, 1H), 6.9 (t, 1H), 8.0 (dd, 1H), 8.3 (d, 1H).

### Reference Example 33

### 3-Chloro-5-nitro-1-(2-pyridylmethyl)indole

2-Picolyl chloride hydrochloride (3.26 g) was added to a stirred mixture of 3-chloro-5-nitroindole **(reference example 30)** (1.97 g) and potassium carbonate (13.8 g) in DMF (50 ml). The mixture was heated to 50°C and stirred for 2 hours, after which time the solvent was removed *in vacuo.* The residue was dissolved in DCM, then washed with water, and dried over sodium sulphate. Concentration gave the product as a yellow solid (2.53 g, 88%); NMR spectrum (CDCl₃) 5.43 (s, 2H), 6.90 (d, 1H), 7.23 (dd, 1H), 7.35 (s, 1H), 7.37 (d, 1H), 7.62 (dt, 1H), 8.12 (dd, 1H), 8.60 (m, 2H).

The procedure described above was repeated using the appropriate heterocycle and alkyl halide. Thus were obtained the compounds described below:

### Reference Example 33.1

### 3-Chloro-5-nitro-1-(2-pyridinylmethyl)indazole

Obtained from 3-chloro-5-nitroindazole and 2-picolyl chloride hydrochloride in 74% yield; NMR spectrum (CDCl₃) 5.32 (s, 2H), 6.80 (d, 1H), 6.89 (d, 1H), 7.01 (dt, 1H), 7.28 (m, 3H), 8.12 (dd, 1H), 8.61 (d, 1H).

### Reference Example 34

### 2-Chloro-1-(3-fluorobenzyloxy)-4-nitrobenzene

To a solution of 2-chloro-4-nitrophenol (20.0 g) in acetone (400 ml) was added potassium carbonate (47.76 g) followed by the dropwise addition of 3-fluorobenzyl bromide (32.67 g) over 15 minutes. The reaction mixture was then stirred at room temperature for 16 hours and filtered to remove insoluble material. The solvent was then concentrated *in vacuo* and the solid remaining was purified by chromatography using 30-80% DCM / isohexane as eluent to give the title compound (30.96 g, 95%); NMR spectrum (DMSO-d⁶) 5.39 (s, 2H), 7.18 (t, 1H), 7.30 (m, 2H), 7.45 (m, 2H), 8.23 (dd, 1H), 8.32 (d, 1H); Mass spectrum M-H⁺ 280.

The procedure described above was repeated using the appropriate phenol and alkyl halide. Thus were obtained the compounds described below:

### Reference Example 34.1

### 4-(2-Fluorobenzyloxy)-3-iodonitrobenzene

Obtained from 2-fluorobenzyl bromide and 4-hydroxy-3-iodonitrobenzene in 85% yield; Mass spectrum M-H⁺ 372.

### Reference Example 34.2

### 4-(3-Fluorobenzyloxy)-3-iodonitrobenzene

Obtained by reacting 4-hydroxy-3-iodonitrobenzene and 3-fluorobenzyl bromide in 99% yield; Mass spectrum M-H⁺ 372.

### Reference Example 35

### 4-(2-Fluorobenzyloxy)-3-(trimethylsilylethynyl)nitrobenzene

To a solution of 4-(2-fluorobenzyloxy)-3-iodonitrobenzene (0.49 g) **(reference example 34.1)** in acetonitrile (10 ml) was added trimethylsilylacetylene (0.54 ml), copper(I) iodide (5 mg), bis(triphenylphosphine)-dichloropalladium (18 mg) and triethylamine (10 ml) under nitrogen and the mixture stirred at room temperature for 4 hours. The reaction was concentrated *in vacuo* and the residue purified by chromatography using DCM as eluent to give the tide compound as a yellow solid (0.33 g, 73%); Mass spectrum M-H⁺ 342.

The procedure described above was repeated using the appropriate halobenzene. Thus were obtained the compounds described below:

### Reference Example 35.1

### 4-(3-Fluorobenzyloxy)-3-(trimethylsilylethynyl)nitrobenzene

Obtained from 4-(3-fluorobenzyloxy)-3-iodonitrobenzene **(reference example 34.2)**; Mass spectrum M-H⁺ 342.

### Reference Example 36

### 4-Hydroxytetrahydrothiopyran

Sodium borohydride (60 mg) was added to 2M NaOH (100 µl) and the resulting solution diluted with water (0.75 ml). This solution was added dropwise to tetrahydrothiopyran-4-one (0.5 g) in methanol (5 ml) using an ice bath to maintain the internal temperature at 18-25°C. A further 0.13 equivalents of sodium borohydride was added and after stirring at room temperature for 30 minutes the reaction was concentrated *in vacuo* to a minimum volume and water (5 ml) was added. The solution was extracted with diethyl ether (6 x 20ml), dried and concentrated *in vacuo* to afford the title compound as a colourless oil (0.48 g, 94%); NMR spectrum (DMSO-d6) 1.5 (m, 2H), 2.0 (m, 2H), 2.4 (m, 2H), 2.7 (m, 2H), 3.4 (m, 1H), 4.6 (d, 1H).

### Reference Example 37

### 1-(2-Chloro-4-nitrobenzoyl)azepane

To a solution of 2-chloro-5-nitrobenzoic acid (4.02 g) and triethylamine (2.20 g) in DCM (150 ml) was added O-(7-azabenztriazol-1-yl)-N,N,N',N'-tetramethyluronium hexafluorophosphate (8.36 g). This mixture was stirred at room temperature under an atmosphere of nitrogen for 3 hours. Hexamethyleneimine (2.18 g) was added and this mixture stirred at room temperature for 2 hours. The solvent was removed *in vacuo* and the residue purified by chromatography using 0-10% ethyl acetate in DCM as eluent to give the title compound as a colourless oil which crystallised upon standing (5.09 g, 90%); NMR spectrum (CDCl₃) 1.53 - 1.77 (m, 6H), 1.79 -1.93 (m, 2H), 3.15 - 3.27 (m, 2H), 3.60 - 3.84 (m, 2H), 7.48 (d, 1H), 8.18 (dd, 1H), 8.30 (d, 1H); Mass spectrum MH⁺ 283.

### Reference Example 38

### 5-Nitroindole-3-carbonitrile

5-Nitroindole (2 g) was dissolved in diethyl ether (100 ml) and cooled to -10°C. Chlorosulphonyl isocyanate (5.37 ml) was added dropwise maintaining an internal temperature of -10°C to afford a white precipitate. This was filtered and washed with ether before adding to DMF (100 ml). The resulting solution was stirred at room temperature for 1 hour, then poured into water (500 ml) to give a yellow solid, which was filtered and dried. This solid was stirred in ethyl acetate (250 ml) for 30 minutes, then filtered. The filtrate was evaporated *in vacuo* to afford the title compound as a pale yellow solid (1.35 g, 60%); NMR spectrum (DMSO-d6) 7.7 (d, 1H), 8.2 (dd, 1H), 8.5 (m, 2H); Mass spectrum M-H⁺ 186.

### Reference Example 39

### Ethyl 4-(1-(tert-butoxycarbonyl)piperidine)carboxylate

While maintaining the temperature in the range 0-5°C, a solution of di-*tert*-butyl dicarbonate (41.7 g) in ethyl acetate (75 ml) was added in portions to a solution of ethyl 4-piperidinecarboxylate (30 g) in ethyl acetate (150 ml) cooled at 5°C. After stirring for 48 hours at ambient temperature, the mixture was poured into water (300 ml). The organic layer was separated, washed successively with water (200 ml), 0.1N aqueous hydrochloric acid (200 ml), saturated aqueous sodium hydrogen carbonate (200 ml) and brine (200 ml), dried and evaporated to give the title compound (48 g, 98%); NMR spectrum (CDCl₃) 1.25 (t, 3H), 1.45 (s, 9H), 1.55 - 1.70 (m, 2H), 1.8 - 2.0 (d, 2H), 2.35 - 2.5 (m, 1H), 2.7 - 2.95 (t, 2H), 3.9 - 4.1 (bs, 2H), 4.15 (q, 2H).

### Reference Example 40

### 1-(tert-Butoxycarbonyl)-4-hydroxymethylpiperidine

A solution of 1M lithium aluminium hydride in THF (133 ml) was added in portions to a solution of ethyl 4-(1-(tert-butoxycarbonyl)piperidine)carboxylate **(reference example 39)** (48 g) in dry THF (180 ml) cooled at 0°C. After stirring at 0°C for 2 hours, water (30 ml) was added followed by 2N sodium hydroxide (10 ml). The precipitate was removed by filtration through diatomaceous earth and washed with ethyl acetate. The filtrate was washed with water, brine, dried and evaporated to give the title compound (36.3 g, 89%); NMR spectrum (CDCl₃) 1.05 - 1.2 (m, 2H), 1.35 - 1.55 (m, 10H), 1.6 - 1.8 (m, 2H), 2.6 - 2.8 (t, 2H), 3.4 - 3.6 (t, 2H), 4.0 - 4.2 (bs, 2H); Mass spectrum M⁺ 215.

### Reference Example 41

### 1-(tert-Butoxycarbonyl-4-tosyloxymethylpiperidine

1, 4-Diazabicyclo[2.2.2]octane (42.4 g) was added to a solution of 1-(*tert-*butoxycarbonyl)-4-hydroxymethylpiperidine **(reference example 40)** (52.5 g) in *tert*-butyl methyl ether (525 ml). After stirring for 15 minutes at ambient temperature, the mixture was cooled to 5°C and a solution of 4-toluenesulphonyl chloride (62.8 g) in *tert-*butyl methyl ether (525 ml) was added in portions over 2 hours while maintaining the temperature at 0°C. After stirring for 1 hour at ambient temperature, petroleum ether (11) was added. The precipitate was removed by filtration. The filtrate was evaporated to give a solid. The solid was dissolved in ether and washed successively with 0.5N aqueous hydrochloric acid (2 x 500 ml), water, saturated aqueous sodium hydrogen carbonate and brine, dried and evaporated to give the title compound as a white solid (76.7 g, 85%); NMR spectrum (CDCl₃) 1.0 - 1.2 (m, 2H), 1.45 (s, 9H), 1.65 (d, 2H), 1.75 -1.9 (m, 2H), 2.45 (s, 3H), 2.55 - 2.75 (m, 2H), 3.85 (d, 1H), 4.0 - 4.2 (bs, 2H), 7.35 (d, 2H), 7.8 (d, 2H); Mass spectrum M+Na⁺ 392.

### Reference Example 42

### 3-Ethynyl-4-(2-fluorobenzyloxy)aniline

A solution of 4-(2-fluorobenzyloxy)-3-(trimethylsilylethynyl)nitrobenzene (310 mg) **(reference example 35)** and 10% Pt/C in ethyl acetate / ethanol (9:1, 10 ml) was stirred under an atmosphere of hydrogen for 20 minutes. The catalyst was removed by filtration and the solution concentrated *in vacuo* to give a green solid. This was dissolved in methanol (100 ml) and DCM (50 ml), potassium carbonate (0.375 g) added and the solution stirred for 30 minutes. The reaction was filtered and concentrated *in vacuo.* The residue was purified by chromatography using DCM as eluent to give the title compound as a yellow oil (0.13 g, 62%); Mass spectrum MH⁺ 283.

The procedure described above was repeated using the appropriate nitrobenzene. Thus was obtained the compound described below:

### Reference Example 42.1

### 3-Ethynyl-4-(3-fluorobenzyloxy)aniline

Obtained from 4-(3-fluorobenzyloxy)-3-(trimethylsilylethynyl)nitrobenzene **(reference example 35.1);** Mass spectrum MH⁺ 283.

### Reference Example 43

### 3-Fluoro-4-(1-methyl-1H-imidazolyl-2-ylthio)nitrobenzene

To a stirred solution of 2-mercapto-1-methylimidazole (1.14 g), in DMF (20 ml), was added sodium hydride (0.44 g) in small portions and the reaction stirred at ambient temperature until effervescence ceased. To this was added a solution of 3,4-difluoronitrobenzene (1.59 g) in DMF (10 ml), and the solution stirred at 80°C for 4 hours. The reaction was poured into water (150 ml) and organic material extracted into ethyl acetate (150 ml). The organic layer was washed successively with water (3 x 150 ml), brine (150 ml) and dried. Evaporation of the solvent gave an oil which was purified by chromatography using ethyl acetate and then 10% methanol / ethyl acetate as eluent to give title compound as a solid (1.8 g, 70%); NMR spectrum (DMSO-d6) 2.5 (s, 3H), 6.7 - 6.9 (t, 1H), 7.2 (t, 1H), 7.6 (s, 1H), 7.95 - 8.05 (dd, 1H), 8.15 - 8.25 (dd, 1H); Mass spectrum MH⁺ 254.

## Claims

1. A quinazoline derivative of the Formula I 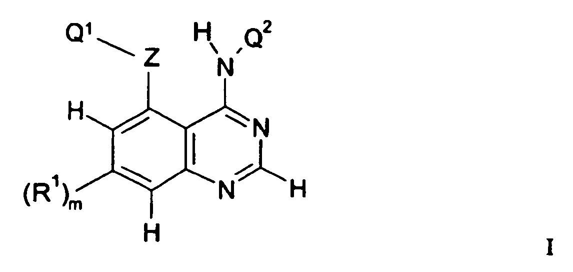 wherein:
m is 0 or 1 and the R¹ group, when present, is selected from hydroxy, amino, methyl, ethyl, propyl, methoxy, ethoxy, propoxy, butoxy, pentoxy, pyrrolidin-1-yl, 2-pyrrolidin-1-ylethoxy, 3-pyrrolidin-1-ylpropoxy, 2-piperidinoethoxy, 3-piperidinopropoxy, 2-piperidin-3-ylethoxy, -3-piperidin-3-ylpropoxy, 2-piperidin-4-ylethoxy, 3-piperidin-4-ylpropoxy, 2-piperazin-1-ylethoxy, 3-piperazin-1-ylpropoxy, 2-morpholinoethoxy, 3-morpholinopropoxy, 2-homopiperidinoethoxy, 3-homopiperidinopropoxy, 2-homopiperazin-1-ylethoxy and 3-homopiperazin-1-ylpropoxy
and wherein adjacent carbon atoms in any (2-6C)alkoxy chain within a R¹ substituent are optionally separated by the insertion into the chain of a group selected from O, NH and N(CH₃),
and wherein any terminal CH₃ group within a (1-6C)alkoxy chain in a R¹ substituent optionally bears on the terminal CH₃ group a substituent selected from hydroxy, amino and N-(1-methylpyrrolidin-3-yl)-N-methylamino,
and wherein any pyrrolidinyl or piperidinyl group within a R¹ substituent optionally bears a substituent selected from hydroxy, methyl, amino, methylamino and dimethylamino,
and wherein any piperazin-1-yl or homopiperazin-1-yl group within a R¹ substituent optionally bears a substituent at the 4-position selected from methyl, ethyl, isopropyl, 2-methoxyethyl, tetrahydrofurfuryl, 2-morpholinoethyl and 1-methylpiperidin-4-yl;
the Q¹-Z- group is selected from cyclopentyloxy, tetrahydrofuran-3-yloxy, tetrahydropyran-4-yloxy, tetrahydrothiopyran-4-yloxy, 1;1-dioxotetrahydrothiopyran-4-yloxy, 1-oxotetrahydrothiopyran-4-yloxy, tetrahydrothien-3-yloxy, 1,1-dioxotetrahydrothien-3-yloxy, 1-oxotetrahydrothien-3-yloxy, pyrrolidin-3-yloxy, pyrrolidin-2-yloxy, piperidin-3-yloxy, piperidin-4-yloxy, homopiperidin-3-yloxy, homopiperidin-4-yloxy and azetidin-3-yloxy,
and wherein the azetidinyl, pyrrolidinyl, piperidinyl or homopiperidinyl group within the Q¹-Z- group is optionally N- substituted by a substituent selected from methyl, ethyl, n-propyl, isopropyl, n-butyl, isobutyl, tert-butyl, allyl, 2-propynyl, acetyl, propionyl, methoxycarbonyl, ethoxycarbonyl, propoxycarbonyl, tert-butoxycarbonyl, methylsulphonyl, ethylsulphonyl, 2-methoxyethyl, carbamoylmethyl, N-methylcarbamoylmethyl, N,N-dimethylcarbamoylmethyl, 2-carbamoylethyl, 2-(N-methylcarbamoyl)ethyl, 2-(N,N-dimethylcarbamoyl)ethyl, acetylmethyl, 2-acetylethyl, methoxycarbonylmethyl and 2-methoxycarbonylethyl,
and wherein any heterocyclyl group within the Q¹-Z- group optionally bears 1 or 2 oxo substituents; and
Q² is an aryl group of formula Ib 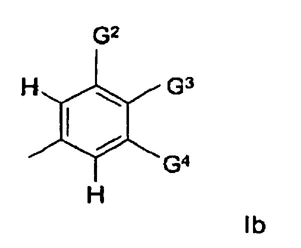 wherein G³ and G⁴ together form a group of formula:- -CH=CH-NH-, -NH-CH=CH-, -NH-N=CH- or -CH=N-NH-,
and the 9-membered bicyclic heteroaryl ring formed when G³ and G⁴ are linked together optionally bears on a NH group of the heteroaryl portion of the bicyclic ring a group of the formula:
-X¹²-Q¹¹
wherein X¹² is a direct bond or is selected from SO₂ and CO, wherein Q¹¹ is phenyl, benzyl, 2-phenylethyl, 2-furyl, furfuryl, 3-furyl, 3-furylmethyl, 2-oxazolyl, 4-oxazolyl, 2-oxazolylmethyl, 4-oxazolylmethyl, 2-imidazolyl, 4-imidazolyl, 2-imidazolylmethyl, 4-imidazolylmethyl, 2-, 3-or 4-pyridyl, 2-, 3-or 4-pyridylmethyl, 2-(2-, 3-or 4-pyridyl)ethyl, 2-, 4- or 5-pyrimidinyl, 2-, 4- or 5-pyrimidinylmethyl, 2-(2-, 4- or 5-pyrimidinyl)ethyl, 1,2,4-triazol-3-yl, 1,2,4-triazol-5-ylmethyl, triazol-3-ylmethyl, 1,2,4-triazol-5-yl, 2-thienyl, 3-thienyl, 2-thienylmethyl, 3-thienylmethyl, 2-(2-thienyl)ethyl, 2-(3-thienyl)ethyl, 2-thiazolyl, 4-thiazolyl, 2-thiazolylmethyl, 4-thiazolylmethyl, 1,2,5-thiadiazol-3-yl, 1,2,5-thiadiazol-3-ylmethyl, or 2-(1,2,5-thiadiazol-3-yl)ethyl, which optionally bears 1 or 2 substituents, which may be the same or different, selected from fluoro, chloro, bromo, cyano, hydroxy, methyl and ethyl,
and the 9- membered bicyclic heteroaryl ring formed when G³ and G⁴ together are linked optionally bears on an available carbon atom in the heteroaryl portion of the bicyclic ring 1 substituent selected from fluoro, chloro, bromo, cyano, hydroxy, amino, methyl, ethyl, vinyl, ethynyl, methylamino and di-methylamino,
and G² is selected from hydrogen, fluoro, chloro, bromo, trifluoromethyl, cyano, hydroxy, amino, methyl, ethyl, vinyl, ethynyl, methylamino and dimethylamino;
or a pharmaceutically acceptable salt thereof.

2. A quinazoline derivative according to claim 1, wherein
R¹, when present, is methoxy,
Q¹-Z- is 1-methylpiperidin-4-yloxy, and Q² is an aryl group of formula Ib wherein G² is hydrogen, and G³ and G⁴ together form a group of formula:- -NH-CH=CH- or -NH-N=CH-,
and the 9-membered bicyclic heteroaryl ring formed when G³ and G⁴ are linked together optionally bears on a NH group of the heteroaryl portion of the bicyclic ring a group of the formula:
-X¹²-Q¹¹
wherein X¹² is a direct bond or is SO₂, and Q¹¹ is phenyl, benzyl, or 2-pyridylmethyl which optionally bears a fluoro substituent,
and the 9-membered bicyclic heteroaryl ring formed when G³ and G⁴ together are linked optionally bears at the 3-position a chloro substituent;
or a pharmaceutically acceptable salt thereof.

3. A quinazoline derivative according to claim 2, wherein Q² is selected from
1-benzenesulphonylindol-5-yl, 1-benzylindol-5-yl,
1-(2-pyridylmethyl)indol-5-yl, 1-(2-pyridylmethyl)indazol-5-yl and
1-(3-fluorobenzyl)indazol-S-yl;
or a pharmaceutically acceptable salt thereof.

4. A quinazoline derivative according to claim 1, wherein:
R¹, when present, is methoxy;
Q¹-Z- is 1-methylpiperidin-4-yloxy; and
Q²N(H) is a group of the formula Ic: 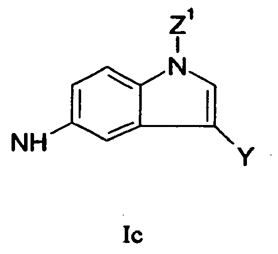
wherein Z¹ is hydrogen, and Y is selected from hydrogen, chloro and bromo; or a pharmaceutically acceptable salt thereof.

5. A quinazoline derivative according to claim 1, wherein
m is 1 and R¹ is methoxy;
Q¹-Z- 1-methylpiperidin-4-yloxy;
Q² is a group of formula Ib wherein G² is hydrogen, and G³ and G⁴ together form a group of the formula: -NH-CH=CH-, and the indolyl ring so formed by G³ and G⁴ together with the carbon atoms to which they are attached is substituted at the 1-position by a group of the formula:
-X¹²-Q¹¹
wherein X¹² is a direct bond and Q¹¹ is benzyl which is optionally substituted by 1 fluoro substituent;
or a pharmaceutically acceptable salt thereof.

6. A quinazoline derivative according to claim 5, wherein Q¹¹ is 2-fluorobenzyl or 3-fluorobenzyl;
or a pharmaceutically acceptable salt thereof.

7. A quinazoline derivative of the formula I as defined in claim 1 selected from:
4-(3-Chloroindol-5-ylamino)-5-(1-methylpiperidin-4-yloxy)quinazoline;
4-(3-Chloroindol-5-ylamino)-7-methoxy-5-(1-methylpiperidin-4-yloxy)quinazoline;
4-(Indol-5-ylamino)-7-methoxy-5-(1-methylpiperidin-4-yloxy)quinazoline;
or a pharmaceutically acceptable acid addition salt thereof.

8. A quinazoline derivative of the formula I as defined in claim 1 selected from:
4-(3-Bromoindol-5-ylamino)-7-methoxy-5-(1-methylpiperidin-4-yloxy)quinazoline;
4-(1-(3-Fluorobenzyl)indol-5-ylamino)-7-methoxy-5-(1-methylpiperidin-4-yloxy)quinazoline;
4-(1-(2-Fluorobenzyl)indol-5-ylamino)-7-methoxy-5-(1-methylpiperidin-4-yloxy)quinazoline;
or a pharmaceutically acceptable acid addition salt thereof.

9. A process for the preparation of a quinazoline derivative of the formula I, or a salt thereof, according to claim 1 which comprises:
(a) the reaction of a quinazoline of the Formula II 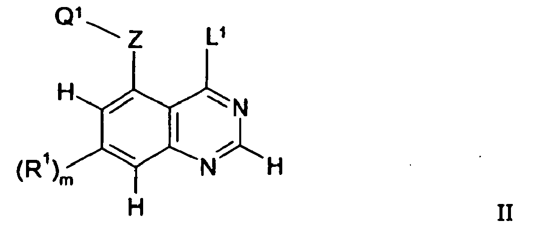 wherein L¹ is a displaceable group and Q¹, Z, m and R¹ and are as defined in claim 1 except that any functional group is protected if necessary, with a compound of the Formula:
Q²NH₂
wherein Q² is as defined in claim 1 except that any functional group is protected if necessary, whereafter any protecting group that is present is removed by conventional means; or
(b) the coupling, conveniently in the presence of a suitable dehydrating agent, of an alcohol of the Formula:
Q¹-OH
wherein Q¹ is as defined in claim 1 except that any functional group is protected if necessary, with a quinazoline of the Formula VI 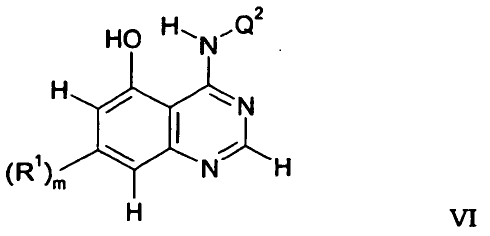 wherein m, R¹ and Q² are as defined in claim 1 except that any functional group is protected if necessary, whereafter any protecting group that is present is removed by conventional means; or
(c) the reaction of an alcohol of the Formula
Q¹-OH
wherein Q¹ is as defined in claim 1 except that any functional group is protected if necessary with a quinazoline of the Formula VIII 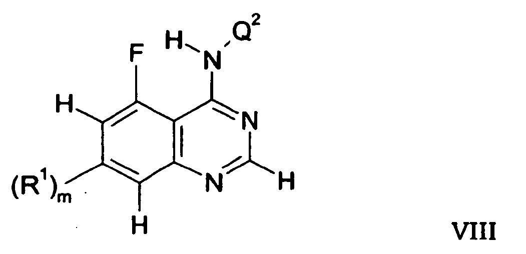 wherein m, R¹ and Q² are as defined in claim 1 except that any functional group is protected if necessary, whereafter any protecting group that is present is removed by conventional means; or
(d) for the production of those compounds of the Formula I wherein m is 1 and R¹ is a group of the formula
Q³-X¹-
wherein Q³ is a heterocyclyl-(1-6C)alkyl group as defined in claim 1 and X¹ is O, the coupling of a quinazoline of the Formula XI 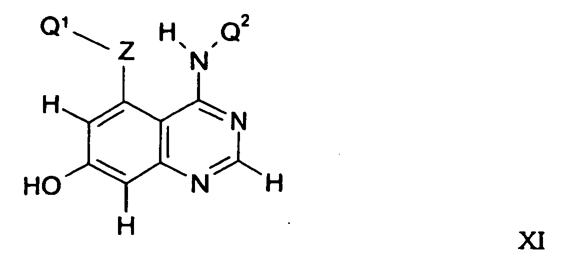 wherein Q¹, Z and Q² are as defined in claim 1 except that any functional group is protected if necessary, with an alcohol of the formula Q³OH wherein any functional group in Q³ is protected if necessary, whereafter any protecting group that is present is removed by conventional means; or
(e) for the production of those compounds of the formula I wherein R¹ is a hydroxy group, the cleavage of a quinazoline derivative of the formula I wherein R¹ is a (1-6C)alkoxy group; or
(f) for the production of those compounds of the formula I wherein Q¹, R¹ or Q² contains a primary or secondary amino group, the cleavage of the corresponding compound of Formula I wherein Q¹, R¹ or Q² contains a protected primary or secondary amino group; or
(g) for the production of those compounds of the Formula I wherein Q¹, R¹ or Q² contains a (1-6C)alkoxy or substituted (1-6C)alkoxy group or a (1-6C)alkylamino or substituted (1-6C)alkylamino group, the alkylation of a quinazoline derivative of the formula I wherein Q¹, R¹ or Q² contains a hydroxy group or a primary or secondary amino group as appropriate; or
(h) for the production of those compounds of the Formula I wherein Q¹, R¹ or Q² contains an amino-hydroxy-disubstituted (1-6C)alkoxy group, the reaction of a compound of the formula I wherein Q¹, R¹ or Q² contains an epoxy-substituted (1-6C)alkoxy group with a heterocyclyl compound or an appropriate amine; or
(i) the reaction of a quinazoline of the formula XII 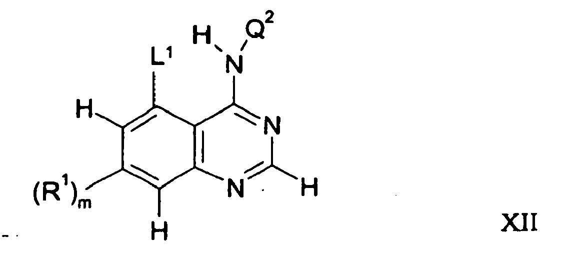 wherein L¹ is a displaceable group and m, R¹ and Q² are as defined in claim 1 except that any functional group is protected if necessary, with a compound of the Formula:
Q¹ZH
wherein Q¹ and Z are as defined in claim 1 except that any functional group is protected if necessary, whereafter any protecting group that is present is removed by conventional means; or
(j) for the production of those compounds of the formula I wherein Q¹, R¹ or Q² contains an amino-substituted (1-6C)alkoxy group, the reaction of a compound of the Formula I wherein Q¹, R¹ or Q² contains a halogeno-substituted (1-6C)alkoxy group with a heterocyclyl compound or an appropriate amine; or
(k) for the production of those compounds of the formula I wherein a heterocyclyl group in R¹ or Q¹ contains an S- or N-oxide the oxidation of a ring N or S atom in a compound of the formula (I); or
(l) for the production of those compounds of the Formula I wherein R¹, Q¹ or Q² contains an (1-6C)alkylamino or substituted (1-6C)alkylamino group or a nitrogen linked heterocyclyl group, the reductive amination of an aldehyde or ketone group in a compound of formula 1, with a (1-6C)alkylamine, substituted (1-6C)alkylamine group or a heterocycle containing an NH group in the presence of a suitable reducing agent; or
(m) the conversion of one compound of the Formula I into another compound of the Formula I;
and when a pharmaceutically acceptable salt of a quinazoline derivative of the formula I is required it may be obtained using a conventional procedure.

10. A pharmaceutical composition which comprises a quinazoline derivative of the Formula I, or a pharmaceutically acceptable thereof, as defined in claim 1 in association with a pharmaceutically acceptable diluent or carrier.

11. A quinazoline derivative of the Formula I, or a pharmaceutically acceptable salt thereof, as defined in claim 1 for use in a method of treatment of the human or animal body by therapy.

12. The use of a quinazoline derivative of the Formula I, or a pharmaceutically acceptable salt thereof, as defined in claim 1 in the manufacture of a medicament for use in the prevention or treatment of tumours which are sensitive to the inhibition of one or more erbB receptor tyrosine kinases.

13. The use of a quinazoline derivative of the formula I, or a pharmaceutically acceptable salt thereof, as defined in claim 1 in the manufacture of a medicament for use in the treatment of a cancer selected from leukaemia multiple myeloma, lymphoma, bile duct, bone, bladder, brain /CNS, breast, colorectal, endometrial, gastric, head and neck, hepatic, lung, neuronal, oesophageal, Ovarian, pancreatic, prostate, renal, skin, testicular, thyroid, uterine and vulval cancer.

## Patentansprüche

1. Chinazolinderivat der Formel I 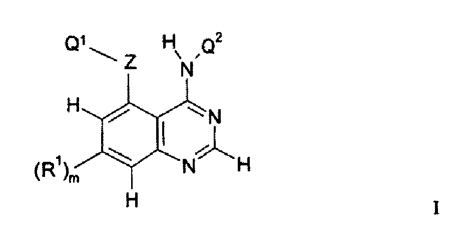 worin:
m für 0 oder 1 steht und die Gruppe R¹, sofern vorhanden, unter Hydroxy, Amino, Methyl, Ethyl, Propyl, Methoxy, Ethoxy, Propoxy, Butoxy, Pentoxy, Pyrrolidin-1-yl, 2-Pyrrolidin-1-ylethoxy, 3-Pyrrolidin-1-ylpropoxy, 2-Piperidinoethoxy, 3-Piperidinopropoxy, 2-Piperidin-3-ylethoxy, 3-Piperidin-3-ylpropoxy, 2-Piperidin-4-ylethoxy, 3-Piperidin-4-ylpropoxy, 2-Piperazin-1-ylethoxy, 3-Piperazin-1-ylpropoxy, 2-Morpholinoethoxy, 3-Morpholinopropoxy, 2-Homopiperidinoethoxy, 3-Homopiperidinopropoxy, 2-Homopiperazin-1-ylethoxy und 3-Homopiperazin-1-ylpropoxy ausgewählt ist,
und worin benachbarte Kohlenstoffatome in jeder C₂₋₆-Alkoxykette in einem Substituenten R¹ gegebenenfalls durch Einschub einer unter O, NH und N(CH₃) ausgewählten Gruppe in die Kette getrennt sind,
und worin jede endständige CH₃-Gruppe in einer C₁₋₆-Alkoxykette in einem Substituenten R¹ gegebenenfalls an der endständigen CH₃-Gruppe einen unter Hydroxy, Amino und N-(1-Methylpyrrolidin-3-yl)-N-methylamino ausgewählten Substituenten trägt,
und worin jede Pyrrolidinyl- oder Piperidinylgruppe in einem Substituenten R¹ gegebenenfalls einen unter Hydroxy, Methyl, Amino, Methylamino und Dimethylamino ausgewählten Substituenten trägt,
und worin jede Piperazin-1-yl- oder Homopiperazin-1-ylgruppe in einem Substituenten R¹ gegebenenfalls in der 4-Position einen unter Methyl, Ethyl, Isopropyl, 2-Methoxyethyl, Tetrahydrofurfuryl, 2-Morpholinoethyl und 1-Methylpiperidin-4-yl ausgewählten Substituenten trägt;
die Gruppe Q¹-Z- unter Cyclopentyloxy, Tetrahydrofuran-3-yloxy, Tetrahydropyran-4-yloxy, Tetra-hydrothiopyran-4-yloxy, 1,1-Dioxotetrahydrothio-pyran-4-yloxy, 1-Oxotetrahydrothiopyran-4-yloxy, Tetrahydrothien-3-yloxy, 1,1-Dioxotetrahydrothien-3-yloxy, 1-Oxotetrahydrothien-3-yloxy, Pyrrolidin-3-yloxy, Pyrrolidin-2-yloxy, Piperidin-3-yloxy, Piperidin-4-yloxy, Homopiperidin-3-yloxy, Homopiperidin-4-yloxy und Azetidin-3-yloxy ausgewählt ist,
und worin die Azetidinyl-, Pyrrolidinyl-, Piperidinyl- oder Homopiperidinylgruppe in der Gruppe Q¹-Z- gegebenenfalls durch einen unter Methyl, Ethyl, n-Propyl, Isopropyl, n-Butyl, Isobutyl, tert-Butyl, Allyl, 2-Propinyl, Acetyl, Propionyl, Methoxycarbonyl, Ethoxycarbonyl, Propoxycarbonyl, tert-Butoxycarbonyl, Methylsulfonyl, Ethylsulfonyl, 2-Methoxyethyl, Carbamoylmethyl, N-Methylcarbamoylmethyl, N,N-Dimethylcarbamoylmethyl, 2-Carbamoylethyl, 2-(N-Methylcarbamoyl)ethyl, 2-(N,N-Dimethylcarbamoyl)-ethyl, Acetylmethyl, 2-Acetylethyl, Methoxycarbonylmethyl und 2-Methoxycarbonylethyl ausgewählten Substituenten N-substituiert ist,
und worin jede Heterocyclylgruppe in der Gruppe Q¹-Z- gegebenenfalls 1 oder 2 Oxosubstituenten trägt; und
Q² für eine Arylgruppe der Formel Ib 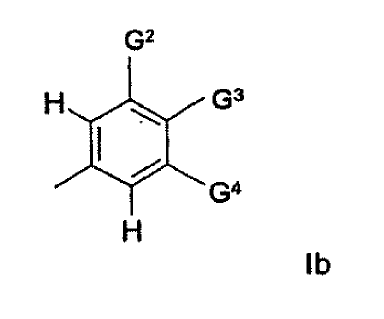
worin G³ und G⁴ gemeinsam eine Gruppe der Formel -CH=CH-NH, -NH-CH=CH-, -NH-N=CH- oder -CH=N-NH-bilden und der bei Verknüpfung von G³ und G⁴ gebildete 9-gliedrige bicyclische Heteroarylring gegebenenfalls an einer NH-Gruppe des Heteroarylteils der bicyclischen Gruppe eine Gruppe der Formel:
-X¹²-Q¹¹
worin X¹² für eine direkte Bindung steht oder unter SO₂ und CO ausgewählt ist, worin Q¹¹ für Phenyl, Benzyl, 2-Phenylethyl, 2-Furyl, Furfuryl, 3-Furyl, 3-Furylmethyl, 2-Oxazolyl, 4-Oxazolyl, 2-Oxazolylmethyl, 4-Oxazolylmethyl, 2-Imidazolyl, 4-Imidazolyl, 2-Imidazolylmethyl, 4-Imidazolylmethyl, 2-, 3- oder 4-Pyridyl, 2-, 3- oder 4-Pyridylmethyl, 2-(2-, 3- oder 4-Pyridyl)ethyl, 2-, 4- oder 5-Pyrimidinyl, 2-, 4- oder 5-Pyrimidinylmethyl, 2-(2-, 4- oder 5-Pyrimidinyl)ethyl, 1,2,4-Triazol-3-yl, 1,2,4-Triazol-5-ylmethyl, Triazol-3-ylmethyl, 1,2,4-Triazol-5-yl, 2-Thienyl, 3-Thienyl, 2-Thienylmethyl, 3-Thienylmethyl, 2-(2-Thienyl)ethyl, 2-(3-Thienyl)ethyl, 2-Thiazolyl, 4-Thiazolyl, 2-Thiazolylmethyl, 4-Thiazolylmethyl, 1,2,5-Thiadiazol-3-yl, 1,2,5-Thiadiazol-3-ylmethyl oder 2-(1,2,5-Thiadiazol-3-yl)ethyl, das gegebenenfalls 1 oder 2 Substituenten, die gleich oder verschieden sind und unter Fluor, Chlor, Brom, Cyano, Hydroxy, Methyl und Ethyl ausgewählt sind, trägt, steht, trägt;
und der bei Verknüpfung von G³ und G⁴ gebildete 9-gliedrige bicyclische Heteroarylring gegebenenfalls an einem verfügbaren Kohlenstoffatom im Heteroarylteil des bicyclischen Rings 1 unter Fluor, Chlor, Brom, Cyano, Hydroxy, Amino, Methyl, Ethyl, Vinyl, Ethinyl, Methylamino und Dimethylamino ausgewählten Substituenten trägt,
und G² unter Wasserstoff, Fluor, Chlor, Brom, Trifluormethyl, Cyano, Hydroxy, Amino, Methyl, Ethyl, Vinyl, Ethinyl, Methylamino und Dimethylamino ausgewählt ist;
oder ein pharmazeutisch annehmbares Salz davon.

2. Chinazolinderivat nach Anspruch 1, worin
R¹, sofern vorhanden, für Methoxy steht,
Q¹-Z- für 1-Methylpiperidin-4-yloxy steht und Q² für eine Arylgruppe der Formel Ib, worin G² für Wasserstoff steht und G³ und G⁴ gemeinsam eine Gruppe der Formel -NH-CH=CH- oder -NH-N=CH-bilden, steht,
und der bei Verknüpfung von G³ und G⁴ gebildete 9-gliedrige bicyclische Heteroarylring gegebenenfalls an einer NH-Gruppe des Heteroarylteils des bicyclischen Rings eine Gruppe der Formel:
-X¹²-Q¹¹
worin X¹² für eine direkte Bindung oder SO₂ steht und Q¹¹ für Phenyl, Benzyl oder 2-Pyridylmethyl, das gegebenenfalls einen Fluorsubstituenten trägt,
steht,
trägt;
und der bei Verknüpfung von G³ und G⁴ gebildete 9-gliedrige bicyclische Heteroarylring gegebenenfalls in der 3-Position einen Chlorsubstituenten trägt;
oder ein pharmazeutisch annehmbares Salz davon.

3. Chinazolinderivat nach Anspruch 2, worin Q² unter 1-Benzolsulfonylindol-5-yl, 1-Benzylindol-5-yl, 1-(2-Pyridylmethyl)indol-5-yl, 1-(2-Pyridylmethyl)-indazol-5-yl und 1-(3-Fluorbenzyl)indazol-5-yl ausgewählt ist;
oder ein pharmazeutisch annehmbares Salz davon.

4. Chinazolinderivat nach Anspruch 1, worin:
R¹, sofern vorhanden, für Methoxy steht,
Q¹-Z- für 1-Methylpiperidin-4-yloxy steht und
Q²N(H) für eine Gruppe der Formel Ic: 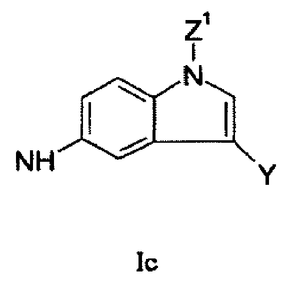
worin Z¹ für Wasserstoff steht und Y unter Wasserstoff, Chlor und Brom ausgewählt ist, steht; oder ein pharmazeutisch annehmbares Salz davon.

5. Chinazolinderivat nach Anspruch 1, worin:
m für 1 steht und R¹ für Methoxy steht,
Q¹-Z- für 1-Methylpiperidin-4-yloxy steht und
Q² für eine Gruppe der Formel Ib, worin G² für Wasserstoff steht und G³ und G⁴ gemeinsam eine Gruppe der Formel -NH-CH=CH- bilden und der so durch G³ und G⁴ gemeinsam mit den Kohlenstoffatomen, an die sie gebunden sind, gebildete Indolylring in der 1-Position durch eine Gruppe der Formel:
-X¹²-Q¹¹
worin X¹² für eine direkte Bindung steht und Q¹¹ für Benzyl, das gegebenenfalls durch 1 Fluorsubstituenten substituiert ist, steht, substituiert ist, steht; oder ein pharmazeutisch annehmbares Salz davon.

6. Chinazolinderivat nach Anspruch 5, worin Q¹¹ für 2-Fluorbenzyl oder 3-Fluorbenzyl steht;
oder ein pharmazeutisch annehmbares Salz davon.

7. Chinazolinderivat der Formel I gemäß Anspruch 1, ausgewählt unter:
4-(3-Chlorindol-5-ylamino)-5-(1-methylpiperidin-4-yloxy)chinazolin;
4-(3-Chlorindol-5-ylamino)-7-methoxy-5-(1-methylpiperidin-4-yloxy)chinazolin;
4-(Indol-5-ylamino)-7-methoxy-5-(1-methylpiperidin-4-yloxy)chinazolin;
oder ein pharmazeutisch annehmbares Säureadditionssalz davon.

8. Chinazolinderivat der Formel I gemäß Anspruch 1, ausgewählt unter:
4-(3-Bromindol-5-ylamino)-7-methoxy-5-(1-methylpiperidin-4-yloxy)chinazolin;
4-(1-(3-Fluorbenzyl)indol-5-ylamino)-7-methoxy-5-(1-methylpiperidin-4-yloxy)chinazolin;
4-(1-(2-Fluorbenzyl)indol-5-ylamino)-7-methoxy-5-(1-methylpiperidin-4-yloxy)chinazolin;
oder ein pharmazeutisch annehmbares Säureadditionssalz davon.

9. Verfahren zur Herstellung eines Chinazolinderivats der Formel I oder eines Salzes davon nach Anspruch 1, bei dem man:
(a) ein Chinazolin der Formel II 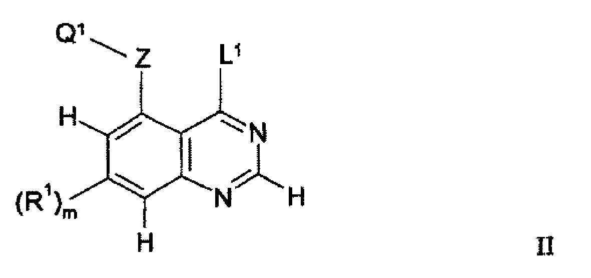 worin L¹ für eine austauschbare Gruppe steht und Q¹, Z, m und R¹ die in Anspruch 1 angegebene Bedeutung besitzen, wobei jedoch jede funktionelle Gruppe gegebenenfalls geschützt ist, mit einer Verbindung der Formel
Q²NH₂
worin Q² die in Anspruch 1 angegebene Bedeutung besitzt, wobei jedoch jede funktionelle Gruppe gegebenenfalls geschützt ist, umsetzt und danach jede vorhandene Schutzgruppe mit herkömmlichen Mitteln abspaltet; oder
(b) einen Alkohol der Formel
Q¹-OH
worin Q¹ die in Anspruch 1 angegebene Bedeutung besitzt, wobei jedoch jede funktionelle Gruppe gegebenenfalls geschützt ist, mit einem Chinazolin der Formel VI 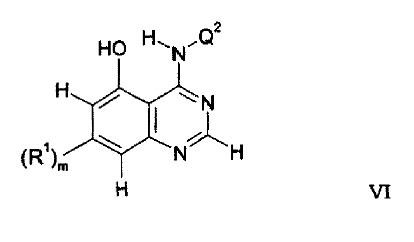 worin m, R¹ und Q² die in Anspruch 1 angegebene Bedeutung besitzt, wobei jedoch jede funktionelle Gruppe gegebenenfalls geschützt ist, kuppelt, zweckmäßigerweise in Gegenwart eines geeigneten Dehydratisierungsmittels, und danach jede vorhandene Schutzgruppe mit herkömmlichen Mitteln abspaltet; oder
(c) einen Alkohol der Formel
Q¹-OH
worin Q¹ die in Anspruch 1 angegebene Bedeutung besitzt, wobei jedoch jede funktionelle Gruppe gegebenenfalls geschützt ist, mit einem Chinazolin der Formel VIII 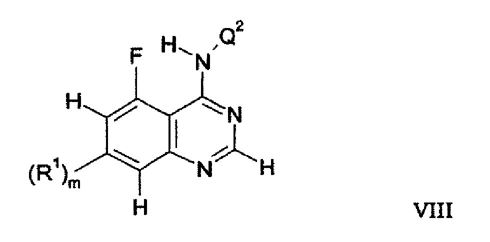 worin m, R¹ und Q² die in Anspruch 1 angegebene Bedeutung besitzt, wobei jedoch jede funktionelle Gruppe gegebenenfalls geschützt ist, umsetzt und danach jede vorhandene Schutzgruppe mit herkömmlichen Mitteln abspaltet; oder
(d) zur Herstellung derjenigen Verbindungen der Formel I, worin m für 1 steht und R¹ für eine Gruppe der Formel
Q³-X¹-
worin Q³ für eine Heterocyclyl-C₁₋₆-alkylgruppe gemäß Anspruch 1 steht und X¹ für 0 steht, steht, ein Chinazolin der Formel XI 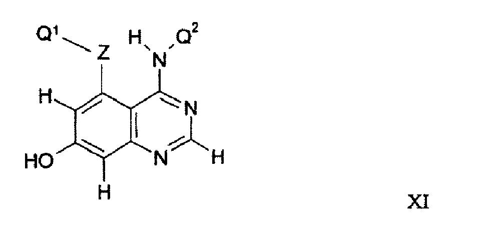 worin Q¹, Z und Q² die in Anspruch 1 angegebene Bedeutung besitzt, wobei jedoch jede funktionelle Gruppe gegebenenfalls geschützt ist, mit einem Alkohol der Formel Q³OH, wobei jede funktionelle Gruppe in Q³ gegebenenfalls geschützt ist, kuppelt und danach jede vorhandene Schutzgruppe mit herkömmlichen Mitteln abspaltet; oder
(e) zur Herstellung derjenigen Verbindungen der Formel I, worin R¹ für eine Hydroxygruppe steht, ein Chinazolinderivat der Formel I, worin R¹ für eine C₁₋₆-Alkoxygruppe steht, spaltet; oder
(f) zur Herstellung derjenigen Verbindungen der Formel I, worin Q¹, R¹ oder Q² eine primäre oder sekundäre Aminogruppe enthält, die entsprechende Verbindung der Formel (I), worin Q¹, R¹ oder Q² eine geschützte primäre oder sekundäre Aminogruppe enthält, spaltet; oder
(g) zur Herstellung derjenigen Verbindungen der Formel I, worin Q¹, R¹ oder Q² eine C₁₋₆-Alkoxy-oder substituierte C₁₋₆-Alkoxygruppe oder eine C₁₋₆-Alkylamino- oder substituierte C₁₋₆-Alkylaminogruppe enthält, ein Chinazolinderivat der Formel I, worin Q¹, R¹ oder Q² eine Hydroxygruppe bzw. eine primäre oder sekundäre Aminogruppe enthält, alkyliert; oder
(h) zur Herstellung derjenigen Verbindungen der Formel I, worin Q¹, R¹ oder Q² eine amino-hydroxydisubstituierte C₁₋₆-Alkoxygruppe enthält, eine Verbindung der Formel I, worin Q¹, R¹ oder Q² eine epoxysubstituierte C₁₋₆-Alkoxygruppe enthält, mit einer Heterocyclylverbindung oder einem geeigneten Amin umsetzt; oder
(i) ein Chinazolin der Formel XII 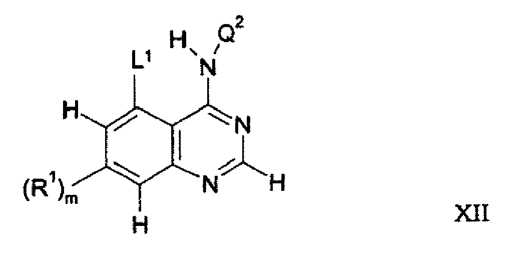 worin L¹ für eine austauschbare Gruppe steht und m, R¹ und Q² die in Anspruch 1 angegebene Bedeutung besitzen, wobei jedoch jede funktionelle Gruppe gegebenenfalls geschützt ist, mit einer Verbindung der Formel
Q¹ZH
worin Q¹ und Z die in Anspruch 1 angegebene Bedeutung besitzen, wobei jedoch jede funktionelle Gruppe gegebenenfalls geschützt ist, umsetzt und danach jede vorhandene Schutzgruppe mit herkömmlichen Mitteln abspaltet; oder
(j) zur Herstellung derjenigen Verbindungen der Formel I, worin Q¹, R¹ oder Q² eine aminosubstituierte C₁₋₆-Alkoxygruppe enthält, eine Verbindung der Formel I, worin Q¹, R¹ oder Q² eine halogensubstituierte C₁₋₆-Alkoxygruppe enthält, mit einer Heterocyclylverbindung oder einem geeigneten Amin umsetzt; oder
(k) zur Herstellung derjenigen Verbindungen der Formel I, worin eine Heterocyclylgruppe in R¹ oder Q¹ ein S- oder N-Oxid enthält, ein Ring-N-Atom oder Ring-S-Atom in einer Verbindung der Formel (I) oxidiert; oder
(l) zur Herstellung derjenigen Verbindungen der Formel I, worin R¹, Q¹ oder Q² eine C₁₋₆-Alkylamino-oder substituierte C₁₋₆-Alkylaminogruppe oder eine über Stickstoff gebundene Heterocyclylgruppe enthält, eine Aldehyd- oder Ketongruppe in einer Verbindung der Formel I mit einer C₁₋₆-Alkylamino-oder substitiuerten C₁₋₆-Alkylaminogruppe oder einem Heterocyclus mit einer NH-Gruppe in Gegenwart eines geeigneten Reduktionsmittels reduktiv aminiert; oder
(m) eine Verbindung der Formel I in eine andere Verbindung der Formel I umwandelt; und ein gegebenenfalls gewünschtes pharmazeutisch annehmbares Salz eines Chinazolinderivats der Formel I nach einer herkömmlichen Vorgehensweise erhältlich ist.

10. Pharmazeutische Zusammensetzung, die ein Chinazolinderivat der Formel I oder ein pharmazeutisch annehmbares Salz davon nach Anspruch 1 zusammen mit einem pharmazeutisch annehmbaren Verdünnungsmittel oder Träger enthält.

11. Chinazolinderivat der Formel I oder ein pharmazeutisch annehmbares Salz davon nach Anspruch 1 zur Verwendung bei einem Verfahren zur Behandlung des menschlichen oder tierischen Körpers durch Therapie.

12. Verwendung eines Chinazolinderivats der Formel I oder eines pharmazeutisch annehmbaren Salzes davon nach Anspruch 1 bei der Herstellung eines Arzneimittels zur Verwendung bei der Prävention oder Behandlung von Tumoren, die gegenüber der Inhibierung einer oder mehrerer erbB-Rezeptor-Tyrosinkinasen empfindlich sind.

13. Verwendung eines Chinazolinderivats der Formel I oder eines pharmazeutisch annehmbaren Salzes davon nach Anspruch 1 bei der Herstellung eines Arzneimittels zur Verwendung bei der Behandlung einer unter Leukämie, multiplem Myelom, Lymphom, Gallengangs-, Knochen-, Blasen-, Hirn/ZNS-, Brust-, Kolorektal-, Endometrial-, Magen-, Kopf- und Hals-, Leber-, Lungen-, Nerven-, Speiseröhren-, Eierstock-, Bauchspeicheldrüsen-, Prostata-, Nieren-, Haut-, Hoden-, Schilddrüsen-, Gebärmutter- und Vulvakrebs ausgewählten Krebserkrankung.

## Revendications

1. Dérivé de quinazoline de formule I 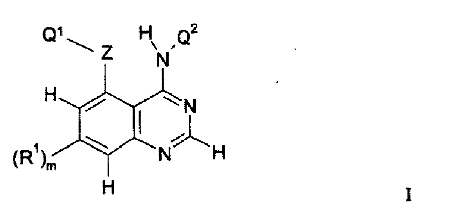 dans laquelle :
m vaut 0 ou 1 et le groupe R¹, lorsqu'il est présent, est choisi parmi un groupe hydroxy, un groupe amino, un groupe méthyle, un groupe éthyle, un groupe propyle, un groupe méthoxy, un groupe éthoxy, un groupe propoxy, un groupe butoxy, un groupe pentoxy, un groupe pyrrolidin-1-yle, un groupe 2-pyrrolidin-1-yléthoxy, un groupe 3-pyrrolidin-1-ylpropoxy, un groupe 2-pipéridinoéthoxy, un groupe 3-pipéridinopropoxy, un groupe 2-pipéridin-3-yléthoxy, un groupe 3-pipéridin-3-ylpropoxy, un groupe 2-pipéridin-4-yléthoxy, un groupe 3-pipéridin-4-ylpropoxy, un groupe 2-pipérazin-1-yléthoxy, un groupe 3-pipérazin-1-ylpropoxy, un groupe 2-morpholinoéthoxy, un groupe 3-morpholinopropoxy, un groupe 2-homopipéridinoéthoxy, un groupe 3-homopipéridinopropoxy, un groupe 2-homopipérazin-1-yléthoxy et un groupe 3-homopipérazin-1-ylpropoxy,
et où des atomes de carbone adjacents dans toute chaîne alcoxy en (2-6C) dans un substituant R¹ sont éventuellement séparés par l'insertion dans la chaîne d'un groupe choisi parmi un atome d'oxygène, un groupe NH et un groupe N(CH₃),
et où tout groupe CH₃ terminal dans une chaîne alcoxy en (1-6C) dans un substituant R¹ porte éventuellement sur le groupe CH₃ terminal un substituant choisi parmi un groupe hydroxy, un groupe amino et un groupe N-(1-méthylpyrrolidin-3-yl)-N-méthylamino,
et où tout groupe pyrrolidinyle ou pipéridinyle dans un substituant R¹ porte éventuellement un substituant choisi parmi un groupe hydroxy, un groupe méthyle, un groupe amino, un groupe méthylamino et un groupe diméthylamino,
et où tout groupe pipérazin-1-yle ou homopipérazin-1-yle dans un substituant R¹ porte éventuellement un substituant en position 4, choisi parmi un groupe méthyle, un groupe éthyle, un groupe isopropyle, un groupe 2-méthoxyéthyle, un groupe tétrahydrofurfuryle, un groupe 2-morpholinoéthyle et un groupe 1-méthylpipéridin-4-yle ;
le groupe Q¹-Z- est choisi parmi un groupe cyclopentyloxy, un groupe tétrahydrofuran-3-yloxy, un groupe tétrahydropyran-4-yloxy, un groupe tétrahydrothiopyran-4-yloxy, un groupe 1,1-dioxotétrahydrothiopyran-4-yloxy, un groupe 1-oxotétrahydrothiopyran-4-yloxy, un groupe tétrahydrothién-3-yloxy, un groupe 1,1-dioxotétrahydrothién-3-yloxy, un groupe 1-oxotétrahydrothién-3-yloxy, un groupe pyrrolidin-3-yloxy, un groupe pyrrolidin-2-yloxy, un groupe pipéridin-3-yloxy, un groupe pipéridin-4-yloxy, un groupe homopipéridin-3-yloxy, un groupe homopipéridin-4-yloxy et un groupe azétidin-3-yloxy,
et où le groupe azétidinyle, pyrrolidinyle, pipéridinyle ou homopipéridinyle dans le groupe Q¹-Z- est éventuellement N-substitué par un substituant choisi parmi un groupe méthyle, un groupe éthyle, un groupe n-propyle, un groupe isopropyle, un groupe n-butyle, un groupe isobutyle, un groupe tert-butyle, un groupe allyle, un groupe 2-propynyle, un groupe acétyle, un groupe propionyle, un groupe méthoxycarbonyle, un groupe éthoxycarbonyle, un groupe propoxycarbonyle, un groupe tert-butoxycarbonyle, un groupe méthylsulfonyle, un groupe éthylsulfonyle, un groupe 2-méthoxyéthyle, un groupe carbamoylméthyle, un groupe N-méthylcarbamoylméthyle, un groupe N,N-diméthylcarbamoylméthyle, un groupe 2-carbamoyléthyle, un groupe 2-(N-méthylcarbamoyl)éthyle, un groupe 2-(N,N-diméthylcarbamoyl)éthyle, un groupe acétylméthyle, un groupe 2-acétyléthyle, un groupe méthoxycarbonylméthyle et un groupe 2-méthoxycarbonyléthyle,
et où tout groupe hétérocyclyle dans le groupe Q¹-Z- porte éventuellement 1 ou 2 substituants oxo ; et
Q² est un groupe aryle de formule Ib 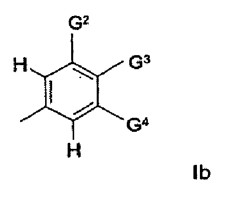
dans laquelle G³ et G⁴ forment conjointement un groupe de formule : -CH=CH-NH-, -NH-CH=CH-, -NH-N=CH- ou -CH=N-NH-,
et le noyau hétéroaryle bicyclique à 9 chaînons formé lorsque G³ et G⁴ sont liés conjointement porte éventuellement, sur un groupe NH de la portion hétéroaryle du noyau bicyclique, un groupe de formule :
-X¹²-Q¹¹
dans laquelle X¹² est une liaison directe ou est choisi parmi un groupe SO₂ et un groupe CO, dans laquelle Q¹¹ est un groupe phényle, un groupe benzyle, un groupe 2-phényléthyle, un groupe 2-furyle, un groupe furfuryle, un groupe 3-furyle, un groupe 3-furylméthyle, un groupe 2-oxazolyle, un 4-oxazolyle, un groupe 2-oxazolylméthyle, un groupe 4-oxazolylméthyle, un groupe 2-imidazolyle, un groupe 4-imidazolyle, un groupe 2-imidazolylméthyle, un groupe 4-imidazolylméthyle, un groupe 2-, 3- ou 4-pyridyle, un groupe 2-, 3-ou 4-pyridylméthyle, un groupe 2-(2-, 3- ou 4-pyridyl)éthyle, un groupe 2-, 4- ou 5-pyrimidinyle, un groupe 2-, 4- ou 5-pyrimidinylméthyle, un groupe 2-(2-, 4- ou 5-pyrimidinyl) éthyle, un groupe 1,2,4-triazol-3-yle, un groupe 1,2,4-triazol-5-ylméthyle, un groupe triazol-3-ylméthyle, un groupe 1,2,4-triazol-5-yle, un groupe 2-thiényle, un groupe 3-thiényle, un groupe 2-thiénylméthyle, un groupe 3-thiénylméthyle, un groupe 2-(2-thiényl)éthyle, un groupe 2-(3-thiényl)éthyle, un groupe 2-thiazolyle, un groupe 4-thiazolyle, un groupe 2-thiazolylméthyle, un groupe 4-thiazolylméthyle, un groupe 1,2,5-thiadiazol-3-yle, un groupe 1,2,5-thiadiazol-3-ylméthyle, ou un groupe 2-(1,2,5-thiadiazol-3-yl)éthyle, qui porte éventuellement 1 ou 2 substituants, qui peuvent être identiques ou différents, choisis parmi un groupe fluoro, un groupe chloro, un groupe bromo, un groupe cyano, un groupe hydroxy, un groupe méthyle et un groupe éthyle,
et le noyau hétéroaryle bicyclique à 9 chaînons formé lorsque G³ et G⁴ sont conjointement liés, porte éventuellement, sur un atome de carbone disponible dans la portion hétéroaryle du noyau bicyclique, 1 substituant choisi parmi un groupe fluoro, un groupe chloro, un groupe bromo, un groupe cyano, un groupe hydroxy, un groupe amino, un groupe méthyle, un groupe éthyle, un groupe vinyle, un groupe éthynyle, un groupe méthylamino et un groupe di-méthylamino,
et G² est choisi parmi un atome d'hydrogène, un groupe fluoro, un groupe chloro, un groupe bromo, un groupe trifluorométhyle, un groupe cyano, un groupe hydroxy, un groupe amino, un groupe méthyle, un groupe éthyle, un groupe vinyle, un groupe éthynyle, un groupe méthylamino et un groupe diméthylamino ;
ou un sel pharmaceutiquement acceptable de celui-ci .

2. Dérivé de quinazoline selon la revendication 1, dans lequel
R¹, lorsqu'il est présent, est un groupe méthoxy,
Q¹-Z- est un groupe 1-méthylpipéridin-4-yloxy, et Q² est un groupe aryle de formule Ib dans laquelle G² est un atome d' hydrogène, et G³ et G⁴ forment conjointement un groupe de formule :
-NH-CH=CH- ou -NH-N=CH-,
et le noyau hétéroaryle bicyclique à 9 chaînons formé lorsque G³ et G⁴ sont liés conjointement porte éventuellement, sur un groupe NH de la portion hétéroaryle du noyau bicyclique, un groupe de formule :
-X¹²-Q¹¹
dans laquelle X¹² est une liaison directe ou est un groupe SO₂, et Q¹¹ est un groupe phényle, un groupe benzyle ou un groupe 2-pyridylméthyle qui porte éventuellement un substituant fluoro,
et le noyau hétéroaryle bicyclique à 9 chaînons formé lorsque G³ et G⁴ sont conjointement liés porte éventuellement un substituant chloro en position 3 ;
ou un sel pharmaceutiquement acceptable de celui-ci .

3. Dérivé de quinazoline selon la revendication 2, dans lequel Q² est choisi parmi un groupe 1-benzènesulfonylindol-5-yle, un groupe 1-benzylindol-5-yle, un groupe 1-(2-pyridylméthyl)indol-5-yle, un groupe 1-(2-pyridylméthyl)indazol-5-yle et un groupe 1-(3-fluorobenzyl)indazol-5-yle ;
ou un sel pharmaceutiquement acceptable de celui-ci .

4. Dérivé de quinazoline selon la revendication 1, dans lequel :
R¹, lorsqu'il est présent, est un groupe méthoxy ;
Q¹-Z- est un groupe 1-méthylpipéridin-4-yloxy ; et
Q²N (H) est un groupe de formule Ic : 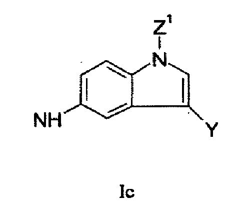
dans laquelle Z¹ est un atome d'hydrogène, et Y est choisi parmi un atome d'hydrogène, un groupe chloro et un groupe bromo ;
ou un sel pharmaceutiquement acceptable de celui-ci .

5. Dérivé de quinazoline selon la revendication 1, dans lequel
m vaut 1 et R¹ est un groupe méthoxy ;
Q¹-Z- est un groupe 1-méthylpipéridin-4-yloxy ;
Q² est un groupe de formule Ib dans laquelle G² est un atome d'hydrogène, et G³ et G⁴ forment conjointement un groupe de formule : -NH-CH=CH-, et le noyau indolyle ainsi formé par G³ et G⁴, conjointement avec les atomes de carbone auxquels ils sont fixés, est substitué en position 1 par un groupe de formule :
-X¹²-Q¹¹
dans laquelle X¹² est une liaison directe et Q¹¹ est un groupe benzyle qui est éventuellement substitué par 1 substituant fluoro ;
ou un sel pharmaceutiquement acceptable de celui-ci .

6. Dérivé de quinazoline selon la revendication 5,
dans lequel Q¹¹ est un groupe 2-fluorobenzyle ou un groupe 3-fluorobenzyle ;
ou un sel pharmaceutiquement acceptable de celui-ci.

7. Dérivé de quinazoline de formule I tel que défini dans la revendication 1, choisi parmi :
la 4- (3-chloroindol-5-ylamino) -5- (1-méthylpipéridin-4-yloxy)quinazoline ;
la 4-(3-chloroindol-5-ylamino)-7-méthoxy-5-(1-méthylpipéridin-4-yloxy)quinazoline ;
la 4-(indol-5-ylamino)-7-méthoxy-5-(1-méthylpipéridin-4-yloxy)quinazoline ;
ou un sel pharmaceutiquement acceptable d'addition à un acide de celui-ci.

8. Dérivé de quinazoline de formule I tel que défini dans la revendication 1, choisi parmi :
la 4-(3-bromoindol-5-ylamino)-7-méthoxy-5-(1-méthylpipéridin-4-yloxy)quinazoline ;
la 4-(1-(3-fluorobenzyl)indol-5-ylamino)-7-méthoxy-5-(1-méthylpipéridin-4-yloxy)quinazoline ;
la 4-(1-(2-fluorobenzyl)indol-5-ylamino)-7-méthoxy-5-(1-méthylpipéridin-4-yloxy)quinazoline ;
ou un sel pharmaceutiquement acceptable d'addition à un acide de celui-ci.

9. Procédé de préparation d'un dérivé de quinazoline de formule I, ou d'un sel de celui-ci, selon la revendication 1, qui comprend :
(a) la réaction d'une quinazoline de formule II 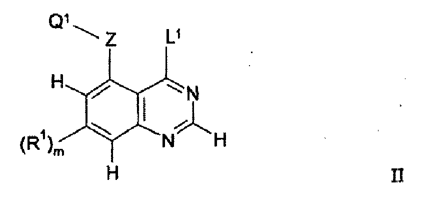 dans laquelle L¹ est un groupe remplaçable et Q¹, Z, m et R¹ sont tels que définis dans la revendication 1, excepté que tout groupe fonctionnel est protégé, le cas échéant, avec un composé de formule :
Q²NH₂
dans laquelle Q² est tel que défini dans la revendication 1, excepté que tout groupe fonctionnel est protégé, le cas échéant, après quoi tout groupe protecteur qui est présent est éliminé par des moyens traditionnels ; ou
(b) le couplage, de façon commode en présence d'un agent de déshydratation approprié, d'un alcool de formule :
Q¹OH
dans laquelle Q¹ est tel que défini dans la revendication 1, excepté que tout groupe fonctionnel est protégé, le cas échéant, avec une quinazoline de formule VI 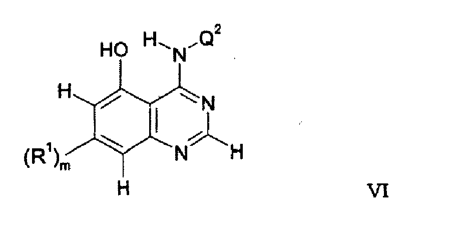 dans laquelle m, R¹ et Q² sont tels que définis dans la revendication 1, excepté que tout groupe fonctionnel est protégé, le cas échéant, après quoi tout groupe protecteur qui est présent est éliminé par des moyens traditionnels ; ou
(c) la réaction d'un alcool de formule
Q¹OH
dans laquelle Q¹ est tel que défini dans la revendication 1, excepté que tout groupe fonctionnel est protégé, le cas échéant, avec une quinazoline de formule VIII 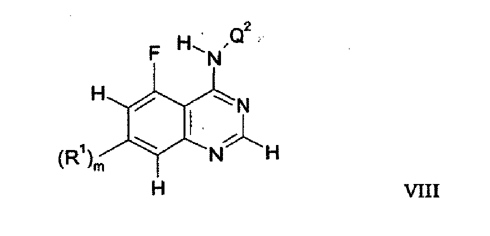 dans laquelle m, R¹ et Q² sont tels que définis dans la revendication 1, excepté que tout groupe fonctionnel est protégé, le cas échéant, après quoi tout groupe protecteur qui est présent est éliminé par des moyens traditionnels ; ou
(d) pour la production des composés de formule I dans laquelle m vaut 1 et R¹ est un groupe de formule
Q³X¹-
dans laquelle Q³ est un groupe hétérocyclyl-(1-6C)alkyle tel que défini dans la revendication 1 et X¹ est un atome d'oxygène, le couplage d'une quinazoline de formule XI 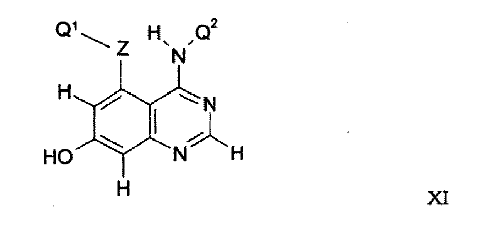 dans laquelle Q¹, Z et Q² sont tels que définis dans la revendication 1, excepté que tout groupe fonctionnel est protégé, le cas échéant, avec un alcool de formule Q³OH dans laquelle tout groupe fonctionnel dans Q³ est protégé, le cas échéant, après quoi tout groupe protecteur qui est présent est éliminé par des moyens traditionnels ; ou
(e) pour la production des composés de formule I dans laquelle R¹ est un groupe hydroxy, le clivage d'un dérivé de quinazoline de formule I dans laquelle R¹ est un groupe alcoxy en (1-6C) ; ou
(f) pour la production des composés de formule I dans laquelle Q¹, R¹ ou Q² renferme un groupe amino primaire ou secondaire, le clivage du composé correspondant de formule I dans laquelle Q¹, R¹ ou Q² renferme un groupe amino primaire ou secondaire protégé ; ou
(g) pour la production des composés de formule I dans laquelle Q¹, R¹ ou Q² renferme un groupe alcoxy en (1-6C) ou un groupe alcoxy en (1-6C) substitué, ou un groupe alkylamino en (1-6C) ou un groupe alkylamino en (1-6C) substitué, l'alkylation d'un dérivé de quinazoline de formule I dans laquelle Q¹, R¹ ou Q² renferme un groupe hydroxy ou un groupe amino primaire ou secondaire tel qu'approprié ; ou
(h) pour la production des composés de formule I dans laquelle Q¹, R¹ ou Q² renferme un groupe alcoxy en (1-6C) amino-hydroxy-disubstitué, la réaction d'un composé de formule I dans laquelle Q¹, R¹ ou Q² renferme un groupe alcoxy en (1-6C) époxy-substitué avec un composé hétérocyclyle ou une amine appropriée ; ou
(i) la réaction d'une quinazoline de formule XII 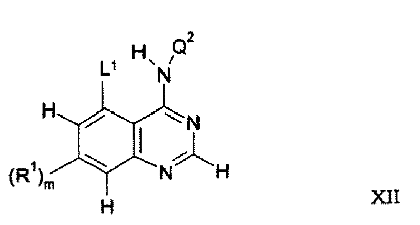 dans laquelle L¹ est un groupe remplaçable et m, R¹ et Q² sont tels que définis dans la revendication 1, excepté que tout groupe fonctionnel est protégé, le cas échéant, avec un composé de formule :
Q¹ZH
dans laquelle Q¹ et Z sont tels que définis dans la revendication 1, excepté que tout groupe fonctionnel est protégé, le cas échéant, après quoi tout groupe protecteur qui est présent est éliminé par des moyens traditionnels ; ou
(j) pour la production des composés de formule I dans laquelle Q¹, R¹ ou Q² renferme un groupe alcoxy en (1-6C) amino-substitué, la réaction d'un composé de formule I dans laquelle Q¹, R¹ ou Q² renferme un groupe alcoxy en (1-6C) halogéno-substitué avec un composé hétérocyclyle ou une amine appropriée ; ou
(k) pour la production des composés de formule I dans laquelle un groupe hétérocyclyle dans R¹ ou Q¹ renferme un groupe S- ou N-oxyde, l'oxydation d'un atome d'azote ou de soufre du noyau dans un composé de formule (I) ; ou
(l) pour la production des composés de formule I dans laquelle R¹, Q¹ ou Q² renferme un groupe alkylamino en (1-6C) ou un groupe alkylamino en (1-6C) substitué, ou un groupe hétérocyclyle lié à un atome d'azote, l'amination réductive d'un groupe aldéhyde ou cétone dans un composé de formule I, avec un groupe alkylamine en (1-6C), un groupe alkylamine en (1-6C) substitué ou un hétérocycle renfermant un groupe NH en présence d'un réducteur approprié ; ou
(m) la conversion d'un composé de formule I en un autre composé de formule I ;
et lorsqu'un sel pharmaceutiquement acceptable d'un dérivé de quinazoline de formule I est requis, il peut être obtenu en employant un mode opératoire traditionnel.

10. Composition pharmaceutique comprenant un dérivé de quinazoline de formule I, ou un sel pharmaceutiquement acceptable de celui-ci, tel que défini dans la revendication 1, en association avec un diluant ou un support pharmaceutiquement acceptable.

11. Dérivé de quinazoline de formule I, ou un sel pharmaceutiquement acceptable de celui-ci, tel que défini dans la revendication 1, destiné à être utilisé dans une méthode de traitement du corps humain ou animal par thérapie.

12. Utilisation d'un dérivé de quinazoline de formule I, ou d'un sel pharmaceutiquement acceptable de celui-ci, tel que défini dans la revendication 1, pour la fabrication d'un médicament destiné à être utilisé pour la prévention ou le traitement de tumeurs qui sont sensibles à l'inhibition d'une ou de plusieurs tyrosine kinases récepteurs erbB.

13. Utilisation d'un dérivé de quinazoline de formule I, ou d'un sel pharmaceutiquement acceptable de celui-ci, tel que défini dans la revendication 1, pour la fabrication d'un médicament destiné à être utilisé pour le traitement d'un cancer choisi parmi une leucémie, un myélome multiple, un lymphome, un cancer de la voie biliaire, osseux, de la vessie, du cerveau/SNC, du sein, colorectal, endométrial, gastrique, de la tête et du cou, hépatique, du poumon, neuronal, oesophagien, ovarien, pancréatique, de la prostate, rénal, de la peau, testiculaire, de la thyroïde, de l'utérus et de la vulve.
